# EUROPEAN PATENT APPLICATION

(11) **EP 4 606 799 A1**
(43) Date of publication of application: **27.08.2025**
(21) Application number: 23880274.8
(22) Date of filing: 19.10.2023
(51) Int. Cl.: C07D 417/14, A61K 31/497, A61P 35/00, A61P 35/02, C07D 491/107, C07D 471/10

(54) **COMPOUND FOR SHP2 PROTEIN DEGRADATION AND MEDICAL USES THEREOF**

(30) Priority: 19.10.2022 KR 20220135265; 07.02.2023 KR 20230016449
(71) Applicant: Ubix Therapeutics, Inc., Seoul 05836 (KR); SK Biopharmaceuticals Co., Ltd., Seongnam-si, Gyeonggi-do 13494 (KR)
(72) Inventor: RYU, Je Ho, Seongnam-si, Gyeonggi-do 13470 (KR); LEE, Ji Hye, Incheon 22008 (KR); AHN, Jung Min, Incheon 21984 (KR); BAE, Onnuri, Incheon 22001 (KR); HAN, Jeong Hwa, Incheon 21562 (KR); LEE, Song Hee, Seoul 05823 (KR); KIM, Ji Young, Seongnam-si, Gyeonggi-do 13121 (KR); KIM, Hyun Hwi, Yongin-si, Gyeonggi-do 16940 (KR); LEE, Soo Hyun, Anyang-si, Gyeonggi-do 14074 (KR); PARK, Ji Youn, Seongnam-si, Gyeonggi-do 13236 (KR); KIM, So Hyun, Seongnam-si, Gyeonggi-do 13494 (KR); SONG, Ji Young, Seongnam-si, Gyeonggi-do 13494 (KR); SAGONG, Hye Yeon, Seongnam-si, Gyeonggi-do 13494 (KR); KIM, Jung Eun, Seongnam-si, Gyeonggi-do 13494 (KR); JUNG, Ji Eun, Seongnam-si, Gyeonggi-do 13494 (KR); HONG, Beom Jin, Seongnam-si, Gyeonggi-do 13494 (KR); PARK, Geon Tae, Seongnam-si, Gyeonggi-do 13494 (KR); LIM, Seona, Seongnam-si, Gyeonggi-do 13494 (KR); SHIN, Yong Je, Seongnam-si, Gyeonggi-do 13494 (KR); MOON, Mi Jin, Seongnam-si, Gyeonggi-do 13494 (KR); LEE, Dong Ho, Seongnam-si, Gyeonggi-do 13494 (KR); NA, Jung Tae, Seongnam-si, Gyeonggi-do 13494 (KR); LIM, Ye Seul, Seoul 05384 (KR); WOO, Yae Jin, Seongnam-si, Gyeonggi-do 13160 (KR); KANG, Ju Young, Namyangju-si, Gyeonggi-do 12104 (KR)
(74) Representative: Engelhard, Markus
(86) International application number: PCT/KR2023/016301
(87) International publication number: WO 2024/085699

(57) **Abstract**

The present disclosure provides a compound of a specific chemical structure, an optical isomer, stereoisomer, isotopic variant, hydrate or solvate thereof, or a pharmaceutically acceptable salt thereof, which has excellent activity in terms of degradation of SHP2 protein. The present disclosure also provides a composition comprising such a compound, an optical isomer, stereoisomer, isotopic variant, hydrate or solvate thereof, or a pharmaceutically acceptable salt thereof. The present disclosure also provides medical uses of a composition comprising the compound according to the present disclosure, an optical isomer, stereoisomer, isotopic variant, hydrate or solvate thereof, or a pharmaceutically acceptable salt thereof, for the treatment or prevention of SHP2-associated diseases (for example, cancer). The present disclosure also provides a method for treating or preventing SHP2-associated diseases (for example, cancer), the method comprising administering, to an individual in need of treatment, an effective amount of a composition comprising the compound according to the present disclosure, an optical isomer, stereoisomer, isotopic variant, hydrate or solvate thereof, or a pharmaceutically acceptable salt thereof.

## Description

### [Technical Field]

This application claims priority to Korean Patent Application No. 10-2022-0135265, filed October 19, 2022, and Korean Patent Application No. 10-2023-0016449, filed February 7, 2023, the entire contents of which are incorporated herein by reference.

The present disclosure relates to a group of compounds having SHP2 proteolytic activity. In particular, the present disclosure relates to a group of compounds having a specific structure and having activity of degrading SHP2 protein. The present disclosure also relates to compositions for or methods of treating diseases related to SHP2 protein using such compounds. That is, the present disclosure relates to the medical use of the compounds according to the present disclosure for treating or preventing SHP2 protein related diseases.

### [Background Art]

Src homology 2 domain-containing phosphatase (SHP2) is a protein tyrosine phosphatase. Mutations in SHP2 are prevalent in Noonan syndrome and LEOPARD syndrome. Activated mutations in SHP2 have also been identified in childhood myelomonocytic leukemia, myelodysplastic syndrome, B-cell acute lymphoblastic leukemia and acute myeloid leukemia. Somatic activating mutations in SHP2 are associated with several types of solid tumors, including lung adenocarcinoma, colon cancer, neuroblastoma, glioblastoma, melanoma, hepatocellular carcinoma, prostate cancer, and breast cancer (Bentires-Alj et al., Cancer. Res. 2004, 64, 8816-8820).

It has been known that SHP2 is involved in various signaling processes such as RAS-ERK, JAK-STAT, PI3K-AKT, NF-κB and mTOR pathways within cancer cells. In the RAS-ERK pathway, SHP2 acts as a positive regulator in the upstream to promote RAS-RAF-MEK-ERK kinase cascade signaling. Therefore, SHP2 inhibition inhibits the oncogenic function of the RAS-RAF-MEK-ERK pathway, resulting in cell growth inhibition and apoptosis in cancer cells. SHP2 also contributes to immune evasion as a key protein in the PD-1/PD-L1-mediated T cell activation inhibitory signaling pathway in immune cells. In summary, SHP2 is a very attractive cancer therapeutic target. For example, the SHP2 inhibitor SHP099 has been shown to selectively block SHP2 phosphatase activity and inhibit cancer cell growth *in vitro* and tumor growth in xenograft mouse models (Chen et al., Nature 2016, 535, 148-152; Garcia Fortanet et al., J. Med. Chem. 2016, 59, 7773-7782).

Allosteric SHP2 inhibitors have been shown to be effective in a preclinical model of Kirsten rat sarcoma (KRAS)-mutant human cancer. Also, it has been demonstrated that SHP2 inactivation by CRISPR-Cas9 induces senescence and inhibits tumor growth in xenograft models of KRAS-mutant tumors.

### [Disclosure]

### [Technical Problem]

Deletion of SHP2 protein may provide an alternative and more effective strategy for inhibiting SHP2 activity. Therefore, a problem to be solved by the present invention is to provide a compound having Src homology 2 domain-containing phosphatase (SHP2) degrading activity, a pharmaceutical composition comprising the same as an active ingredient, and a medical use for the treatment or prevention of SHP2-related diseases (cancer, tumor, etc.).

Another problem to be solved by the present invention is to provide a method for treating or preventing SHP2-related diseases (preferably, cancer or tumor), characterized in that it degrades SHP2 and consequently lowers SHP2 level, and it comprises administering to a patient in need of treatment, improvement or prevention of SHP2 related diseases the compound according to the present invention.

### [Technical Solution]

The present disclosure relates to a SHP2 targeted protein degradation (TPD) compound capable of targeting and degrading SHP2 protein through ubiquitination of E3 ubiquitin ligase, a pharmaceutical composition comprising the compound as an active ingredient, and a method for treating or preventing SHP2-related diseases using the compound.

### Compound of the present disclosure

A compound according to an embodiment of the present invention is a target protein degradation compound of 'SHP2 binding ligand-linker (-W-L-)-VHL ligand'. In one embodiment, the present application relates to a bifunctional compound useful for modulating protein activity by inducing degradation of a target protein. In some embodiments, the bifunctional compound comprises a VHL ligand, which is an E3 ubiquitin ligase binding moiety, and a target protein binding moiety, preferably linked via a connecting (linker) moiety, as described herein. E3 ubiquitin ligases refer to a family of proteins that target substrate proteins by transferring ubiquitin for degradation. VHL is an E3 ubiquitin ligase protein that, in combination with an E2 ubiquitin conjugating enzyme, attaches ubiquitin to lysine on a target protein, thereby inducing degradation by the proteasome. Thus, the bifunctional compound simultaneously binds to VHL and the target protein, thereby inducing degradation via ubiquitination by bringing the target protein into proximity to VHL, and consequently inhibits/suppresses the activity of the target protein. In certain embodiments, the bifunctional compound comprises a SHP2 binding ligand and a VHL ligand that are covalently, directly or indirectly linked to, for example, a chemical linker -W-L-.

One embodiment of the present invention provides a compound of the following chemical formula 1, an optical isomer, a stereoisomer, an isotope variant, a hydrate, a solvate, or a pharmaceutically acceptable salt thereof.

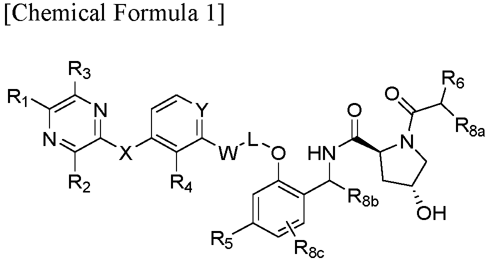

In the Chemical Formula 1, 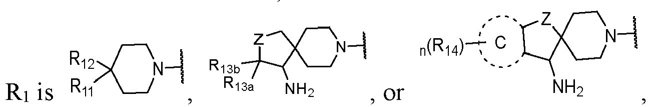
R₁₁ is H, C₁₋₆alkyl, hydroxy, halogen, C₁₋₆hydroxyalkyl, C₁₋₆haloalkyl, C₃₋₁₀cycloalkyl, heterocycle, aryl, or heteroaryl, wherein one or more hydrogens in the aryl and heteroaryl are optionally and each independently substituted with C₁₋₆alkyl, hydroxy, halogen, C₁₋₃hydroxyalkyl, C₁₋₃haloalkyl, or C₁₋₃alkoxy,
R₁₂ is amino, C₁₋₆aminoalkyl, or C₁₋₆alkylamino,
R₁₃ₐ and R_{13b} are each independently H, C₁₋₆alkyl (preferably methyl), hydroxy, halogen, C₁₋₆hydroxyalkyl, C₁₋₆haloalkyl, or C₁₋₆alkoxy,
R₁₄ is each independently H, C₁₋₆alkyl, hydroxyl, halogen, or CN,
Z is each independently -O-, -S-, -NH-, or -CH₂-,
Ring C is aryl (preferably benzene) or heteroaryl (preferably pyridine),
n is an integer from 0 to 5,
R₂ is H, C₁₋₆alkyl, or -NH₂,
R₃ is H, C₁₋₆alkyl, C₁₋₆hydroxyalkyl, C₁₋₆haloalkyl, -C(O)R₉ₐ, -C(O)OR₉ₐ, -C(O)NR₉ₐR_{9b}, or CN,
R₄ is H, C₁₋₆alkyl, halogen (preferably Cl), C₁₋₆haloalkyl, or C₁₋₆alkoxy, or is connected to R₉ₐ of W, which is -N(R₉ₐ)-, to form a heterocycle (preferably pyrrolidine, morpholine) or heteroaryl (preferably pyrrole), wherein one or more hydrogens in the heterocycle and heteroaryl are optionally and each independently substituted with C₁₋₆alkyl, halogen, or C₁₋₃haloalkyl,
R₅ is H, C₁₋₆alkyl, C₂₋₆alkynyl (preferably, acetylenyl), halogen, -CN, or heteroaryl (preferably thiazole, pyrazole, pyrrole, oxazole, or triazole), wherein one or more hydrogens in the heteroaryl are optionally substituted with C₁₋₆alkyl (preferably methyl, ethyl), halogen, or C₁₋₃haloalkyl,
R₆ is -NHC(O)R₇ or heteroaryl (preferably triazole, pyrazole, isoxazole), wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, C₃₋₁₀cycloalkyl, or heterocycle, wherein one or more hydrogens in the cycloalkyl and heterocycle are optionally and each independently substituted with C₁₋₆alkyl, halogen, or -CN,
R₈ₐ is H, C₁₋₆alkyl, or C₃₋₆cycloalkyl,
R_{8b} and R_{8c} are each independently H, C₁₋₆alkyl, C₁₋₆alkoxy, or halogen,
X is -S-, -O-, -N(R₉ₐ)-, -CH₂-, -CHCH-, -CC-, -CH₂O-, or -OCH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 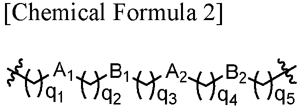
in the Chemical Formula 2,
A₁ and A₂ are each independently direct bond, C₃₋₁₀cycloalkyl (preferably cyclohexane, cyclobutane), heterocycle (preferably piperidine, piperazine, azetidine, 7-azaspiro[3.5]nonane), aryl, or heteroaryl, wherein one or more hydrogens in the cycloalkyl, heterocycle, aryl, or heteroaryl are optionally and each independently substituted with C₁₋₆alkyl, halogen, C₁₋₃haloalkyl, -OH, or =O,
B₁ and B₂ are each independently direct bond, -O-, -N(R₁₀)- -C(O)-, -C(O)N(R₁₀)-, or - N(R₁₀)C(O)-, wherein R₁₀ is each independently H or C₁₋₆alkyl, and
q₁, q₂, q₃, q₄, and q₅ are each independently an integer from 0 to 10.

A preferred embodiment of the present invention provides a compound represented by the Chemical Formula 1, an optical isomer, a stereoisomer, an isotope variant, a hydrate, a solvate, or a pharmaceutically acceptable salt thereof, wherein
R₁ is 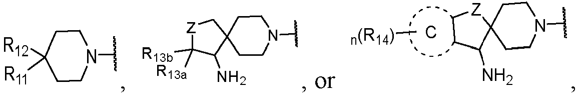
R₁₁ is H, C₁₋₆alkyl, hydroxy, halogen, C₁₋₆hydroxyalkyl, or C₁₋₆haloalkyl,
R₁₂ is amino, C₁₋₆aminoalkyl, or C₁₋₆alkylamino,
R₁₃ₐ and R_{13b} are each independently H, C₁₋₆alkyl (preferably methyl), hydroxy, halogen, C₁₋₆hydroxyalkyl, C₁₋₆haloalkyl, or C₁₋₆alkoxy,
R₁₄ is each independently H, C₁₋₆alkyl, hydroxyl, halogen, or CN,
Z is each independently -O-, -S-, -NH-, or -CH₂-,
Ring C is aryl (preferably benzene) or heteroaryl (preferably pyridine),
n is an integer from 0 to 5,
R₂ is H, C₁₋₆alkyl, or -NH₂,
R₃ is H, C₁₋₆alkyl, C₁₋₆hydroxyalkyl, -C(O)NR₉ₐR_{9b}, or CN,
R₄ is H, C₁₋₆alkyl, halogen (preferably Cl), C₁₋₆haloalkyl, or C₁₋₆alkoxy, or is connected to R₉ₐ of W, which is -N(R₉ₐ)-, to form a heterocycle (preferably pyrrolidine, morpholine) or heteroaryl (preferably pyrrole), wherein one or more hydrogens in the heterocycle and heteroaryl are optionally and each independently substituted with C₁₋₆alkyl, halogen, or C₁₋₃haloalkyl,
R₅ is H, C₁₋₆alkyl, C₂₋₆alkynyl (preferably acetylenyl), halogen, -CN, or heteroaryl (preferably thiazole, pyrazole, pyrrole, oxazole, or triazole), wherein one or more hydrogens in the heteroaryl are optionally substituted with C₁₋₆alkyl (preferably methyl, ethyl), halogen, or C₁₋₃haloalkyl,
R₆ is -NHC(O)R₇ or heteroaryl (preferably triazole, pyrazole, isoxazole), wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, C₃₋₁₀cycloalkyl, or heterocycle, wherein one or more hydrogens in the cycloalkyl and heterocycle are optionally and each independently substituted with C₁₋₆alkyl, halogen, or -CN,
R₈ₐ is H, C₁₋₆alkyl, or C₃₋₆cycloalkyl,
R_{8b} and R_{8c} are each independently H, C₁₋₆alkyl, C₁₋₆alkoxy, or halogen,
X is -S-, -O-, -N(R₉ₐ)-, or -CH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 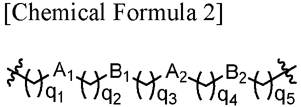
in the Chemical Formula 2,
A₁ and A₂ are each independently direct bond, C₃₋₁₀cycloalkyl (preferably cyclohexane, cyclobutane), heterocycle (preferably piperidine, piperazine, azetidine, 7-azaspiro[3.5]nonane), aryl, or heteroaryl, wherein one or more hydrogens in the cycloalkyl, heterocycle, aryl, or heteroaryl are optionally and each independently substituted with C₁₋₆alkyl, halogen, C₁₋₃haloalkyl, -OH, or =O,
B₁ and B₂ are each independently direct bond, -O-, -N(R₁₀)-, -C(O)-, -C(O)N(R₁₀)-, or - N(R₁₀)C(O)-, wherein R₁₀ is each independently H or C₁₋₆alkyl, and
q₁, q₂, q₃, q₄, and q₅ are each independently an integer from 0 to 10.

The compounds of the present disclosure have desirable characteristics in terms of SHP2 degradation activity, stability in plasma, metabolic stability in the body, stability during storage, pharmacological properties, physicochemical aspects, etc.

In addition, the compounds of the present invention have advantageous characteristics in terms of cancer cell proliferation inhibition activity, (water) solubility, cell permeability, dosage requirement (good effect is exhibited even at low dosages due to good activity, solubility, etc.), bioavailability, body distribution, cancer tissue distribution, pharmacodynamic properties, pharmacodynamic activity, duration of effect, drug interaction, etc., in addition to the aspects of the compounds of the present invention mentioned above.

More preferably, one embodiment of the present invention provides a compound represented by the Chemical Formula 1, an optical isomer, a stereoisomer, an isotope variant, a hydrate, a solvate, or a pharmaceutically acceptable salt thereof, wherein
R₁ is 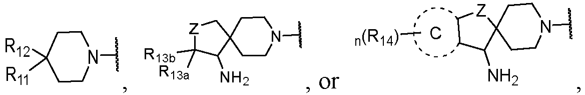
R₁₁ is H, C₁₋₆alkyl, hydroxy, halogen, C₁₋₃hydroxyalkyl, or C₁₋₃haloalkyl,
R₁₂ is amino, C₁₋₃aminoalkyl, or C₁₋₃alkylamino,
R₁₃ₐ and R_{13b} are each independently H, C₁₋₆alkyl (preferably methyl), hydroxy, halogen, C₁₋₃hydroxyalkyl, C₁₋₃haloalkyl, or C₁₋₃alkoxy,
R₁₄ is each independently H, C₁₋₃alkyl, hydroxyl, halogen, or CN,
Z is each independently -O-, -NH-, or -CH₂-,
Ring C is aryl (preferably benzene) or heteroaryl (preferably pyridine),
n is an integer from 0 to 3,
R₂ is H, C₁₋₆alkyl, or -NH₂,
R₃ is H, C₁₋₆alkyl, C₁₋₆hydroxyalkyl, -C(O)NR₉ₐR_{9b}, or CN,
R₄ is H, C₁₋₆alkyl, halogen (preferably Cl), C₁₋₆haloalkyl, or C₁₋₆alkoxy, or is connected to R₉ₐ of W, which is -N(R₉ₐ)-, to form a heterocycle (preferably pyrrolidine, morpholine) or heteroaryl (preferably pyrrole), wherein one or more hydrogens in the heterocycle and heteroaryl are optionally and each independently substituted with C₁₋₃alkyl, halogen, or C₁₋₃haloalkyl,
R₅ is H, C₁₋₆alkyl, acetylenyl, halogen, -CN, or heteroaryl (preferably thiazole, pyrazole, pyrrole, oxazole, or triazole), wherein one or more hydrogens in the heteroaryl are optionally substituted with C₁₋₆alkyl (preferably methyl, ethyl), halogen, or C₁₋₃haloalkyl,
R₆ is -NHC(O)R₇ or heteroaryl (preferably triazole, pyrazole, isoxazole), wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, or C₃₋₁₀cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₆alkyl, or halogen,
X is -S-, -O-, -N(R₉ₐ)-, or -CH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 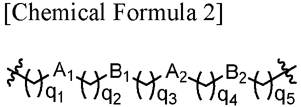
in the Chemical Formula 2,
A₁ and A₂ are each independently direct bond, C₃₋₁₀cycloalkyl (preferably cyclohexane, cyclobutane), or heterocycle (preferably piperidine, piperazine, azetidine, 7-azaspiro[3.5]nonane), wherein one or more hydrogens in the cycloalkyl and heterocycle are optionally and each independently substituted with C₁₋₆alkyl, halogen, C₁₋₃haloalkyl, -OH, or =O,
B₁ and B₂ are each independently direct bond, -O-, -N(R₁₀)-, or -C(O)-, wherein R₁₀ is each independently H or C₁₋₆alkyl, and
q₁, q₂, q₃, q₄, and q₅ are each independently an integer from 0 to 5.

Even more preferably, one embodiment of the present invention provides a compound represented by the Chemical Formula 1, an optical isomer, a stereoisomer, an isotope variant, a hydrate, a solvate, or a pharmaceutically acceptable salt thereof, wherein
R₁ is 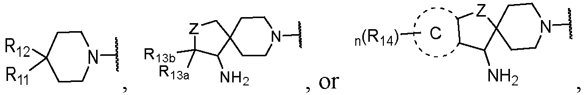
R₁₁ is H, C₁₋₆alkyl, hydroxy, or C₁₋₃hydroxyalkyl,
R₁₂ is amino or C₁₋₃aminoalkyl,
R₁₃ₐ and R_{13b} are each independently H, C₁₋₆alkyl (preferably methyl), hydroxy, C₁₋₃hydroxyalkyl, or C₁₋₃alkoxy,
R₁₄ is each independently H, C₁₋₃alkyl, hydroxyl, halogen, or CN,
Z is each independently -O-, -NH-, or -CH₂-,
Ring C is aryl (preferably benzene) or heteroaryl (preferably pyridine),
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen (preferably Cl), C₁₋₆haloalkyl, or C₁₋₆alkoxy, or is connected to R₉ₐ of W, which is -N(R₉ₐ)-, to form a heterocycle (preferably pyrrolidine, morpholine) or heteroaryl (preferably pyrrole), wherein one or more hydrogens in the heterocycle and heteroaryl are optionally and each independently substituted with C₁₋₃alkyl, halogen, or C₁₋₃haloalkyl,
R₅ is H, C₁₋₆alkyl, acetylenyl, halogen, -CN, thiazole, pyrazole, pyrrole, oxazole, or triazole, wherein one or more hydrogens in the thiazole, pyrazole, pyrrole, oxazole, and triazole are optionally substituted with C₁₋₆alkyl (preferably methyl, ethyl), halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond, -O-, or -N(R₉ₐ)-,
R₉ₐ and R_{9b} are each independently H, C₁₋₃alkyl, or C₁₋₃haloalkyl,
L is the following Chemical Formula 2, 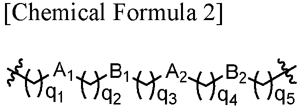
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, C₁₋₃haloalkyl, -OH, or =O,
B₁ and B₂ are each independently direct bond, -O-, or -C(O)-, and
q₁, q₂, q₃, q₄, and q₅ are each independently an integer from 0 to 5.

Even more preferably, one embodiment of the present invention provides a compound represented by the Chemical Formula 1, an optical isomer, a stereoisomer, an isotope variant, a hydrate, a solvate, or a pharmaceutically acceptable salt thereof, wherein
R₁ is 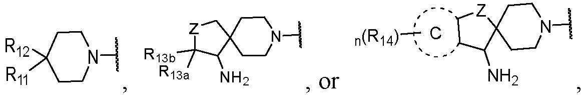
R₁₁ is H, C₁₋₆alkyl, or hydroxy,
R₁₂ is amino or C₁₋₃aminoalkyl,
R₁₃ₐ and R_{13b} are each independently H, C₁₋₆alkyl, hydroxy, or C₁₋₃hydroxyalkyl,
R₁₄ is each independently H, C₁₋₃alkyl, or halogen,
Z is each independently -O- or -CH₂-,
Ring C is aryl or heteroaryl,
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₃alkyl or halogen,
R₅ is H, C₁₋₆alkyl, acetylenyl, halogen, -CN, thiazole, pyrazole, or pyrrole, wherein one or more hydrogens in the thiazole, pyrazole, and pyrrole are optionally substituted with C₁₋₆alkyl, halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond, -O-, or -N(R₉ₐ)-,
R₉ₐ and R_{9b} are each independently H, C₁₋₃alkyl, or C₁₋₃haloalkyl,
L is the following Chemical Formula 2, 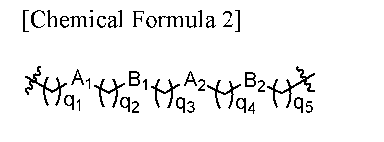
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, -OH, or =O,
B₁ and B₂ are each independently direct bond or -C(O)-, and
q₁, q₂, q₃, q₄, and q₅ are each independently an integer from 0 to 3.

Even more preferably, one embodiment of the present invention provides a compound represented by the Chemical Formula 1, an optical isomer, a stereoisomer, an isotope variant, a hydrate, a solvate, or a pharmaceutically acceptable salt thereof, wherein
R₁ is 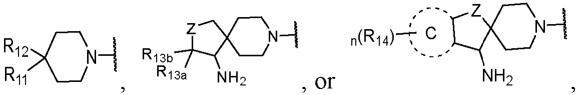
R₁₁ is H or C₁₋₆alkyl,
R₁₂ is amino or C₁₋₃aminoalkyl,
R₁₃ₐ and R_{13b} are each independently H or C₁₋₆alkyl,
R₁₄ is each independently H or C₁₋₃alkyl,
Z is each independently -O- or -CH₂-,
Ring C is aryl or heteroaryl,
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, or C₁₋₆haloalkyl, or is connected to R₉ₐ of W, which is - N(R₉ₐ)-, to form a heterocycle or heteroaryl, wherein one or more hydrogens in the heterocycle and heteroaryl are optionally and each independently substituted with C₁₋₃alkyl or halogen,
R₅ is H, C₁₋₆alkyl, halogen, -CN, thiazole, or pyrazole, wherein one or more hydrogens in the thiazole and pyrazole are optionally substituted with C₁₋₆alkyl, halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with halogen or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond or -N(R₉ₐ)-,
R₉ₐ and R_{9b} are each independently H, C₁₋₃alkyl, or C₁₋₃haloalkyl,
L is the following Chemical Formula 2, 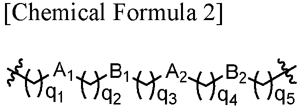
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, -OH, or =O,
B₁ and B₂ are each independently direct bond or -C(O)-, and
q₁, q₂, q₃, q₄, and q₅ are each independently an integer from 0 to 3.

Preferably, in the SHP2 target protein degradation compound according to the present invention, the moiety (SHP2 ligand) on the left side of the linker (-W-L-) has any of the following structures. When it has the structure as follows, not only is the SHP2 degradation activity excellent, but it is also suitable for various purposes of the present invention as mentioned above.

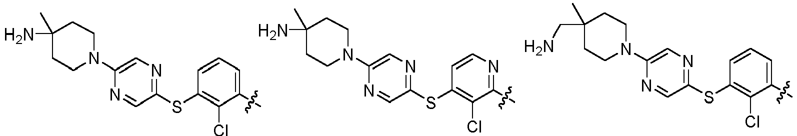

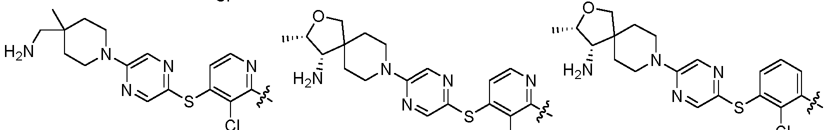

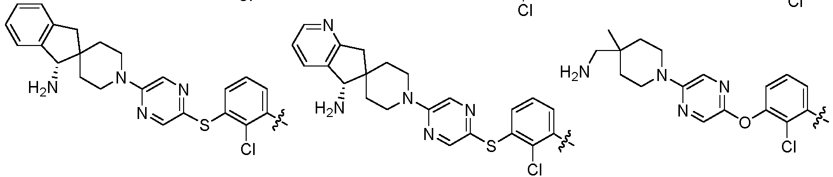

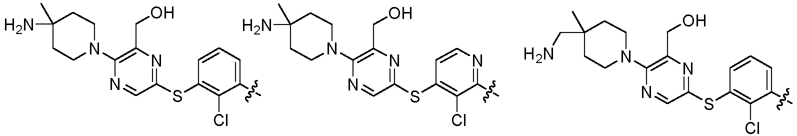

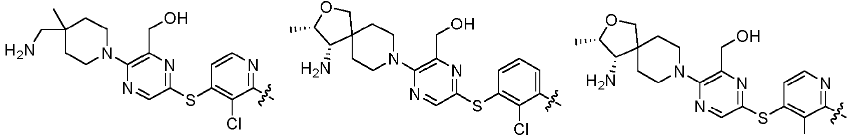

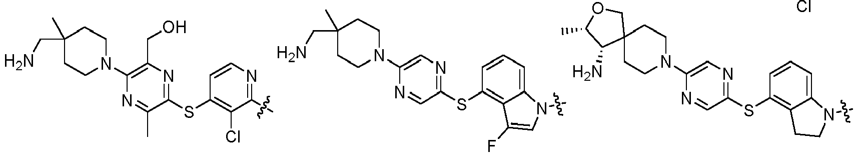

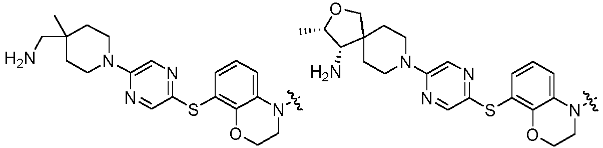

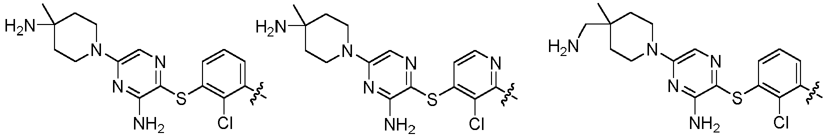

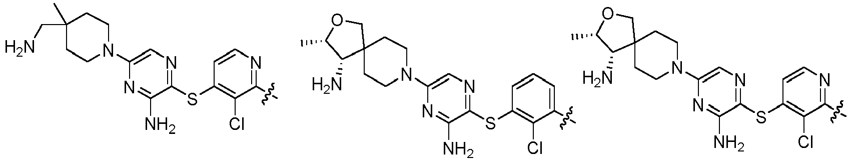

Preferably, in the SHP2 target protein degradation compound according to the present invention, the linker (-W-L-) is any one of the following linkers. When the compound has a linker as described below, not only is the SHP2 degradation activity excellent, but it is also suitable for various purposes of the present invention as mentioned above, particularly metabolic stability, stability during storage, etc.

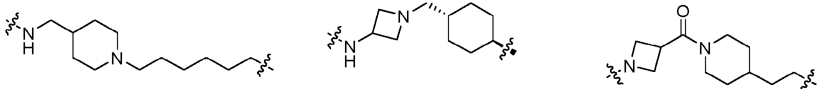

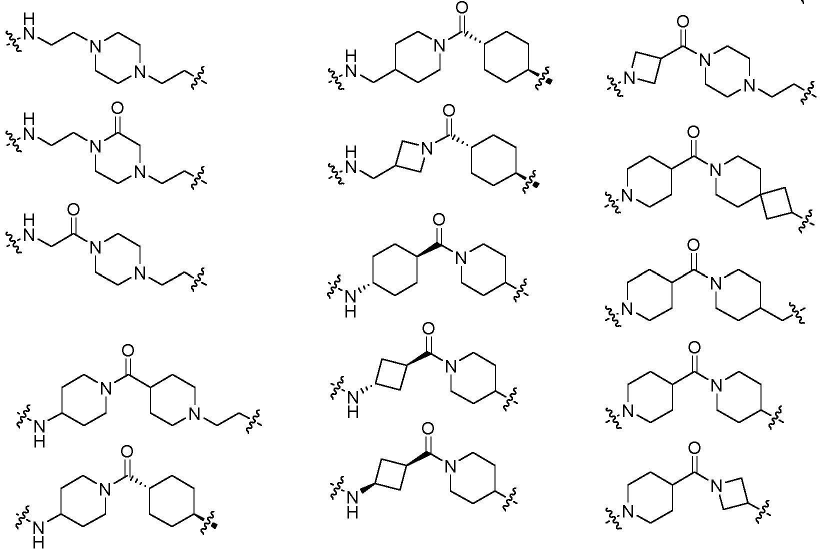

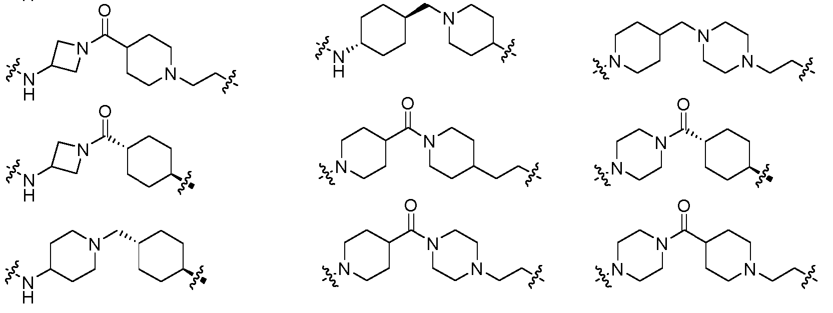

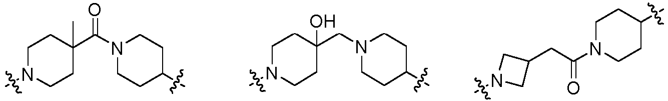

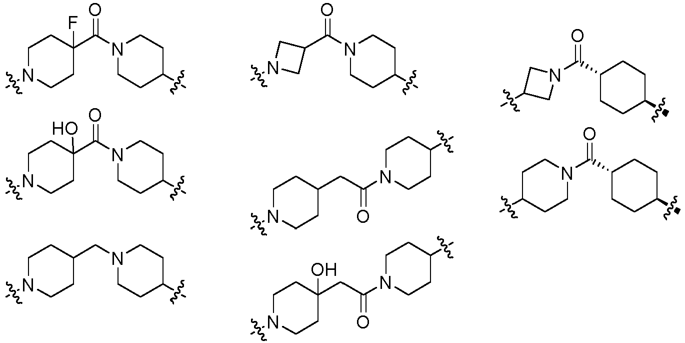

Preferably, in the SHP2 target protein degradation compound according to the present invention, the moiety (VHL ligand) on the right side of the linker (-W-L-) has any of the following structures. When it has the following structure, the SHP2 degradation activity is excellent and it is more suitable for various purposes of the present invention mentioned above.

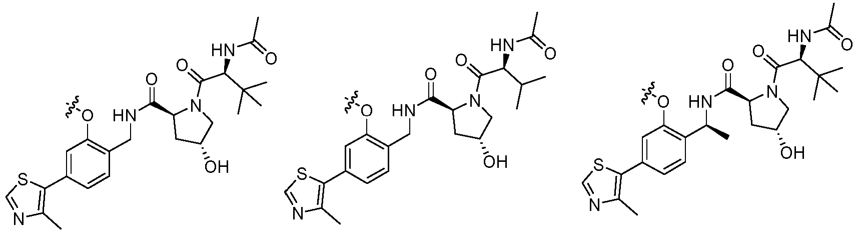

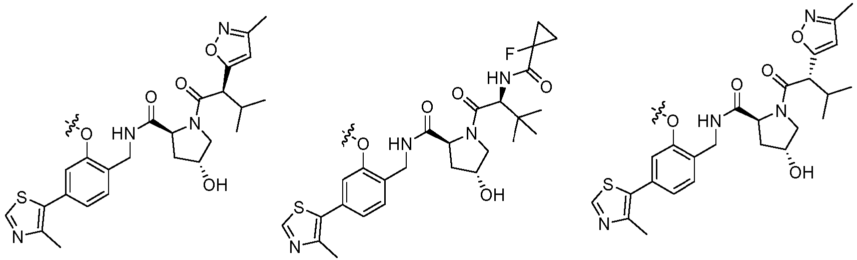

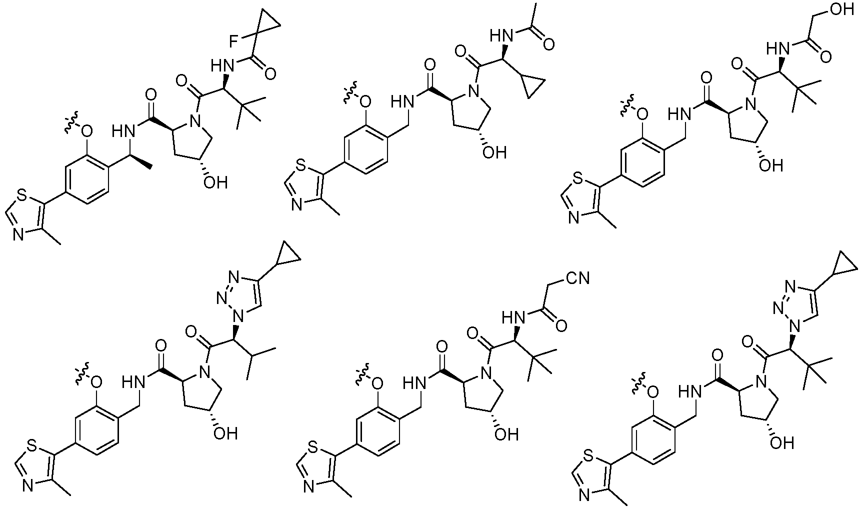

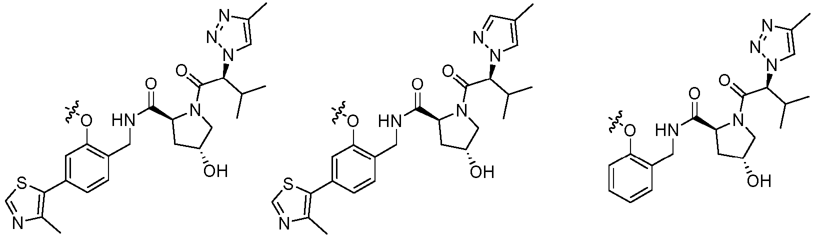

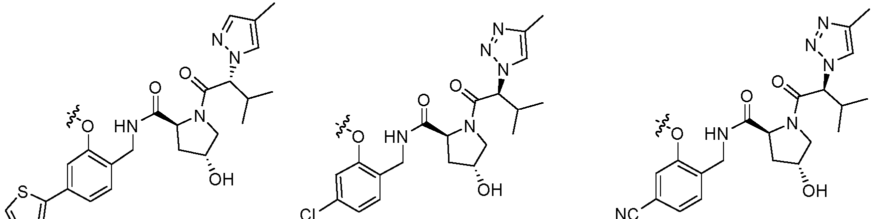

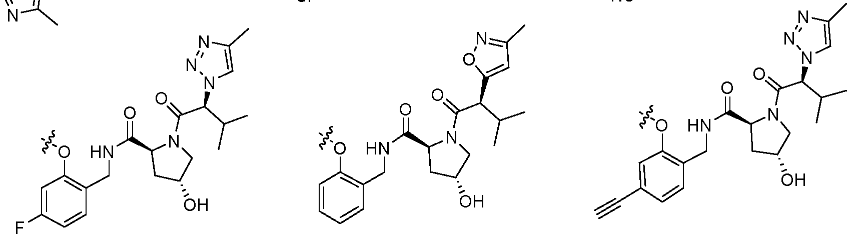

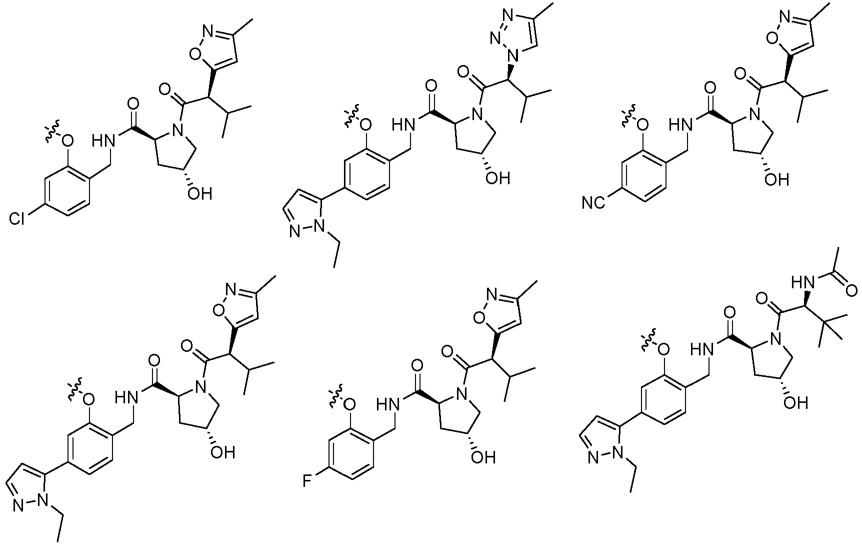

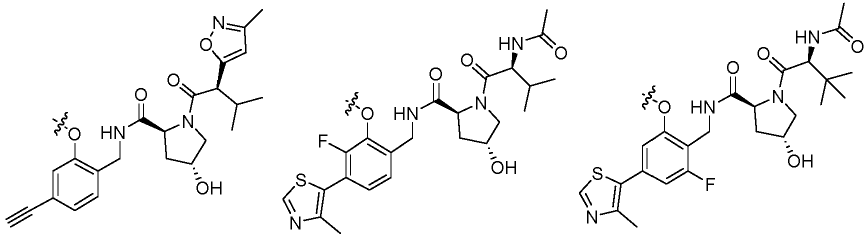

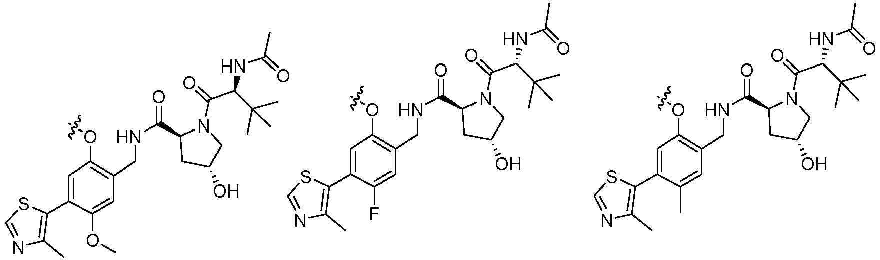

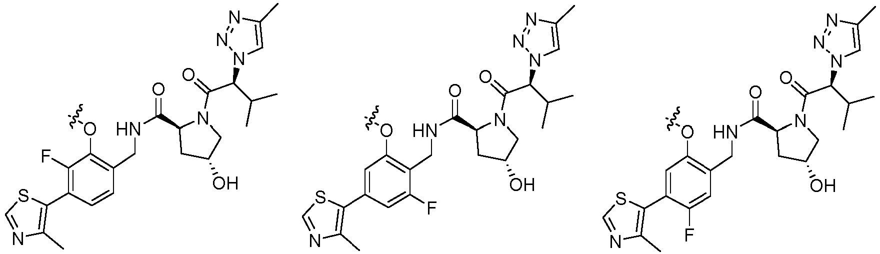

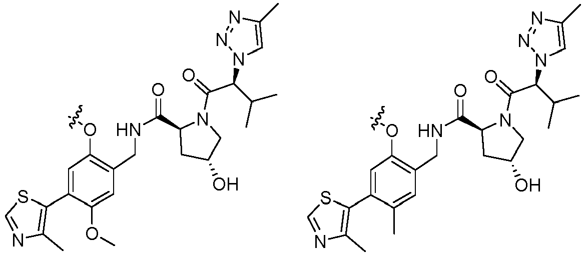

One embodiment of the present invention provides various forms of SHP2 target protein degradation compounds, optical isomers, stereoisomers, isotopic variants, hydrates, solvates or pharmaceutically acceptable salts thereof, which combine one of the aforementioned (i) SHP2 ligand moieties, (ii) one of the linker (-W-L-) moieties, and (iii) one of the VHL ligand moieties.

Non-limiting examples of compounds of chemical formula 1 according to the present disclosure are the compounds prepared in Examples 1 to 124 described below. Each Example number corresponds to a compound number. For example, the number of the final compound prepared in Example 101 is Compound 101.

One embodiment of the present invention provides compounds which are described in the following table, optical isomers, stereoisomers, isotopic variants, hydrates, solvates or pharmaceutically acceptable salts thereof.

| IUPAC Name |
|---|
| (2S,4R)-N-(2-((6-(4-(((3-((5-(4-(aminomethyl)-4-methylpiperidin-1-yl)pyrazin-2-yl)thio)-2-chlorophenyl)amino)methyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(2-((3-((5-(4-(aminomethyl)-4-methylpiperidin-1-yl)pyrazin-2-yl)thio)-2-chlorophenyl)amino)ethyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(2-((3-((5-(4-(aminomethyl)-4-methylpiperidin-1-yl)pyrazin-2-yl)thio)-2-chlorophenyl)amino)ethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-((3-((5-(4-(aminomethyl)-4-methylpiperidin-1-yl)pyrazin-2-yl)thio)-2-chlorophenyl)glycyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-((3-((3-((5-(4-(aminomethyl)-4-methylpiperidin-1-yl)pyrazin-2-yl)thio)-2-chlorophenyl)amino)azetidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-((4-((3-((5-(4-(aminomethyl)-4-methylpiperidin-1-yl)pyrazin-2-yl)thio)-2-chlorophenyl)amino)piperidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(((1R,4S)-4-((4-((5-(4-(aminomethyl)-4-methylpiperidin-1-yl)pyrazin-2-yl)thio)-3-chloropyridin-2-yl)amino)cyclohexyl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-((1-(4-((5-(4-(aminomethyl)-4-methylpiperidin-1-yl)pyrazin-2-yl)thio)-3-chloropyridin-2-yl)piperidin-4-yl)methyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1-(4-((5-(4-(aminomethyl)-4-methylpiperidin-1-yl)pyrazin-2-yl)thio)-3-chloropyridin-2-yl)piperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1-(4-((5-(4-(aminomethyl)-4-methylpiperidin-1-yl)pyrazin-2-yl)thio)-3-chloropyridin-2-yl)-4-hydroxypiperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((S)-1-amino-1,3-dihydrospiro[indene-2,4'-piperidin]-l'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-(aminomethyl)-4-methylpiperidin-1-yl)pyrazin-2-yl)oxy)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((3-((5-(4-(aminomethyl)-4-methylpiperidin-1-yl)pyrazin-2-yl)thio)-2-chlorophenyl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-(((3-((5-(4-(aminomethyl)-4-methylpiperidin-1-yl)pyrazin-2-yl)thio)-2-chlorophenyl)amino)methyl)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-(((3-((5-(4-(aminomethyl)-4-methylpiperidin-1-yl)pyrazin-2-yl)thio)-2-chlorophenyl)amino)methyl)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-(4-((5-(4-(aminomethyl)-4-methylpiperidin-1-yl)pyrazin-2-yl)thio)-3-chloropyridin-2-yl)piperazine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-((S)-1-amino-1,3-dihydrospiro[indene-2,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-(4-((5-((3S,4S)-4-amino-3-methyl-2-oxa-8-azaspiro[4.5]decan-8-yl)pyrazin-2-yl)thio)indolin-1-yl)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(4-((5-((3S,4S)-4-amino-3-methyl-2-oxa-8-azaspiro[4.5]decan-8-yl)pyrazin-2-yl)thio)indolin-1-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-(8-((5-(4-(aminomethyl)-4-methylpiperidin-1-yl)pyrazin-2-yl)thio)-2,3-dihydro-4H-benzo[b][1,4]oxazin-4-yl)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(4-(8-((5-(4-(aminomethyl)-4-methylpiperidin-1-yl)pyrazin-2-yl)thio)-2,3-dihydro-4H-benzo[b][1,4]oxazin-4-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(8-((5-((3S,4S)-4-amino-3-methyl-2-oxa-8-azaspiro[4.5]decan-8-yl)pyrazin-2-yl)thio)-2H-benzo[b][1,4]oxazin-4(3H)-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(4-(4-((5-(4-(aminomethyl)-4-methylpiperidin-1-yl)pyrazin-2-yl)thio)-3-fluoro-1H-indol-1-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-(aminomethyl)-4-methylpiperidin-1-yl)-6-(hydroxymethyl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-(aminomethyl)-4-methylpiperidin-1-yl)-6-(hydroxymethyl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(3-((5-(4-(aminomethyl)-4-methylpiperidin-1-yl)pyrazin-2-yl)thio)-2-chlorophenyl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(4-((5-(4-(aminomethyl)-4-methylpiperidin-1-yl)pyrazin-2-yl)thio)-3-chloropyridin-2-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-((S)-1-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)phenyl)ethyl)-4-hydroxypyrrolidine-2-carboxamide |
| (2S,4R)-1-((S)-2-acetamido-3,3-dimethylbutanoyl)-N-((S)-1-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)phenyl)ethyl)-4-hydroxypyrrolidine-2-carboxamide |
| (2S,4R)-N-(2-(2-(4-(3-((3-((5-(4-(aminomethyl)-4-methylpiperidin-1-yl)pyrazin-2-yl)thio)-2-chlorophenyl)amino)azetidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(4-((3-((5-(4-(aminomethyl)-4-methylpiperidin-1-yl)pyrazin-2-yl)thio)-2-chlorophenyl)amino)piperidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(4-(4-((5-(4-(aminomethyl)-4-methylpiperidin-1-yl)pyrazin-2-yl)thio)-3-chloropyridin-2-yl)piperazine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(1-(4-((5-(4-(aminomethyl)-4-methylpiperidin-1-yl)pyrazin-2-yl)thio)-3-chloropyridin-2-yl)azetidine-3-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(1-(4-((5-(4-(aminomethyl)-4-methylpiperidin-1-yl)pyrazin-2-yl)thio)-3-chloropyridin-2-yl)piperidine-4-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((7-(1-(4-((5-(4-(aminomethyl)-4-methylpiperidin-1-yl)pyrazin-2-yl)thio)-3-chloropyridin-2-yl)piperidine-4-carbonyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1s,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1S,3R)-3-((4-((5-(4-amino-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1r,3S)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1R,4S)-4-((4-((5-(4-(aminomethyl)-4-methylpiperidin-1-yl)pyrazin-2-yl)thio)-3-chloropyridin-2-yl)amino)cyclohexanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(2-(1-(4-((5-(4-(aminomethyl)-4-methylpiperidin-1-yl)pyrazin-2-yl)thio)-3-chloropyridin-2-yl)azetidin-3-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-methylpiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-fluoropiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-hydroxypiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(2-(1-(4-((5-(4-(aminomethyl)-4-methylpiperidin-1-yl)pyrazin-2-yl)thio)-3-chloropyridin-2-yl)piperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(2-(1-(4-((5-(4-(aminomethyl)-4-methylpiperidin-1-yl)pyrazin-2-yl)thio)-3-chloropyridin-2-yl)-4-hydroxypiperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)azetidin-3-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-1-((S)-2-acetamido-3,3-dimethylbutanoyl)-N-(2-((1-((1S,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)-3-methylpyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-(4-(aminomethyl)-4-methylpiperidin-1-yl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-(4-(aminomethyl)-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-(4-amino-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-((3S,4S)-4-amino-3-methyl-2-oxa-8-azaspiro[4.5]decan-8-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-1-((S)-2-(1-fluorocyclopropane-1-carboxamido)-3,3-dimethylbutanoyl)-4-hydroxypyrrolidine-2-carboxamide |
| (2S,4R)-1-((S)-2-acetamido-3,3-dimethylbutanoyl)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(2-cyanoacetamido)-3,3-dimethylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-2-(2-hydroxyacetamido)-3,3-dimethylbutanoyl)pyrrolidine-2-carboxamide |
| (2S,4R)-1-(acetyl-L-valyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-2-cyclopropylacetyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(4-methyl-1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(4-methyl-1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chIoropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |

As used herein, the terms "substituent", "radical", "group", "moiety", and "fragment" may be used interchangeably.

If a substituent is described as "optionally substituted", it means that the substituent may be unsubstituted or substituted with one or more of the defined substituents. If the substitutable position is unsubstituted, the default substituent is hydrogen.

As used herein, the term "alkyl" means a saturated straight chain or branched non-cyclic hydrocarbon, unless the context clearly dictates otherwise, having from 1 to 10 carbon atoms or from 1 to 6 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, -n-nonyl and -n-decyl, while saturated branched alkyls include -isopropyl, - sec-butyl, -isobutyl, -tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, and the like. In a preferred embodiment of the present invention, alkyl is methyl, ethyl, or n-propyl.

As used herein, the term "alkynyl" means a straight chain or branched non-cyclic hydrocarbon, unless the context clearly dictates otherwise, having 2 to 10 carbon atoms or 2 to 6 carbon atoms and containing at least one carbon-carbon triple bond. Representative linear or branched (C₂-C₁₀)alkynyl is -acetylenyl, -propynyl, -1-butynyl, -2-butynyl, -1-pentynyl, -2-pentynyl, -3- methyl-1-butynyl, -4-pentynyl, -1-hexynyl, -2-hexynyl, -5-hexynyl, -1-heptynyl, -2-heptynyl, -6-heptynyl, -1-octynyl, -2-octynyl, -7-octynyl, -1-noninyl, -2-noninyl, -8-noninyl, -1-decynyl, -2-decynyl, and -9-decynyl. These alkynyl groups may be optionally substituted.

As used herein, the term "alkoxy" means -O-(alkyl) including -OCH₃, -OCH₂CH₃, - O(CH₂)₂CH₃, -OC(CH₃)₂H, -OC(CH₃)₃, and the like, wherein alkyl is as defined above.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a preferred embodiment of the present invention, the halogen is chlorine or fluorine.

As used herein, the term "haloalkyl" means an alkyl group, wherein one or more hydrogen atoms are substituted with halogen atoms. For example, the haloalkyl includes -CF₃, -CHF₂, -CH₂F, -CBr₃, -CHBr₂, -CH₂Br, -CC1₃, -CHC1₂, -CH₂CI, -CI₃, -CHI₂, -CH₂I, -CH₂-CF₃, -CH₂-CHF₂, - CH₂-CH₂F, -CH₂-CBr₃, -CH₂-CHBr₂, -CH₂-CH₂Br, -CH₂-CC1₃, -CH₂-CHC1₂, -CH₂-CH₂CI, - CH₂-CI₃, -CH₂-CHI₂, -CH₂-CH₂I, and the like, wherein alkyl and halogen are as described above. In a preferred embodiment of the present invention, haloalkyl is -CF₃.

As used herein, the term "hydroxyalkyl" means linear or branched C₁₋₁₀alkyl or C₁₋₆alkyl substituted with one or more hydroxy groups. Examples of hydroxyalkyl group include hydroxymethyl, hydroxyethyl, hydroxypropyl and hydroxybutyl, but is not limited thereto.

As used herein, the term "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings such as octahydropentalene and decahydronaphthalene; spiro rings such as spiro[3.3]heptane, spiro[3.4]octane, spiro[3.5]nonane, spiro[4.4]nonane, spiro[4.5]decane, and spiro[5.5]undecane; and bridged bicycle rings such as bicyclo[2.1.1]hexane, bicyclo[2.2.1]heptane, and bicyclo[2.2.2]octane. A cycloalkyl group can be unsubstituted or optionally substituted. In a preferred embodiment of the present invention, cycloalkyl is cyclopropyl.

The term "heterocycle" or "heterocycloalkyl" means a 5- to 7-membered monocyclic, or 7- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quaternized. Heterocycles include heteroaryls as defined above. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyran, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms. For example, when the heteroatom is nitrogen, these include, but are limited to, fused heterobicyclic rings such as octahydrocyclopenta[c]pyrrole, octahydropyrrolo[3,4-c]pyrrole, decahydroisoquinoline, and decahydro-2,6-naphthyridine; spiro rings such as 2-azaspiro[3.3]heptane, 2,6-diazaspiro[3.3]heptane, 2-azaspiro[3.4]octane, 2,6-diazaspiro[3.4]octane, 2-azaspiro[3.5]nonane, 2,7-diazaspiro[3.5]nonane, 2-azaspiro[4.4]nonane, 2,7-diazaspiro[4.4]nonane, 8-azaspiro[4.5]decane, 2,8-diazaspiro[4.5]decane, 3-azaspiro[5.5]undecane, and 3,9-diazaspiro[5.5]undecane; and bridged heterobicyclic rings such as 2-azabicyclo[2.1.1]hexane, 2-azabicyclo[2.2.1]heptane, 2,5-diazabicyclo[2.2.1]heptane, 2-azabicyclo[2.2.2]octane, and 2,5-diazabicyclo[2.2.2]octane. In a preferred embodiment of the present invention, heterocycle is any one selected from azetidine, diazetidine, piperidine, piperazine, azaspiro[3.5]nonane, and diazabicyclo[3.2.1]octane.

As used herein, the term "aryl" means a carbocyclic aromatic group containing from 5 to 10 ring atoms. Representative examples include, but are not limited to, phenyl, tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, oxazole, isoxazole, thiazole, isothiazole, oxadiazole, benzofuran, benzothiophene, quinoline, indole, benzoxazole, benzimidazole, benzothiazole, cinnoline, phthalazine, quinazoline, 1H-azepine, etc. In a preferred embodiment of the present invention, heteroaryl is thiazole, pyridine, or pyrazine.

In this specification, * or 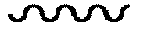 means connected to another moiety.

As used herein, the term "pharmaceutically acceptable salt(s)" refers to a salt prepared from active compounds according to the present disclosure with relatively non-toxic acids or bases, depending on the particular substituents of those compounds. When the compounds of the present disclosure have a relatively acidic group, base-added salts can be obtained by contacting the neutral compounds with a sufficient amount of the desired base and a pure or inert solvent. Suitable pharmaceutically acceptable base addition salts include, but are not limited to sodium, potassium, calcium, aluminum, organic amino, magnesium salts and the like. When the compounds of the present disclosure have a relatively basic group, acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphric acid, dihydrogenphosphoric acid, sulfuric acid, monohydrogensulfuric acid, hydrogen iodide, phosphorous acid and the like. Also, it includes a salt of amino acid such as arginate or its analogues, and it also includes analogues of organic acid such as glucuronic or galacturonic acid. Some specific compounds of this disclosure have both basic and acidic functionality for the conversion of compounds with a basic or acidic portion (addition) salts. Other examples of salts are well known through literature known in the art to which the present invention pertains.

As used herein, the term "compound of the present disclosure" is meant to include not only each compound of chemical formula 1, but also optical isomers, stereoisomers, isotopic variants, hydrates, or solvates thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of chemical formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "isomer" refers to compounds having the same molecular formula but differing in the nature or order of their atomic bonding or in the arrangement of their atoms in space.

As used herein, the term "stereoisomer" refers to isomers having different arrangements of their atoms in space.

Stereoisomers that are not mirror images of each other are termed "diastereomers," and stereoisomers that are non-superimposable mirror images of each other are termed "enantiomers." For example, if a compound has an asymmetric center, a pair of enantiomers are possible, bonded to four different groups. Enantiomers can be characterized by the absolute arrangement of the asymmetric center, described by the R and S sequencing rules, or by the way the molecule rotates the plane of polarization and is designated as dextrorotatory or levorotatory (i.e., (+)- or (-)-isomers, respectively). Chiral compounds can exist as individual enantiomers or as mixtures of these. A mixture containing equal proportions of enantiomers is called a "racemic mixture."

As used herein, a pure enantiomeric compound is one in which the other enantiomer or stereoisomer of the compound is substantially free. This state is called enantiomeric excess. In other words, the "S" form of the compound is substantially free of the "R" form of the compound, and is therefore in enantiomeric excess of the "R" form. The term "enantiomerically pure" or "pure enantiomer" means that the compound contains greater than 95 wt%, greater than 96 wt%, greater than 97 wt%, greater than 98 wt%, greater than 98.5 wt%, greater than 99 wt%, greater than 99.2 wt%, greater than 99.5 wt%, greater than 99.6 wt%, greater than 99.7 wt%, greater than 99.8 wt% or greater than 99.9 wt%. In certain embodiments, the weight is based on the total weight of all enantiomers or stereoisomers of the compound.

As used herein, unless otherwise indicated, the term "enantiomerically pure (R)-compound" means at least about 95 wt% of the (R)-compound, no more than about 5 wt% of the (S), at least about 99 wt% of the (R)-compound and up to about 1 wt% of the (S)-compound, or at least about 99.9 wt% of the (R)-compound and up to about 0.1 wt% of the (S)-compound. In certain embodiments, the weight is based on the total weight of the compound.

In the compositions provided herein, the enantiomerically pure compound or a pharmaceutically acceptable salt, solvate, hydrate or prodrug thereof can be present together with other active or inactive ingredients. For example, a pharmaceutical composition comprising an enantiomerically pure (R)-compound can comprise, for example, about 90% excipients and about 10% enantiomerically pure (R)-compound. In certain embodiments, the enantiomerically pure (R)-compound in such compositions can comprise, for example, greater than or equal to about 95% by total weight of the (R)-compound and up to about 5% by weight of the (S)-compound. For example, a pharmaceutical composition comprising an enantiomerically pure (S)-compound can comprise, for example, about 90% by total weight of the excipients and about 10% by weight of the enantiomerically pure (S)-compound. In certain embodiments, the enantiomerically pure (S)-compound in such compositions can comprise, for example, greater than or equal to about 95% by weight of the (S)-compound and up to about 5% by weight of the (R)-compound. In certain embodiments, the active ingredient can be formulated with little or no excipients or carriers.

Unless otherwise specified, the description or designation of a particular compound in the specification and claims is intended to encompass both individual enantiomers and mixtures, racemates, or other materials thereof.

In certain embodiments, the compounds described herein exist as geometric isomers. In certain embodiments, the compounds described herein have one or more double bonds. The compounds described herein include all cis, trans, syn, anti, entgegen (E) and zusammen (Z) isomers, as well as corresponding mixtures thereof. All geometrical forms of the compounds described herein are contemplated and are within the scope of the present disclosure.

In certain embodiments, the compounds described herein have one or more chiral centers, each center being in the R configuration or the S configuration. The compounds described herein include all diastereomeric, enantiomeric and epimeric forms, as well as corresponding mixtures thereof. All diastereomeric, enantiomeric and epimeric forms of the compounds described herein are contemplated and are within the scope of the present disclosure.

In additional embodiments of the compounds and methods provided herein, mixtures of enantiomers and/or diastereomers produced from a single preparation step, combination or interconversion are useful for the applications described herein. In certain embodiments, the compounds described herein are prepared as individual stereoisomers by reacting a racemic mixture of compounds with an optically active resolving agent to form a pair of diastereomeric compounds, separating the diastereomers, and recovering the optically pure enantiomers. In certain embodiments, dissociative complexes are preferred. In certain embodiments, diastereomers have distinct physical properties (e.g., melting points, boiling points, solubilities, reactivity, etc.) and are separated by exploiting these differences. In certain embodiments, diastereomers are separated by chiral chromatography, or preferably by a separation/resolution technique based on differences in solubility. In certain embodiments, the optically pure enantiomers are recovered with the resolving agent.

In certain embodiments, the compounds described herein exist as tautomers. The compounds described herein include all possible tautomers within the chemical formulae described herein.

Tautomers are compounds that are interconvertible by the movement of hydrogen atoms, which involves the conversion of a single bond to an adjacent double bond. In a bonding arrangement where tautomerism is possible, a chemical equilibrium of tautomers exists. The tautomeric form may be relevant to achieving optimal chemical reactivity and biological activity of the compound of interest. All tautomeric forms of the compounds disclosed herein are contemplated and are within the scope of the present disclosure. The exact ratio of tautomers varies depending on several factors, including temperature, solvent, and pH.

As used herein, the term "isotopic variant" means a compound that contains an unusual ratio of isotopes at one or more of the atoms constituting the compound. For example, an isotopic variant of a compound may be radiolabeled, for example, the hydrogen atoms may be selected from hydrogen, deuterium, and tritium, and may contain carbon-13 (¹³C), nitrogen-15 (¹⁵N), etc.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "purified" means that when isolated, the isolate is greater than 90% pure, in one embodiment greater than 95% pure, in another embodiment greater than 99% pure and in another embodiment greater than 99.9% pure.

### Medical uses and methods of treatment of the compounds according to the present invention

One aspect of the present invention further provides methods for treating a disease or condition in a subject having or susceptible to having such a disease or condition, by administering to the subject a therapeutically-effective amount of one or more compounds as described above. In one embodiment, the treatment is preventative treatment. In another embodiment, the treatment is palliative treatment. In another embodiment, the treatment is restorative treatment.

As used herein, the term "prevention" or "preventing" describes preventing the onset of symptoms or complications of a disease, condition or disorder.

### 1. Diseases or conditions

The compounds of the present disclosure are useful for various therapeutic or prophylactic uses (e.g., cancer). These compounds can be used to degrade SHP2 to lower SHP2 activity, and can also be used to treat SHP2-related diseases or to prevent exacerbation of these diseases. Accordingly, one aspect of the present disclosure provides a method for degrading SHP2 in a cell. In this method the cells are contacted with an effective amount of the compound of the present disclosure. In one embodiment, the cell is present in a subject. The method of the present disclosure comprises administering to a subject in need of treatment or prevention a pharmaceutical composition comprising a therapeutically or prophylactically effective amount of a compound according to the present disclosure.

In one embodiment, one aspect of the present invention provides a method of degrading SHP2 in a cell of a SHP2-associated disease. For example, one embodiment of the present invention can provide a method for lowering the SHP2 activity by degrading SHP2 in cells of a subject having a SHP2-related disease, which will be described later. In another embodiment of the present invention, the compounds of the present disclosure can be used to degrade SHP2 in cells of cancer, in particular leukemia, lymphoma, lung cancer, head and neck cancer, esophageal cancer, stomach cancer, colorectal cancer, pancreatic cancer, liver cancer, breast cancer, ovarian cancer, cervical cancer, bladder cancer, melanoma, neuroblastoma, glioma, sarcoma and so on.

In another embodiment, the present disclosure provides a method of treating a SHP2-related disease, comprising administering to a subject a therapeutically effective amount of a compound of chemical formula 1, an optical isomer, a stereoisomer, an isotope variant, a hydrate, a solvate, or a pharmaceutically acceptable salt thereof. Such a method comprises administering to a subject in need of treatment an amount of a compound of the present disclosure sufficient to degrade SHP2, i.e., a therapeutically effective amount. In such a method, a compound of the present disclosure may be administered to the subject in the form of a pharmaceutical composition described herein.

In one embodiment of the present invention, the SHP2-related disease may be cancer, cancer metastasis, cardiovascular disease, immune disorder, or ocular disorder.

In one embodiment of the present invention, the SHP2-related disease is cancer. For example, it may be any one of the cancers described in Table 2 of International Patent Application Publication No. WO 2021/236775, but is not limited thereto. Specifically, the cancer may be at least one selected from blood cancer, acute lymphoblastic leukemia, chronic lymphoblastic leukemia, acute myeloid leukemia, monocytic leukemia, chronic myeloid leukemia, chronic lymphocytic leukemia, mixed lineage leukemia, NUT-midline cancer, multiple myeloma, small cell lung cancer, non-small cell lung cancer, neuroblastoma, lymphoma, cervical cancer, head and neck cancer, head cancer, esophageal cancer, stomach cancer, pancreatic cancer, liver cancer, melanoma, colon cancer, colon cancer, prostate cancer, breast cancer, ovarian cancer, bladder cancer, glioma, sarcoma, papillary thyroid carcinoma and combinations thereof. The disclosures of Table 2 of WO 2021/236775 are all incorporated herein by reference. In another embodiment, the cancer is a solid tumor. In another embodiment, the cancer is a hematologic cancer. Exemplary hematologic cancers include, but are not limited to, the cancers listed in Table 3 of WO 2021/236775. The disclosures of Table 3 of WO 2021/236775 are all incorporated herein by reference. In another embodiment, the hematologic cancer is acute lymphocytic leukemia, chronic lymphocytic leukemia (including B-cell chronic lymphocytic leukemia), or acute myeloid leukemia. In another embodiment, the cancer is a leukemia, e.g., a leukemia selected from acute monocytic leukemia, acute myeloid leukemia, chronic myelogenous leukemia, chronic lymphocytic leukemia and mixed lineage leukemia (MLL). In another embodiment, the cancer is NUT-midline carcinoma. In another embodiment, the cancer is multiple myeloma. In another embodiment, the cancer is lung cancer, such as small cell lung cancer (SCLC). In another embodiment, the cancer is neuroblastoma. In another embodiment, the cancer is Burkitt's lymphoma. In another embodiment, the cancer is cervical cancer. In another embodiment, the cancer is head and neck cancer. In another embodiment, the cancer is esophageal cancer. In another embodiment, the cancer is stomach cancer. In another embodiment, the cancer is pancreatic cancer. In another embodiment, the cancer is liver cancer. In another embodiment, the cancer is melanoma. In another embodiment, the cancer is ovarian cancer. In another embodiment, the cancer is colorectal cancer. In another embodiment, the cancer is prostate cancer. In another embodiment, the cancer is breast cancer.

In another embodiment, the cancer according to the present invention is selected from the group consisting of acute monocytic leukemia, acute myeloid leukemia, chronic myelogenous leukemia, chronic lymphocytic leukemia, mixed lineage leukemia, lymphoma, NUT-midline carcinoma, multiple myeloma, small cell lung cancer, non-small cell lung cancer, neuroblastoma, Burkitt's lymphoma, cervical cancer, head and neck cancer, esophageal cancer, stomach cancer, pancreatic cancer, liver cancer, melanoma, ovarian cancer, colorectal cancer, prostate cancer, breast cancer, bladder cancer, glioma, sarcoma, esophageal squamous cell carcinoma and papillary thyroid carcinoma.

That is, the aspect of the present invention provides a medical use of the compound of chemical formula 1, an optical isomer, a stereoisomer, an isotope variant, a hydrate, a solvate, or a pharmaceutically acceptable salt thereof for treating or preventing the above diseases.

### 2. Subjects

Suitable subjects to be treated according to the present invention include mammalian subjects. Mammals according to the present disclosure include, but are not limited to, human, canine, feline, bovine, caprine, equine, ovine, porcine, rodents, lagomorphs, primates, and the like, and encompass mammals *in utero.*

In one embodiment, the suitable subject to be treated according to the present invention is human.

### 3. Administration and Dosing

The compounds of the present disclosure are generally administered in a therapeutically effective amount.

As used herein, "effective amount" refers to an amount of a compound of the present disclosure sufficient to prevent a SHP2-related disease; to slow or minimize the progression of a SHP2-related disease; or to provide a therapeutic benefit in the treatment or management of a SHP2-related disease. "Effective amount" also refers to an amount sufficient to inhibit or reduce SHP2 activity, either *in vitro* or *in vivo.*

The compounds of the present disclosure can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

### 4. Other embodiments related to medical use

Based on the foregoing description, one embodiment of the present invention provides a method for inhibiting or degrading Src homology 2 domain-containing phosphatase (SHP2), comprising administering to a subject in need thereof a therapeutically effective amount of a compound according to the present invention, an optical isomer, a stereoisomer, an isotopic variant, a hydrate, a solvate, or a pharmaceutically acceptable salt thereof.

Based on the foregoing description, one embodiment of the present invention provides a method for inhibiting or degrading Src homology 2 domain-containing phosphatase (SHP2) in a patient or a biological sample, comprising contacting the patient or the biological sample with a compound according to the present invention, an optical isomer, a stereoisomer, an isotopic variant, a hydrate, a solvate, or a pharmaceutically acceptable salt thereof.

Based on the foregoing description, one embodiment of the present invention provides a use of a compound according to the present invention, an optical isomer, a stereoisomer, an isotopic variant, a hydrate, a solvate, or a pharmaceutically acceptable salt thereof, for the production of a medicament for degrading Src homology 2 domain-containing phosphatase (SHP2) in a patient or a biological sample.

Based on the foregoing description, one embodiment of the present invention provides a use of a compound according to the present invention, an optical isomer, a stereoisomer, an isotopic variant, a hydrate, a solvate, or a pharmaceutically acceptable salt thereof, for the production of a medicament for treating a Src homology 2 domain-containing phosphatase (SHP2)-related disease.

### Pharmaceutical compositions of the compounds of the present disclosure

In another embodiment, the present invention provides a pharmaceutical composition comprising the compound of chemical formula 1, an optical isomer, a stereoisomer, an isotope variant, a hydrate, a solvate, or a pharmaceutically acceptable salt thereof, and a pharmaceutically acceptable carrier or excipient. In one embodiment of the present invention, the pharmaceutical composition is used for the treatment or prevention of SHP2-related diseases, specifically cancer, which is described above.

The term "pharmaceutically acceptable" means suitable for use as a pharmaceutical preparation, and generally considered safe for such use. The term also means that it has been officially approved by the governing body of a country for this use, or is listed in the Korean Pharmacopoeia or the United States Pharmacopoeia.

### Pharmaceutical compositions, dosage forms and administration routes

For the treatment of the diseases or conditions referred to above, the compounds described herein can be administered as follows:

### 1. Oral administration

The compounds of the present disclosure may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### 2. Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water.

### 3. Topical Administration

Compounds of the present disclosure may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

The terms "combination administration" and "the step of co-administering" or "combination therapy" refer to both simultaneous administration (administration of two or more therapeutic agents at the same time) and staggered administration (administration of one or more therapeutic agents at a different time than the administration of the additional therapeutic agent or agent), so long as the therapeutic agents are simultaneously present in the patient's body to some extent, preferably in an effective amount. In certain preferred embodiments, one or more compounds of the present disclosure as described herein are co-administered in combination with at least one additional bioactive agent, particularly including an anti-cancer agent. In certain preferred embodiments, the co-administration of the compounds results in synergistic activity and/or treatment, including anti-cancer therapy.

### [Advantageous Effects]

The present disclosure provides compounds capable of exhibiting various pharmacological activities due to very good SHP2 degradation activity, pharmaceutical compositions comprising the compound as an active ingredient, their medical uses (especially cancer or tumors), and methods of treatment or prevention comprising administering the compound to a subject in need of such treatment or prevention. The compound according to the present disclosure can provide a compound having excellent SHP2 degradation activity by introducing the SHP2 binding ligand to the VHL ligand via the linker of the present disclosure, and thus can provide an effective treatment option for tumor cells, especially cancers having KRAS-mutations. In addition, the compound according to the present disclosure is excellent in various aspects such as SHP2 degradation activity, plasma stability, metabolic stability, stability during storage, pharmacological properties, and physicochemical properties.

### [Mode for Invention]

Hereinafter, in order to help understand the present invention, examples and the like will be described in detail. However, the examples according to the present invention may be modified in various different forms, and the scope of the present invention should not be construed as being limited to the following examples. The examples of the present invention are provided to more completely explain the present invention to a person having average knowledge in the field to which the present invention belongs.

### Preparation of compounds of the present invention

Hereinafter, the synthesis process of some compounds of the present disclosure will be described, and the other compounds not mentioned below can be prepared by substituting starting materials, intermediates and/or reactants in a similar manner.

### Intermediate 1. Tert-butyl ((1-(5-((3-amino-2-chlorophenyl)thio)pyrazin-2-yl)-4-methylpiperidin-4-yl)methyl)carbamate

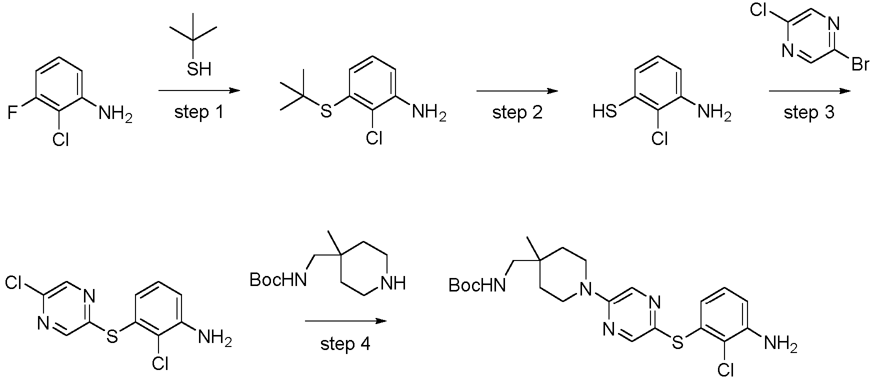

### Step 1: Synthesis of 3-(tert-butylthio)-2-chloroaniline

2-Chloro-3-fluoroaniline (5 g, 34.34 mmol), 2-methylpropane-2-thiol (11.6 ml, 103.02 mmol), and Cs₂CO₃ (22 g, 68.68 mmol) were suspended in DMF (60 ml) and then stirred at 20 °C for 16 hours. The reaction solution was diluted with EtOAc (400 ml) and washed with distilled water (150 ml x 3) and brine (150 ml x 3). The organic layer was dried over anhydrous sodium sulfate, filtered and concentrated under reduced pressure to give 7.8 g (quant.) of brown oil.

### Step 2: Synthesis of 3-amino-2-chlorobenzenethiol

3-(tert-Butylthio)-2-chloroaniline (7.8 g, 34.34 mmol) was suspended in conc. HCl (126 ml) and then stirred at 90 °C for 6 hours. The reaction solution was cooled, and the resulting solid was filtered and dried to give 5.3 g (97%) of an ivory solid.

### Step 3: Synthesis of 2-chloro-3-((5-chloropyrazin-2-yl)thio)aniline

3-Amino-2-chlorobenzenethiol (5.3 g, 33.20 mmol), 2-bromo-5-chloropyrazine (6.4 g, 33.20 mmol), Pd₂(dba)₃ (304 mg, 0.33 mmol), Xantphos (384 mg, 0.66 mmol), and DIPEA (11.6 ml, 66.40 mmol) were suspended in 1,4-dioxane (60 ml) and then stirred at 95 °C for 1 hour. The reaction solution was filtered, and the filtrate was concentrated under reduced pressure. The obtained residue was subjected to MPLC (3-15% EtOAc/hexane) to give 5.93 g (66%) of an ivory solid.

### Step 4: Synthesis of tert-butyl ((1-(5-((3-amino-2-chlorophenyl)thio)pyrazin-2-yl)-4-methylpiperidin-4-yl)methyl)carbamate

2-Chloro-3-((5-chloropyrazin-2-yl)thio)aniline (2.5 g, 9.19 mmol), tert-butyl ((4-methylpiperidin-4-yl)methyl)carbamate (2.3 g, 10.11 mmol), and DIPEA (7.2 ml, 41.34 mmol) were suspended in NMP (9 ml) and then stirred at 130 °C for 16 hours. Distilled water (150 ml) was added to the reaction solution, followed by extraction with EtOAc (150 ml). The organic layer was washed with brine (150 ml), dried over anhydrous sodium sulfate, filtered and concentrated under reduced pressure. The obtained residue was subjected to MPLC (3-30% EtOAc/hexane) to give 3.84 g (90%) of an ivory solid.

Intermediates 2 to 5 were synthesized using the same method as that used for synthesizing Intermediate 1.

| | |
|---|---|
| Intermediate 2. Tert-butyl (1-(5-((3-amino-2-chlorophenyl)thio)pyrazin-2-yl)-4-methylpiperidin-4-yl)carbamate | 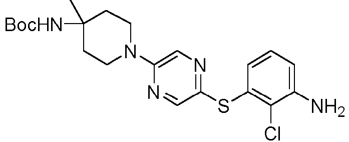 |
| Intermediate 3. Tert-butyl ((3S,4S)-8-(5-((3-amino-2-chlorophenyl)thio)pyrazin-2-yl)-3-methyl-2-oxa-8-azaspiro[4.5]decan-4-yl)carbamate | 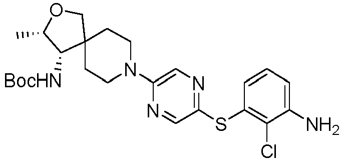 |
| Intermediate 4. (S)-tert-butyl (1'-(5-((3-amino-2-chlorophenyl)thio)pyrazin-2-yl)-1,3-dihydrospiro[indene-2,4'-piperidin]-1-yl)carbamate | 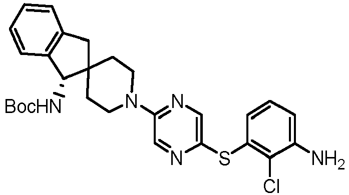 |
| Intermediate 5. N-((S)-1'-(5-((3-amino-2-chlorophenyl)thio)pyrazin-2-yl)-5,7-dihydrospiro[cyclopenta[b]pyridine-6,4'-piperidin]-5-yl)-2-methylpropane-2-sulfinamide | 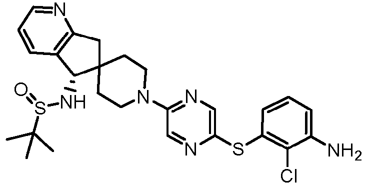 |

### Intermediate 6. Tert-butyl ((1-(5-(3-amino-2-chlorophenoxy)pyrazin-2-yl)-4-methylpiperidin-4-yl)methyl)carbamate

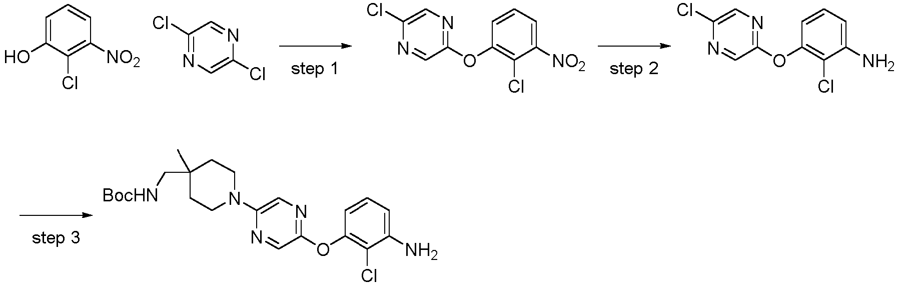

### Step 1: Synthesis of 2-chloro-5-(2-chloro-3-nitrophenoxy)pyrazine

2,5-Dichloropyrazine (1.72 g, 11.50 mmol), 2-chloro-3-nitrophenol (2.00 g, 11.50 mmol), and K₂CO₃ (3.19 g, 23.00 mmol) were suspended in DMF (20 ml) and then stirred at 100 °C for 4 hours. The reaction solution was stopped by adding sat. NH₄Cl (aq.), diluted with brine (200 ml), and extracted with EtOAc (400 ml), dried over anhydrous magnesium sulfate, filtered, and concentrated under reduced pressure. The obtained residue was subjected to MPLC (0-20% EtOAc/hexane) to give 1.91 g (58%) of a white solid.

### Step 2: Synthesis of 2-chloro-3-((5-chloropyrazin-2-yl)oxy)aniline

2-Chloro-5-(2-chloro-3-nitrophenoxy)pyrazine (1.91 g, 6.68 mmol) was suspended in THF (100 ml), sat. NH₄Cl (aq.) was added, and the mixture was stirred at 60 °C for 48 hours. The reaction solution was cooled to room temperature, and the resulting dark suspension was filtered and diluted with EtOAc (100 ml). The organic layer was extracted, washed with brine (50 ml x 2), dried over anhydrous magnesium sulfate, filtered, and concentrated under reduced pressure. The obtained residue was subjected to MPLC (0-50% EtOAc/hexane) to give 1.28 g (75%) of a grayish white solid.

### Step 3: Synthesis of tert-butyl ((1-(5-(3-amino-2-chlorophenoxy)pyrazin-2-yl)-4-methylpiperidin-4-yl)methyl)carbamate

2-Chloro-3-((5-chloropyrazin-2-yl)oxy)aniline (500 mg, 1.95 mmol) was suspended in NMP (2.5 ml). Then, tert-butyl ((4-methylpiperidin-4-yl)methyl)carbamate (535 mg, 2.34 mmol) and DIPEA (3.40 ml, 19.50 mmol) were added, and the mixture was stirred at 130 °C for one day. The reaction solution was cooled to room temperature and diluted with distilled water (50 ml) and EtOAc (80 ml). The organic layer was extracted, washed with brine, dried over anhydrous magnesium sulfate, filtered, and concentrated under reduced pressure. The obtained residue was subjected to MPLC (0-50% EtOAc/hexane) to give 316 mg (36%) of a white solid.

### Intermediate 7. (R)-N-((3S,4S)-8-(5-(indolin-4-ylthio)pyrazin-2-yl)-3-methyl-2-oxa-8-azaspiro[4.5]decan-4-yl)-2-methylpropane-2-sulfinamide

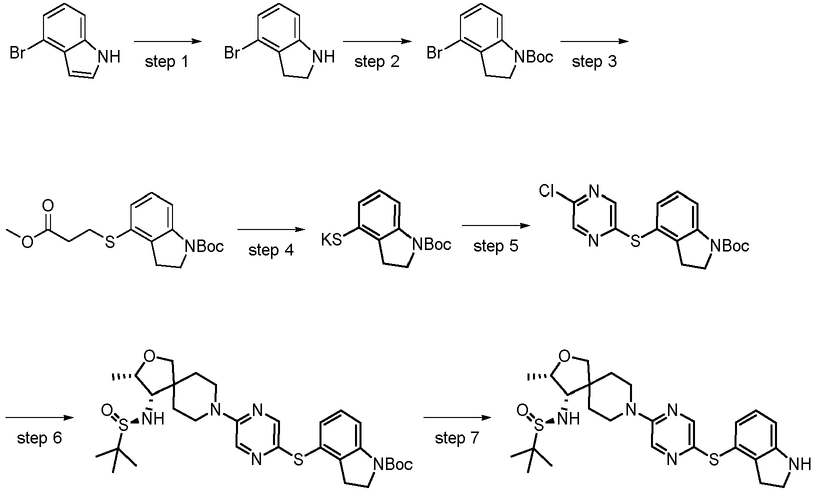

### Step 1: Synthesis of 4-bromoindoline

4-Bromo-1H-indole (1.28 ml, 10.20 mmol) was suspended in TFA (20 ml), triethylsilane (4.89 ml, 30.61 mmol) was added at 0 °C, and the mixture was stirred at 50 °C for 1 hour. The reaction solution was concentrated under reduced pressure to obtain a residue. After adjusting the pH to 10 with 2 M NaOH (aq.), distilled water (50 ml) was added, and then extracted with EtOAc (50 ml x 3). The organic layer was washed with brine (50 ml x 2) and dried over anhydrous sodium sulfate. The residue was filtered and concentrated under reduced pressure to give 1.90 g (crude) of yellow oil.

### Step 2: Synthesis of Tert-butyl 4-bromoindoline-1-carboxylate

4-Bromoindoline (1.90 g, 9.59 mmol) was suspended in DCM (30 ml), di-tert-butyl dicarbonate (3.14 g, 14.39 mmol) and TEA (4.01 ml, 28.78 mmol) were added, and the mixture was stirred at room temperature for 16 hours. The reaction solution was washed with distilled water (30 ml) and brine (30 ml x 2), dried over anhydrous sodium sulfate, filtered, and concentrated under reduced pressure. The obtained residue was subjected to MPLC (0-6% EtOAc/Petroleum ether) to give 2.80 g (91%) of yellow oil.

### Step 3: Synthesis of Tert-butyl 4-((3-methoxy-3-oxopropyl)thio)indoline-1-carboxylate

Tert-Butyl 4-bromoindoline-1-carboxylate (2.80 g, 9.39 mmol), methyl 3-mercaptopropanoate (3.05 ml, 28.17 mmol), TEA (3.92 ml, 28.17 mmol), Xantphos (1.09 g, 1.88 mmol), and Pd₂(dba)₃ (0.86 g, 0.94 mmol) were suspended in toluene (56 ml). And, degassing and N₂ purging were repeated three times, and then stirred at 100 °C for 16 hours. Distilled water (100 ml) was added to the reaction solution, extracted with EtOAc (80 ml x 3), and the organic layer was washed with brine (50 ml), dried over anhydrous sodium sulfate, filtered, and concentrated under reduced pressure. The obtained residue was subjected to MPLC (0-10% EtOAc/Petroleum ether) to give 1.46 g (46%) of yellow solid.

### Step 4: Synthesis of potassium 1-(tert-butoxycarbonyl)indoline-4-thiolate

Tert-Butyl 4-((3-methoxy-3-oxopropyl)thio)indoline-1-carboxylate (1.45 g, 4.30 mmol) was suspended in THF (29 ml), t-BuOK (1 M, 8.59 ml) was added at -65 °C, and the mixture was stirred at the same temperature for 2 hours. The reaction solution was concentrated under reduced pressure to give 1.20 g (crude) of yellow liquid.

### Step 5: Synthesis of Tert-butyl 4-((5-chloropyrazin-2-yl)thio)indoline-1-carboxylate

2,5-Dichloropyrazine (1.30 g, 8.73 mmol) was suspended in THF (12 ml), potassium 1-(tert-butoxycarbonyl)indoline-4-thiolate (1.20 g, 4.15 mmol) was added, and the mixture was stirred at room temperature for 2 hours. After adding sat. NH4Cl (aq.) (30 ml) to the reaction solution, the mixture was extracted with EtOAc (20 ml x 3), and the organic layer was washed with brine (20 ml x 2), dried over anhydrous sodium sulfate, filtered, and concentrated under reduced pressure. The obtained residue was subjected to MPLC (0-15% EtOAc/Petroleum ether) to give 601 mg (40%) of a white solid.

### Step 6: Synthesis of Tert-butyl 4-((5-((3S,4S)-4-(((R)-tert-butylsulfinyl)amino)-3-methyl-2-oxa-8-azaspiro[4.5]decan-8-yl)pyrazin-2-yl)thio)indoline-1-carboxylate

(R)-2-Methyl-N-((3S,4S)-3-methyl-2-oxa-8-azaspiro[4.5]decan-4-yl)propane-2-sulfinamide (181 mg, 0.66 mmol) was suspended in NMP (2 ml), DIPEA (0.23 ml, 1.32 mmol) was added, and tert-butyl 4-((5-chloropyrazin-2-yl)thio)indoline-1-carboxylate (120 mg, 0.33 mmol) was added. The mixture was stirred at 130 °C for 5 hours. After adding sat. NH₄Cl (aq.) to the reaction solution, it was extracted with EtOAc (30 ml x 3), and the organic layer was washed with brine (20 ml x 2), dried over anhydrous magnesium sulfate, filtered, and concentrated under reduced pressure. The obtained residue was subjected to MPLC (0-100% EtOAc/hexane) to give 116 mg (58%) of colorless oil.

### Step 7: Synthesis of (R)-N-((3S,4S)-8-(5-(indolin-4-ylthio)pyrazin-2-yl)-3-methyl-2-oxa-8-azaspiro [4.5] decan-4-yl)-2-methylpropane-2-sulfinamide

Tert-Butyl 4-((5-((3S,4S)-4-(((R)-tert-butylsulfinyl)amino)-3-methyl-2-oxa-8-azaspiro[4.5]decan-8-yl)pyrazin-2-yl)thio)indoline-1-carboxylate (116 mg, 0.19 mmol) was suspended in DCM (10 ml), TFA (2.5 ml) was added, and the mixture was stirred at room temperature for 1 hour. The reaction solution was concentrated under reduced pressure, diluted with EtOAc (50 ml), and the organic layer was basified with 1 M NaOH (aq.), washed with brine (30 ml), dried over anhydrous magnesium sulfate, filtered, and concentrated under reduced pressure. The obtained residue was subjected to MPLC (0-10% MeOH/DCM + 0.1% NH₄OH) to give 71 mg (73%) of colorless oil.

### Intermediate 8. Tert-butyl ((1-(5-((3,4-dihydro-2H-benzo[b][1,4]oxazin-8-yl)thio)pyrazin-2-yl)-4-methylpiperidin-4-yl)methyl)carbamate

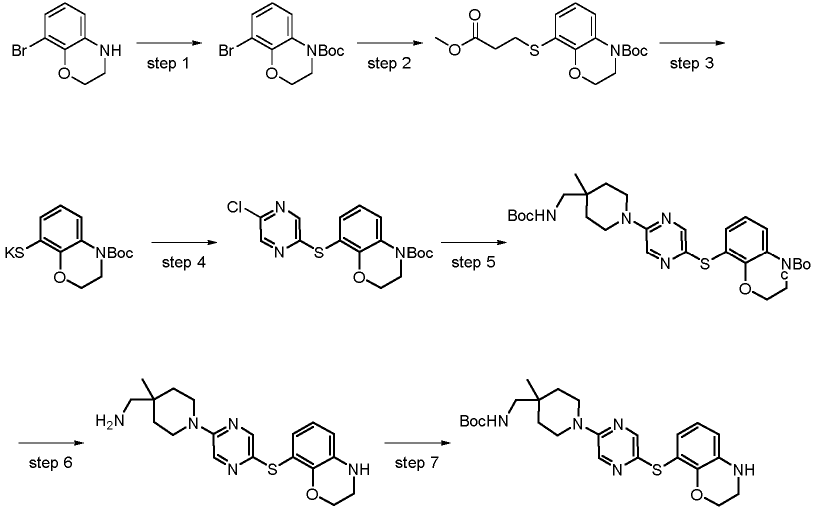

### Step 1: Synthesis of Tert-butyl 8-bromo-2,3-dihydro-4H-benzo[b][1,4]oxazine-4-carboxylate

8-Bromo-3,4-dihydro-2H-benzo[b][1,4]oxazine (3 g, 14.01 mmol) and di-tert-butyl dicarbonate (3.67 g, 16.82 mmol) were suspended in DCM (20 ml), and TEA (5.85 ml, 42.04 mmol) and DMAP (171 mg, 1.40 mmol) were added, and the mixture was stirred at room temperature for 12 hours. The reaction solution was washed with distilled water (30 ml) and brine (30 ml x 2), dried over anhydrous sodium sulfate, filtered, and concentrated under reduced pressure. The obtained residue was subjected to MPLC (0-20% EtOAc/Petroleum ether) to give 3.40 g (77%) of yellow oil.

### Step 2: Synthesis of Tert-butyl 8-((3-methoxy-3-oxopropyl)thio)-2,3-dihydro-4H-benzo[b][1,4]oxazine-4-carboxylate

Tert-Butyl 8-bromo-2,3-dihydro-4H-benzo[b][1,4]oxazine-4-carboxylate (3 g, 9.55 mmol), methyl 3-mercaptopropanoate (2.59 ml, 23.87 mmol), TEA (3.99 ml, 28.65 mmol), Xantphos (1.11 g, 1.91 mmol), and Pd₂(dba)₃ (0.87 g, 0.95 mmol) were suspended in toluene (30 ml), and degassing and N₂ purging were repeated three times, and the mixture was stirred at 100°C for 12 hours. Distilled water (40 ml) was added to the reaction solution, and the mixture was extracted with EtOAc (30 ml x 2). The organic layer was dried over anhydrous sodium sulfate, filtered, and concentrated under reduced pressure. The obtained residue was subjected to MPLC (0-25% EtOAc/Petroleum ether) to give 2.40 g (68%) of yellow oil.

### Step 3: Synthesis of potassium 4-(tert-butoxycarbonyl)-3,4-dihydro-2H-benzo[b][1,4]oxazine-8-thiolate

tert-Butyl 8-((3-methoxy-3-oxopropyl)thio)-2,3-dihydro-4H-benzo[b][1,4]oxazine-4-carboxylate (1.45 g, 4.30 mmol) was suspended in THF (29 ml), t-BuOK (1 M, 8.59 ml) was added at -65 °C, and the mixture was stirred at the same temperature for 2 hours. The reaction solution was concentrated under reduced pressure to give 1.20 g (crude) of yellow liquid.

### Step 4: Synthesis of tert-butyl 8-((5-chloropyrazin-2-yl)thio)-2,3-dihydro-4H-benzo[b][1,4]oxazine-4-carboxylate

2,5-Dichloropyrazine (3.51 g, 23.57 mmol) was suspended in THF (20 ml), potassium 4-(tert-butoxycarbonyl)-3,4-dihydro-2H-benzo[b][1,4]oxazine-8-thiolate (2.40 g, 7.86 mmol) was added, and the mixture was stirred at room temperature for 2 hours. Distilled water (20 ml) was added to the reaction solution, and extracted with EtOAc (20 ml x 3). The organic layer was washed with brine (10 ml), dried over anhydrous sodium sulfate, filtered, and concentrated under reduced pressure. The obtained residue was subjected to MPLC (0-30% EtOAc/Petroleum ether) to give 1.70 g (56%) of a yellow solid.

### Step 5: Synthesis of tert-butyl 8-((5-(4-(((tert-butoxycarbonyl)amino)methyl)-4-methylpiperidin-1-yl)pyrazin-2-yl)thio)-2,3-dihydro-4H-benzo[b][1,4]oxazine-4-carboxylate

tert-Butyl N-[(4-methyl-4-piperidyl)methyl]carbamate (1.12 g, 4.92 mmol), tert-butyl 8-((5-chloropyrazin-2-yl)thio)-2,3-dihydro-4H-benzo[b][1,4]oxazine-4-carboxylate (1.70 g, 4.48 mmol), and K₂CO₃ (1.86 g, 13.43 mmol) were suspended in DMSO (20 ml), and degassing and N₂ purging were repeated three times, and the mixture was stirred at 80 °C for 3 hours. After adding sat. NH₄Cl (aq.) to the reaction solution, it was extracted with EtOAc (20 ml x 2). Then, the organic layer was washed with brine (20 ml x 2), dried over anhydrous sodium sulfate, filtered, and concentrated under reduced pressure. The obtained residue was subjected to MPLC (0-30% EtOAc/hexane) to give 2.00 g (80%) of a yellow solid.

### Step 6: Synthesis of (1-(5-((3,4-dihydro-2H-benzo[b][1,4]oxazin-8-yl)thio)pyrazin-2-yl)-4-methylpiperidin-4-yl)methanamine

tert-Butyl 8-((5-(4-(((tert-butoxycarbonyl)amino)methyl)-4-methylpiperidin-1-yl)pyrazin-2-yl)thio)-2,3-dihydro-4H-benzo[b][1,4]oxazine-4-carboxylate (2 g, 3.50 mmol) was suspended in MeOH (25 ml), 4 M HCl in MeOH (8.75 ml) was added, and the mixture was stirred at room temperature for 3 hours. The reaction solution was concentrated under reduced pressure, diluted with EtOAc (80 ml), and the organic layer was basified with 1 M NaOH (aq.), washed with brine (50 ml), dried over anhydrous sodium sulfate, filtered, and concentrated under reduced pressure. The obtained residue was subjected to MPLC (20-70% EtOAc/Petroleum ether) to give 780 mg (60%) of a yellow solid.

### Step 7: Synthesis of tert-butyl ((1-(5-((3,4-dihydro-2H-benzo[b][1,4]oxazin-8-yl)thio)pyrazin-2-yl)-4-methylpiperidin-4-yl)methyl)carbamate

(1-(5-((3,4-Dihydro-2H-benzo[b][1,4]oxazin-8-yl)thio)pyrazin-2-yl)-4-methylpiperidin-4-yl)methanamine (700 mg, 1.88 mmol), di-tert-butyl dicarbonate (411 mg, 1.88 mmol), and TEA (0.52 ml, 3.77 mmol) were suspended in DCM (7 ml) and stirred at room temperature for 2 hours. The reaction solution was concentrated under reduced pressure, sat. NH₄Cl (aq.) was added, and EtOAc (10 ml x 2) was extracted. The organic layer was washed with brine (10 ml x 2), dried over anhydrous sodium sulfate, filtered, and concentrated under reduced pressure. The obtained residue was subjected to prep-HPLC (column: Waters Xbridge Prep OBD C18 150 mm x 40 mm x 10 um; mobile phase: [H₂O (0.1% NH₄HCO₃)-ACN]; B%: 40%-70%, 15 min) to give 290 mg (32%) of a yellow solid.

Intermediate 9 was synthesized using the same method as that used for synthesizing Intermediate 8.

| | |
|---|---|
| Intermediate 9. tert-butyl ((3S,4S)-8-(5-((3,4-dihydro-2H-benzo[b][1,4]oxazin-8-yl)thio)pyrazin-2-yl)-3-methyl-2-oxa-8-azaspiro[4.5]decan-4-yl)carbamate | 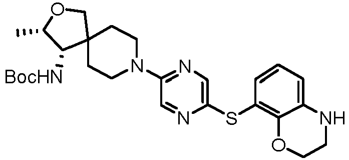 |

### Intermediate 10. tert-butyl ((1-(5-((3-fluoro-1H-indol-4-yl)thio)pyrazin-2-yl)-4-methylpiperidin-4-yl)methyl)carbamate

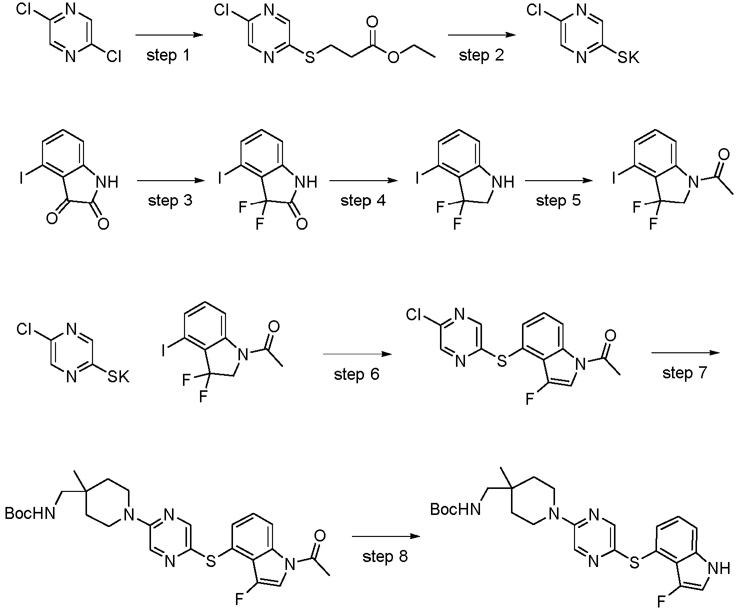

### Step 1: Synthesis of ethyl 3-((5-chloropyrazin-2-yl)thio)propanoate

2,5-Dichloropyrazine (500 mg, 3.36 mmol) and ethyl 3-mercaptopropanoate (455 mg, 3.39 mmol) were suspended in DMF (2.5 ml), K₂CO₃ (464 mg, 3.36 mmol) was added, and the mixture was stirred at room temperature for 16 hours. Distilled water (10 ml) was added to the reaction solution, and extracted with EtOAc (5 ml x 5). The organic layer was washed with brine (5 ml x 5), dried over anhydrous sodium sulfate, filtered, and concentrated under reduced pressure. The obtained residue was subjected to MPLC (0-25% EtOAc/Petroleum ether) to give 593 mg (68%) of colorless oil.

### Step 2: Synthesis of potassium 5-chloropyrazine-2-thiolate

Ethyl 3-((5-chloropyrazin-2-yl)thio)propanoate (1.60 g, 6.49 mmol) was suspended in THF (25 ml), and t-BuOK (1.46 g, 12.97 mmol) was added at -30 °C, and stirred at the same temperature for 2 hours. The reaction solution was concentrated under reduced pressure to give 1.20 g (crude) of an orange solid.

### Step 3: Synthesis of 3,3-difluoro-4-iodoindolin-2-one

4-Iodoindoline-2,3-dione (10 g, 36.63 mmol) was suspended in DCM (350 ml), DAST (17.71 g, 109.88 mmol) was added at 0 °C, and the mixture was stirred at room temperature for 24 hours. Distilled water (100 ml) was added to the reaction solution, and extracted with DCM (50 ml x 3). The organic layer was washed with brine (50 ml x 2), dried over anhydrous sodium sulfate, filtered, and concentrated under reduced pressure. The obtained residue was subjected to recrystallization from PE at room temperature for 30 min to give 11.20 g (93%) of a brown solid.

### Step 4: Synthesis of 3,3-difluoro-4-iodoindoline

3,3-Difluoro-4-iodoindolin-2-one (5 g, 16.95 mmol) was suspended in THF (30 ml), and BH₃-THF (1 M, 59.32 ml) was added dropwise at 0 °C, and stirred at room temperature for 2 hours. 10% citric acid (aq.) (100 ml) was added to the reaction solution at 0 °C, distilled water (200 ml) was added, and then extracted with EtOAc (150 ml x 3). The organic layer was washed with brine (200 ml x 2), dried over anhydrous sodium sulfate, filtered, and concentrated under reduced pressure to give 4.76 g (crude) of yellow oil.

### Step 5: Synthesis of 1-(3,3-difluoro-4-iodoindolin-1-yl)ethan-1-one

3,3-Difluoro-4-iodoindoline (4.76 g, 16.94 mmol) was suspended in EtOAc (60 ml), and then DIPEA (7.38 ml, 42.34 mmol) and AcCl (3.01 ml, 42.34 mmol) were added dropwise at 0 °C, and the mixture was stirred at the same temperature for 2 hours. Distilled water (150 ml) was added to the reaction solution, and extracted with EtOAc (80 ml x 3). The organic layer was washed with brine (80 ml), dried over anhydrous sodium sulfate, filtered, and concentrated under reduced pressure. The obtained residue was subjected to MPLC (0-25% EtOAc/Petroleum ether) to give 2.87 g (51%) of a green solid.

### Step 6: Synthesis of 1-(4-((5-chloropyrazin-2-yl)thio)-3,3-difluoroindolin-1-yl)ethan-1-one

1-(3,3-Difluoro-4-iodoindolin-1-yl)ethan-1-one (1.50 g, 4.64 mmol), potassium 5-chloropyrazine-2-thiolate (1.03 g, 5.57 mmol), DIPEA (1.62 ml, 9.29 mmol), Xantphos (269 mg, 0.46 mmol), and Pd₂(dba)₃ (213 mg, 0.23 mmol) were suspended in 1,4-dioxane (25 ml), and degassing and N₂ purging were repeated three times, and then the mixture was stirred at 70 °C for 5 hours. Distilled water (80 ml) was added to the reaction solution, extracted with EtOAc (30 ml x 3), and the organic layer was washed with brine (30 ml), dried over anhydrous sodium sulfate, filtered, and concentrated under reduced pressure. The obtained residue was subjected to MPLC (0-25% EtOAc/Petroleum ether) to give 830 mg (42%) of a yellow solid.

### Step 7: Synthesis of tert-butyl ((1-(5-((1-acetyl-3,3-difluoroindolin-4-yl)thio)pyrazin-2-yl)-4-methylpiperidin-4-yl)methyl)carbamate

1-(4-((5-Chloropyrazin-2-yl)thio)-3,3-difluoroindolin-1-yl)ethan-1-one (830 mg, 2.43 mmol) and tert-butyl ((4-methylpiperidin-4-yl)methyl)carbamate (665 mg, 2.91 mmol) were suspended in DMSO (8 ml), K₂CO₃ (1.01 g, 7.29 mmol) was added, and the mixture was stirred at 80 °C for 12 hours. Distilled water (20 ml) was added to the reaction mixture, and the mixture was extracted with EtOAc (10 ml x 3). The organic layer was washed with brine (10 ml), dried over anhydrous sodium sulfate, filtered, and concentrated under reduced pressure. The obtained residue was subjected to MPLC (0-40% EtOAc/Petroleum ether) to give 510 mg (39%) of a yellow solid.

### Step 8: Synthesis of tert-butyl ((1-(5-((3-fluoro-1H-indol-4-yl)thio)pyrazin-2-yl)-4-methylpiperidin-4-yl)methyl)carbamate

Tert-Butyl ((1-(5-((1-acetyl-3,3-difluoroindolin-4-yl)thio)pyrazin-2-yl)-4-methylpiperidin-4-yl)methyl)carbamate (450 mg, 0.84 mmol) was suspended in MeOH (2.7 ml) and H₂O (2.7 ml), and NaOH (134.91 mg, 3.37 mmol) was added and stirred at 50 °C for 4 hours. The reaction solution was concentrated under reduced pressure, sat. NH₄Cl (aq.) (5 ml) was added, extracted with EtOAc (5 ml x 3), and the organic layer was washed with brine (10 ml), dried over anhydrous sodium sulfate, filtered, and concentrated under reduced pressure. The obtained residue was subjected to MPLC (0-45% EtOAc/Petroleum ether) to give 250 mg (62%) of a yellow solid.

### Intermediate 11. tert-butyl ((1-(5-((3-(azetidin-3-ylamino)-2-chlorophenyl)thio)pyrazin-2-yl)-4-methylpiperidin-4-yl)methyl)carbamate

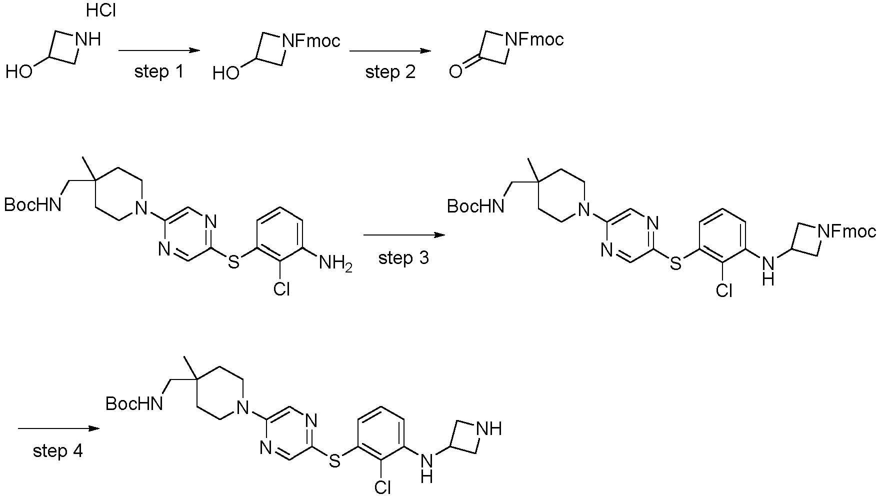

### Step 1: Synthesis of (9H-fluoren-9-yl)methyl 3-hydroxyazetidine-1-carboxylate

3-Hydroxyazetidine hydrochloride (1 g, 9.13 mmol), and Na₂CO₃ (2.9 g, 27.38 mmol) were suspended in a mixed solvent of H₂O: 1,4-dioxane (1:1 (80 ml)) and stirred at 0 °C for 10 min. Fmoc-Cl (2.36 g, 9.13 mmol) was added and stirred at room temperature for 2 hours. Distilled water (30 ml) was added to the reaction solution and extracted with EtOAc (30 ml). The organic layer was washed with brine (30 ml), dried over anhydrous sodium sulfate, filtered, and concentrated under reduced pressure. The obtained residue was subjected to MPLC (1-60% EtOAc/hexane) to give 2.52 g (93%) of a white solid.

### Step 2: Synthesis of (9H-fluoren-9-yl)methyl 3-oxoazetidine-1-carboxylate

(9H-Fluoren-9-yl)methyl 3-hydroxyazetidine-1-carboxylate (1.11 g, 3.76 mmol) and DMP (1.75 g, 4.13 mmol) were suspended in DCM (17 ml) and stirred at room temperature for 2 hours. After adding sat. Na₂S₂O₃ (aq.) (40 ml) to the reaction solution, it was extracted with DCM (50 ml). The organic layer was washed with distilled water (40 ml), dried over anhydrous sodium sulfate, filtered, and concentrated under reduced pressure. The obtained residue was subjected to MPLC (1-25% EtOAc/hexane) to give 1.1 g (100%) of a white solid.

### Step 3: Synthesis of (9H-fluoren-9-yl)methyl 3-((3-((5-(4-(((tert-butoxycarbonyl)amino)methyl)-4-methylpiperidin-1-yl)pyrazin-2-yl)thio)-2-chlorophenyl)amino)azetidine-1-carboxylate

Tert-Butyl ((1-(5-((3-amino-2-chlorophenyl)thio)pyrazin-2-yl)-4-methylpiperidin-4-yl)methyl)carbamate (Intermediate 1, 1.50 g, 3.23 mmol), (9H-fluoren-9-yl)methyl 3-oxoazetidine-1-carboxylate (2.37 g, 8.08 mmol), and Ti(O*i*-Pr)₄ (1.9 ml, 6.47 mmol) were suspended in THF (12 ml) and stirred at 70 °C for 16 hours. NaBH₄ (306 mg, 8.08 mmol) and MeOH (4 ml) were added, and the mixture was stirred at room temperature for 1 hour. Distilled water (50 ml) was added to the reaction solution, extracted with DCM (50 ml), and the organic layer was dried over anhydrous sodium sulfate, filtered, and concentrated under reduced pressure. The obtained residue was subjected to MPLC (0-15% EtOAc/DCM) to give 1.62 g (68%) of an ivory solid.

### Step 4: Synthesis of tert-butyl ((1-(5-((3-(azetidin-3-ylamino)-2-chlorophenyl)thio)pyrazin-2-yl)-4-methylpiperidin-4-yl)methyl)carbamate

(9H-Fluoren-9-yl)methyl 3-((3-((5-(4-(((tert-butoxycarbonyl)amino)methyl)-4-methylpiperidin-1-yl)pyrazin-2-yl)thio)-2-chlorophenyl)amino)azetidine-1-carboxylate (900 mg, 1.21 mmol) and Et₂NH (6.3 ml, 60.70 mmol) were suspended in DCM (6.3 ml) and stirred at room temperature for 3 hours. Distilled water (20 ml) was added to the reaction mixture, and it was extracted with DCM (30 ml). The organic layer was dried over anhydrous sodium sulfate, filtered and concentrated under reduced pressure to give 739 mg (crude) of an ivory solid.

Intermediates 12 to 16 were synthesized using the same method as that used for synthesizing Intermediate 11.

| | |
|---|---|
| Intermediate 12. tert-butyl (1-(5-((3-(azetidin-3-ylamino)-2-chlorophenyl)thio)pyrazin-2-yl)-4-methylpiperidin-4-yl)carbamate | 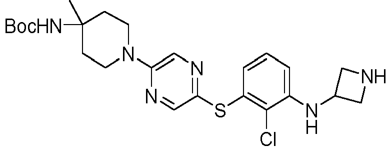 |
| Intermediate 13. tert-butyl ((3S,4S)-8-(5-((3-(azetidin-3-ylamino)-2-chlorophenyl)thio)pyrazin-2-yl)-3-methyl-2-oxa-8-azaspiro[4.5]decan-4-yl)carbamate | 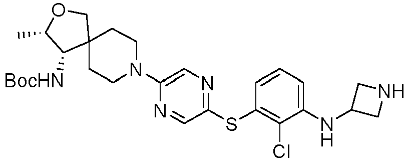 |
| Intermediate 14. (S)-tert-butyl (1'-(5-((3-(azetidin-3-ylamino)-2-chlorophenyl)thio)pyrazin-2-yl)-1,3-dihydrospiro[indene-2,4'-piperidin]-1-yl)carbamate | 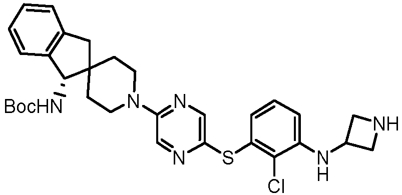 |
| Intermediate 15. N-((S)-1'-(5-((3-(azetidin-3-ylamino)-2-chlorophenyl)thio)pyrazin-2-yl)-5,7-dihydrospiro[cyclopenta[b]pyridine-6,4'-piperidin]-5-yl)-2-methylpropane-2-sulfinamide | 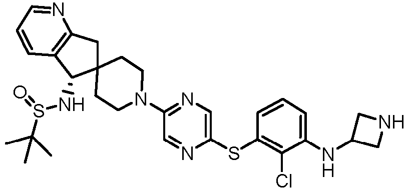 |
| Intermediate 16. tert-butyl ((1-(5-(3-(azetidin-3-ylamino)-2-chlorophenoxy)pyrazin-2-yl)-4-methylpiperidin-4-yl)methyl)carbamate | 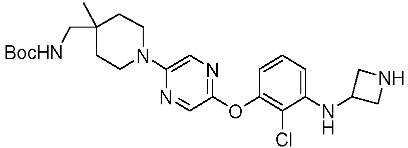 |

### Intermediate 17. tert-butyl ((1-(5-((2-chloro-3-(piperidin-4-ylamino)phenyl)thio)pyrazin-2-yl)-4-methylpiperidin-4-yl)methyl)carbamate

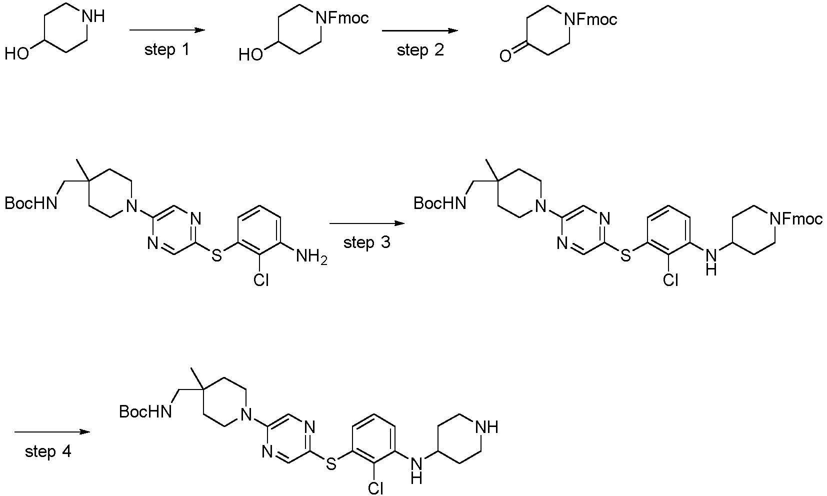

### Step 1: Synthesis of (9H-fluoren-9-yl)methyl 4-hydroxypiperidine-1-carboxylate

4-Hydroxypiperidine (1 g, 9.89 mmol) and Na₂CO₃ (3.14 g, 29.66 mmol) were suspended in a mixed solvent of H₂O: 1,4-dioxane (1:1, 82 ml) and stirred at 0 °C for 10 min. Fmoc-Cl (2.56 g, 9.89 mmol) was added and stirred at room temperature for 16 hours. Distilled water (30 ml) was added to the reaction solution and extracted with EtOAc (30 ml). The organic layer was washed with brine (30 ml), dried over anhydrous sodium sulfate, filtered, and concentrated under reduced pressure. The obtained residue was subjected to MPLC (20-70% EtOAc/hexane) to give 2.95 g (92%) of a white solid.

### Step 2: Synthesis of (9H-fluoren-9-yl)methyl 4-oxopiperidine-1-carboxylate

(9H-Fluoren-9-yl)methyl 4-hydroxypiperidine-1-carboxylate (2.71 g, 8.38 mmol) and DMP (3.90 g, 9.22 mmol) were suspended in DCM (38 ml) and stirred at room temperature for 3 hours. After adding sat. Na₂S₂O₃ (aq.) (40 ml) to the reaction mixture, it was extracted with DCM (50 ml). The organic layer was washed with distilled water (40 ml), dried over anhydrous sodium sulfate, filtered, and concentrated under reduced pressure. The obtained residue was subjected to MPLC (0-35% EtOAc/hexane) to give 2.76 g (quant.) of a white solid.

### Step 3: Synthesis of (9H-fluoren-9-yl)methyl 4-((3-((5-(4-(((tert-butoxycarbonyl)amino)methyl)-4-methylpiperidin-1-yl)pyrazin-2-yl)thio)-2-chlorophenyl)amino)piperidine-1-carboxylate

Tert-Butyl ((1-(5-((3-amino-2-chlorophenyl)thio)pyrazin-2-yl)-4-methylpiperidin-4-yl)methyl)carbamate (Intermediate 1, 150 mg, 0.32 mmol), (9H-fluoren-9-yl)methyl 4-oxopiperidine-1-carboxylate (260 mg, 0.81 mmol), NaBH(OAc)₃ (206 mg, 0.97 mmol), and acetic acid (0.3 ml) were suspended in a mixed solvent of ACN: DCM (1:1, 2.1 ml), and the mixture was stirred at room temperature for 4 hours. After adding sat. NaHCO₃ (aq.) (10 ml) to the reaction solution, it was extracted with DCM (10 ml x 2), and the organic layer was dried over anhydrous sodium sulfate, filtered, and concentrated under reduced pressure. The obtained residue was subjected to MPLC (1-40% EtOAc/hexane) to give 334 mg (crude) of an ivory solid.

### Step 4: Synthesis of tert-butyl ((1-(5-((2-chloro-3-(piperidin-4-ylamino)phenyl)thio)pyrazin-2-yl)-4-methylpiperidin-4-yl)methyl)carbamate

(9H-Fluoren-9-yl)methyl 4-((3-((5-(4-(((tert-butoxycarbonyl)amino)methyl)-4-methylpiperidin-1-yl)pyrazin-2-yl)thio)-2-chlorophenyl)amino)piperidine-1-carboxylate (334 mg, crude) and piperidine (0.64 ml, 6.47 mmol) were suspended in DCM (1.5 ml) and stirred at room temperature for 2 hours. Distilled water (10 ml) was added to the reaction solution, and the mixture was extracted with DCM (10 ml). The organic layer was washed with brine (10 ml), dried over anhydrous sodium sulfate, filtered and concentrated under reduced pressure. The obtained residue was subjected to MPLC (amine-silica, 0-3% MeOH/DCM) to give 80.1 mg (45%, 2 steps) of an ivory solid.

Intermediates 18 to 24 were synthesized using the same method as that used for synthesizing Intermediate 17.

| | |
|---|---|
| Intermediate 18. tert-butyl ((1-(5-((3-((azetidin-3-ylmethyl)amino)-2-chlorophenyl)thio)pyrazin-2-yl)-4-methylpiperidin-4-yl)methyl)carbamate | 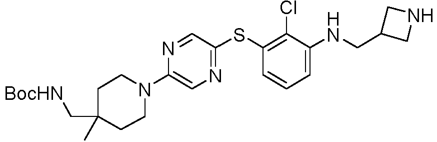 |
| Intermediate 19. tert-butyl ((1-(5-((2-chloro-3-((piperidin-4-ylmethyl)amino)phenyl)thio)pyrazin-2-yl)-4-methylpiperidin-4-yl)methyl)carbamate | 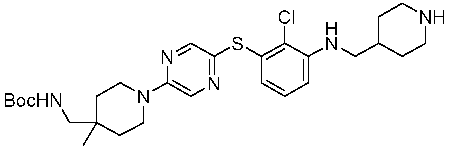 |
| Intermediate 20. tert-butyl ((1-(5-((2-chloro-3-((2-(piperazin-1-yl)ethyl)amino)phenyl)thio)pyrazin-2-yl)-4-methylpiperidin-4-yl)methyl)carbamate | 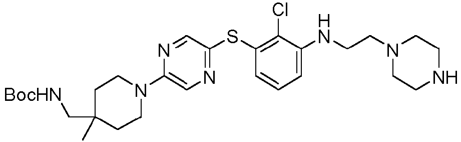 |
| Intermediate 21. tert-butyl ((1-(5-((2-chloro-3-((2-(2-oxopiperazin-1-yl)ethyl)amino)phenyl)thio)pyrazin-2-yl)-4-methylpiperidin-4-yl)methyl)carbamate | 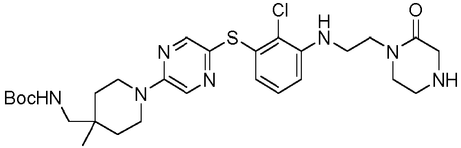 |
| Intermediate 22. (R)-N-((3S,4S)-8-(5-((1-(azetidin-3-yl)indolin-4-yl)thio)pyrazin-2-yl)-3-methyl-2-oxa-8-azaspiro[4.5]decan-4-yl)-2-methylpropane-2-sulfinamide | 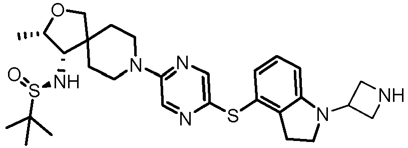 |
| Intermediate 23. (R)-2-methyl-N-((3S,4S)-3-methyl-8-(5-((1-(piperidin-4-yl)indolin-4-yl)thio)pyrazin-2-yl)-2-oxa-8-azaspiro[4.5]decan-4-yl)propane-2-sulfinamide | 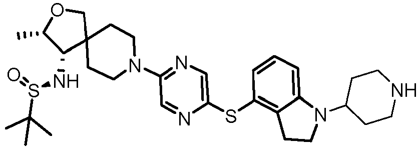 |
| Intermediate 24. tert-butyl ((1-(5-((4-(azetidin-3-yl)-3,4-dihydro-2H-benzo[b][1,4]oxazin-8-yl)thio)pyrazin-2-yl)-4-methylpiperidin-4-yl)methyl)carbamate | 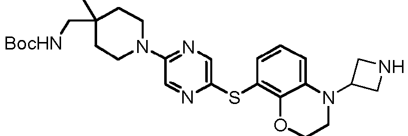 |

### Intermediate 25. tert-butyl ((4-methyl-1-(5-((4-(piperidin-4-yl)-3,4-dihydro-2H-benzo[b][1,4]oxazin-8-yl)thio)pyrazin-2-yl)piperidin-4-yl)methyl)carbamate

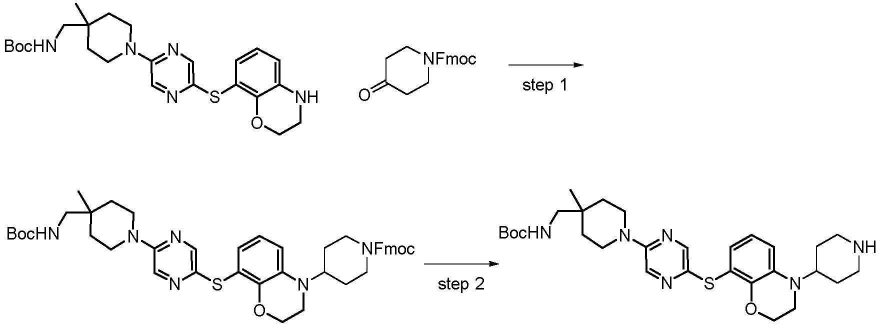

### Step 1: Synthesis of (9H-fluoren-9-yl)methyl 4-(8-((5-(4-(((tert-butoxycarbonyl)amino)methyl)-4-methylpiperidin-1-yl)pyrazin-2-yl)thio)-2,3-dihydro-4H-benzo[b][1,4]oxazin-4-yl)piperidine-1-carboxylate

Tert-Butyl ((1-(5-((3,4-dihydro-2H-benzo[b][1,4]oxazin-8-yl)thio)pyrazin-2-yl)-4-methylpiperidin-4-yl)methyl)carbamate (Intermediate 8, 30 mg, 0.06 mmol), (9H-fluoren-9-yl)methyl 4-oxopiperidine-1-carboxylate (102 mg, 0.32 mmol), phenylsilane (34 mg, 0.32 mmol), and TFA (24 µl, 0.32 mmol) were suspended in ACN (2 ml) and stirred at room temperature for 3 hours. After adding sat. NaHCO₃ (aq.) (5 ml) to the reaction solution, it was extracted with DCM (5 ml x 2). The organic layer was dried over anhydrous sodium sulfate, filtered, and concentrated under reduced pressure. The obtained residue was subjected to MPLC (0-40% EtOAc/hexane) to give 37 mg (75%) of an ivory solid.

### Step 2: Synthesis of tert-butyl ((4-methyl-1-(5-((4-(piperidin-4-yl)-3,4-dihydro-2H-benzo[b][1,4]oxazin-8-yl)thio)pyrazin-2-yl)piperidin-4-yl)methyl)carbamate

((9H-Fluoren-9-yl)methyl 4-(8-((5-(4-(((tert-butoxycarbonyl)amino)methyl)-4-methylpiperidin-1-yl)pyrazin-2-yl)thio)-2,3-dihydro-4H-benzo[b][1,4]oxazin-4-yl)piperidine-1-carboxylate (40 mg, 0.05 mmol) was suspended in DCM (0.8 ml) and piperidine (0.1 ml, 1.03 mmol) was added. The mixture was stirred at room temperature for 2 hours. Distilled water (10 ml) was added to the reaction solution, and extracted with DCM (10 ml). The organic layer was washed with brine (10 ml), dried over anhydrous sodium sulfate, filtered, and concentrated under reduced pressure. The obtained residue was subjected to MPLC (amine-silica, 0-3% MeOH/DCM) to give 24 mg (84%) of a white solid.

Intermediate 26 was synthesized using the same method as that used for synthesizing Intermediate 25.

| | |
|---|---|
| Intermediate 26. tert-butyl ((3S,4S)-3-methyl-8-(5-((4-(piperidin-4-yl)-3,4-dihydro-2H-benzo[b][1,4]oxazin-8-yl)thio)pyrazin-2-yl)-2-oxa-8-azaspiro[4.5]decan-4-yl)carbamate | 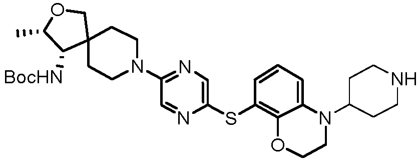 |

### Intermediate 27. tert-butyl ((1-(5-((3-fluoro-1-(piperidin-4-yl)-1H-indol-4-yl)thio)pyrazin-2-yl)-4-methylpiperidin-4-yl)methyl)carbamate

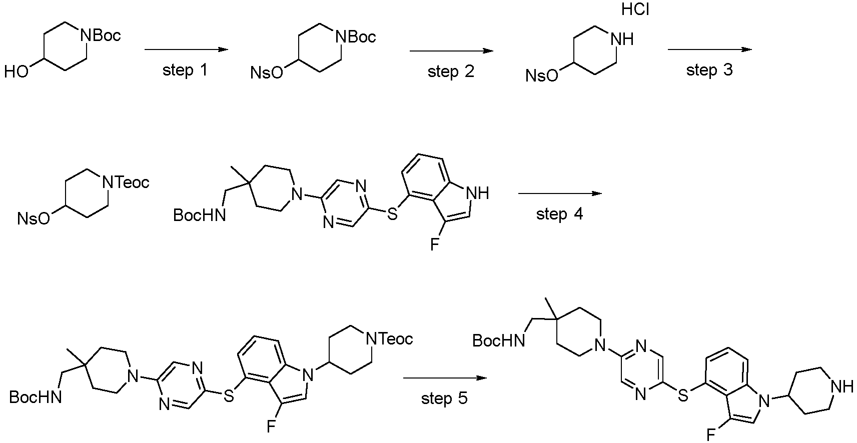

### Step 1: Synthesis of tert-butyl 4-(((2-nitrophenyl)sulfonyl)oxy)piperidine-1-carboxylate

Tert-Butyl 4-hydroxypiperidine-1-carboxylate (5 g, 24.84 mmol), 2-nitrobenzenesulfonyl chloride (6.6 g, 29.81 mmol), TEA (6.9 ml, 49.68 mmol), and DMAP (303 mg, 2.48 mmol) were suspended in DCM (48 ml) and stirred at room temperature for 3 hours. Distilled water (50 ml) was added to the reaction solution, and the mixture was extracted with DCM (50 ml). The organic layer was dried over anhydrous sodium sulfate, filtered, and concentrated under reduced pressure. The obtained residue was subjected to MPLC (0-30% EtOAc/hexane) to give 5.89 g (61%) of a white solid.

### Step 2: Synthesis of piperidin-4-yl 2-nitrobenzenesulfonate hydrochloride

Tert-Butyl 4-(((2-nitrophenyl)sulfonyl)oxy)piperidine-1-carboxylate (300 mg, 0.78 mmol) was suspended in DCM (5.2 ml), 4 M HCl in dioxane (1.9 ml) was added, and the mixture was stirred at room temperature for 1 hour. The reaction solution was concentrated under reduced pressure to give 243 mg (quant.) of a white solid.

### Step 3: Synthesis of 2-(trimethylsilyl)ethyl 4-(((2-nitrophenyl)sulfonyl)oxy)piperidine-1-carboxylate

Piperidin-4-yl 2-nitrobenzenesulfonate hydrochloride (243 mg, 0.78 mmol) and TEA (0.43 ml, 3.11 mmol) were suspended in DCM (2.6 ml), 1-[2-(trimethylsilyl)ethoxycarbonyloxy]pyrrolidin-2,5-dione (221 mg, 0.85 mmol) was added, and the mixture was stirred at room temperature for 1 hour. Distilled water (20 ml) was added to the reaction solution, and the mixture was extracted with DCM (20 ml). The organic layer was dried over anhydrous sodium sulfate, filtered, and concentrated under reduced pressure. The obtained residue was subjected to MPLC (0-40% EtOAc/hexane) to give 327 mg (98%) of a white solid.

### Step 4: Synthesis of 2-(trimethylsilyl)ethyl 4-(4-((5-(4-(((tert-butoxycarbonyl)amino)methyl)-4-methylpiperidin-1-yl)pyrazin-2-yl)thio)-3-fluoro-1H-indol-1-yl)piperidine-1-carboxylate

Tert-Butyl ((1-(5-((3-fluoro-1H-indol-4-yl)thio)pyrazin-2-yl)-4-methylpiperidin-4-yl)methyl)carbamate (Intermediate 10, 10 mg, 0.02 mmol), 2-(trimethylsilyl)ethyl 4-(((2-nitrophenyl)sulfonyl)oxy)piperidine-1-carboxylate (27 mg, 0.06 mmol), and Cs₂CO₃ (14 mg, 0.04 mmol) were suspended in DMF (0.05 ml) and the mixture was stirred at 70 °C for 16 hours. Distilled water (5 ml) was added to the reaction solution, and extracted with EtOAc (5 ml). The organic layer was washed with brine (5 ml), dried over anhydrous sodium sulfate, filtered, and concentrated under reduced pressure. The obtained residue was subjected to MPLC (0-40% EtOAc/hexane), (0-10% EtOAc/DCM), to give 10.6 mg (crude) of an ivory solid.

### Step 5: Synthesis of tert-butyl ((1-(5-((3-fluoro-1-(piperidin-4-yl)-1H-indol-4-yl)thio)pyrazin-2-yl)-4-methylpiperidin-4-yl)methyl)carbamate

2-(Trimethylsilyl)ethyl 4-(4-((5-(4-(((tert-butoxycarbonyl)amino)methyl)-4-methylpiperidin-1-yl)pyrazin-2-yl)thio)-3-fluoro-1H-indol-1-yl)piperidine-1-carboxylate (10.6 mg, crude) was suspended in THF (0.05 ml), TBAF (1.0 M in THF, 30 µl, 0.03 mmol) was added, and the mixture was stirred at 50 °C for 1 h. Distilled water (5 ml) was added to the reaction solution, and then extracted with EtOAc (5 ml). The organic layer was washed with brine (5 ml), dried over anhydrous sodium sulfate, filtered, and concentrated under reduced pressure. The obtained residue was subjected to MPLC (amine-silica, 0-5% MeOH/DCM) to give 3.7 mg (25%, 2 steps) of an ivory solid.

### Intermediate 28. tert-butyl ((1-(5-((2-(azetidin-3-ylamino)-3-chloropyridin-4-yl)thio)pyrazin-2-yl)-4-methylpiperidin-4-yl)methyl)carbamate

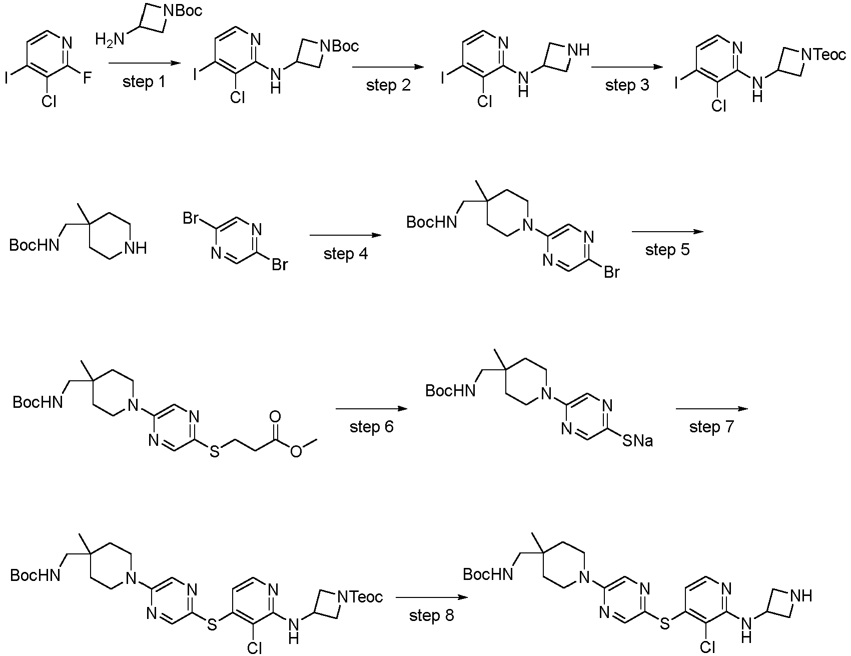

### Step 1: Synthesis of tert-butyl 3-((3-chloro-4-iodopyridin-2-yl)amino)azetidine-1-carboxylate

3-Chloro-2-fluoro-4-iodopyridine (500 mg, 1.94 mmol), 1-Boc-3-aminoazetidine (669 mg, 3.88 mmol), and DIPEA (0.68 ml, 3.88 mmol) were suspended in DMSO (7.5 ml) and stirred at 100 °C for 2 hours. Distilled water (30 ml) was added to the reaction solution and extracted with EtOAc (30 ml). The organic layer was washed with brine (30 ml), dried over anhydrous sodium sulfate, filtered, and concentrated under reduced pressure. The obtained residue was subjected to MPLC (1-15% EtOAc/hexane) to give 540 mg (68%) of a white solid.

### Step 2: Synthesis of N-(azetidin-3-yl)-3-chloro-4-iodopyridin-2-amine

Tert-Butyl 3-((3-chloro-4-iodopyridin-2-yl)amino)azetidine-1-carboxylate (440 mg, 1.07 mmol) was suspended in DCM (10.6 ml), TFA (2.6 ml) was added, and the mixture was stirred at room temperature for 1 hour. The reaction solution was concentrated under reduced pressure to give 800 mg (quant.) of brown oil.

### Step 3: Synthesis of 2-(trimethylsilyl)ethyl 3-((3-chloro-4-iodopyridin-2-yl)amino)azetidine-1-carboxylate

N-(Azetidin-3-yl)-3-chloro-4-iodopyridin-2-amine (800 mg, 1.07 mmol) and TEA (0.60 ml, 4.30 mmol) were suspended in DCM (3.8 ml), and 1-[2-(trimethylsilyl)ethoxycarbonyloxy]pyrrolidin-2,5-dione (279 mg, 1.07 mmol) was added at 0 °C, and the mixture was stirred at room temperature for 1 hour. Distilled water (15 ml) was added to the reaction solution, and the mixture was extracted with DCM (20 ml). The organic layer was dried over anhydrous sodium sulfate, filtered, and concentrated under reduced pressure. The obtained residue was subjected to MPLC (1-15% EtOAc/hexane) to give 468 mg (96%) of colorless oil.

### Step 4: Synthesis of tert-butyl ((1-(5-bromopyrazin-2-yl)-4-methylpiperidin-4-yl)methyl)carbamate

tert-Butyl ((4-methylpiperidin-4-yl)methyl)carbamate (504 mg, 2.21 mmol), 2,5-dibromopyrazine (500 mg, 2.10 mmol), and DIPEA (1.1 ml, 6.31 mmol) were suspended in NMP (2 ml) and stirred at 130 °C for 1 hour. Distilled water (20 ml) was added to the reaction solution and extracted with EtOAc (30 ml). The organic layer was washed with brine (20 ml), dried over anhydrous sodium sulfate, filtered, and concentrated under reduced pressure. The obtained residue was subjected to MPLC (1-15% EtOAc/hexane) to give 705 mg (87%) of a yellow solid.

### Step 5: Synthesis of methyl 3-((5-(4-(((tert-butoxycarbonyl)amino)methyl)-4-methylpiperidin-1-yl)pyrazin-2-yl)thio)propanoate

tert-Butyl ((1-(5-bromopyrazin-2-yl)-4-methylpiperidin-4-yl)methyl)carbamate (705 mg, 1.83 mmol), methyl 3-mercaptopropionate (0.22 ml, 2.01 mmol), Pd₂(dba)₃ (84 mg, 0.09 mmol), Xantphos (106 mg, 0.18 mmol), and DIPEA (0.64 ml, 3.66 mmol) were suspended in 1,4-dioxane (3.6 ml) and stirred at 90 °C for 3 hours. The reaction solution was filtered, and the filtrate was concentrated under reduced pressure. The obtained residue was subjected to MPLC (1-35% EtOAc/hexane) to give 680 mg (88%) of yellow oil.

### Step 6: Synthesis of sodium 5-(4-(((tert-butoxycarbonyl)amino)methyl)-4-methylpiperidin-1-yl)pyrazine-2-thiolate

Methyl 3-((5-(4-(((tert-butoxycarbonyl)amino)methyl)-4-methylpiperidin-1-yl)pyrazin-2-yl)thio)propanoate (243 mg, 0.57 mmol) was suspended in THF (1.28 ml), and NaOEt (ca. 20% in EtOH, 0.21 ml, 0.63 mmol) was added. The mixture was stirred at room temperature for 2 hours. The reaction solution was concentrated under reduced pressure to give 278 mg (crude) of a yellow solid.

### Step 7: Synthesis of 2-(trimethylsilyl)ethyl 3-((4-((5-(4-(((tert-butoxycarbonyl)amino)methyl)-4-methylpiperidin-1-yl)pyrazin-2-yl)thio)-3-chloropyridin-2-yl)amino)azetidine-1-carboxylate

2-(Trimethylsilyl)ethyl 3-((3-chloro-4-iodopyridin-2-yl)amino)azetidine-1-carboxylate (200 mg, 0.44 mmol), sodium 5-(4-(((tert-butoxycarbonyl)amino)methyl)-4-methylpiperidin-1-yl)pyrazine-2-thiolate (278 mg, crude, 0.57 mmol), Pd₂(dba)₃ (20 mg, 0.02 mmol), Xantphos (25 mg, 0.04 mmol), and DIPEA (0.15 ml, 0.88 mmol) were suspended in 1,4-dioxane (1 ml), and stirred at 90 °C for 16 hours. Distilled water (15 ml) was added to the reaction solution, and the mixture was extracted with EtOAc (20 ml). The organic layer was washed with brine (15 ml), dried over anhydrous sodium sulfate, filtered, and concentrated under reduced pressure. The obtained residue was subjected to MPLC (1-40% EtOAc/hexane) to give 278 mg (95%) of a yellow solid.

### Step 8: Synthesis of tert-butyl ((1-(5-((2-(azetidin-3-ylamino)-3-chloropyridin-4-yl)thio)pyrazin-2-yl)-4-methylpiperidin-4-yl)methyl)carbamate

2-(Trimethylsilyl)ethyl 3-((4-((5-(4-(((tert-butoxycarbonyl)amino)methyl)-4-methylpiperidin-1-yl)pyrazin-2-yl)thio)-3-chloropyridin-2-yl)amino)azetidine-1-carboxylate (37.8 mg, 0.06 mmol) and TBAF (1.0 M in THF, 0.17 ml, 0.17 mmol) were suspended in THF (0.43 ml) and stirred at 50 °C for 2 hours. Distilled water (5 ml) was added to the reaction solution, extracted with DCM (5 ml), and the organic layer was dried over anhydrous sodium sulfate, filtered and concentrated under reduced pressure to give 28.5 mg (96%) of an ivory solid.

Intermediates 29 to 34 were synthesized using the same method as that used for synthesizing Intermediate 28.

| | |
|---|---|
| Intermediate 29. tert-butyl (1-(5-((2-(azetidin-3-ylamino)-3-chloropyridin-4-yl)thio)pyrazin-2-yl)-4-methylpiperidin-4-yl)carbamate | 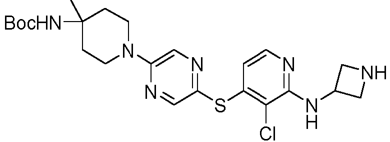 |
| Intermediate 30. tert-butyl ((3S,4S)-8-(5-((2-(azetidin-3-ylamino)-3-chloropyridin-4-yl)thio)pyrazin-2-yl)-3-methyl-2-oxa-8-azaspiro[4.5]decan-4-yl)carbamate | 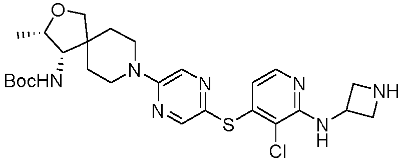 |
| Intermediate 31. tert-butyl ((1-(5-((3-chloro-2-(piperidin-4-ylamino)pyridin-4-yl)thio)pyrazin-2-yl)-4-methylpiperidin-4-yl)methyl)carbamate | 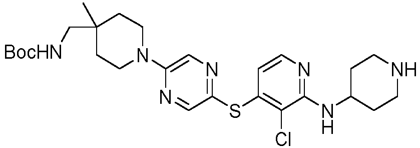 |
| Intermediate 32. tert-butyl (1-(5-((3-chloro-2-(piperidin-4-ylamino)pyridin-4-yl)thio)pyrazin-2-yl)-4-methylpiperidin-4-yl)carbamate | 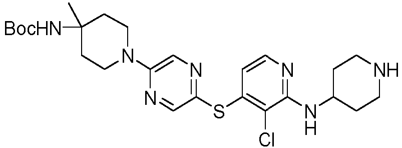 |
| Intermediate 33. tert-butyl ((38,48)-8-(5-((3-chloro-2-(piperidin-4-ylamino)pyridin-4-yl)thio)pyrazin-2-yl)-3-methyl-2-oxa-8-azaspiro[4.5]decan-4-yl)carbamate | 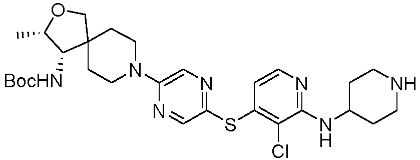 |
| Intermediate 34. tert-butyl ((1-(5-((3-chloro-2-(piperazin-1-yl)pyridin-4-yl)thio)pyrazin-2-yl)-4-methylpiperidin-4-yl)methyl)carbamate | 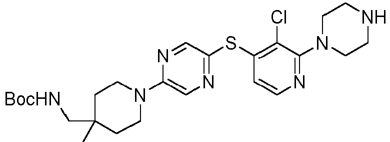 |

### Intermediate 35. (1S,3S)-3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylic acid

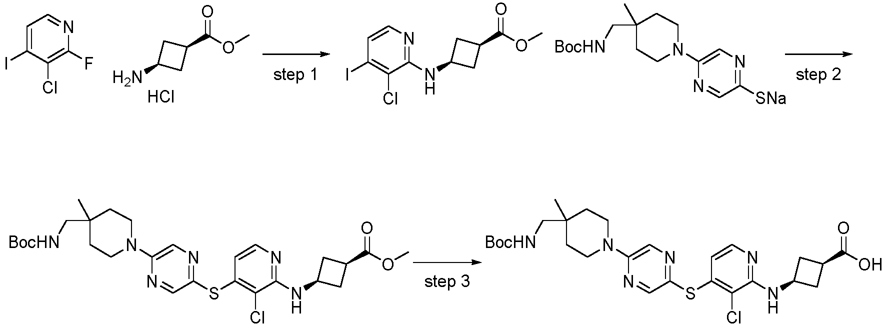

### Step 1: Synthesis of (1S,3S)-methyl 3-((3-chloro-4-iodopyridin-2-yl)amino)cyclobutane-1-carboxylate

3-Chloro-2-fluoro-4-iodopyridine (1 g, 3.88 mmol), methyl (1S,3S)-3-aminocyclobutane-1-carboxylate hydrochloride (644 mg, 3.88 mmol), and DIPEA (2 ml, 11.65 mmol) were suspended in DMSO (12 ml) and stirred at 100 °C for 4 hours. Distilled water (30 ml) was added to the reaction solution and extracted with EtOAc (30 ml x 2). The organic layer was washed with brine (30 ml x 2), dried over anhydrous sodium sulfate, filtered, and concentrated under reduced pressure. The obtained residue was subjected to MPLC (0-10% EtOAc/Hex) to give 600 mg (42%) of a yellow solid.

### Step 2: Synthesis of (1S,3S)-methyl 3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylate

Sodium 5-(4-(((tert-butoxycarbonyl)amino)methyl)-4-methylpiperidin-1-yl)pyrazine-2-thiolate (905 mg, 2.51 mmol), (1S,3S)-methyl 3-((3-chloro-4-iodopyridin-2-yl)amino)cyclobutane-1-carboxylate (460 mg, 1.25 mmol), Pd₂(dba)₃ (14 mg, 0.06 mmol), Xantphos (73 mg, 0.13 mmol), and DIPEA (0.43 ml, 2.51 mmol) was suspended in 1,4-dioxane (4 ml) and stirred at 90 °C for 16 hours. The reaction solution was filtered, and the filtrate was concentrated under reduced pressure. The obtained residue was subjected to MPLC (0-50% EtOAc/hexane) to give 680 mg (94%) of an ivory solid.

### Step 3: Synthesis of (1S,3S)-3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylic acid

(1S,3S)-Methyl 3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylate (377 mg, 0.65 mmol) and LiOH (41 mg, 0.98 mmol) were suspended in a mixed solvent of THF: H₂O: MeOH (4: 1: 1, 2 ml) and stirred at room temperature for 2 hours. The reaction solution was adjusted to pH 1-2 with 1 M HCl (aq.) and extracted with EtOAc (15 ml x 2). The organic layer was dried over anhydrous sodium sulfate, filtered, and concentrated under reduced pressure. The obtained residue was subjected to MPLC (0-66% EtOAc/hexane) to give 241 mg (66%) of an orange solid.

Intermediates 36 to 48 were synthesized using the same method as that used for synthesizing Intermediate 35.

| | |
|---|---|
| Intermediate 36. (18,38)-3-((4-((5-(4-((tert-butoxycarbonyl)amino)-4-methylpiperidin-1-yl)pyrazin-2-yl)thio)-3-chloropyridin-2-yl)amino)cyclobutane-1-carboxylic acid | 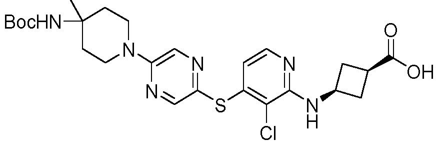 |
| Intermediate 37. (1R,3R)-3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylic acid | 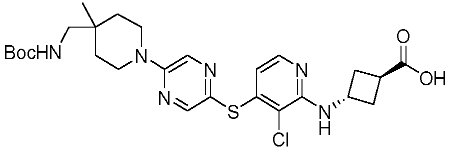 |
| Intermediate 38. (1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexane-1-carboxylic acid | 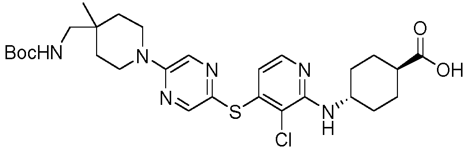 |
| Intermediate 39. 1-(4-((5-(4-(((tert-butoxycarbonyl)amino)methyl)-4-methylpiperidin-1-yl)pyrazin-2-yl)thio)-3-chloropyridin-2-yl)azetidine-3-carboxylic acid | 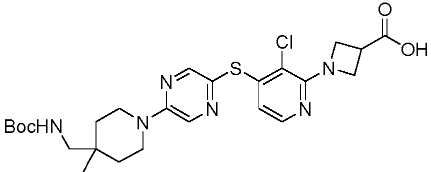 |
| Intermediate 40. 2-(1-(4-((5-(4-(((tert-butoxycarbonyl)amino)methyl)-4-methylpiperidin-1-yl)pyrazin-2-yl)thio)-3-chloropyridin-2-yl)azetidin-3-yl)acetic acid | 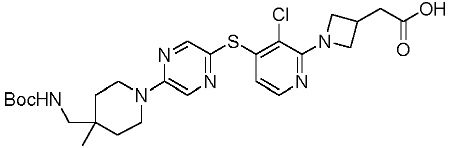 |
| Intermediate 41. 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid | 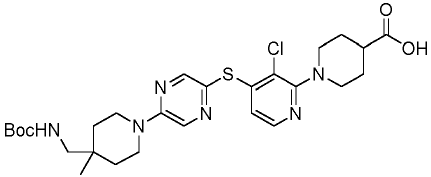 |
| Intermediate 42. 1-(4-((5-(4-((tert-butoxycarbonyl)amino)-4-methylpiperidin-1-yl)pyrazin-2-yl)thio)-3-chloropyridin-2-yl)piperidine-4-carboxylic acid | 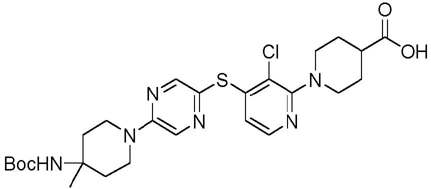 |
| Intermediate 43. 1-(4-((5-((3S,4S)-4-((tert-butoxycarbonyl)amino)-3-methyl-2-oxa-8-azaspiro[4.5]decan-8-yl)pyrazin-2-yl)thio)-3-chloropyridin-2-yl)piperidine-4-carboxylic acid | 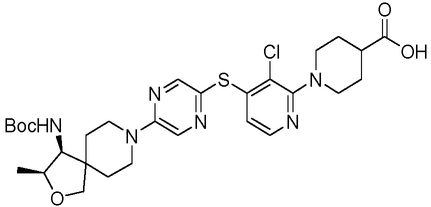 |
| Intermediate 44. 1-(4-((5-(4-(((tert-butoxycarbonyl)amino)methyl)-4-methylpiperidin-1-yl)pyrazin-2-yl)thio)-3-chloropyridin-2-yl)-4-methylpiperidine-4-carboxylic acid | 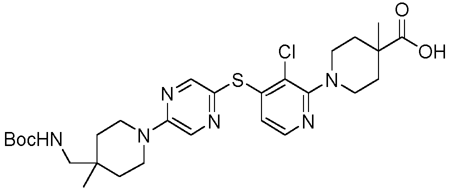 |
| Intermediate 45. 1-(4-((5-(4-(((tert-butoxycarbonyl)amino)methyl)-4-methylpiperidin-1-yl)pyrazin-2-yl)thio)-3-chloropyridin-2-yl)-4-fluoropiperidine-4-carboxylic acid | 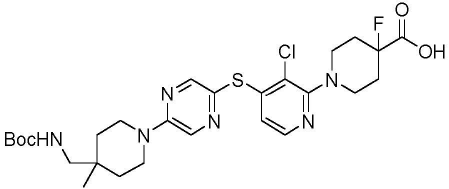 |
| Intermediate 46. 1-(4-((5-(4-(((tert-butoxycarbonyl)amino)methyl)-4-methylpiperidin-1-yl)pyrazin-2-yl)thio)-3-chloropyridin-2-yl)-4-hydroxypiperidine-4-carboxylic acid | 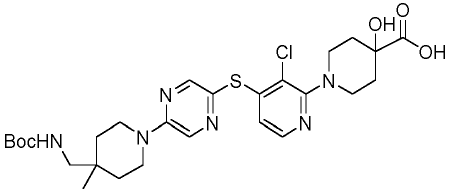 |
| Intermediate 47. 2-(1-(4-((5-(4-(((tert-butoxycarbonyl)amino)methyl)-4-methylpiperidin-1-yl)pyrazin-2-yl)thio)-3-chloropyridin-2-yl)piperidin-4-yl)acetic acid | 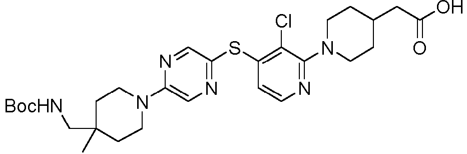 |
| Intermediate 48. 2-(1-(4-((5-(4-(((tert-butoxycarbonyl)amino)methyl)-4-methylpiperidin-1-yl)pyrazin-2-yl)thio)-3-chloropyridin-2-yl)-4-hydroxypiperidin-4-yl)acetic acid | 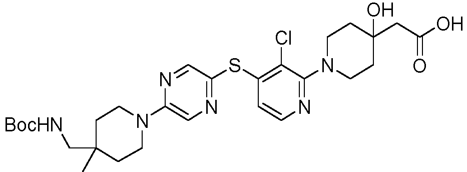 |

### Intermediate 49. tert-butyl ((1-(5-((2-(azetidin-3-ylamino)-3-chloropyridin-4-yl)thio)-3-(hydroxymethyl)pyrazin-2-yl)-4-methylpiperidin-4-yl)methyl)carbamate

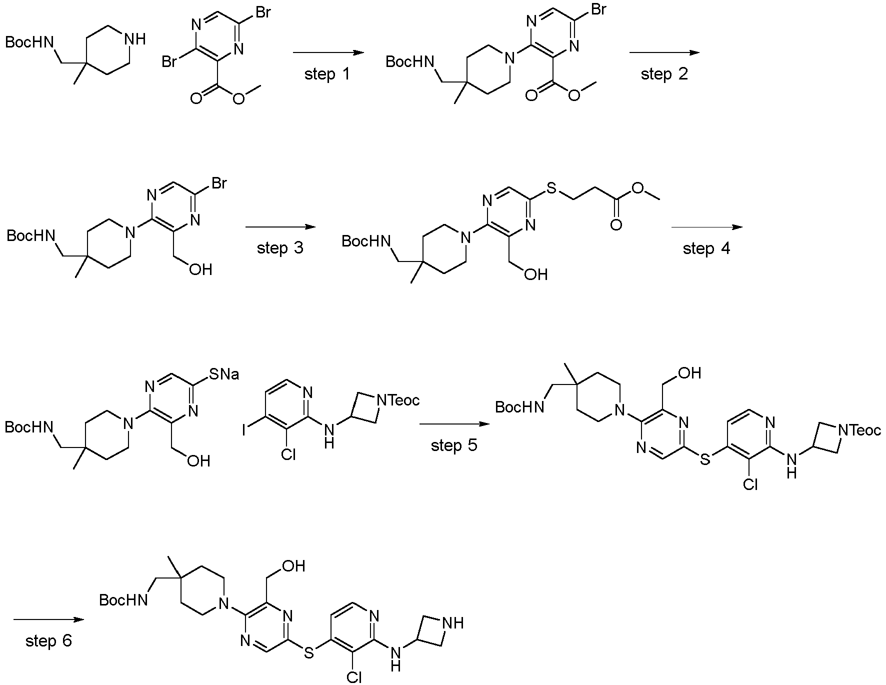

### Step 1: Synthesis of methyl 6-bromo-3-(4-(((tert-butoxycarbonyl)amino)methyl)-4-methylpiperidin-1-yl)pyrazine-2-carboxylate

tert-Butyl ((4-methylpiperidin-4-yl)methyl)carbamate (4.63 g, 20.28 mmol), methyl 3,6-dibromopyrazine-2-carboxylate (5 g, 16.9 mmol), and TEA (4.7 ml, 33.8 mmol) were suspended in DMF (75 ml) and stirred at 80 °C for 2 hours. Distilled water (50 ml) was added to the reaction solution and extracted with EtOAc (50 ml x 2). The organic layer was washed with brine (50 ml x 2), dried over anhydrous sodium sulfate, filtered, and concentrated under reduced pressure. The obtained residue was subjected to MPLC (0-15% EtOAc/Hex) to give 7.18 g (96%) of a yellow solid.

### Step 2: Synthesis of tert-butyl ((1-(5-bromo-3-(hydroxymethyl)pyrazin-2-yl)-4-methylpiperidin-4-yl)methyl)carbamate

Methyl 6-bromo-3-(4-(((tert-butoxycarbonyl)amino)methyl)-4-methylpiperidin-1-yl)pyrazine-2-carboxylate (3 g, 6.77 mmol) was suspended in EtOH (16 ml), and NaBH₄ (640 mg, 16.92 mmol) and CaCl₂ (1.5 mg, 13.53 mmol) were added at 0 °C, and the mixture was stirred at room temperature for 16 hours. After adding sat. NH₄Cl (aq.) (40 ml) to the reaction solution, it was extracted with EtOAc (40 ml). The organic layer was washed with brine (40 ml), dried over anhydrous sodium sulfate, filtered and concentrated under reduced pressure. The obtained residue was subjected to MPLC (1-20% EtOAc/hexane) to give 1.08 g (39%) of a yellow solid.

### Step 3: Synthesis of methyl 3-((5-(4-(((tert-butoxycarbonyl)amino)methyl)-4-methylpiperidin-1-yl)-6-(hydroxymethyl)pyrazin-2-yl)thio)propanoate

tert-Butyl ((1-(5-bromo-3-(hydroxymethyl)pyrazin-2-yl)-4-methylpiperidin-4-yl)methyl)carbamate (1.07 g, 2.58 mmol), methyl 3-mercaptopropionate (0.31 ml, 2.83 mmol), Pd₂(dba)₃ (118 mg, 0.13 mmol), Xantphos (149 mg, 0.26 mmol), and DIPEA (0.93 ml, 5.15 mmol) were suspended in 1,4-dioxane (10 ml) and stirred at 90 °C for 1 hour. The reaction solution was filtered, and the filtrate was concentrated under reduced pressure. The obtained residue was subjected to MPLC (1-40% EtOAc/hexane) to give 1.07 g (82%) of yellow oil.

### Step 4: Synthesis of sodium 5-(4-(((tert-butoxycarbonyl)amino)methyl)-4-methylpiperidin-1-yl)-6-(hydroxymethyl)pyrazine-2-thiolate

Methyl 3-((5-(4-(((tert-butoxycarbonyl)amino)methyl)-4-methylpiperidin-1-yl)-6-(hydroxymethyl)pyrazin-2-yl)thio)propanoate (167 mg, 0.37 mmol) was suspended in THF (1.2 ml), and NaOEt (ca. 20% in EtOH, 0.16 ml, 0.40 mmol) was added. The mixture was stirred at room temperature for 2 hours. The reaction solution was concentrated under reduced pressure to give 143 mg (crude) of a yellow solid.

### Step 5: Synthesis of 2-(trimethylsilyl)ethyl 3-((4-((5-(4-(((tert-butoxycarbonyl)amino)methyl)-4-methylpiperidin-1-yl)-6-(hydroxymethyl)pyrazin-2-yl)thio)-3-chloropyridin-2-yl)amino)azetidine-1-carboxylate

2-(Trimethylsilyl)ethyl 3-((3-chloro-4-iodopyridin-2-yl)amino)azetidine-1-carboxylate (111 mg, 0.24 mmol), sodium 5-(4-(((tert-butoxycarbonyl)amino)methyl)-4-methylpiperidin-1-yl)-6-(hydroxymethyl)pyrazine-2-thiolate (143 mg, crude, 0.37 mmol), Pd₂(dba)₃ (11 mg, 0.01 mmol), Xantphos (14 mg, 0.24 mmol), and DIPEA (0.13 ml, 0.73 mmol) were suspended in 1,4-dioxane (0.8 ml), and stirred at 90 °C for 16 hours. Distilled water (15 ml) was added to the reaction solution, and the mixture was extracted with EtOAc (15 ml). The organic layer was washed with brine (15 ml), dried over anhydrous sodium sulfate, filtered, and concentrated under reduced pressure. The obtained residue was subjected to MPLC (1-20% EtOAc/hexane) to give 71 mg (55%) of a yellow solid.

### Step 6: Synthesis of tert-butyl ((1-(5-((2-(azetidin-3-ylamino)-3-chloropyridin-4-yl)thio)-3-(hydroxymethyl)pyrazin-2-yl)-4-methylpiperidin-4-yl)methyl)carbamate

2-(Trimethylsilyl)ethyl 3-((4-((5-(4-(((tert-butoxycarbonyl)amino)methyl)-4-methylpiperidin-1-yl)-6-(hydroxymethyl)pyrazin-2-yl)thio)-3-chloropyridin-2-yl)amino)azetidine-1-carboxylate (50 mg, 0.09 mmol) and TBAF (1.0 M in THF, 0.28 ml, 0.28 mmol) were suspended in THF (0.93 ml) and stirred at 50°C for 2 hours. Distilled water (5 ml) was added to the reaction solution, extracted with DCM (5 ml), and the organic layer was dried over anhydrous sodium sulfate. After filtration and concentration under reduced pressure, 51 mg (crude) of an ivory solid was obtained.

Intermediate 50 was synthesized using the same method as that used for synthesizing Intermediate 49.

| | |
|---|---|
| Intermediate 50. tert-butyl ((1-(5-((3-chloro-2-(piperidin-4-ylamino)pyridin-4-yl)thio)-3-(hydroxymethyl)pyrazin-2-yl)-4-methylpiperidin-4-yl)methyl)carbamate | 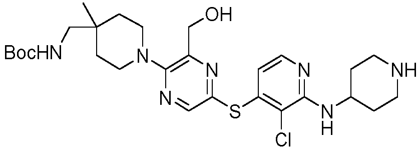 |

### Intermediate 51. 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid

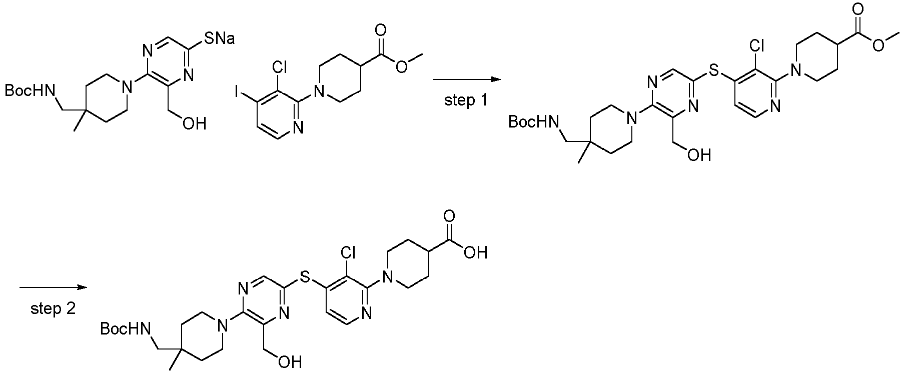

### Step 1: Synthesis of methyl 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylate

Sodium 5-(4-(((tert-butoxycarbonyl)amino)methyl)-4-methylpiperidin-1-yl)-6-(hydroxymethyl)pyrazine-2-thiolate (132 mg, 0.34 mmol), methyl 1-(3-chloro-4-iodopyridin-2-yl)piperidine-4-carboxylate (86 mg, 0.23 mmol), Pd2(dba)3 (10 mg, 0.01 mmol), Xantphos (12 mg, 0.02 mmol), and DIPEA (0.11 ml, 0.68 mmol) were suspended in 1,4-dioxane (1.1 ml), and stirred at 90 °C for 16 hours. The reaction solution was filtered, and the filtrate was concentrated under reduced pressure. The obtained residue was subjected to MPLC (0-50% EtOAc/hexane) to give 104 mg (74%) of an ivory solid.

### Step 2: Synthesis of 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid

Methyl 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylate (102 mg, 0.16 mmol) and LiOH (10 mg, 0.25 mmol) were suspended in a mixed solvent of THF:H₂O (3:1, 0.72 ml) and stirred at room temperature for 2 hours. The reaction solution was adjusted to pH 1~2 with 1 M HCl (aq.) and extracted with EtOAc (15 ml x 2). The organic layer was dried over anhydrous sodium sulfate, filtered and concentrated under reduced pressure to give 104 mg (99%) of an orange solid.

Intermediates 52 to 54 were synthesized using the same method as that used for synthesizing Intermediate 51.

| | |
|---|---|
| Intermediate 52. 1-(4-((5-(4-((tert-butoxycarbonyl)amino)-4-methylpiperidin-1-yl)-6-(hydroxymethyl)pyrazin-2-yl)thio)-3-chloropyridin-2-yl)piperidine-4-carboxylic acid | 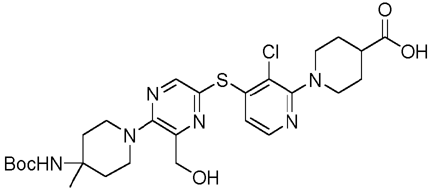 |
| Intermediate 53. 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid | 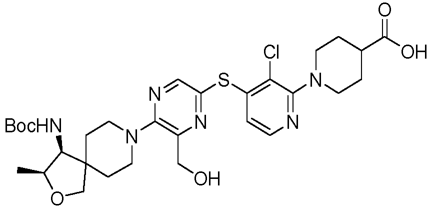 |
| Intermediate 54. 1-(4-((5-(4-(((tert-butoxycarbonyl)amino)methyl)-4-methylpiperidin-1-yl)-6-(hydroxymethyl)-3-methylpyrazin-2-yl)thio)-3-chloropyridin-2-yl)piperidine-4-carboxylic acid | 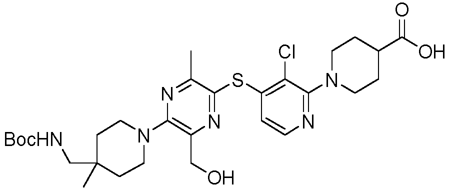 |

### Intermediate 55. tert-butyl ((1-(5-((2-chloro-3-(piperidin-4-yl)phenyl)thio)pyrazin-2-yl)-4-methylpiperidin-4-yl)methyl)carbamate

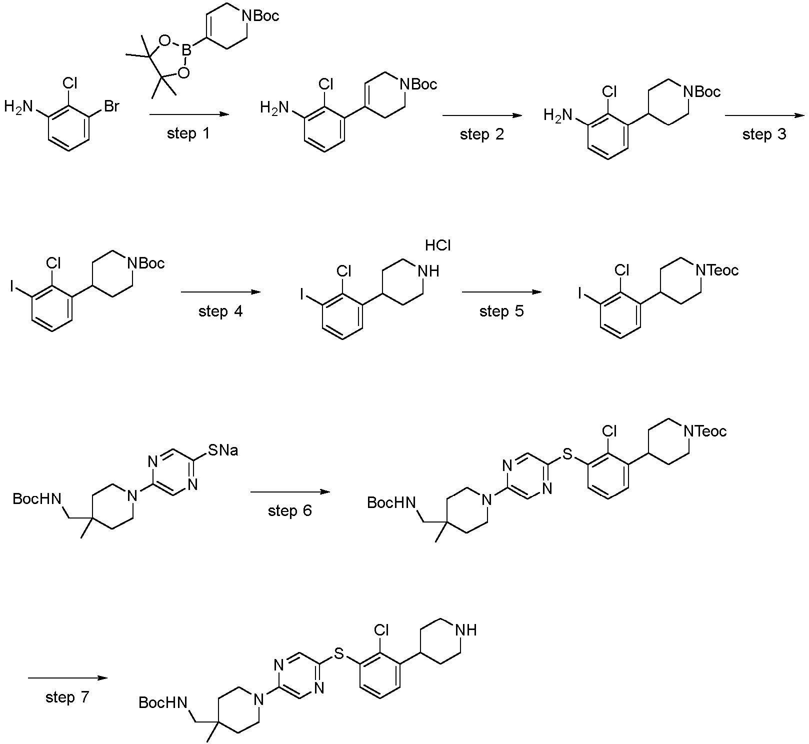

### Step 1: Synthesis of tert-butyl 4-(3-amino-2-chlorophenyl)-3,6-dihydropyridine-1(2H)-carboxylate

3-Bromo-2-chloroaniline (200 mg, 0.97 mmol), tert-butyl 4-(4,4,5,5-tetramethyl-1,3,2-dioxaborolan-2-yl)-3,6-dihydropyridine-1(2H)-carboxylate (329 mg, 1.07 mmol), Pd(dppf)₂ (79 mg, 0.10 mmol), and Cs₂CO₃ (631 mg, 1.94 mmol) were suspended in a mixed solvent of 1,4-dioxane : H₂O (4 : 1, 4 ml) and stirred at 90 °C for 2 hours. The reaction solution was filtered through celite, and the filtrate was concentrated under reduced pressure. The obtained residue was subjected to MPLC (1-10% EtOAc/hexane) to give 270 mg (90%) of yellow gum.

### Step 2: Synthesis of tert-butyl 4-(3-amino-2-chlorophenyl)piperidine-1-carboxylate

tert-Butyl 4-(3-amino-2-chlorophenyl)-3,6-dihydropyridine-1(2H)-carboxylate (201 mg, 0.65 mmol) was suspended in MeOH (4.6 ml), Pd/C (23 mg, 10% purity) was added, and the mixture was stirred at room temperature for 3.5 hours under a stream of hydrogen. The reaction solution was filtered through celite, and the filtrate was concentrated under reduced pressure. The obtained residue was subjected to MPLC (1-10% EtOAc/hexane) to give 41.8 mg (21%) of colorless oil.

### Step 3: Synthesis of tert-butyl 4-(2-chloro-3-iodophenyl)piperidine-1-carboxylate

tert-Butyl 4-(3-amino-2-chlorophenyl)piperidine-1-carboxylate (41.8 mg, 0.13 mmol) was suspended in a mixed solvent of 2 M HCl (aq.) : dioxane (2 : 1, 0.87 ml), and sodium nitrite (10 mg, 0.15 mmol) and H₂O (0.03 ml) were added at 0 °C, and the mixture was stirred at 0 °C for 15 min. NaI (22 mg, 0.15 mmol) was added to the reaction solution, and the mixture was stirred at room temperature for 10 min. Distilled water (5 ml) was added to the reaction solution, and then extracted with EtOAc (5 ml). The organic layer was dried over anhydrous sodium sulfate, filtered, and concentrated under reduced pressure. The obtained residue was subjected to MPLC (0-10% EtOAc/hexane) to give 24.2 mg (43%) of brown oil.

### Step 4: Synthesis of 4-(2-chloro-3-iodophenyl)piperidine hydrochloride

tert-Butyl 4-(2-chloro-3-iodophenyl)piperidine-1-carboxylate (24.2 mg, 0.06 mmol) was suspended in DCM (0.57 ml), 4 M HCl in dioxane (0.14 ml) was added, and the mixture was stirred at room temperature for 1 hour. The reaction solution was concentrated under reduced pressure to obtain 19.8 mg (quant.) of a white solid.

### Step 5: Synthesis of 2-(trimethylsilyl)ethyl 4-(2-chloro-3-iodophenyl)piperidine-1-carboxylate

4-(2-Chloro-3-iodophenyl)piperidine hydrochloride (19.8 mg, 0.06 mmol) and TEA (32 µl, 0.23 mmol) were suspended in DCM (0.2 ml), 1-[2-(trimethylsilyl)ethoxycarbonyloxy]pyrrolidin-2,5-dione (14.9 mg, 0.06 mmol) was added, and the mixture was stirred at room temperature for 1 hour. Distilled water (5 ml) was added to the reaction solution, and the mixture was extracted with DCM (5 ml). The organic layer was dried over anhydrous sodium sulfate, filtered, and concentrated under reduced pressure. The obtained residue was subjected to MPLC (0-10% EtOAc/hexane) to obtain 22.6 mg (85%) of brown oil.

### Step 6: Synthesis of 2-(trimethylsilyl)ethyl 4-(3-((5-(4-(((tert-butoxycarbonyl)amino)methyl)-4-methylpiperidin-1-yl)pyrazin-2-yl)thio)-2-chlorophenyl)piperidine-1-carboxylate

2-(Trimethylsilyl)ethyl 4-(2-chloro-3-iodophenyl)piperidine-1-carboxylate (22.6 mg, 0.05 mmol), sodium 5-(4-(((tert-butoxycarbonyl)amino)methyl)-4-methylpiperidin-1-yl)pyrazine-2-thiolate (crude, 0.05 mmol), Pd₂(dba)₃ (2.2 mg, 0.002 mmol), Xantphos (2.8 mg, 0.04 mmol), and DIPEA (17 µl, 0.10 mmol) were suspended in 1,4-dioxane (0.11 ml) and stirred at 90 °C for 1 hour. Distilled water (5 ml) was added to the reaction solution, and the mixture was extracted with EtOAc (5 ml). The organic layer was dried over anhydrous sodium sulfate, filtered, and concentrated under reduced pressure. The obtained residue was subjected to MPLC (0-30% EtOAc/hexane) to give 16.5 mg (50%) of an ivory solid.

### Step 7: Synthesis of tert-butyl ((1-(5-((2-chloro-3-(piperidin-4-yl)phenyl)thio)pyrazin-2-yl)-4-methylpiperidin-4-yl)methyl)carbamate

2-(Trimethylsilyl)ethyl 4-(3-((5-(4-(((tert-butoxycarbonyl)amino)methyl)-4-methylpiperidin-1-yl)pyrazin-2-yl)thio)-2-chlorophenyl)piperidine-1-carboxylate (16.5 mg, 0.02 mmol) was suspended in THF (0.05 ml), TBAF (1.0 M in THF, 30 µl, 0.03 mmol) was added, and the mixture was stirred at 50 °C for 1 hour. Distilled water (5 ml) was added to the reaction solution, and then extracted with DCM (5 ml). The organic layer was dried over anhydrous sodium sulfate, filtered and concentrated under reduced pressure to obtain 16 mg (quant.) of an ivory solid.

### Intermediate 56. tert-butyl ((1-(5-((3-chloro-2-(piperidin-4-yl)pyridin-4-yl)thio)pyrazin-2-yl)-4-methylpiperidin-4-yl)methyl)carbamate

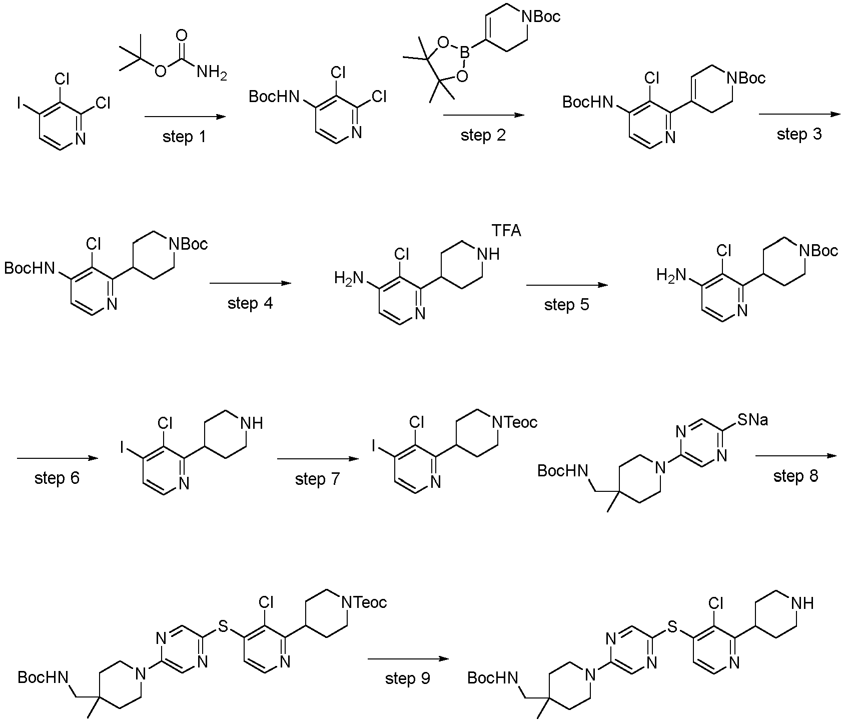

### Step 1: Synthesis of tert-butyl (2,3-dichloropyridin-4-yl)carbamate

2,3-Dichloro-4-iodopyridine (500 mg, 1.83 mmol), tert-butyl carbamate (257 mg, 2.19 mmol), Pd₂(dba)₃ (84 mg, 0.09 mmol), Xantphos (106 mg, 0.18 mmol), and Cs₂CO₃ (1.19 g, 3.65 mmol) were suspended in toluene (8 ml) and stirred at 90 °C for 16 hours. Distilled water (30 ml) was added to the reaction solution and extracted with EtOAc (30 ml). The organic layer was washed with brine (30 ml), dried over anhydrous sodium sulfate, filtered, and concentrated under reduced pressure. The obtained residue was subjected to MPLC (0-5% EtOAc/hexane) to give 380 mg (79%) of an ivory solid.

### Step 2: Synthesis of tert-butyl 4-(3-amino-2-chlorophenyl)-3,6-dihydropyridine-1(2H)-carboxylate

tert-Butyl 4-((tert-butoxycarbonyl)amino)-3-chloro-3',6'-dihydro-[2,4'-bipyridine]-1'(2'H)-carboxylate (356 mg, 1.35 mmol), tert-butyl 4-(4,4,5,5-tetramethyl-1,3,2-dioxaborolan-2-yl)-3,6-dihydropyridine-1(2H)-carboxylate (920 mg, 2.98 mmol), Pd(dppf)₂ (220 mg, 0.27 mmol), and Cs₂CO₃ (882 mg, 2.71 mmol) were suspended in a mixed solvent of 1,4-dioxane : H₂O (4:1, 5.6 ml) and reacted in a microwave reactor at 130°C for 1.5 hours. The reaction solution was filtered with celite, and the filtrate was concentrated under reduced pressure. The obtained residue was subjected to MPLC (0-30% EtOAc/hexane) to obtain 369 mg (67%) of brown gum.

### Step 3: Synthesis of tert-butyl 4-(4-((tert-butoxycarbonyl)amino)-3-chloropyridin-2-yl)piperidine-1-carboxylate

tert-Butyl 4-(3-amino-2-chlorophenyl)-3,6-dihydropyridine-1(2H)-carboxylate (369 mg, 0.90 mmol) was suspended in MeOH (3.6 ml), platinum(IV) oxide (21 mg) was added, and the mixture was stirred at room temperature for 2 hours under a stream of hydrogen. The reaction solution was filtered through celite, and the filtrate was concentrated under reduced pressure. The obtained residue was subjected to MPLC (0-10% EtOAc/hexane) to give 204 mg (55%) of a white solid.

### Step 4: Synthesis of 4-(2-chloro-3-iodophenyl)piperidine trifluoroacetic acid

tert-Butyl 4-(4-((tert-butoxycarbonyl)amino)-3-chloropyridin-2-yl)piperidine-1-carboxylate (204 mg, 0.50 mmol) was suspended in DCM (4.34 ml), TFA (1.86 ml) was added, and the mixture was stirred at room temperature for 3 hours. The reaction solution was concentrated under reduced pressure to obtain 415 mg (quant.) of brown oil.

### Step 5: Synthesis of tert-butyl 4-(4-amino-3-chloropyridin-2-yl)piperidine-1-carboxylate

4-(2-Chloro-3-iodophenyl)piperidine trifluoroacetic acid (415 mg, 0.50 mmol) and TEA (0.28 ml, 1.98 mmol) were suspended in DCM (1.8 ml), di-tert-butyl dicarbonate (113 mg, 0.52 mmol) was added, and the mixture was stirred at room temperature for 1 hour. Distilled water (10 ml) was added to the reaction solution, and the mixture was extracted with DCM (10 ml). The organic layer was dried over anhydrous sodium sulfate, filtered, and concentrated under reduced pressure. The obtained residue was subjected to MPLC (0-40% EtOAc/hexane) to obtain 147 mg (95%) of a white solid.

### Step 6: Synthesis of 3-chloro-4-iodo-2-(piperidin-4-yl)pyridine

tert-Butyl 4-(4-amino-3-chloropyridin-2-yl)piperidine-1-carboxylate (60 mg, 0.19 mmol) was suspended in 50% sulfuric acid (aq.) (0.96 ml), sodium nitrite (14.6 mg, 0.21 mmol) and H₂O (0.1 ml) were added at 0 °C, and the mixture was stirred at 0 °C for 30 minutes. KI (160 mg, 0.96 mmol) was added to the reaction solution, and the mixture was stirred at room temperature for 30 minutes. The reaction solution was adjusted to pH 8 with sat. NaHCO₃ (aq.), and extracted with DCM (10 x 2 ml). The organic layer was dried over anhydrous sodium sulfate, filtered, and concentrated under reduced pressure. The obtained residue was subjected to MPLC (amine-silica, 0-3% MeOH/DCM) to give 29.8 mg (48%) of an ivory solid.

### Step 7: Synthesis of 2-(trimethylsilyl)ethyl 4-(3-chloro-4-iodopyridin-2-yl)piperidine-1-carboxylate

3-Chloro-4-iodo-2-(piperidin-4-yl)pyridine (33.5 mg, 0.10 mmol) and TEA (43 µl, 0.31 mmol) were suspended in DCM (0.35 ml), 1-[2-(trimethylsilyl)ethoxycarbonyloxy]pyrrolidin-2,5-dione (28.3 mg, 0.11 mmol) was added, and the mixture was stirred at room temperature for 1 hour. Distilled water (5 ml) was added to the reaction mixture, and the mixture was extracted with DCM (5 ml). The organic layer was dried over anhydrous sodium sulfate, filtered, and concentrated under reduced pressure. The obtained residue was subjected to MPLC (0-15% EtOAc/hexane) to obtain 34.9 mg (72%) of a white solid.

### Step 8: Synthesis of 2-(trimethylsilyl)ethyl 4-(4-((5-(4-(((tert-butoxycarbonyl)amino)methyl)-4-methylpiperidin-1-yl)pyrazin-2-yl)thio)-3-chloropyridin-2-yl)piperidine-1-carboxylate

2-(Trimethylsilyl)ethyl 4-(3-chloro-4-iodopyridin-2-yl)piperidine-1-carboxylate (34.9 mg, 0.07 mmol), sodium 5-(4-(((tert-butoxycarbonyl)amino)methyl)-4-methylpiperidin-1-yl)pyrazine-2-thiolate (crude, 0.09 mmol), Pd₂(dba)₃ (3.4 mg, 0.004 mmol), Xantphos (4.3 mg, 0.07 mmol), and DIPEA (26 µl, 0.15 mmol) were suspended in 1,4-dioxane (0.25 ml) and stirred at 90 °C for 1 hour. Distilled water (5 ml) was added to the reaction solution, and the mixture was extracted with EtOAc (5 ml). The organic layer was dried over anhydrous sodium sulfate, filtered, and concentrated under reduced pressure. The obtained residue was subjected to MPLC (0-40% EtOAc/hexane) to give 43 mg (83%) of an ivory solid.

### Step 9: Synthesis of tert-butyl ((1-(5-((3-chloro-2-(piperidin-4-yl)pyridin-4-yl)thio)pyrazin-2-yl)-4-methylpiperidin-4-yl)methyl)carbamate

2-(Trimethylsilyl)ethyl 4-(4-((5-(4-(((tert-butoxycarbonyl)amino)methyl)-4-methylpiperidin-1-yl)pyrazin-2-yl)thio)-3-chloropyridin-2-yl)piperidine-1-carboxylate (42 mg, 0.06 mmol) was suspended in THF (0.41 ml), TBAF (1.0 M in THF, 0.19 ml, 0.19 mmol) was added, and the mixture was stirred at 50 °C for 2 hours. Distilled water (5 ml) was added to the reaction solution, and then extracted with DCM (5 ml). The organic layer was dried over anhydrous sodium sulfate, filtered and concentrated under reduced pressure. The obtained residue was subjected to MPLC (amine-silica, 0-3% MeOH/DCM) to give 32 mg (97%) of an ivory solid.

### Intermediate 57. 1-(3-((5-(4-(((tert-butoxycarbonyl)amino)methyl)-4-methylpiperidin-1-yl)pyrazin-2-yl)thio)-2-chlorophenyl)piperidine-4-carboxylic acid

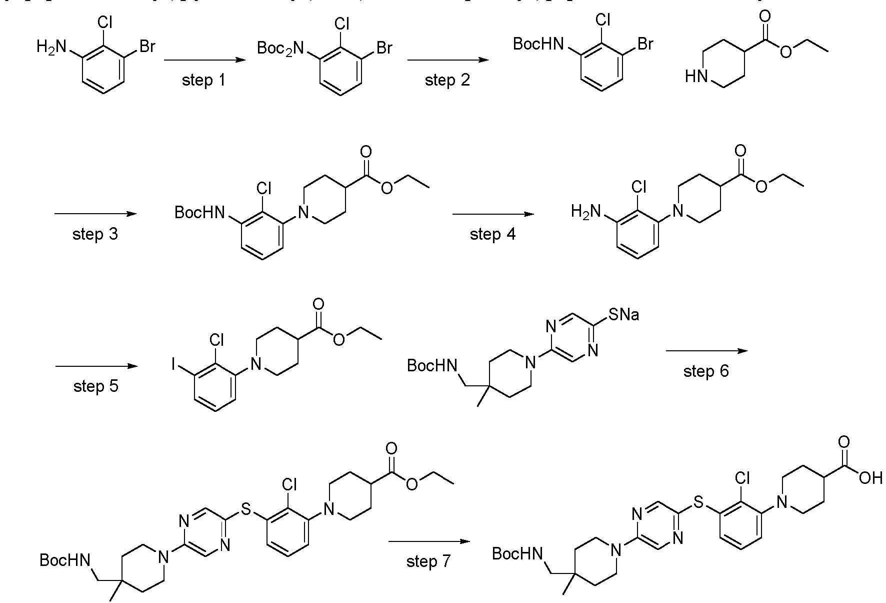

### Step 1: Synthesis of tert-butyl (3-bromo-2-chlorophenyl)(tert-butoxycarbonyl)carbamate

3-Bromo-2-chloroaniline (300 mg, 1.45 mmol) and DMAP (18 mg, 0.14 mmol) were suspended in THF (2.6 ml), di-tert-butyl dicarbonate (951 mg, 4.36 mmol) was added, and the mixture was heated and refluxed for 1 hour. Distilled water (20 ml) was added to the reaction mixture, and the mixture was extracted with EtOAc (20 ml). The organic layer was dried over anhydrous sodium sulfate, filtered, and concentrated under reduced pressure. The obtained residue was subjected to MPLC (0-15% EtOAc/hexane) to obtain 582 mg (98%) of a white solid.

### Step 2: Synthesis of tert-butyl (3-bromo-2-chlorophenyl)carbamate

tert-Butyl (3-bromo-2-chlorophenyl)(tert-butoxycarbonyl)carbamate (550 mg, 1.35 mmol) and K₂CO₃ (561 mg, 4.06 mmol) were suspended in MeOH (2.4 ml) and heated to reflux for 2 hours. Distilled water (20 ml) was added to the reaction solution, and it was extracted with EtOAc (20 ml). The organic layer was dried over anhydrous sodium sulfate, filtered, and concentrated under reduced pressure. The obtained residue was subjected to MPLC (0-5% EtOAc/hexane) to give 582 mg (75%) of an ivory solid.

### Step 3: Synthesis of ethyl 1-(3-((tert-butoxycarbonyl)amino)-2-chlorophenyl)piperidine-4-carboxylate

tert-Butyl (3-bromo-2-chlorophenyl)carbamate (243 mg, 0.79 mmol), ethyl piperidine-4-carboxylate (249 mg, 1.59 mmol), Pd(OAc)₂ (8.9 mg, 0.04 mmol), BINAP (49 mg, 0.08 mmol), and Cs₂CO₃ (516 mg, 1.59 mmol) were suspended in 1,4-dioxane (2.6 ml) and stirred at 90 °C for 16 hours. Distilled water (20 ml) was added to the reaction solution, and the mixture was extracted with EtOAc (20 ml). The organic layer was dried over anhydrous sodium sulfate, filtered, and concentrated under reduced pressure. The obtained residue was subjected to MPLC (0-10% EtOAc/hexane) to give 254 mg (84%) of an ivory solid.

### Step 4: Synthesis of ethyl 1-(3-amino-2-chlorophenyl)piperidine-4-carboxylate

Ethyl 1-(3-((tert-butoxycarbonyl)amino)-2-chlorophenyl)piperidine-4-carboxylate (254 mg, 0.66 mmol) was suspended in DCM (6.6 ml), TFA (1.65 ml) was added, and the mixture was stirred at room temperature for 1 hour. The reaction solution was adjusted to pH 8 with sat. NaHCO₃ (aq.), and extracted with DCM (10 x 2 ml). The organic layer was dried over anhydrous sodium sulfate, filtered, and concentrated under reduced pressure to yield 185 mg (99%) of an ivory solid.

### Step 5: Synthesis of ethyl 1-(2-chloro-3-iodophenyl)piperidine-4-carboxylate

Ethyl 1-(3-amino-2-chlorophenyl)piperidine-4-carboxylate (100 mg, 0.35 mmol) was suspended in a mixed solvent of 2 M HCl (aq.) : dioxane (2:1, 2.29 ml), and sodium nitrite (26.8 mg, 0.39 mmol) and H₂O (0.08 ml) were added at 0 °C, and the mixture was stirred at 0 °C for 15 min. NaI (58 mg, 0.39 mmol) was added to the reaction solution, and the mixture was stirred at room temperature for 30 min. Distilled water (5 ml) was added to the reaction solution, and the mixture was extracted with EtOAc (5 ml). The organic layer was dried over anhydrous sodium sulfate, filtered, and concentrated under reduced pressure. The obtained residue was subjected to MPLC (0-5% EtOAc/hexane) to give 86 mg (62%) of brown oil.

### Step 6: Synthesis of ethyl 1-(3-((5-(4-(((tert-butoxycarbonyl)amino)methyl)-4-methylpiperidin-1-yl)pyrazin-2-yl)thio)-2-chlorophenyl)piperidine-4-carboxylate

Ethyl 1-(2-chloro-3-iodophenyl)piperidine-4-carboxylate (86 mg, 0.22 mmol), sodium 5-(4-(((tert-butoxycarbonyl)amino)methyl)-4-methylpiperidin-1-yl)pyrazine-2-thiolate (crude, 0.22 mmol), Pd₂(dba)₃ (10 mg, 0.01 mmol), Xantphos (12.6 mg, 0.02 mmol), and DIPEA (76 µl, 0.44 mmol) were suspended in 1,4-dioxane (0.5 ml), and the mixture was stirred at 90 °C for 1 hour. Distilled water (5 ml) was added to the reaction solution, and extracted with EtOAc (5 ml). The organic layer was dried over anhydrous sodium sulfate, filtered, and concentrated under reduced pressure. The obtained residue was subjected to MPLC (0-30% EtOAc/hexane) to obtain 88 mg (67%) of an ivory solid.

### Step 7: Synthesis of 1-(3-((5-(4-(((tert-butoxycarbonyl)amino)methyl)-4-methylpiperidin-1-yl)pyrazin-2-yl)thio)-2-chlorophenyl)piperidine-4-carboxylic acid

Ethyl 1-(3-((5-(4-(((tert-butoxycarbonyl)amino)methyl)-4-methylpiperidin-1-yl)pyrazin-2-yl)thio)-2-chlorophenyl)piperidine-4-carboxylate (88 mg, 0.15 mmol) was suspended in a mixed solvent of THF: MeOH: H₂O (3:2:1, 1 ml), LiOH·H₂O (12 mg, 0.29 mmol) was added, and the mixture was stirred at room temperature for 2 hours. The reaction solution was adjusted to pH 2 with 1 M HCl (aq.) and extracted with EA (5 ml). The organic layer was dried over anhydrous sodium sulfate, filtered and concentrated under reduced pressure to give 84 mg (100%) of an ivory solid.

Intermediates 58 to 60 were synthesized using the same method as that used for synthesizing Intermediate 57.

| | |
|---|---|
| Intermediate 58. 1-(3-((5-(4-(((tert-butoxycarbonyl)amino)methyl)-4-methylpiperidin-1-yl)-6-(hydroxymethyl)pyrazin-2-yl)thio)-2-chlorophenyl)piperidine-4-carboxylic acid | 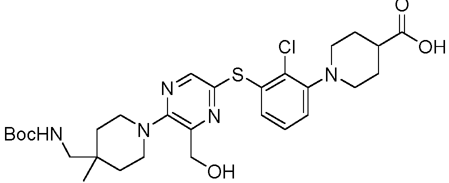 |
| Intermediate 59. 1-(3-((5-(4-((tert-butoxycarbonyl)amino)-4-methylpiperidin-1-yl)-6-(hydroxymethyl)pyrazin-2-yl)thio)-2-chlorophenyl)piperidine-4-carboxylic acid | 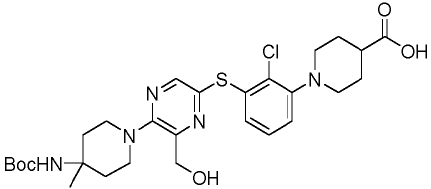 |
| Intermediate 60. 1-(3-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-2-chlorophenyl)piperidine-4-carboxylic acid | 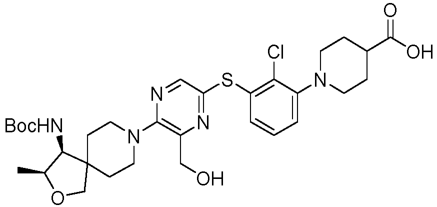 |

### Intermediate 61. (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide

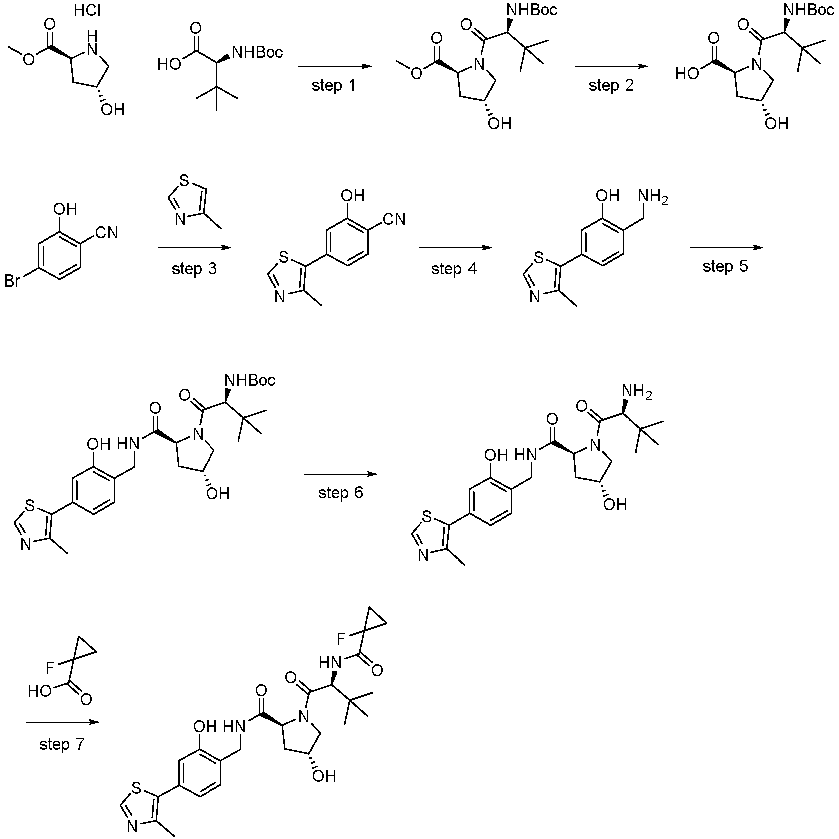

### Step 1: Synthesis of (2S,4R)-methyl 1-((S)-2-((tert-butoxycarbonyl)amino)-3,3-dimethylbutanoyl)-4-hydroxypyrrolidine-2-carboxylate

(2S,4R)-Methyl 4-hydroxypyrrolidine-2-carboxylate hydrochloride (7.85 g, 43.23 mmol), (S)-2-((tert-butoxycarbonyl)amino)-3,3-dimethylbutanoic acid (10 g, 43.23 mmol), EDCI (16.57 g, 86.46 mmol), HOBt (7 g, 86.46 mmol), and DIPEA (45 ml, 259.38 mmol) were suspended in DMF (216 ml) and stirred at room temperature for 16 hours. The reaction solution was washed with sat. NH₄Cl (aq.) (300 ml) and sat. NaHCO₃ (aq.) (300 ml) and extracted with EtOAc (150 ml x 2). The organic layer was washed with brine (150 ml x 2), dried over anhydrous sodium sulfate, filtered and concentrated under reduced pressure. The obtained residue was subjected to MPLC (0-5% MeOH/DCM) to give 11 g (71%) of an ivory solid.

### Step 2: Synthesis of (2S,4R)-1-((S)-2-((tert-butoxycarbonyl)amino)-3,3-dimethylbutanoyl)-4-hydroxypyrrolidine-2-carboxylic acid

(2S,4R)-Methyl 1-((S)-2-((tert-butoxycarbonyl)amino)-3,3-dimethylbutanoyl)-4-hydroxypyrrolidine-2-carboxylate (781 mg, 2.18 mmol) and LiOH (366 mg, 8.72 mmol) were suspended in a mixed solvent of H₂O:THF (1: 1, 10 ml) and stirred at room temperature for 3 hours. The reaction solution was adjusted to pH 1-2 with 1 M HCl (aq.) and extracted with EtOAc (15 ml x 2). The organic layer was dried over anhydrous sodium sulfate, filtered and concentrated under reduced pressure to obtain 680 mg (91%) of a white solid.

### Step 3: Synthesis of 2-hydroxy-4-(4-methylthiazol-5-yl)benzonitrile

4-Bromo-2-hydroxybenzonitrile (2 g, 10.15 mmol) and 4-methylthiazole (1.83 ml, 20.20 mmol) were suspended in DMAC (40 ml), KOAc (1.98 g, 20.20 mmol) and Pd(OAc)₂ (45 mg, 0.20 mmol) were added, and the mixture was heated and refluxed for 16 hours. Distilled water (100 ml) was added to the reaction solution, and the mixture was extracted with EtOAc (50 ml x 2). The organic layer was washed with brine (50 ml x 2), dried over anhydrous magnesium sulfate, filtered, and concentrated under reduced pressure. The obtained residue was subjected to MPLC (0-50% EtOAc/hexane) to give 980 mg (45%) of an ivory solid.

### Step 4: Synthesis of 2-(aminomethyl)-5-(4-methylthiazol-5-yl)phenol

2-Hydroxy-4-(4-methylthiazol-5-yl)benzonitrile (864 mg, 3.99 mmol) was suspended in THF (22 ml), LAH (2.0 M in THF, 8 ml, 15.98 mmol) was slowly added at 0 °C, and the mixture was heated and refluxed for 3 hours. After lowering the temperature of the reaction solution to 0 °C, distilled water (0.5 ml) was slowly added, neutralized with Rochelle salt solution (20 ml), and filtered through a celite filter. The filtrate was subjected to MPLC (DCM: MeOH: NH4OH = 15:1:0.1) to obtain 416 mg (47%) of a yellow solid.

### Step 5: Synthesis of tert-butyl ((S)-1-((2S,4R)-4-hydroxy-2-((2-hydroxy-4-(4-methylthiazol-5-yl)benzyl)carbamoyl)pyrrolidin-1-yl)-3,3-dimethyl-1-oxobutan-2-yl)carbamate

2-(Aminomethyl)-5-(4-methylthiazol-5-yl)phenol (416 mg, 1.89 mmol), (2S,4R)-1-((S)-2-((tert-butoxycarbonyl)amino)-3,3-dimethylbutanoyl)-4-hydroxypyrrolidine-2-carboxylic acid (650 mg, 1.89 mmol), HATU (789 mg, 2.08 mmol), and DIPEA (0.98 ml, 5.66 mmol) were suspended in DMF (6 ml) and stirred at room temperature for 16 hours. Distilled water (25 ml) was added to the reaction solution, and then extracted with EtOAc (15 ml x 2). The organic layer was washed with brine (15 ml x 2), dried over anhydrous sodium sulfate, filtered, and concentrated under reduced pressure. The obtained residue was subjected to MPLC (0-20% MeOH/DCM) to give 898 mg (87%) of a pale yellow solid.

### Step 6: Synthesis of (2S,4R)-1-((S)-2-amino-3,3-dimethylbutanoyl)-4-hydroxy-N-(2-hydroxy-4-(4-methylthiazol-5-yl)benzyl)pyrrolidine-2-carboxamide

tert-Butyl ((S)-1-((2S,4R)-4-hydroxy-2-((2-hydroxy-4-(4-methylthiazol-5-yl)benzyl)carbamoyl)pyrrolidin-1-yl)-3,3-dimethyl-1-oxobutan-2-yl)carbamate (885 mg, 1.62 mmol) was suspended in DCM (30 ml), 4 M HCl in dioxane (8 ml) was added, and the mixture was stirred at room temperature for 2 hours. The reaction solution was neutralized by adding sat. NaHCO₃ (aq.) (20 ml), and then extracted with DCM (10 ml x 2). The organic layer was washed with brine (10 ml x 2), dried over anhydrous magnesium sulfate, filtered and concentrated under reduced pressure. The obtained residue was subjected to MPLC (amine-silica, 0-10% MeOH/DCM) to give 522 mg (72%) of a yellow solid.

### Step 7: Synthesis of (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide

(2S,4R)-1-((S)-2-Amino-3,3-dimethylbutanoyl)-4-hydroxy-N-(2-hydroxy-4-(4-methylthiazol-5-yl)benzyl)pyrrolidine-2-carboxamide (399 mg, 0.89 mmol), 1-fluorocyclopropane-1-carboxylic acid (92.6 mg, 0.89 mmol), HATU (381 mg, 0.89 mmol), and DIPEA (0.8 ml, 4.47 mmol) were suspended in DMF (4 ml) and stirred at room temperature for 16 hours. Distilled water (10 ml) was added to the reaction solution, and EtOAc (11 ml x 2) was extracted. The organic layer was washed with brine (10 ml x 2), dried over anhydrous sodium sulfate, filtered, and concentrated under reduced pressure. The obtained residue was subjected to MPLC (0-10% MeOH/DCM) to give 377 mg (79%) of a white solid.

Intermediates 62 to 64 were synthesized using the same method as that used for synthesizing Intermediate 61.

| | |
|---|---|
| Intermediate 62. (2S,4R)-1-((S)-2-acetamido-3,3-dimethylbutanoyl)-4-hydroxy-N-(2-hydroxy-4-(4-methylthiazol-5-yl)benzyl)pyrrolidine-2-carboxamide | 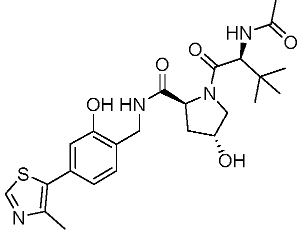 |
| Intermediate 63. (2S,4R)-1-((S)-2-(1-fluorocyclopropane-1-carboxamido)-3,3-dimethylbutanoyl)-4-hydroxy-N-((S)-1-(2-hydroxy-4-(4-methylthiazol-5-yl)phenyl)ethyl)pyrrolidine-2-carboxamide | 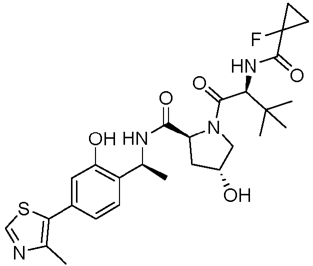 |
| Intermediate 64. (2S,4R)-1-((S)-2-acetamido-3,3-dimethylbutanoyl)-4-hydroxy-N-((8)-1-(2-hydroxy-4-(4-methylthiazol-5-yl)phenyl)ethyl)pyrrolidine-2-carboxamide | 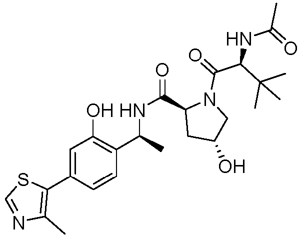 |

### Intermediate 65. (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid

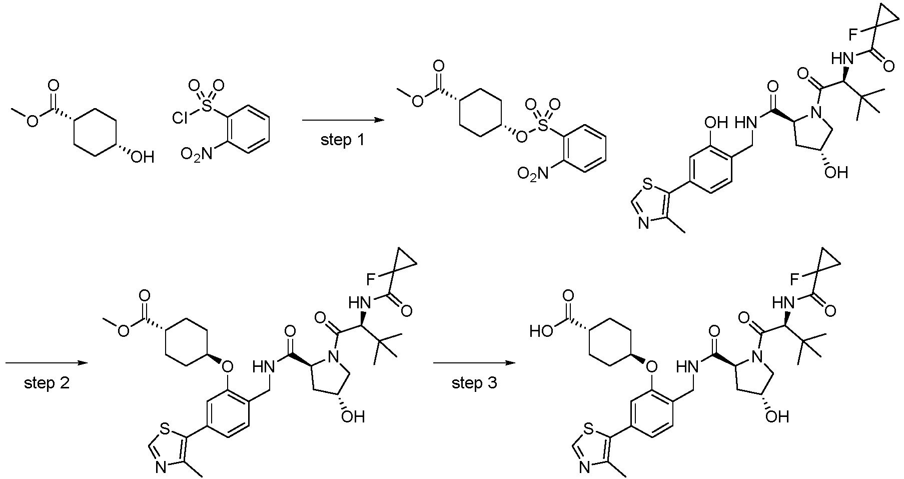

### Step 1: Synthesis of (1S,4S)-methyl 4-(((2-nitrophenyl)sulfonyl)oxy)cyclohexane-1-carboxylate

Methyl (1S,4S)-4-hydroxycyclohexane-1-carboxylate (4 g, 25.29 mmol), TEA (7 ml, 50.57 mmol), and DMAP (309 mg, 2.52 mmol) were suspended in DCM (120 ml). 2-nitrobenzenesulfonyl chloride (22.4 g, 101.14 mmol) was dissolved in DCM (25 ml), and this solution was slowly added to the above suspension at 0 °C and stirred at room temperature for 2 hours. Distilled water (200 ml) was added to the reaction solution, and extracted with DCM (50 ml). The organic layer was dried over anhydrous sodium sulfate, filtered, and concentrated under reduced pressure. The obtained residue was subjected to MPLC (0-20% EtOAc/hexane) to obtain 7.14 g (82%) of colorless oil.

### Step 2: Synthesis of (1S,4R)-methyl 4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylate

(1S,4S)-Methyl 4-(((2-nitrophenyl)sulfonyl)oxy)cyclohexane-1-carboxylate (3.9 g, 11.26 mmol), (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 61, 1.5 g, 2.82 mmol), and Cs₂CO₃ (1.8 g, 5.63 mmol) were suspended in DMF (60 ml) and stirred at 50 °C for 16 hours. Distilled water (100 ml) was added to the reaction solution, and extracted with EtOAc (100 ml x 2). The organic layer was washed with brine (100 ml), dried over anhydrous sodium sulfate, filtered and concentrated under reduced pressure. The obtained residue was subjected to MPLC (amine-silica, 0-5% MeOH/DCM) to give 1.3 g (70%) of an ivory solid.

### Step 3: Synthesis of (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid

(1S,4R)-Methyl 4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexanecarboxylate (1.8 g, 2.75 mmol) and LiOH (231 mg, 5.51 mmol) were suspended in a mixed solvent of THF: H₂O: MeOH (4:1:1, 12 ml) and stirred at room temperature for 4 hours. The reaction solution was adjusted to pH 1-2 with 1 M HCl (aq.) and extracted with EtOAc (20 ml x 2). The organic layer was dried over anhydrous sodium sulfate, filtered, and concentrated under reduced pressure. The obtained residue was subjected to MPLC (0-20% MeOH/DCM) to give 896 mg (49%) of an ivory solid.

Intermediates 66 and 67 were synthesized using the same method as that used for synthesizing Intermediate 65.

| | |
|---|---|
| Intermediate 66. (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid | 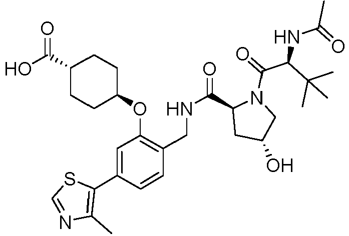 |
| Intermediate 67. (1S,4R)-4-(2-((S)-1-((2S,4R)-1-((S)-2-acetamido-3,3-dimethylbutanoyl)-4-hydroxypyrrolidine-2-carboxamido)ethyl)-5-(4-methylthiazol-5-yl)phenoxy)cyclohexane-1-carboxylic acid | 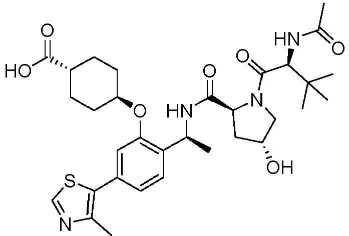 |

### Intermediate 68. (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

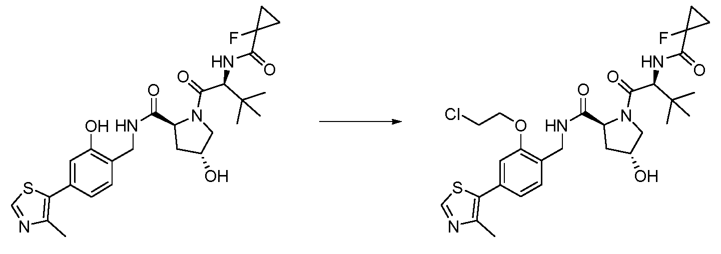

(2S,4R)-1-((S)-2-(1-Fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 61, 350 mg, 0.657 mmol) and 1-bromo-2-chloroethane (0.2 ml, 2.628 mmol) were suspended in DMF (2 ml), and K₂CO₃ (272 mg, 1.971 mmol) and KI (218 mg, 1.314 mmol) were added, and the mixture was stirred at 70 °C for 16 hours. Distilled water (20 ml) was added to the reaction solution, and then extracted with EtOAc (20 ml x 2). The organic layer was washed with brine (20 ml x 2), dried over anhydrous magnesium sulfate, filtered and concentrated under reduced pressure. The obtained residue was subjected to MPLC (0-5% MeOH/DCM) to give 234 mg (60%) of an ivory solid.

Intermediate 69 was synthesized using the same method as that used for synthesizing Intermediate 68.

| | |
|---|---|
| Intermediate 69. (28,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide | 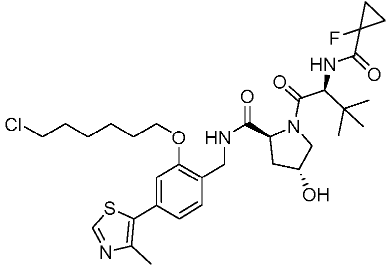 |

### Intermediate 70. 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylic acid

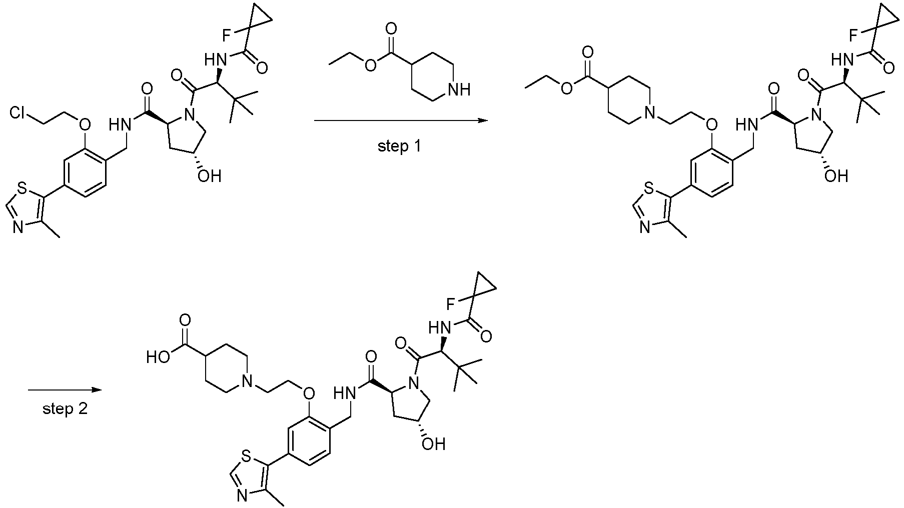

### Step 1: Synthesis of ethyl 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylate

(2S,4R)-N-(2-(2-Chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 68, 100 mg, 0.15 mmol), ethyl piperidine-4-carboxylate (17.6 mg, 0.11 mmol), and K₂CO₃ (46 mg, 0.37 mmol) were suspended in DMF (0.15 ml) and stirred at 70 °C for 16 hours. Distilled water (10 ml) was added to the reaction solution, and then extracted with EtOAc (10 ml). The organic layer was washed with brine (10 ml), dried over anhydrous sodium sulfate, filtered, and concentrated under reduced pressure. The obtained residue was subjected to MPLC (0-5% MeOH/DCM) to give 67 mg (84%) of an ivory solid.

### Step 2: Synthesis of 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylic acid

Ethyl 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylate (67 mg, 0.09 mmol) and LiOH (7.9 mg, 0.19 mmol) were suspended in a mixed solvent of THF: MeOH: H₂O (3:2:1, 0.26 ml) and stirred at room temperature for 2 hours. The reaction solution was adjusted to pH 2 with 1 M HCl (aq.) and concentrated under reduced pressure to obtain 87 mg (crude) of an ivory solid.

### Intermediate 71. (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide

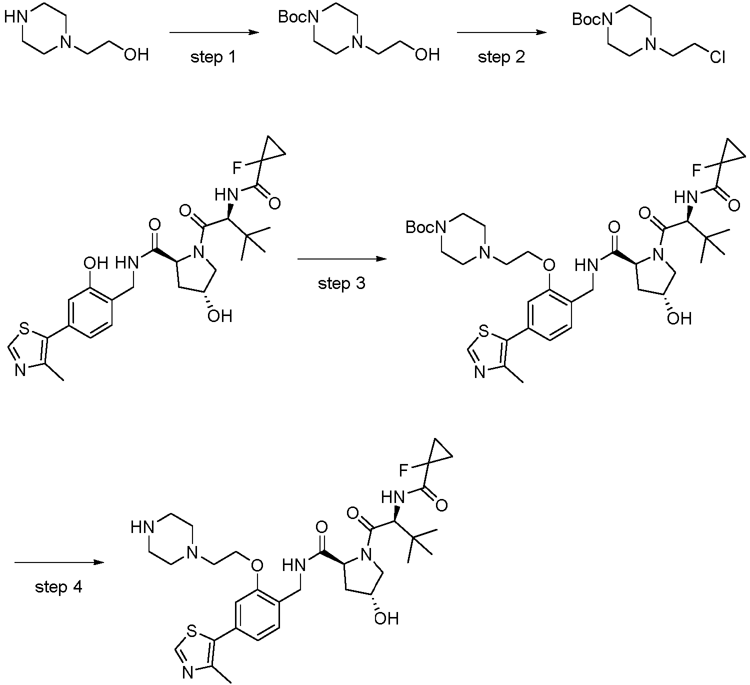

### Step 1: Synthesis of tert-butyl 4-(2-hydroxyethyl)piperazine-1-carboxylate

1-(2-Hydroxyethyl)piperazine (2.8 ml, 23.04 mmol) was suspended in THF (23 ml), Boc₂O (5.8 ml, 25.35 mmol) was added at 0 °C, and the mixture was stirred at room temperature for 1 hour. The reaction solution was neutralized by adding sat. NH₄Cl (aq.) (50 ml), extracted with DCM (50 ml), and the organic layer was dried over anhydrous magnesium sulfate, filtered, and concentrated under reduced pressure to obtain 5.6 g (quant.) of colorless oil.

### Step 2: Synthesis of tert-butyl 4-(2-chloroethyl)piperazine-1-carboxylate

tert-Butyl 4-(2-hydroxyethyl)piperazine-1-carboxylate (5.6 g, 24.66 mmol), TEA (6.8 ml, 49.33 mmol), and DMAP (120 mg, 0.99 mmol) were suspended in DCM (123 ml), and 2-nitrobenzenesulfonyl chloride (5.4 g, 24.66 mmol) dissolved in DCM (25 ml) was slowly added at 0 °C, and the mixture was stirred at room temperature for 2 hours. Distilled water (200 ml) was added to the reaction solution, and extracted with DCM (50 ml). The organic layer was dried over anhydrous sodium sulfate, filtered, and concentrated under reduced pressure. The obtained residue was subjected to MPLC (0-50% EtOAc/hexane) to give 2.9 g (49%) of a yellow solid.

### Step 3: Synthesis of tert-butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1-carboxylate

tert-Butyl 4-(2-chloroethyl)piperazine-1-carboxylate (1.4 g, 5.91 mmol), (2S,4R)-1-((S)-2-(1-fluorocyclopropane-1-carboxamido)-3,3-dimethylbutanoyl)-4-hydroxy-N-(2-hydroxy-4-(4-methylthiazol-5-yl)benzyl)pyrrolidine-2-carboxamide (Intermediate 61, 1.1 g, 1.97 mmol), and Cs₂CO₃ (1.3 g, 3.94 mmol) were suspended in DMF (44 ml) and stirred at 50 °C for 16 hours. Distilled water (100 ml) was added to the reaction solution, and extracted with EtOAc (100 ml x 2). The organic layer was washed with brine (100 ml), dried over anhydrous sodium sulfate, filtered and concentrated under reduced pressure. The obtained residue was subjected to MPLC (amine-silica, 0-5% MeOH/DCM) to give 833 mg (57%) of an ivory solid.

### Step 4: Synthesis of (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide

tert-Butyl 4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazine-1-carboxylate (819 mg, 1.10 mmol) was suspended in DCM (9 ml), 4 M HCl in dioxane (2 ml) was added, and the mixture was stirred at room temperature for 1 hour. The reaction solution was neutralized by adding sat. NaHCO₃ (aq.) (30 ml), extracted with DCM (3 ml x 2), and the organic layer was dried over anhydrous magnesium sulfate, filtered, and concentrated under reduced pressure. The obtained residue was subjected to MPLC (amine-silica, 0-5% MeOH/DCM) to give 739 mg (quant.) of an ivory solid.

Intermediates 72 to 79 were synthesized using the same method as that used for synthesizing Intermediate 71. In the case of Intermediates 72 to 79, O-nosylate was obtained as a result through Step 2.

| | |
|---|---|
| Intermediate 72. (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide | 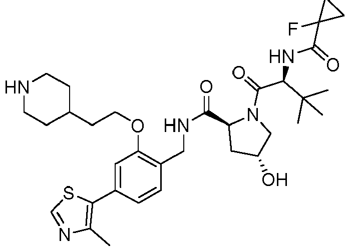 |
| Intermediate 73. (28,4R)-N-(2-(7-azaspiro[3.5]nonan-2-yloxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide | 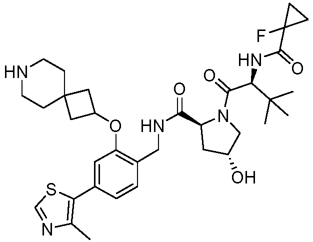 |
| Intermediate 74. (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-ylmethoxy)benzyl)pyrrolidine-2-carboxamide | 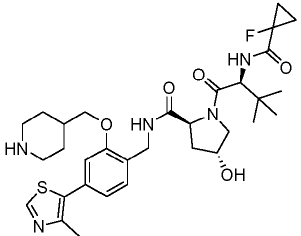 |
| Intermediate 75. (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide | 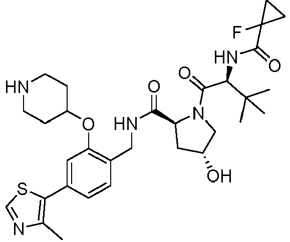 |
| Intermediate 76. (2S,4R)-N-(2-(azetidin-3-yloxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide | 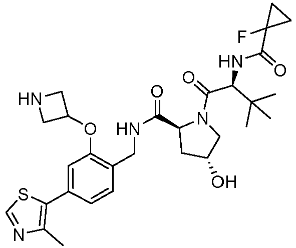 |
| Intermediate 77. (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide | 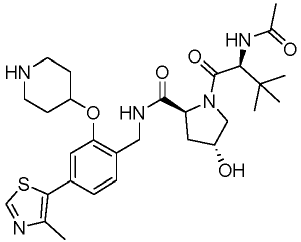 |
| Intermediate 78. (2S,4R)-1-((S)-2-(1-fluorocyclopropane-1-carboxamido)-3,3-dimethylbutanoyl)-4-hydroxy-N-((S)-1-(4-(4-methylthiazol-5-yl)-2-(piperidin-4-yloxy)phenyl)ethyl)pyrrolidine-2-carboxamide | 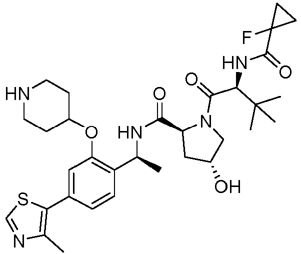 |
| Intermediate 79. (2S,4R)-1-((S)-2-acetamido-3,3-dimethylbutanoyl)-4-hydroxy-N-((S)-1-(4-(4-methylthiazol-5-yl)-2-(piperidin-4-yloxy)phenyl)ethyl)pyrrolidine-2-carboxamide | 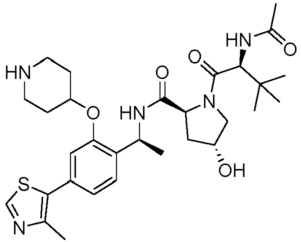 |

### Intermediate 80. (2S,4R)-1-((S)-2-(2-cyanoacetamido)-3,3-dimethylbutanoyl)-4-hydroxy-N-(4-(4-methylthiazol-5-yl)-2-(piperidin-4-yloxy)benzyl)pyrrolidine-2-carboxamide

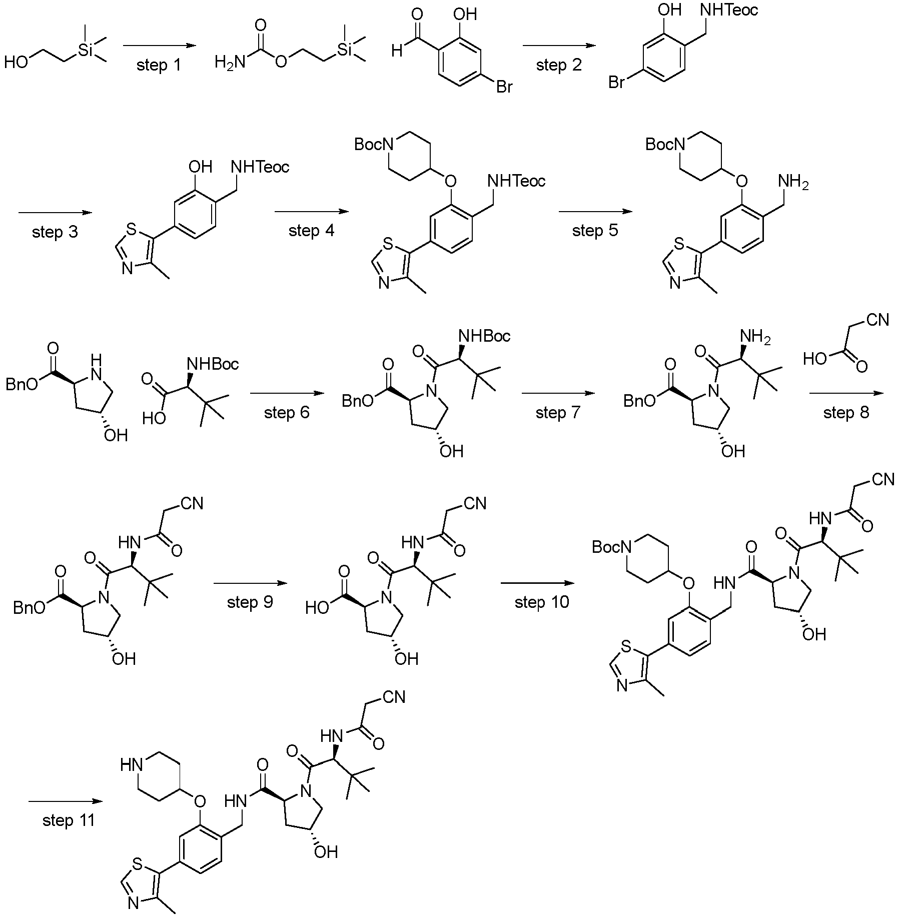

### Step 1: Synthesis of 2-(trimethylsilyl)ethyl carbamate

2-(Trimethylsilyl)ethanol (3 g, 25.4 mmol) was suspended in toluene (42 ml), 1,1-carbonyldiimidazole (8.23 g, 30.48 mmol) was added, and the mixture was stirred at room temperature for 5 hours. Ammonium hydroxide (28% in H₂O, 8.3 mL) was added to the reaction solution, and the mixture was stirred at room temperature for 16 hours. Distilled water (50 ml) was added to the reaction solution, and extracted with EtOAc (50 ml). The organic layer was washed with 1 M HCl (aq.) (30 ml) and sat. NH₄Cl (aq.) (30 ml) and dried over anhydrous magnesium sulfate. The residue was filtered and concentrated under reduced pressure to obtain 4.17 g (100%) of a white solid.

### Step 2: Synthesis of 2-(trimethylsilyl)ethyl 4-bromo-2-hydroxybenzylcarbamate

4-Bromohydroxy benzaldehyde (1.27 g, 6.35 mmol), 2-(trimethylsilyl)ethyl carbamate (4.17 g, 25.4 mmol), and triethylsilane (4.05 ml, 25.4 mmol) were suspended in ACN (30 ml), TFA (3.79 ml, 50.8 mmol) was slowly added, and the mixture was stirred at room temperature for 16 hours. After adding sat. NaHCO₃ (aq.) (50 ml) to the reaction solution, it was extracted with DCM (50 ml), and the organic layer was dried over anhydrous sodium sulfate, filtered, and concentrated under reduced pressure. The obtained residue was subjected to MPLC (0-15% EtOAc/hexane) to give 1.79 g (83%) of a white solid.

### Step 3: Synthesis of 2-(trimethylsilyl)ethyl 2-hydroxy-4-(4-methylthiazol-5-yl)benzylcarbamate

2-(Trimethylsilyl)ethyl 4-bromo-2-hydroxybenzylcarbamate (1.79 g, 5.17 mmol) and 4-methylthiazole (0.94 ml, 10.34 mmol) were suspended in DMAC (7.3 ml), KOAc (1.02 g, 10.34 mmol) and Pd(OAc)₂ (58 mg, 0.26 mmol) were added, and the mixture was heated and refluxed for 16 hours. Distilled water (50 ml) was added to the reaction mixture, and extracted with EtOAc (50 ml x 2). The organic layer was washed with brine (50 ml x 2), dried over anhydrous magnesium sulfate, filtered, and concentrated under reduced pressure. The obtained residue was subjected to MPLC (0-40% EtOAc/hexane) to give 546 mg (30%) of an ivory solid.

### Step 4: Synthesis of tert-butyl 4-(5-(4-methylthiazol-5-yl)-2-((((2-(trimethylsilyl)ethoxy)carbonyl)amino)methyl)phenoxy)piperidine-1-carboxylate

2-(Trimethylsilyl)ethyl 2-hydroxy-4-(4-methylthiazol-5-yl)benzylcarbamate (700 mg, 1.92 mmol), tert-butyl 4-(((2-nitrophenyl)sulfonyl)oxy)piperidine-1-carboxylate (2.23 g, 5.76 mmol), and Cs₂CO₃ (1.36 g, 3.84 mmol) were suspended in DMF (4 ml) and stirred at 50 °C for 16 hours. Distilled water (50 ml) was added to the reaction solution, and extracted with EtOAc (50 ml x 2). The organic layer was washed with brine (50 ml), dried over anhydrous sodium sulfate, filtered, and concentrated under reduced pressure. The obtained residue was subjected to MPLC (0-30% EtOAc/hexane) to give 927 g (88%) of a white solid.

### Step 5: Synthesis of tert-butyl 4-(2-(aminomethyl)-5-(4-methylthiazol-5-yl)phenoxy)piperidine-1-carboxylate

tert-Butyl 4-(5-(4-methylthiazol-5-yl)-2-((((2-(trimethylsilyl)ethoxy)carbonyl)amino)methyl)phenoxy)piperidine-1-carboxylate (150 mg, 0.27 mmol) and TBAF (1.0 M in THF, 0.8 ml, 0.82 mmol) were suspended in THF (2.7 ml) and stirred at 50 °C for 2 hours. Distilled water (10 ml) was added to the reaction mixture, and the mixture was extracted with DCM (10 ml). The organic layer was dried over anhydrous sodium sulfate. The resulting mixture was filtered and concentrated under reduced pressure to obtain 110 mg (crude) of an ivory solid.

### Step 6: Synthesis of (2S,4R)-benzyl 1-((S)-2-((tert-butoxycarbonyl)amino)-3,3-dimethylbutanoyl)-4-hydroxypyrrolidine-2-carboxylate

(2S,4R)-Benzyl 4-hydroxypyrrolidine-2-carboxylate (10 g, 38.8 mmol), (S)-2-((tert-butoxycarbonyl)amino)-3,3-dimethylbutanoic acid (8.97 g, 38.8 mmol), EDCI (8.93 g, 45.56 mmol), HOBt (6.29 g, 45.56 mmol), and DIPEA (25 ml, 194 mmol) were suspended in DCM (60 ml) and stirred at room temperature for 16 hours. The reaction solution was washed with distilled water (100 ml), extracted with DCM (100 ml), dried over anhydrous sodium sulfate, filtered, and concentrated under reduced pressure. The obtained residue was subjected to MPLC (0-50% EtOAc/hexane) to give 9.42 g (56%) of an ivory solid.

### Step 7: Synthesis of (2S,4R)-benzyl 1-((S)-2-amino-3,3-dimethylbutanoyl)-4-hydroxypyrrolidine-2-carboxylate

(2S,4R)-Benzyl 1-((S)-2-((tert-butoxycarbonyl)amino)-3,3-dimethylbutanoyl)-4-hydroxypyrrolidine-2-carboxylate (4.83 g, 11.1 mmol) was suspended in DCM (80 ml), 4 M HCl in dioxane (22 ml) was added, and the mixture was stirred at room temperature for 1 hour. The reaction solution was concentrated under reduced pressure to obtain 3.7 g (crude) of a white solid.

### Step 8: Synthesis of (2S,4R)-benzyl 1-((S)-2-(2-cyanoacetamido)-3,3-dimethylbutanoyl)-4-hydroxypyrrolidine-2-carboxylate

(2S,4R)-Benzyl 1-((S)-2-amino-3,3-dimethylbutanoyl)-4-hydroxypyrrolidine-2-carboxylate (3.7 g, 11.1 mmol), 2-cyanoacetic acid (58 mg, 0.69 mmol), EDCI (159 mg, 0.83 mmol), HOBt (112 mg, 0.83 mmol), and DIPEA (0.6 ml, 3.45 mmol) were suspended in DCM (1.4 ml) and stirred at room temperature for 16 hours. The reaction solution was washed with distilled water (100 ml), extracted with DCM (100 ml), dried over anhydrous sodium sulfate, filtered, and concentrated under reduced pressure. The obtained residue was subjected to MPLC (0-10% MeOH/DCM) to give 112 mg (41%) of a white solid.

### Step 9: Synthesis of (2S,4R)-1-((S)-2-(2-cyanoacetamido)-3,3-dimethylbutanoyl)-4-hydroxypyrrolidine-2-carboxylic acid

(2S,4R)-Benzyl 1-((S)-2-(2-cyanoacetamido)-3,3-dimethylbutanoyl)-4-hydroxypyrrolidine-2-carboxylate (112 mg, 0.28 mmol) and LiOH (23 mg, 0.56 mmol) were suspended in a mixed solvent of H₂O:THF:MeOH (1:1:1, 0.52 ml) and stirred at room temperature for 2 hours. The reaction solution was adjusted to pH 1-2 with 1 M HCl (aq.) and concentrated under reduced pressure to obtain 151 mg (crude) of an ivory solid.

### Step 10: Synthesis of tert-butyl 4-(2-(((2S,4R)-1-((S)-2-(2-cyanoacetamido)-3,3-dimethylbutanoyl)-4-hydroxypyrrolidine-2-carboxamido)methyl)-5-(4-methylthiazol-5-yl)phenoxy)piperidine-1-carboxylate

tert-Butyl 4-(2-(aminomethyl)-5-(4-methylthiazol-5-yl)phenoxy)piperidine-1-carboxylate (140 mg, 0.28 mmol), (2S,4R)-1-((S)-2-(2-cyanoacetamido)-3,3-dimethylbutanoyl)-4-hydroxypyrrolidine-2-carboxylic acid (151 mg, 0.28 mmol), EDCI (64 mg, 0.34 mmol), HOBt (45 g, 0.34 mmol), and DIPEA (0.24 ml, 1.40 mmol) were suspended in DMF (0.2 ml), DCM (0.56 ml), and stirred at room temperature for 16 hours. The reaction solution was washed with distilled water (10 ml), extracted with DCM (10 ml), dried over anhydrous sodium sulfate, filtered, and concentrated under reduced pressure. The obtained residue was subjected to MPLC (0-5% MeOH/DCM) to give 79 mg (41%) of an ivory solid.

### Step 11: Synthesis of (2S,4R)-1-((S)-2-(2-cyanoacetamido)-3,3-dimethylbutanoyl)-4-hydroxy-N-(4-(4-methylthiazol-5-yl)-2-(piperidin-4-yloxy)benzyl)pyrrolidine-2-carboxamide

tert-Butyl 4-(2-(((2S,4R)-1-((S)-2-(2-cyanoacetamido)-3,3-dimethylbutanoyl)-4-hydroxypyrrolidine-2-carboxamido)methyl)-5-(4-methylthiazol-5-yl)phenoxy)piperidine-1-carboxylate (79 mg, 0.11 mmol) was suspended in DCM (1.1 ml), 4 M HCl in dioxane (0.29 ml) was added, and the mixture was stirred at room temperature for 1 hour. The reaction solution was concentrated under reduced pressure to obtain 68 mg (crude) of an ivory solid.

Intermediates 81 to 83 were synthesized using the same method as that used for synthesizing Intermediate 80.

| | |
|---|---|
| Intermediate 81. (2S,4R)-4-hydroxy-1-((S)-2-(2-hydroxyacetamido)-3,3-dimethylbutanoyl)-N-(4-(4-methylthiazol-5-yl)-2-(piperidin-4-yloxy)benzyl)pyrrolidine-2-carboxamide | 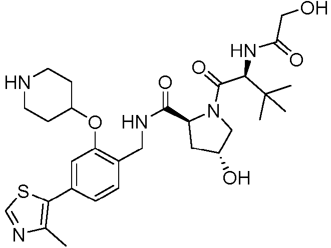 |
| Intermediate 82. (2S,4R)-1-((S)-2-acetamido-3-methylbutanoyl)-4-hydroxy-N-(4-(4-methylthiazol-5-yl)-2-(piperidin-4-yloxy)benzyl)pyrrolidine-2-carboxamide | 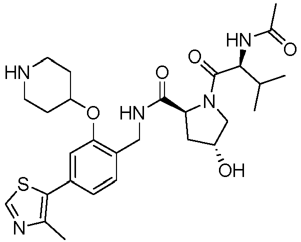 |
| Intermediate 83. (2S,4R)-1-((S)-2-acetamido-2-cyclopropylacetyl)-4-hydroxy-N-(4-(4-methylthiazol-5-yl)-2-(piperidin-4-yloxy)benzyl)pyrrolidine-2-carboxamide | 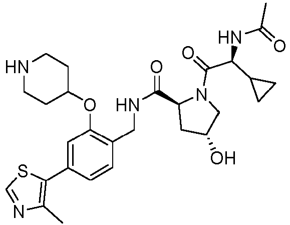 |

### Intermediate 84. (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P1)

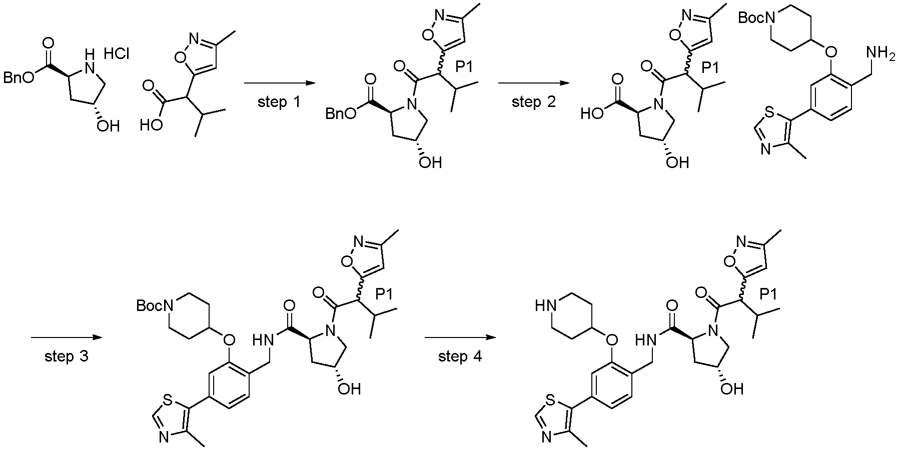

### Step 1: Synthesis of benzyl (2S,4R)-4-hydroxy-1-(3-methyl-2-(3-methylisoxazol-5-yl)butanoyl)pyrrolidine-2-carboxylate (P1)

3-Methyl-2-(3-methylisoxazol-5-yl)butanoic acid (4.50 g, 24.60 mmol) and benzyl (2S,4R)-4-hydroxypyrrolidine-2-carboxylate hydrochloride (6.96 g, 27.00 mmol) were suspended in DMF (27 ml), and HATU (9.34 g, 24.60 mmol) and DIPEA (4.28 ml, 24.60 mmol) were added at 0 °C, and the mixture was stirred at room temperature for 12 hours. 1 M HCl (aq.) was added to the reaction solution, and the mixture was extracted with EtOAc (200 ml x 2). The organic layer was washed with sat. NaHCO₃ (aq.) (200 ml) and concentrated under reduced pressure. The obtained residue was subjected to MPLC (0-100% EtOAc/Petroleum ether) to obtain a mixture. The obtained mixture was purified by SFC (column: REGIS (S,S) WHELK-O1 (250 mm x 25 mm x 10 um); mobile phase: [CO₂-EtOH (0.1% NH₄OH)]; B%: 25%, isocratic elution mode) to obtain 1.50 g (16%, 100%ee) of yellow solid P1 and 2.00 g (20%, 98.8%ee) of its isomer P2.

### Step 2: Synthesis of (2S,4R)-4-hydroxy-1-(3-methyl-2-(3-methylisoxazol-5-yl)butanoyl)pyrrolidine-2-carboxylic acid (P1)

Benzyl (2S,4R)-4-hydroxy-1-(3-methyl-2-(3-methylisoxazol-5-yl)butanoyl)pyrrolidine-2-carboxylate (P1) (1.52 g, 3.88 mmol) was suspended in MeOH (15 ml), Pd/C (150 mg, 10% purity) was added, and the mixture was stirred at room temperature for 2 hours under a stream of hydrogen (15 psi). The reaction solution was filtered through celite, and the filtrate was concentrated under reduced pressure to obtain 995 mg (65%) of a pale yellow solid.

### Step 3: Synthesis of tert-butyl 4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)piperidine-1-carboxylate (P1)

tert-Butyl 4-(2-(aminomethyl)-5-(4-methylthiazol-5-yl)phenoxy)piperidine-1-carboxylate (357 mg, 0.88 mmol), (2S,4R)-4-hydroxy-1-(3-methyl-2-(3-methylisoxazol-5-yl)butanoyl)pyrrolidine-2-carboxylic acid (P1) (262 mg, 0.88 mmol), EDCI (203 mg, 1.06 mmol), HOBt (143 mg, 1.06 mmol), and DIPEA (0.46 ml, 2.65 mmol) were suspended in DCM (2.2 ml) and stirred at room temperature for 16 hours. The reaction solution was washed with distilled water (50 ml), extracted with DCM (50 ml), dried over anhydrous sodium sulfate, filtered, and concentrated under reduced pressure. The obtained residue was subjected to MPLC (0-5% MeOH/DCM) to give 488 mg (81%) of an ivory solid.

### Step 4: Synthesis of (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P1)

tert-Butyl 4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)piperidine-1-carboxylate (P1) (488 mg, 0.72 mmol) was suspended in DCM (7.2 ml), 4 M HCl in dioxane (1.8 ml) was added, and the mixture was stirred at room temperature for 1 hour. The reaction mixture was neutralized by adding sat. NaHCO₃ (aq.) (20 ml), extracted with DCM (20 ml x 2), dried over anhydrous magnesium sulfate, filtered, and concentrated under reduced pressure. The obtained residue was subjected to MPLC (amine-silica, 0-5% MeOH/DCM) to give 375 mg (90%) of a yellow solid.

Intermediate 85 was synthesized using the same method as that used for synthesizing Intermediate 84.

| | |
|---|---|
| Intermediate 85. (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) | 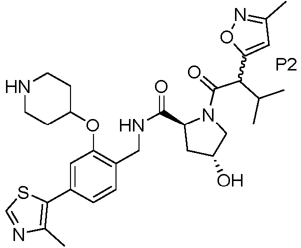 |

### Intermediate 86. (2S,4R)-4-hydroxy-1-(3-methyl-2-(4-methyl-1H-pyrazol-1-yl)butanoyl)-N-(4-(4-methylthiazol-5-yl)-2-(piperidin-4-yloxy)benzyl)pyrrolidine-2-carboxamide (P1)

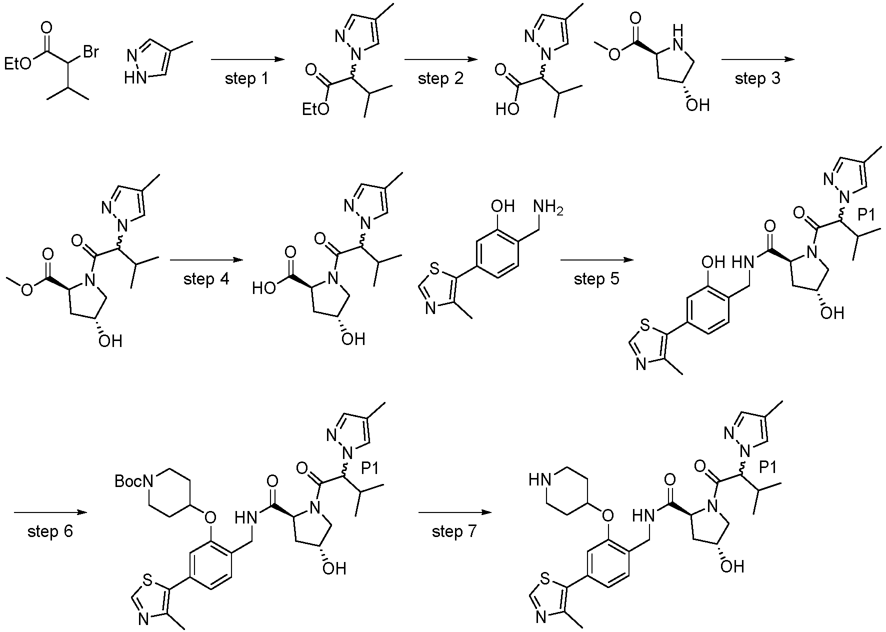

### Step 1: Synthesis of ethyl 3-methyl-2-(4-methyl-1H-pyrazol-1-yl)butanoate

Ethyl 2-bromo-3-methylbutanoate (500 mg, 2.39 mmol) and 4-methylpyrazole (255 mg, 3.11 mmol) were suspended in THF (4 ml), and NaH (60% dispersion in mineral oil, 124 mg, 3.11 mmol) was slowly added at 0 °C and stirred for 48 hours. Distilled water (30 ml) was added to the reaction solution, and extracted with EtOAc (30 ml x 2). Afterwards, the mixture was dried over anhydrous sodium sulfate, filtered, and concentrated under reduced pressure. The obtained residue was subjected to MPLC (0-20% EtOAc/hexane) to give 311 mg (62%) of colorless oil.

### Step 2: Synthesis of 3-Methyl-2-(4-methyl-1H-pyrazol-1-yl)butanoic acid

Ethyl 3-methyl-2-(4-methyl-1H-pyrazol-1-yl)butanoate (295 mg, 1.40 mmol) and LiOH (88 mg, 2.10 mmol) were suspended in a mixed solvent of H₂O:THF (1:1, 6 ml) and stirred at room temperature for 4 hours. The reaction solution was adjusted to pH 1-2 with 1 M HCl (aq.) and extracted with EtOAc (15 ml x 2). The organic layer was dried over anhydrous sodium sulfate, filtered and concentrated under reduced pressure to give 278 mg (crude) of colorless oil.

### Step 3: Synthesis of (2S,4R)-methyl 4-hydroxy-1-(3-methyl-2-(4-methyl-1H-pyrazol-1-yl)butanoyl)pyrrolidine-2-carboxylate

3-Methyl-2-(4-methyl-1H-pyrazol-1-yl)butanoic acid (105 mg, 0.57 mmol), (2S,4R)-methyl 4-hydroxypyrrolidine-2-carboxylate (83 g, 0.57 mmol), EDCI (132 mg, 0.69 mmol), HOBt (93 mg, 0.69 mmol), and DIPEA (0.5 ml, 2.88 mmol) were suspended in DCM (2 ml) and stirred at room temperature for 16 hours. The reaction solution was washed with distilled water (15 ml) and extracted with DCM (15 ml). Afterwards, the solution was dried over anhydrous sodium sulfate, filtered, and concentrated under reduced pressure. The obtained residue was subjected to MPLC (0-5% MeOH/DCM) to give 71 mg (40%) of colorless oil.

### Step 4: Synthesis of (2S,4R)-4-hydroxy-1-(3-methyl-2-(4-methyl-1H-pyrazol-1-yl)butanoyl)pyrrolidine-2-carboxylic acid

(2S,4R)-Methyl 4-hydroxy-1-(3-methyl-2-(4-methyl-1H-pyrazol-1-yl)butanoyl)pyrrolidine-2-carboxylate (94 mg, 0.30 mmol) and 2 M NaOH (aq.) (0.2 ml) were suspended in THF (1 ml) and stirred at 50 °C for 2 hours. The reaction solution was adjusted to pH 1-2 with 1 M HCl (aq.) and extracted with EtOAc (10 ml x 2). The organic layer was dried over anhydrous sodium sulfate, filtered and concentrated under reduced pressure to give 21 mg (crude) of colorless oil.

### Step 5: Synthesis of (2S,4R)-4-hydroxy-N-(2-hydroxy-4-(4-methylthiazol-5-yl)benzyl)-1-(3-methyl-2-(4-methyl-1H-pyrazol-1-yl)butanoyl)pyrrolidine-2-carboxamide (P1)

2-(Aminomethyl)-5-(4-methylthiazol-5-yl)phenol (16 mg, 0.07 mmol), (2S,4R)-4-hydroxy-1-(3-methyl-2-(4-methyl-1H-pyrazol-1-yl)butanoyl)pyrrolidine-2-carboxylic acid (21 g, 0.07 mmol), EDCI (16 mg, 0.08 mmol), HOBt (11 mg, 0.08 mmol), and DIPEA (0.06 ml, 0.36 mmol) were suspended in DCM (0.2 ml) and stirred at room temperature for 16 hours. The reaction solution was washed with distilled water (10 ml) and extracted with DCM (10 ml). Then, the solution was dried over anhydrous sodium sulfate, filtered, and concentrated under reduced pressure. The obtained residue was subjected to MPLC (0-5% MeOH/DCM) to give 14 mg (40%) of white solid P1 and 9 mg (25%) of its isomer P2.

### Step 6: Synthesis of tert-butyl 4-(2-(((2S,4R)-4-hydroxy-1-(3-methyl-2-(4-methyl-1H-pyrazol-1-yl)butanoyl)pyrrolidine-2-carboxamido)methyl)-5-(4-methylthiazol-5-yl)phenoxy)piperidine-1-carboxylate (P1)

(2S,4R)-4-Hydroxy-N-(2-hydroxy-4-(4-methylthiazol-5-yl)benzyl)-1-(3-methyl-2-(4-methyl-1H-pyrazol-1-yl)butanoyl)pyrrolidine-2-carboxamide (P1) (54 mg, 0.11 mmol), tert-butyl 4-(((2-nitrophenyl)sulfonyl)oxy)piperidine-1-carboxylate (127 mg, 0.33 mmol), and Cs₂CO₃ (72 mg, 0.22 mmol) were suspended in DMF (1.1 ml) and stirred at 50 °C for 16 hours. Distilled water (10 ml) was added to the reaction solution, and extracted with EtOAc (10 ml x 2). The organic layer was washed with brine (10 ml), dried over anhydrous sodium sulfate, filtered and concentrated under reduced pressure. The obtained residue was subjected to MPLC (0-10% MeOH/DCM) to give 62 mg (80%) of an ivory solid.

### Step 7: Synthesis of (2S,4R)-4-hydroxy-1-(3-methyl-2-(4-methyl-1H-pyrazol-1-yl)butanoyl)-N-(4-(4-methylthiazol-5-yl)-2-(piperidin-4-yloxy)benzyl)pyrrolidine-2-carboxamide (P1)

tert-Butyl 4-(2-(((2S,4R)-4-hydroxy-1-(3-methyl-2-(4-methyl-1H-pyrazol-1-yl)butanoyl)pyrrolidine-2-carboxamido)methyl)-5-(4-methylthiazol-5-yl)phenoxy)piperidine-1-carboxylate (P1) (62 mg, 0.09 mmol) was suspended in DCM (0.91 ml), 4 M HCl in dioxane (0.23 ml) was added, and the mixture was stirred at room temperature for 1 hour. The reaction solution was neutralized by adding sat. NaHCO₃ (aq.) (15 ml), and extracted with DCM (15 ml x 2). After drying over anhydrous magnesium sulfate, filtering, and concentrating under reduced pressure, 50 mg (crude) of an ivory solid was obtained.

Intermediate 87 was synthesized using the same method as that used for synthesizing Intermediate 86.

| | |
|---|---|
| Intermediate 87. (2S,4R)-4-hydroxy-1-(3-methyl-2-(4-methyl-1H-pyrazol-1-yl)butanoyl)-N-(4-(4-methylthiazol-5-yl)-2-(piperidin-4-yloxy)benzyl)pyrrolidine-2-carboxamide (P2) | 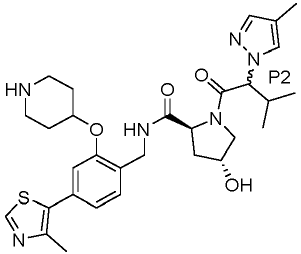 |

### Intermediate 88. (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide

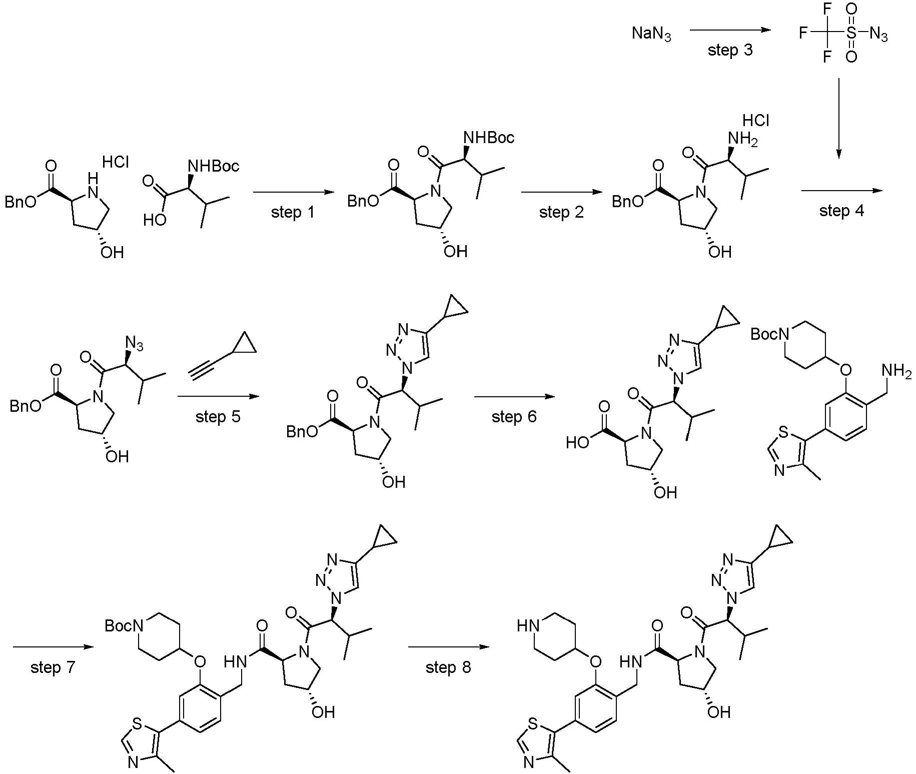

### Step 1: Synthesis of benzyl (2S,4R)-1-((tert-butoxycarbonyl)-L-valyl)-4-hydroxypyrrolidine-2-carboxylate

Benzyl (2S,4R)-4-hydroxypyrrolidine-2-carboxylate hydrochloride (300 mg, 1.16 mmol), (tert-butoxycarbonyl)-L-valine (253 mg, 1.16 mmol), EDCI (268 mg, 1.40 mmol), HOBt (189 mg, 1.40 mmol), and DIPEA (0.8 ml, 4.66 mmol) were suspended in DCM (2.3 ml) and stirred at room temperature for 16 hours. Distilled water (15 ml) was added to the reaction solution, and the mixture was extracted with DCM (15 ml). The organic layer was dried over anhydrous sodium sulfate, filtered, and concentrated under reduced pressure. The obtained residue was subjected to MPLC (0-40% EtOAc/hexane) to give 249 mg (51%) of an ivory solid.

### Step 2: Synthesis of benzyl (2S,4R)-1-(L-valyl)-4-hydroxypyrrolidine-2-carboxylate hydrochloride

Benzyl (2S,4R)-1-((tert-butoxycarbonyl)-L-valyl)-4-hydroxypyrrolidine-2-carboxylate (120 mg, 0.29 mmol) was suspended in DCM (2.8 ml), 4 M HCl in dioxane (0.71 ml) was added, and the mixture was stirred at room temperature for 1 hour. The reaction solution was concentrated under reduced pressure to obtain 114.7 mg (quant.) of a white solid.

### Step 3: Synthesis of trifluoromethanesulfonyl azide

Sodium azide (56 mg, 0.86 mmol) was suspended in pyridine (0.84 ml), trifluoromethanesulfonic anhydride (0.14 ml, 0.86 mmol) was added at 0 °C, and the mixture was stirred at room temperature for 1 hour. The reaction solution was used immediately in the next reaction.

### Step 4: Synthesis of benzyl (2S,4R)-1-((S)-2-azido-3-methylbutanoyl)-4-hydroxypyrrolidine-2-carboxylate

Copper(II) sulfate pentahydrate (10.7 mg, 0.04 mmol) and benzyl (2S,4R)-1-(L-valyl)-4-hydroxypyrrolidine-2-carboxylate hydrochloride (114.7 mg, 0.29 mmol) were suspended in H₂O (0.2 ml), trifluoromethanesulfonyl azide (0.86 mmol) was added, and the mixture was stirred at room temperature for 16 hours. 1 M HCl (aq.) (10 ml) was added to the reaction solution, and then extracted with EtOAc (10 ml). The organic layer was washed with distilled water (10 ml) and brine (10 ml) and dried over anhydrous sodium sulfate. The resultant was filtered and concentrated under reduced pressure to give 101 mg (quant.) of colorless oil.

### Step 5: Synthesis of benzyl (2S,4R)-1-((S)-2-(4-cyclopropyl-1H-1,2,3-triazol-1-yl)-3-methylbutanoyl)-4-hydroxypyrrolidine-2-carboxylate

Benzyl (2S,4R)-1-((S)-2-azido-3-methylbutanoyl)-4-hydroxypyrrolidine-2-carboxylate (101 mg, 0.28 mg), ethynylcyclopropane (36 µl, 0.43 mmol), copper(II) sulfate pentahydrate (93 mg, 0.37 mmol), and sodium L-ascorbate (226 mg, 1.14 mmol) were suspended in a mixed solvent of H₂O: DCM: DMSO (2:1:1, 3 ml), and stirred at room temperature for 2 hours. Distilled water (10 ml) was added to the reaction solution, and then extracted with EtOAc (10 ml). The organic layer was washed with brine (10 ml), dried over anhydrous sodium sulfate, filtered, and concentrated under reduced pressure. The obtained residue was subjected to MPLC (0-3% MeOH/DCM) to give 83.5 mg (71%) of a white solid.

### Step 6: Synthesis of (2S,4R)-1-((S)-2-(4-cyclopropyl-1H-1,2,3-triazol-1-yl)-3-methylbutanoyl)-4-hydroxypyrrolidine-2-carboxylic acid

Benzyl (2S,4R)-1-((S)-2-(4-cyclopropyl-1H-1,2,3-triazol-1-yl)-3-methylbutanoyl)-4-hydroxypyrrolidine-2-carboxylate (83.5 mg, 0.20 mmol) was suspended in MeOH (0.5 ml), Pd/C (2.4 mg, 5% purity) was added, and the mixture was stirred at room temperature for 5 hours under a stream of hydrogen. The reaction solution was filtered through celite, and the filtrate was concentrated under reduced pressure to obtain 71 mg (quant.) of a white solid.

### Step 7: Synthesis of tert-butyl 4-(2-(((2S,4R)-1-((S)-2-(4-cyclopropyl-1H-1,2,3-triazol-1-yl)-3-methylbutanoyl)-4-hydroxypyrrolidine-2-carboxamido)methyl)-5-(4-methylthiazol-5-yl)phenoxy)piperidine-1-carboxylate

tert-Butyl 4-(2-(aminomethyl)-5-(4-methylthiazol-5-yl)phenoxy)piperidine-1-carboxylate (51 mg, 0.10 mmol), (2S,4R)-1-((S)-2-(4-cyclopropyl-1H-1,2,3-triazol-1-yl)-3-methylbutanoyl)-4-hydroxypyrrolidine-2-carboxylic acid (19 mg, 0.10 mmol), EDCI (23 mg, 0.12 mmol), HOBt (16 mg, 0.12 mmol), and DIPEA (0.05 ml, 0.30 mmol) were suspended in DCM (0.3 ml) and stirred at room temperature for 16 hours. The reaction solution was washed with distilled water (10 ml), extracted with DCM (10 ml), dried over anhydrous sodium sulfate, filtered, and concentrated under reduced pressure. The obtained residue was subjected to MPLC (0-5% MeOH/DCM) to give 54 mg (76%) of a white solid.

### Step 8: Synthesis of (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide

tert-Butyl 4-(2-(((2S,4R)-1-((S)-2-(4-cyclopropyl-1H-1,2,3-triazol-1-yl)-3-methylbutanoyl)-4-hydroxypyrrolidine-2-carboxamido)methyl)-5-(4-methylthiazol-5-yl)phenoxy)piperidine-1-carboxylate (54 mg, 0.08 mmol) was suspended in DCM (0.8 ml), 4 M HCl in dioxane (0.19 ml) was added, and the mixture was stirred at room temperature for 1 hour. The reaction solution was neutralized by adding sat. NaHCO₃ (aq.) (10 ml), and extracted with DCM (10 ml x 2). After drying over anhydrous magnesium sulfate, filtering, and concentrating under reduced pressure, 45 mg (97%) of the title of an ivory solid was obtained.

Intermediate 89 was synthesized using the same method as that used for synthesizing Intermediate 88.

| | |
|---|---|
| Intermediate 89. (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)-2-(piperidin-4-yloxy)benzyl)pyrrolidine-2-carboxamide | 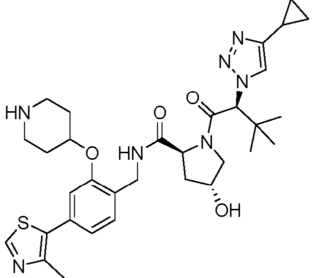 |

### Intermediate 90. (2S,4R)-4-hydroxy-1-((S)-3-methyl-2-(4-methyl-1H-1,2,3-triazol-1-yl)butanoyl)-N-(4-(4-methylthiazol-5-yl)-2-(piperidin-4-yloxy)benzyl)pyrrolidine-2-carboxamide

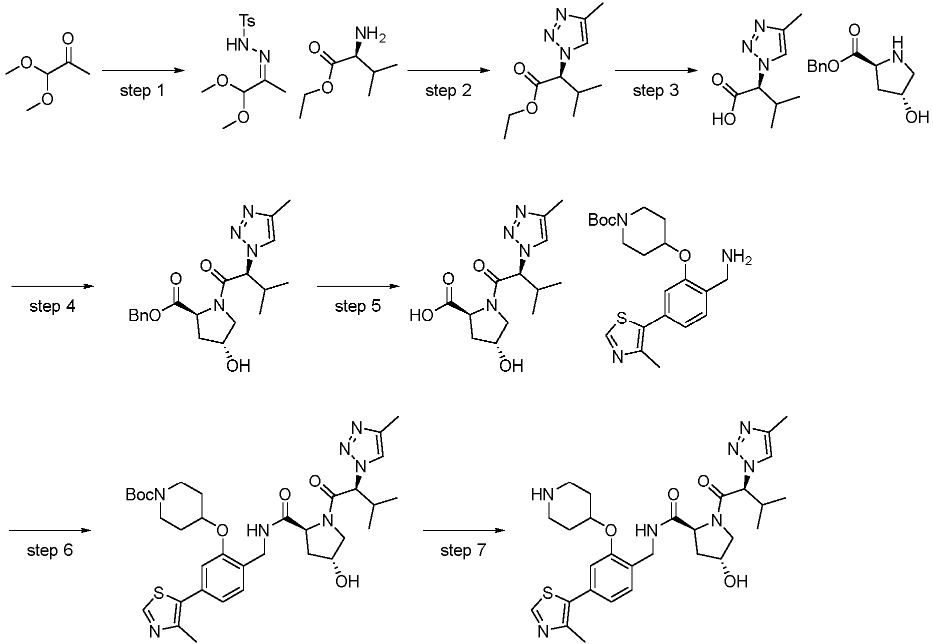

### Step 1: Synthesis of N'-(1,1-dimethoxypropan-2-ylidene)-4-methylbenzenesulfonohydrazide

1,1-Dimethoxypropan-2-one (34 g, 287 mmol) and 4-methylbenzenesulfonohydrazide (48.7 g, 261 mmol) were suspended in MeOH (340 ml), degassing and N₂ purging were repeated three times, and the mixture was stirred at room temperature for 1 hour. The reaction solution was used immediately in the next reaction.

### Step 2: Synthesis of ethyl (S)-3-methyl-2-(4-methyl-1H-1,2,3-triazol-1-yl)butanoate

N'-(1,1-Dimethoxypropan-2-ylidene)-4-methylbenzenesulfonohydrazide (74.92 g, 261 mmol) was suspended in MeOH (340 ml), and ethyl L-valinate hydrochloride (57 g, 313 mmol) and TEA (36.4 ml, 261 mmol) were added. Degassing and N2 purging were repeated three times. Then, the mixture was stirred at 75 °C for 12 hours. The reaction solution was concentrated under reduced pressure, and the residue was subjected to MPLC (2-20% EtOAc/petroleum ether) to obtain 39.20 g (69%) of yellow liquid.

### Step 3: Synthesis of (S)-3-methyl-2-(4-methyl-1H-1,2,3-triazol-1-yl)butanoic acid

Ethyl (S)-3-methyl-2-(4-methyl-1H-1,2,3-triazol-1-yl)butanoate (10 g, 47.3 mmol) was suspended in distilled water (50 ml), conc. HCl (50 ml) was added, and the mixture was stirred at 50 °C for 5 hours. The reaction solution was concentrated under reduced pressure to obtain 12.80 g (crude) of yellow gum.

### Step 4: Synthesis of benzyl (2S,4R)-4-hydroxy-1-((S)-3-methyl-2-(4-methyl-1H-1,2,3-triazol-1-yl)butanoyl)pyrrolidine-2-carboxylate

(S)-3-Methyl-2-(4-methyl-1H-1,2,3-triazol-1-yl)butanoic acid (6.20 g, 27.2 mmol), benzyl (2S,4R)-4-hydroxypyrrolidine-2-carboxylate hydrochloride (7.03 g, 27.2 mmol), and DIPEA (14.2 ml, 81.8 mmol) were suspended in DMF (60 ml), and degassing and N₂ purging were repeated three times. HATU (12.4 g, 32.7 mmol) was added, and the mixture was stirred at room temperature for 2 hours. The reaction solution was diluted with distilled water (150 ml), and extracted with EtOAc (100 ml x 3). The organic layer was washed with distilled water (150 ml x 2), dried over anhydrous sodium sulfate, filtered, and concentrated under reduced pressure. The obtained residue was subjected to MPLC (2-10% EtOAc/Petroleum ether), and the resulting mixture was purified by prep-HPLC (column: Phenomenex luna C18 (250 mm x 70 mm x 10 um); mobile phase: [H₂O (FA)-ACN]; B%: 30%-60%, 20 min) to obtain 1.70 g (16%, 100%ee) of a white solid.

### Step 5: Synthesis of (2S,4R)-4-hydroxy-1-((S)-3-methyl-2-(4-methyl-1H-1,2,3-triazol-1-yl)butanoyl)pyrrolidine-2-carboxylic acid

Benzyl (2S,4R)-4-hydroxy-1-((S)-3-methyl-2-(4-methyl-1H-1,2,3-triazol-1-yl)butanoyl)pyrrolidine-2-carboxylate (1.70 g, 4.40 mmol) was suspended in MeOH (15 ml), Pd/C (170 mg, 10% purity) was added, and the mixture was stirred at room temperature for 3 hours under a stream of hydrogen (15 psi). The reaction solution was filtered through celite, and the filtrate was concentrated under reduced pressure to obtain 1.05 g (81%) of a white solid.

### Step 6: Synthesis of tert-butyl 4-(2-(((2S,4R)-4-hydroxy-1-((S)-3-methyl-2-(4-methyl-1H-1,2,3-triazol-1-yl)butanoyl)pyrrolidine-2-carboxamido)methyl)-5-(4-methylthiazol-5-yl)phenoxy)piperidine-1-carboxylate

tert-Butyl 4-(2-(aminomethyl)-5-(4-methylthiazol-5-yl)phenoxy)piperidine-1-carboxylate (228 mg, 0.57 mmol), (2S,4R)-4-hydroxy-1-((S)-3-methyl-2-(4-methyl-1H-1,2,3-triazol-1-yl)butanoyl)pyrrolidine-2-carboxylic acid (140 mg, 0.47 mmol), EDCI (109 mg, 0.57 mmol), HOBt (77 mg, 0.57 mmol), and DIPEA (0.41 ml, 2.36 mmol) were suspended in DCM (1.6 ml) and stirred at room temperature for 16 hours. The reaction solution was washed with distilled water (15 ml) and extracted with DCM (15 ml). After drying over anhydrous sodium sulfate, filtering and concentrating under reduced pressure, the obtained residue was subjected to MPLC (0-5% MeOH/DCM) to obtain 268 mg (83%) of an ivory solid.

### Step 7: Synthesis of (2S,4R)-4-hydroxy-1-((S)-3-methyl-2-(4-methyl-1H-1,2,3-triazol-1-yl)butanoyl)-N-(4-(4-methylthiazol-5-yl)-2-(piperidin-4-yloxy)benzyl)pyrrolidine-2-carboxamide

tert-butyl 4-(2-(((2S,4R)-4-hydroxy-1-((S)-3-methyl-2-(4-methyl-1H-1,2,3-triazol-1-yl)butanoyl)pyrrolidine-2-carboxamido)methyl)-5-(4-methylthiazol-5-yl)phenoxy)piperidine-1-carboxylate (266 mg, 0.39 mmol) was suspended in DCM (4 ml), 4 M HCl in dioxane (0.98 ml) was added, and the mixture was stirred at room temperature for 1 hour. The reaction solution was neutralized by adding sat. NaHCO₃ (aq.) (20 ml), and extracted with DCM (20 ml x 2). After drying over anhydrous magnesium sulfate, filtering, and concentrating under reduced pressure, 226 mg (crude) of an ivory solid was obtained.

Intermediates 91 to 93 were synthesized using the same method as that used for synthesizing Intermediate 90.

| | |
|---|---|
| Intermediate 91. (2S,4R)-4-hydroxy-1-((S)-3-methyl-2-(4-methyl-1H-1,2,3-triazol-1-yl)butanoyl)-N-(2-(piperidin-4-yloxy)benzyl)pyrrolidine-2-carboxamide | 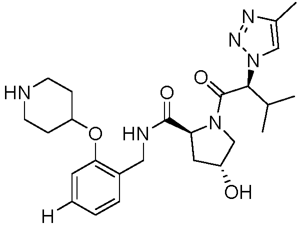 |
| Intermediate 92. (2S,4R)-N-(4-chloro-2-(piperidin-4-yloxy)benzyl)-4-hydroxy-1-((S)-3-methyl-2-(4-methyl-1H-1,2,3-triazol-1-yl)butanoyl)pyrrolidine-2-carboxamide | 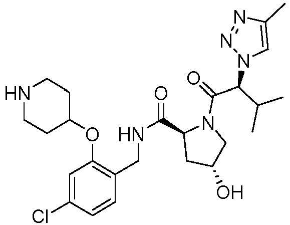 |
| Intermediate 93. (2S, 4R)-N-(4-cuano-2-(piperidin-4-yloxy)benzyl)-4-hydroxy-1-((S)-3-methyl-2-(4-methyl-1H-1,2,3-triazol-1-yl)butanolyl)pyrrolidine-2-carboxamide) | 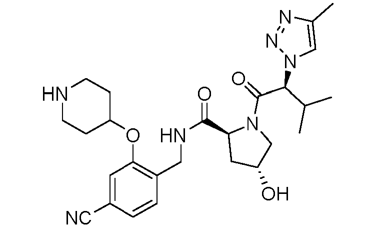 |

### Example 1. (2S,4R)-N-(2-((6-(4-(((3-((5-(4-(aminomethyl)-4-methylpiperidin-1-yl)pyrazin-2-yl)thio)-2-chlorophenyl)amino)methyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

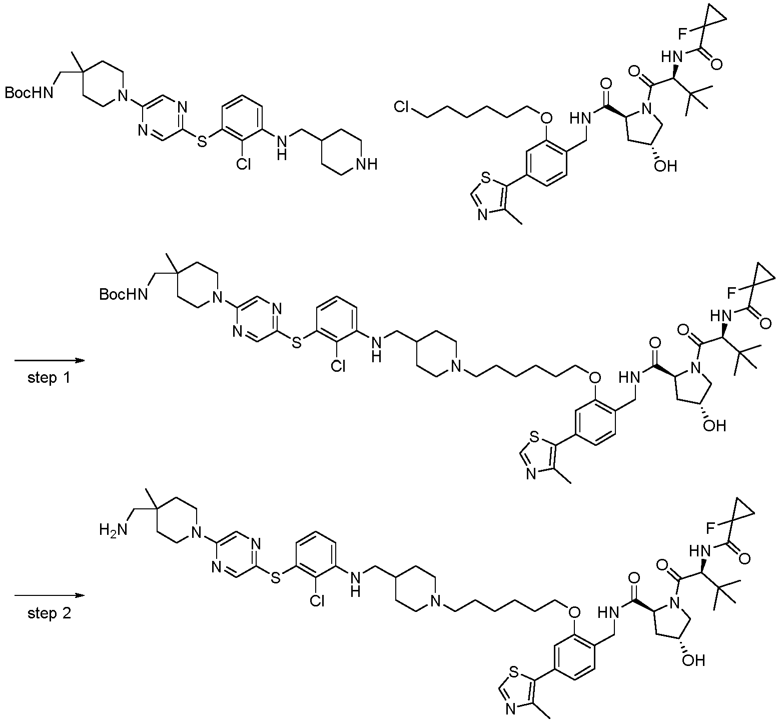

### Step 1: Synthesis of tert-butyl ((1-(5-((2-chloro-3-(((1-(6-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)hexyl)piperidin-4-yl)methyl)amino)phenyl)thio)pyrazin-2-yl)-4-methylpiperidin-4-yl)methyl)carbamate

tert-Butyl ((1-(5-((2-chloro-3-((piperidin-4-ylmethyl)amino)phenyl)thio)pyrazin-2-yl)-4-methylpiperidin-4-yl)methyl)carbamate (Intermediate 19, 33 mg, 0.06 mmol) and (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 69, 46 mg, 0.07 mmol) were suspended in DMF (0.1 ml). KI (13 mg, 0.12 mmol) and K₂CO₃ (24 mg, 0.17 mmol) were added and stirred at 70 °C for 16 hours. Distilled water (10 ml) was added to the reaction solution, and the mixture was extracted with EtOAc (10 ml x 2). The organic layer was washed with brine (10 ml x 2), dried over anhydrous magnesium sulfate, filtered, and concentrated under reduced pressure. The obtained residue was subjected to MPLC (amine-silica, 0-2% MeOH/DCM) to give 49 mg (72%) of an ivory solid.

### Step 2: Synthesis of (2S,4R)-N-(2-((6-(4-(((3-((5-(4-(aminomethyl)-4-methylpiperidin-1-yl)pyrazin-2-yl)thio)-2-chlorophenyl)amino)methyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

tert-Butyl ((1-(5-((2-chloro-3-(((1-(6-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)hexyl)piperidin-4-yl)methyl)amino)phenyl)thio)pyrazin-2-yl)-4-methylpiperidin-4-yl)methyl)carbamate (43 mg, 0.04 mmol) was suspended in DCM (1.0 ml), 4 M HCl in dioxane (0.1 ml) was added, and the mixture was stirred at room temperature for 1 hour. The reaction solution was neutralized by adding sat. NaHCO₃ (aq.) (10 ml), and then extracted with DCM (10 ml x 2). The organic layer was washed with brine (10 ml x 2), dried over anhydrous magnesium sulfate, filtered and concentrated under reduced pressure. The obtained residue was subjected to MPLC (amine-silica, 0-5% MeOH/DCM) to give 30 mg (78%) of an ivory solid.

### Example 2. (2S,4R)-N-(2-(2-(4-(2-((3-((5-(4-(aminomethyl)-4-methylpiperidin-1-yl)pyrazin-2-yl)thio)-2-chlorophenyl)amino)ethyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

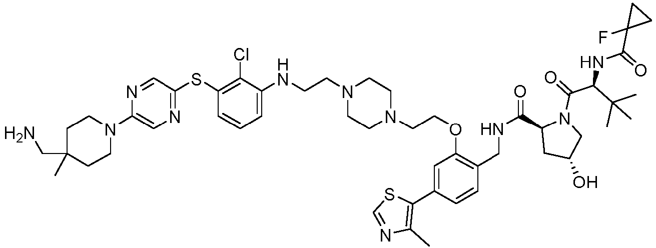

Example 2 was synthesized in the same way as Example 1, using tert-butyl ((1-(5-((2-chloro-3-((2-(piperazin-1-yl)ethyl)amino)phenyl)thio)pyrazin-2-yl)-4-methylpiperidin-4-yl)methyl)carbamate (Intermediate 20) and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 68) instead of tert-butyl ((1-(5-((2-chloro-3-((piperidin-4-ylmethyl)amino)phenyl)thio)pyrazin-2-yl)-4-methylpiperidin-4-yl)methyl)carbamate (Intermediate 19) and (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 69), respectively.

### Example 3. (2S,4R)-N-(2-(2-(4-(2-((3-((5-(4-(aminomethyl)-4-methylpiperidin-1-yl)pyrazin-2-yl)thio)-2-chlorophenyl)amino)ethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

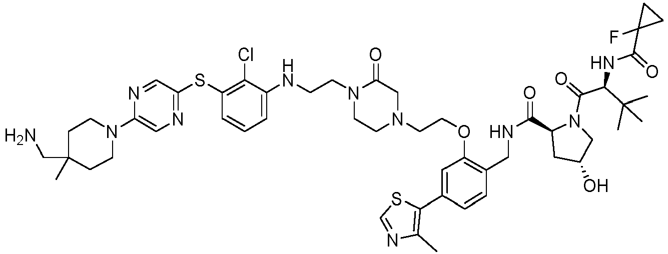

Example 3 was synthesized in the same way as Example 1, using tert-butyl ((1-(5-((2-chloro-3-((2-(2-oxopiperazin-1-yl)ethyl)amino)phenyl)thio)pyrazin-2-yl)-4-methylpiperidin-4-yl)methyl)carbamate (Intermediate 21) and (2S,4R)-N-(2-(2-chloroethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 68) instead of tert-butyl ((1-(5-((2-chloro-3-((piperidin-4-ylmethyl)amino)phenyl)thio)pyrazin-2-yl)-4-methylpiperidin-4-yl)methyl)carbamate (Intermediate 19) and (2S,4R)-N-(2-((6-chlorohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 69), respectively.

### Example 4. (2S,4R)-N-(2-(2-(4-((3-((5-(4-(aminomethyl)-4-methylpiperidin-1-yl)pyrazin-2-yl)thio)-2-chlorophenyl)glycyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

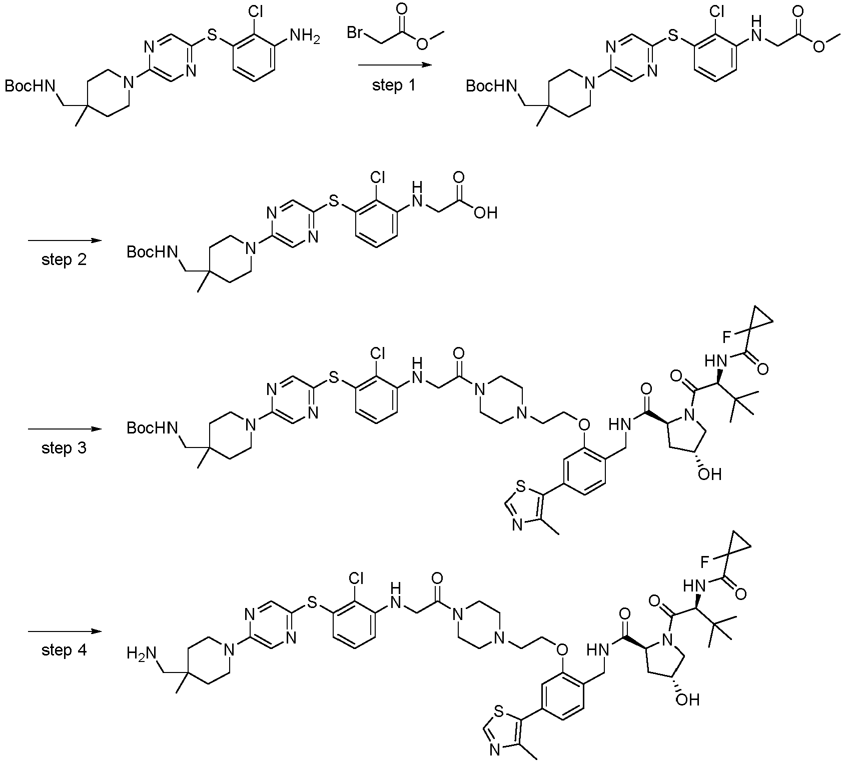

### Step 1: Synthesis of methyl (3-((5-(4-(((tert-butoxycarbonyl)amino)methyl)-4-methylpiperidin-1-yl)pyrazin-2-yl)thio)-2-chlorophenyl)glycinate

tert-Butyl ((1-(5-((3-amino-2-chlorophenyl)thio)pyrazin-2-yl)-4-methylpiperidin-4-yl)methyl)carbamate (Intermediate 1, 30 mg, 0.06 mmol), methyl bromoacetate (33 mg, 0.21 mmol), K₂CO₃ (27 mg, 0.19 mmol), and KI (21 mg, 0.13 mmol) were suspended in DMF (0.4 ml) and stirred at 70 °C for 16 hours. Distilled water (10 ml) was added to the reaction solution, and then extracted with EtOAc (10 ml). The organic layer was washed with brine (10 ml), dried over anhydrous sodium sulfate, filtered, and concentrated under reduced pressure. The obtained residue was subjected to MPLC (1-30% EtOAc/hexane) to give 28.6 mg (82%) of an ivory solid.

### Step 2: Synthesis of (3-((5-(4-(((tert-butoxycarbonyl)amino)methyl)-4-methylpiperidin-1-yl)pyrazin-2-yl)thio)-2-chlorophenyl)glycine

Methyl (3-((5-(4-(((tert-butoxycarbonyl)amino)methyl)-4-methylpiperidin-1-yl)pyrazin-2-yl)thio)-2-chlorophenyl)glycinate (28.6 mg, 0.05 mmol) and LiOH (4.5 mg, 0.11 mmol) were suspended in a mixed solvent of H₂O:THF (2:1, 0.4 ml) and stirred at room temperature for 2 hours. The reaction solution was adjusted to pH 2 with 1 M HCl (aq.) and extracted with DCM (10 ml x 2). The organic layer was dried over anhydrous sodium sulfate, filtered and concentrated under reduced pressure. The obtained residue was subjected to MPLC (0-12% MeOH/DCM) to give 20.3 mg (73%) of an ivory solid.

### Step 3: Synthesis of tert-butyl ((1-(5-((2-chloro-3-((2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)-2-oxoethyl)amino)phenyl)thio)pyrazin-2-yl)-4-methylpiperidin-4-yl)methyl)carbamate

(3-((5-(4-(((tert-Butoxycarbonyl)amino)methyl)-4-methylpiperidin-1-yl)pyrazin-2-yl)thio)-2-chlorophenyl)glycine (19 mg 0.04 mmol), (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (Intermediate 71, 24.5 mg, 0.04 mmol), EDCI (8.4 mg, 0.04 mmol), HOBt (5.9 mg, 0.04 mmol), and DIPEA (0.02 ml, 0.11 mmol) were suspended in DMF. (0.12 ml) and stirred at room temperature for 16 hours. Distilled water (7 ml) was added to the reaction solution, and extracted with EtOAc (10 ml). The organic layer was washed with brine (7 ml), dried over anhydrous sodium sulfate, filtered, and concentrated under reduced pressure. The obtained residue was subjected to MPLC (0-7% MeOH/DCM) to give 24 mg (57%) of an ivory solid.

### Step 4: Synthesis of (2S,4R)-N-(2-(2-(4-((3-((5-(4-(aminomethyl)-4-methylpiperidin-1-yl)pyrazin-2-yl)thio)-2-chlorophenyl)glycyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

tert-Butyl ((1-(5-((2-chloro-3-((2-(4-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperazin-1-yl)-2-oxoethyl)amino)phenyl)thio)pyrazin-2-yl)-4-methylpiperidin-4-yl)methyl)carbamate (24 mg, 0.02 mmol) was suspended in DCM (2.1 ml), 4 M HCl in dioxane (0.052 ml) was added, and the mixture was stirred at room temperature for 2 hours. After adding sat. NaHCO₃ (aq.) (5 ml) to the reaction solution, it was extracted with DCM (10 ml x 2). The organic layer was dried over anhydrous magnesium sulfate, filtered, and concentrated under reduced pressure. The obtained residue was subjected to MPLC (amine-silica, 0-3% MeOH/DCM) to give 17.3 mg (79%) of an ivory solid.

### Example 5. (2S,4R)-N-(2-(((1R,4S)-4-((3-((3-((5-(4-(aminomethyl)-4-methylpiperidin-1-yl)pyrazin-2-yl)thio)-2-chlorophenyl)amino)azetidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

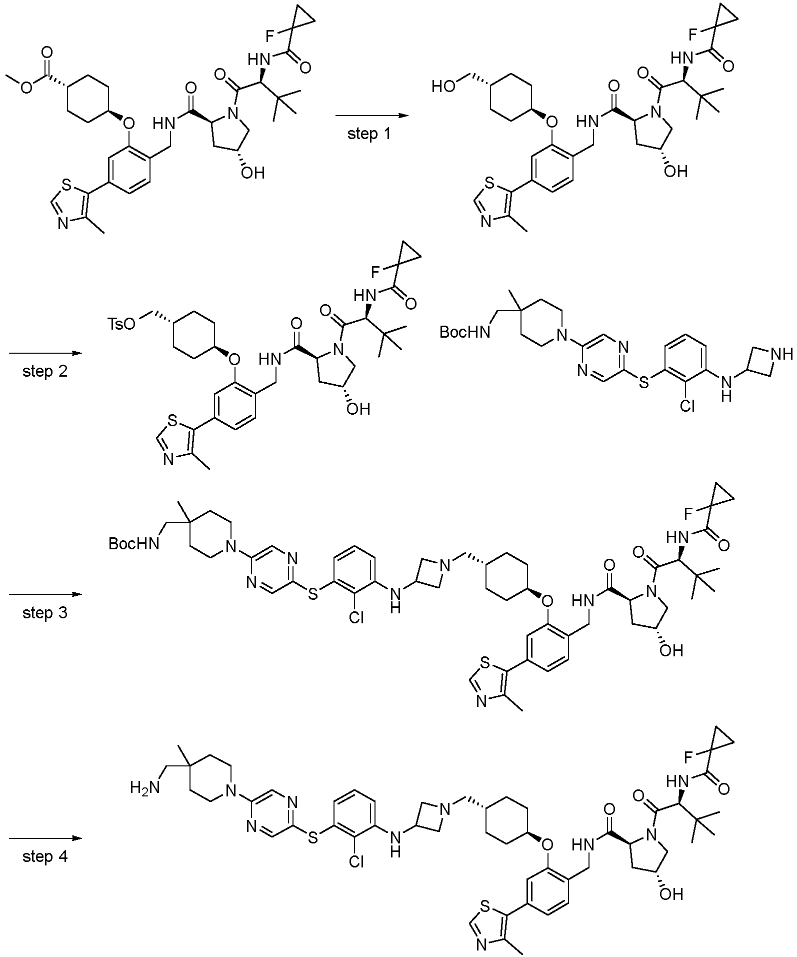

### Step 1: Synthesis of (2S,4R)-1-((S)-2-(1-fluorocyclopropane-1-carboxamido)-3,3-dimethylbutanoyl)-4-hydroxy-N-(2-(((1R,4S)-4-(hydroxymethyl)cyclohexyl)oxy)-4-(4-methylthiazol-5-yl)benzyl)pyrrolidine-2-carboxamide

Methyl (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylate (100 mg, 0.15 mmol) was suspended in THF (2 ml) and LAH (2.0 M in THF, 82 µl, 0.16 mmol) was added at 0 °C, and the mixture was stirred at room temperature for 30 min. 10% NaOH (aq.) (5 ml) was added to the reaction solution, and then extracted with EtOAc (15 ml). The organic layer was washed with distilled water (10 ml) and brine (10 ml), dried over anhydrous sodium sulfate, filtered and concentrated under reduced pressure. The obtained residue was subjected to MPLC (0-5% MeOH/DCM) to give 72.5 mg (76%) of an ivory solid.

### Step 2: Synthesis of ((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexyl)methyl 4-methylbenzenesulfonate

(2S,4R)-1-((S)-2-(1-Fluorocyclopropane-1-carboxamido)-3,3-dimethylbutanoyl)-4-hydroxy-N-(2-(((1R,4S)-4-(hydroxymethyl)cyclohexyl)oxy)-4-(4-methylthiazol-5-yl)benzyl)pyrrolidine-2-carboxamide (72.5 mg, 0.11 mmol) was suspended in DCM (0.48 ml), and TEA (23 µl, 0.17 mmol), DMAP (1.4 mg, 0.01 mmol), and 4-toluenesulfonyl chloride (25.7 mg, 0.13 mmol) were added at 0 °C, and the mixture was stirred at room temperature for 3 hours. Distilled water (10 ml) was added to the reaction solution, extracted with DCM (10 ml), and the organic layer was dried over anhydrous sodium sulfate, filtered, and concentrated under reduced pressure. The obtained residue was subjected to MPLC (0-5% MeOH/DCM) to obtain 55.8 mg (62%) of an ivory solid.

### Step 3: Synthesis of tert-butyl ((1-(5-((2-chloro-3-((1-(((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexyl)methyl)azetidin-3-yl)amino)phenyl)thio)pyrazin-2-yl)-4-methylpiperidin-4-yl)methyl)carbamate

tert-Butyl ((1-(5-((3-(azetidin-3-ylamino)-2-chlorophenyl)thio)pyrazin-2-yl)-4-methylpiperidin-4-yl)methyl)carbamate (Intermediate 11, 26.4 mg, crude), ((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexyl)methyl 4-methylbenzenesulfonate (25 mg, 0.3 mmol), K₂CO₃ (13 mg, 0.09 mmol), and KI (10 mg, 0.06 mmol) were suspended in DMF (0.1 ml) and stirred at 70 °C for 16 hours. Distilled water (7 ml) was added to the reaction solution, and extracted with EtOAc (10 ml). The organic layer was washed with brine (7 ml), dried over anhydrous sodium sulfate, filtered, and concentrated under reduced pressure. The obtained residue was subjected to MPLC (0-7% MeOH/DCM) to obtain 11.6 mg (32%) of an ivory solid.

### Step 4: Synthesis of (2S,4R)-N-(2-(((1R,4S)-4-((3-((3-((5-(4-(aminomethyl)-4-methylpiperidin-1-yl)pyrazin-2-yl)thio)-2-chlorophenyl)amino)azetidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

tert-Butyl ((1-(5-((2-chloro-3-((1-(((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexyl)methyl)azetidin-3-yl)amino)phenyl)thio)pyrazin-2-yl)-4-methylpiperidin-4-yl)methyl)carbamate (11.6 mg, 0.01 mmol) was suspended in DCM (1 ml), 4 M HCl in dioxane (25 µl) was added, and the mixture was stirred at room temperature for 1 h. The reaction solution was added sat. After adding sat. NaHCO₃ (aq.) (3 ml) to the reaction solution, it was extracted with DCM (5 ml x 2). The organic layer was dried over anhydrous magnesium sulfate, filtered, and concentrated under reduced pressure. The obtained residue was subjected to MPLC (amine-silica, 0-3% MeOH/DCM) to give 7.6 mg (72%) of an ivory solid.

### Example 6. (2S,4R)-N-(2-(((1R,4S)-4-((4-((3-((5-(4-(aminomethyl)-4-methylpiperidin-1-yl)pyrazin-2-yl)thio)-2-chlorophenyl)amino)piperidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

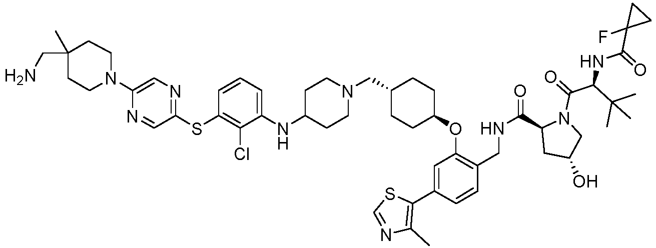

Example 6 was synthesized in the same way as Example 5, using tert-butyl ((1-(5-((2-chloro-3-(piperidin-4-ylamino)phenyl)thio)pyrazin-2-yl)-4-methylpiperidin-4-yl)methyl)carbamate (Intermediate 17) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 7. (2S,4R)-N-(2-((1-(((1R,4S)-4-((4-((5-(4-(aminomethyl)-4-methylpiperidin-1-yl)pyrazin-2-yl)thio)-3-chloropyridin-2-yl)amino)cyclohexyl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

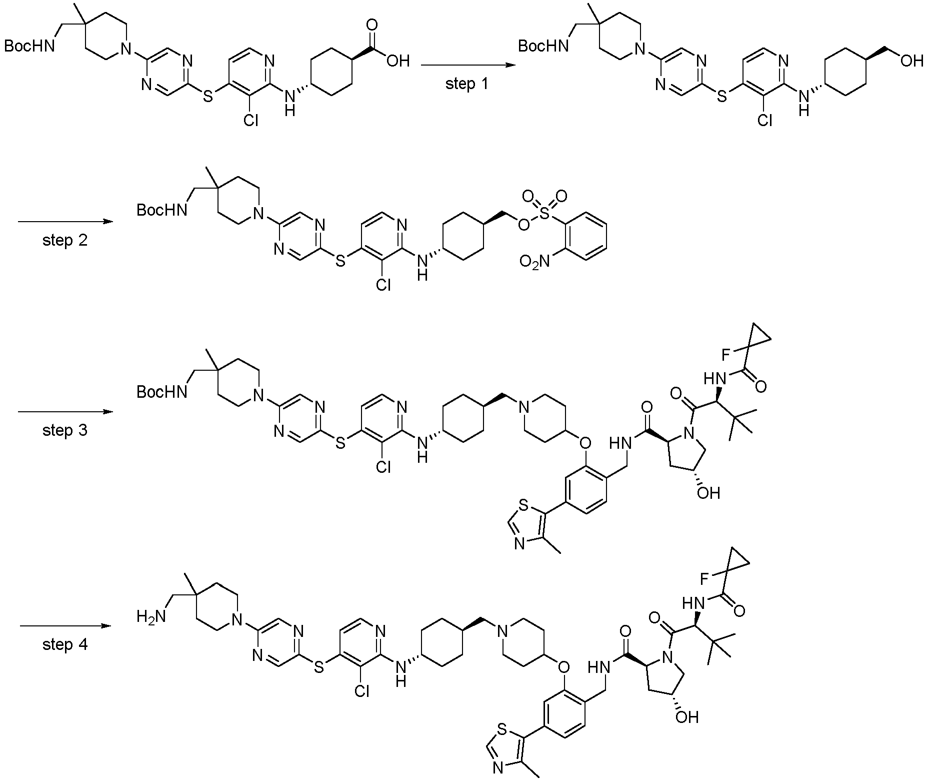

### Step 1: Synthesis of tert-butyl ((1-(5-((3-chloro-2-(((1R,4R)-4-(hydroxymethyl)cyclohexyl)amino)pyridin-4-yl)thio)pyrazin-2-yl)-4-methylpiperidin-4-yl)methyl)carbamate

(1R,4R)-4-((4-((5-(4-(((tert-Butoxycarbonyl)amino)methyl)-4-methylpiperidin-1-yl)pyrazin-2-yl)thio)-3-chloropyridin-2-yl)amino)cyclohexane-1-carboxylic acid (Intermediate 38, 42 mg, 0.07 mmol) was suspended in THF (0.24 ml), LAH (2.0 M in THF, 0.03 ml, 0.07 mmol) was added at 0 °C, and the mixture was stirred at room temperature for 1 hour. Distilled water (0.01 ml) was added to the reaction solution, stirred at room temperature for 5 minutes, and extracted with DCM (10 ml). The organic layer was dried over anhydrous magnesium sulfate, filtered, and concentrated under reduced pressure. The obtained residue was subjected to MPLC (0-5% MeOH/DCM) to give 14 mg (34%) of an ivory solid.

### Step 2: Synthesis of ((1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexyl)methyl 2-nitrobenzenesulfonate

tert-Butyl ((1-(5-((3-chloro-2-(((1R,4R)-4-(hydroxymethyl)cyclohexyl)amino)pyridin-4-yl)thio)pyrazin-2-yl)-4-methylpiperidin-4-yl)methyl)carbamate (16 mg, 0.03 mmol), TEA (0.007 ml, 0.06 mmol), and DMAP (0.13 mg, 0.001 mmol) were suspended in DCM (0.14 ml), stirred at 0 °C for 10 minutes, 2-nitrobenzenesulfonyl chloride (20 mg, 0.09 mmol) was added, and stirred at room temperature for 4 hours. Distilled water (10 ml) was added to the reaction solution, and then extracted with DCM (10 ml x 2). The obtained organic layer was dried over anhydrous sodium sulfate, filtered, and concentrated under reduced pressure. The obtained residue was subjected to MPLC (0-5% MeOH/DCM) to give 15 mg (quant.) of an ivory solid.

### Step 3: Synthesis of tert-butyl ((1-(5-((3-chloro-2-(((1S,4R)-4-((4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)piperidin-1-yl)methyl)cyclohexyl)amino)pyridin-4-yl)thio)pyrazin-2-yl)-4-methylpiperidin-4-yl)methyl)carbamate

((1R,4R)-4-((4-((5-(4-(((tert-Butoxycarbonyl)amino)methyl)-4-methylpiperidin-1-yl)pyrazin-2-yl)thio)-3-chloropyridin-2-yl)amino)cyclohexyl)methyl 2-nitrobenzenesulfonate (14 mg, 0.02 mmol) was dissolved in DMF (0.2 ml). Then (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75, 14 mg, 0.02 mmol) and Cs₂CO₃ (12 mg, 0.04 mmol) were added and stirred at 50 °C for 16 hours. Distilled water (10 ml) was added to the reaction solution, and the mixture was extracted with EtOAc (10 ml x 2). The organic layer was washed with brine (10 ml x 2), dried over anhydrous magnesium sulfate, filtered, and concentrated under reduced pressure. The obtained residue was subjected to MPLC (0-10% MeOH/DCM) to give 6.8 mg (32%) of an ivory solid.

### Step 4: Synthesis of (2S,4R)-N-(2-((1-(((1R,4S)-4-((4-((5-(4-(aminomethyl)-4-methylpiperidin-1-yl)pyrazin-2-yl)thio)-3-chloropyridin-2-yl)amino)cyclohexyl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

tert-Butyl ((1-(5-((3-chloro-2-(((1S,4R)-4-((4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)piperidin-1-yl)methyl)cyclohexyl)amino)pyridin-4-yl)thio)pyrazin-2-yl)-4-methylpiperidin-4-yl)methyl)carbamate (6 mg, 0.005 mmol) was suspended in DCM (1.0 ml), 4 M HCl in dioxane (0.01 ml) was added, and the mixture was stirred at room temperature for 1 hour. The reaction solution was neutralized by adding sat. NaHCO₃ (aq.) (5 ml), and then extracted with DCM (5 ml x 2). The organic layer was washed with brine (5 ml x 2), dried over anhydrous magnesium sulfate, filtered and concentrated under reduced pressure. The obtained residue was subjected to MPLC (amine-silica, 0-5% MeOH/DCM) to give 3.5 mg (65%) of an ivory solid.

### Example 8. (2S,4R)-N-(2-(2-(4-((1-(4-((5-(4-(aminomethyl)-4-methylpiperidin-1-yl)pyrazin-2-yl)thio)-3-chloropyridin-2-yl)piperidin-4-yl)methyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

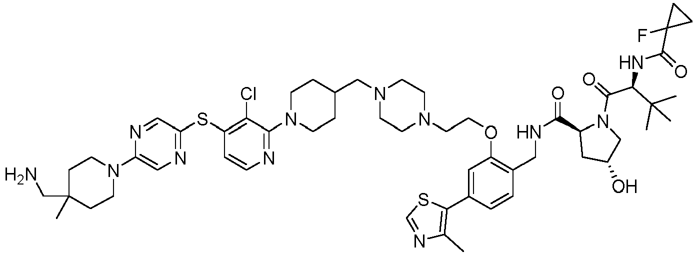

Example 8 was synthesized in the same way as Example 7, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (Intermediate 71) instead of (1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexane-1-carboxylic acid (Intermediate 38) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75), respectively.

### Example 9. (2S,4R)-N-(2-((1-((1-(4-((5-(4-(aminomethyl)-4-methylpiperidin-1-yl)pyrazin-2-yl)thio)-3-chloropyridin-2-yl)piperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

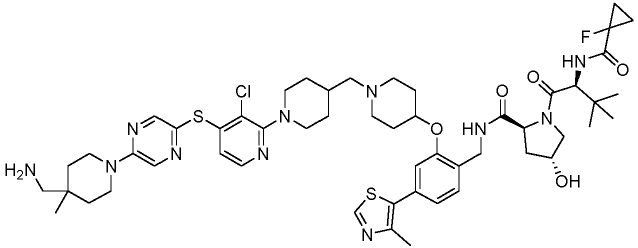

Example 9 was synthesized in the same way as Example 7, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) instead of (1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexane-1-carboxylic acid (Intermediate 38).

### Example 10. (2S,4R)-N-(2-((1-((1-(4-((5-(4-(aminomethyl)-4-methylpiperidin-1-yl)pyrazin-2-yl)thio)-3-chloropyridin-2-yl)-4-hydroxypiperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

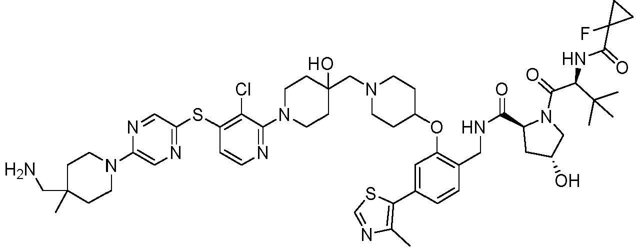

Example 10 was synthesized in the same way as Example 7, using 2-(1-(4-((5-(4-(((tert-butoxycarbonyl)amino)methyl)-4-methylpiperidin-1-yl)pyrazin-2-yl)thio)-3-chloropyridin-2-yl)-4-hydroxypiperidin-4-yl)acetic acid (Intermediate 48) instead of (1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexane-1-carboxylic acid (Intermediate 38).

### Example 11. (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

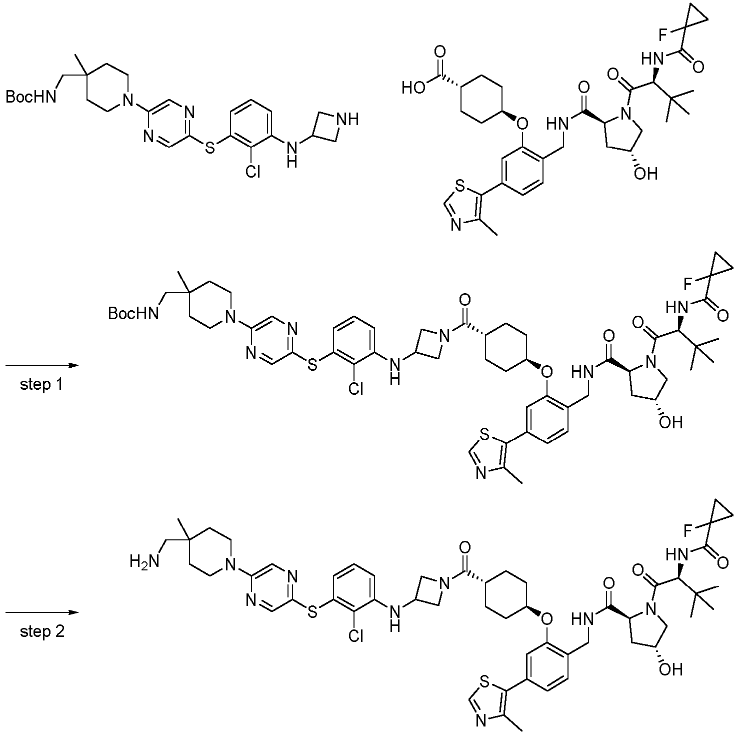

### Step 1: Synthesis of tert-butyl ((1-(5-((2-chloro-3-((1-((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carbonyl)azetidin-3-yl)amino)phenyl)thio)pyrazin-2-yl)-4-methylpiperidin-4-yl)methyl)carbamate

tert-Butyl ((1-(5-((3-(azetidin-3-ylamino)-2-chlorophenyl)thio)pyrazin-2-yl)-4-methylpiperidin-4-yl)methyl)carbamate (Intermediate 11, 278 mg, crude), (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65, 205 mg, 0.31 mmol), EDCI (72 mg, 0.37 mmol), HOBt (54 mg, 0.37 mmol), and DIPEA (0.16 ml, 0.93 mmol) were suspended in DMF (1 ml) and stirred at room temperature for 16 hours. Distilled water (20 ml) was added to the reaction solution, and the mixture was extracted with EtOAc (30 ml). The organic layer was washed with brine (20 ml), dried over anhydrous sodium sulfate, filtered, and concentrated under reduced pressure. The obtained residue was subjected to MPLC (0-7% MeOH/DCM) to give 144 mg (40%) of an ivory solid.

### Step 2: Synthesis of (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

tert-Butyl ((1-(5-((2-chloro-3-((1-((1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carbonyl)azetidin-3-yl)amino)phenyl)thio)pyrazin-2-yl)-4-methylpiperidin-4-yl)methyl)carbamate (100 mg, 0.09 mmol) was suspended in DCM (8.6 ml), 4 M HCl in dioxane (0.22 ml) was added, and the mixture was stirred at room temperature for 1 hour. After adding sat. NaHCO₃ (aq.) (5 ml) to the reaction solution, it was extracted with DCM (10 ml x 2). The organic layer was dried over anhydrous magnesium sulfate, filtered, and concentrated under reduced pressure. The obtained residue was subjected to MPLC (amine-silica, 0-3% MeOH/DCM) to give 80.7 mg (88%) of an ivory solid.

### Example 12. (2S,4R)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

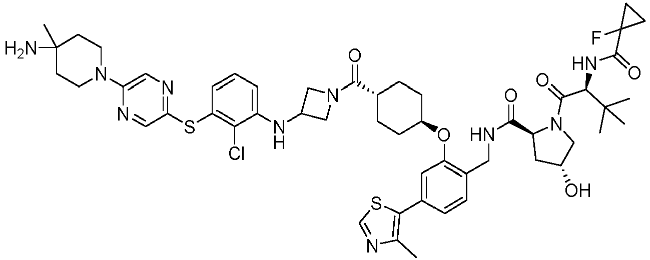

Example 12 was synthesized in the same way as Example 11, using tert-butyl (1-(5-((3-(azetidin-3-ylamino)-2-chlorophenyl)thio)pyrazin-2-yl)-4-methylpiperidin-4-yl)carbamate (Intermediate 12) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 13. (2S,4R)-N-(2-(((1R,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

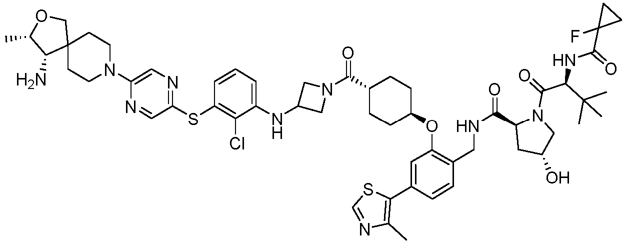

Example 13 was synthesized in the same way as Example 11, using tert-butyl ((3S,4S)-8-(5-((3-(azetidin-3-ylamino)-2-chlorophenyl)thio)pyrazin-2-yl)-3-methyl-2-oxa-8-azaspiro[4.5]decan-4-yl)carbamate (Intermediate 13) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 14. (2S,4R)-N-(2-(((1R,4S)-4-(3-((3-((5-((S)-1-amino-1,3-dihydrospiro[indene-2,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

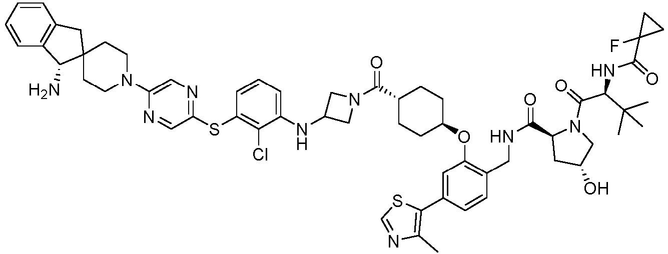

Example 14 was synthesized in the same way as Example 11, using (S)-tert-butyl (1'-(5-((3-(azetidin-3-ylamino)-2-chlorophenyl)thio)pyrazin-2-yl)-1,3-dihydrospiro[indene-2,4'-piperidin]-1-yl)carbamate (Intermediate 14) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 15. (2S,4R)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

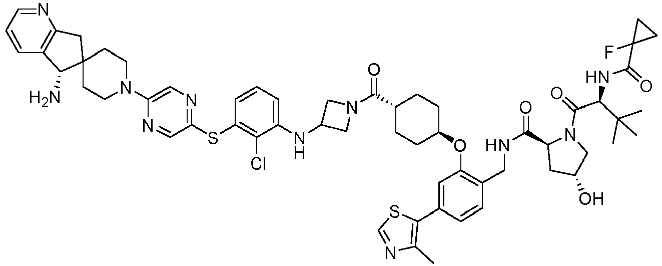

Example 15 was synthesized in the same way as Example 11, using N-((S)-1'-(5-((3-(azetidin-3-ylamino)-2-chlorophenyl)thio)pyrazin-2-yl)-5,7-dihydrospiro[cyclopenta[b]pyridine-6,4'-piperidin]-5-yl)-2-methylpropane-2-sulfinamide (Intermediate 15) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 16. (2S,4R)-N-(2-(((1R,4S)-4-(3-((3-((5-(4-(aminomethyl)-4-methylpiperidin-1-yl)pyrazin-2-yl)oxy)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

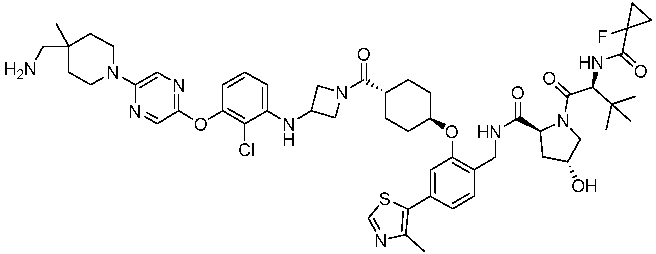

Example 16 was synthesized in the same way as Example 11, using tert-butyl ((1-(5-(3-(azetidin-3-ylamino)-2-chlorophenoxy)pyrazin-2-yl)-4-methylpiperidin-4-yl)methyl)carbamate (Intermediate 16) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 17. (2S,4R)-N-(2-(((1R,4S)-4-(4-((3-((5-(4-(aminomethyl)-4-methylpiperidin-1-yl)pyrazin-2-yl)thio)-2-chlorophenyl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

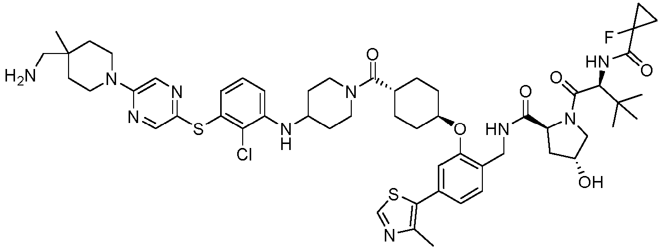

Example 17 was synthesized in the same way as Example 11, using tert-butyl ((1-(5-((2-chloro-3-(piperidin-4-ylamino)phenyl)thio)pyrazin-2-yl)-4-methylpiperidin-4-yl)methyl)carbamate (Intermediate 17) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 18. (2S,4R)-N-(2-(((1R,4S)-4-(3-(((3-((5-(4-(aminomethyl)-4-methylpiperidin-1-yl)pyrazin-2-yl)thio)-2-chlorophenyl)amino)methyl)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

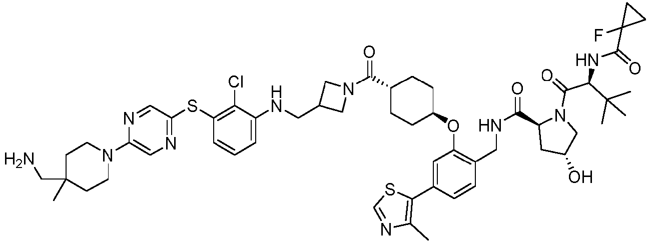

Example 18 was synthesized in the same way as Example 11, using tert-butyl ((1-(5-((3-((azetidin-3-ylmethyl)amino)-2-chlorophenyl)thio)pyrazin-2-yl)-4-methylpiperidin-4-yl)methyl)carbamate (Intermediate 18) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 19. (2S,4R)-N-(2-(((1R,4S)-4-(4-(((3-((5-(4-(aminomethyl)-4-methylpiperidin-1-yl)pyrazin-2-yl)thio)-2-chlorophenyl)amino)methyl)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

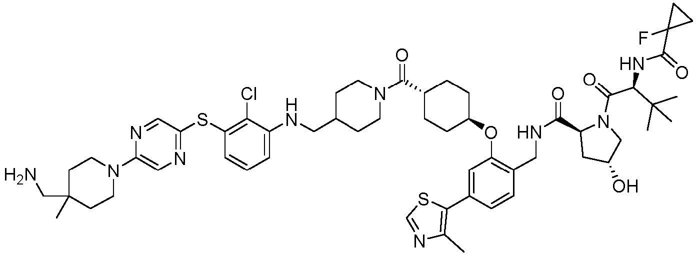

Example 19 was synthesized in the same way as Example 11, using tert-butyl ((1-(5-((2-chloro-3-((piperidin-4-ylmethyl)amino)phenyl)thio)pyrazin-2-yl)-4-methylpiperidin-4-yl)methyl)carbamate (Intermediate 19) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 20. (2S,4R)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

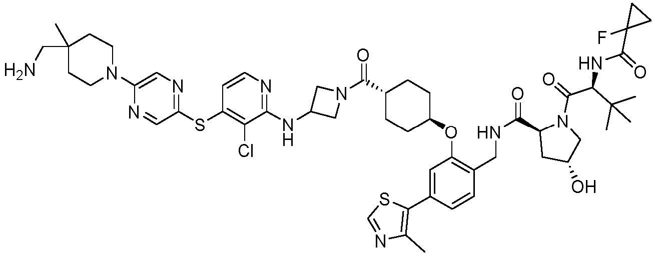

Example 20 was synthesized in the same way as Example 11, using tert-butyl ((1-(5-((2-(azetidin-3-ylamino)-3-chloropyridin-4-yl)thio)pyrazin-2-yl)-4-methylpiperidin-4-yl)methyl)carbamate (Intermediate 28) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 21. (2S,4R)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

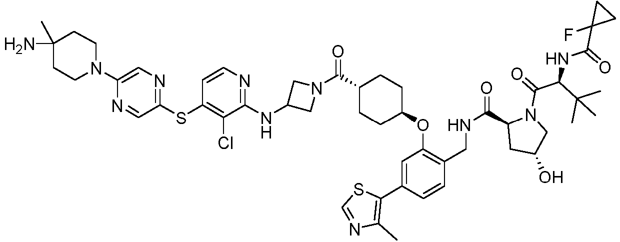

Example 21 was synthesized in the same way as Example 11, using tert-butyl (1-(5-((2-(azetidin-3-ylamino)-3-chloropyridin-4-yl)thio)pyrazin-2-yl)-4-methylpiperidin-4-yl)carbamate (Intermediate 29) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 22. (2S,4R)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

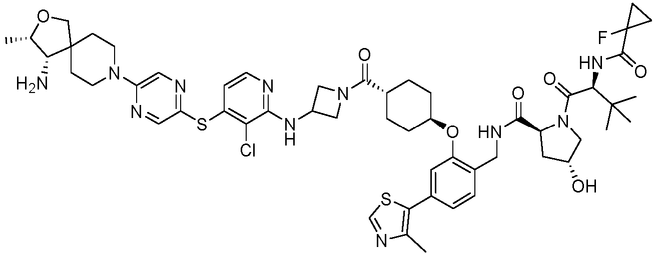

Example 22 was synthesized in the same way as Example 11, using tert-butyl ((3S,4S)-8-(5-((2-(azetidin-3-ylamino)-3-chloropyridin-4-yl)thio)pyrazin-2-yl)-3-methyl-2-oxa-8-azaspiro[4.5]decan-4-yl)carbamate (Intermediate 30) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 23. (2S,4R)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

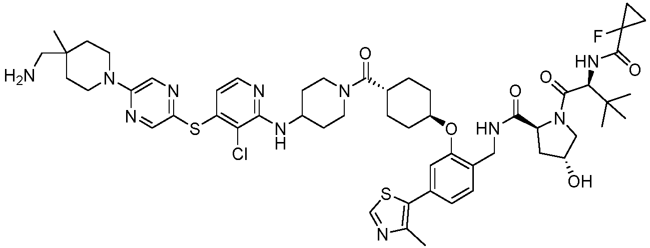

Example 23 was synthesized in the same way as Example 11, using tert-butyl ((1-(5-((3-chloro-2-(piperidin-4-ylamino)pyridin-4-yl)thio)pyrazin-2-yl)-4-methylpiperidin-4-yl)methyl)carbamate (Intermediate 31) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 24. (2S,4R)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

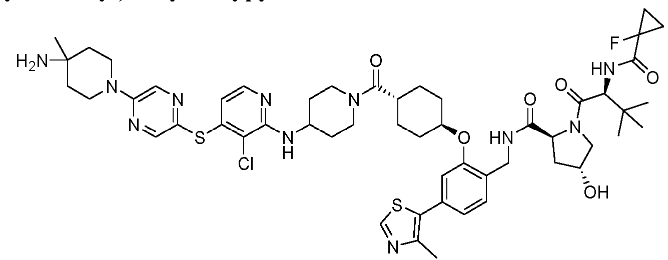

Example 24 was synthesized in the same way as Example 11, using tert-butyl (1-(5-((3-chloro-2-(piperidin-4-ylamino)pyridin-4-yl)thio)pyrazin-2-yl)-4-methylpiperidin-4-yl)carbamate (Intermediate 32) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 25. (2S,4R)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

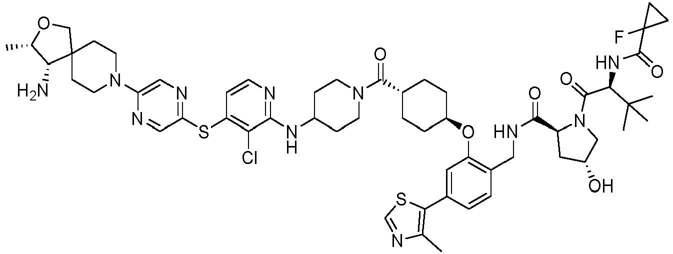

Example 25 was synthesized in the same way as Example 11, using tert-butyl ((3S,4S)-8-(5-((3-chloro-2-(piperidin-4-ylamino)pyridin-4-yl)thio)pyrazin-2-yl)-3-methyl-2-oxa-8-azaspiro[4.5]decan-4-yl)carbamate (Intermediate 33) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 26. (2S,4R)-N-(2-(((1R,4S)-4-(4-(4-((5-(4-(aminomethyl)-4-methylpiperidin-1-yl)pyrazin-2-yl)thio)-3-chloropyridin-2-yl)piperazine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

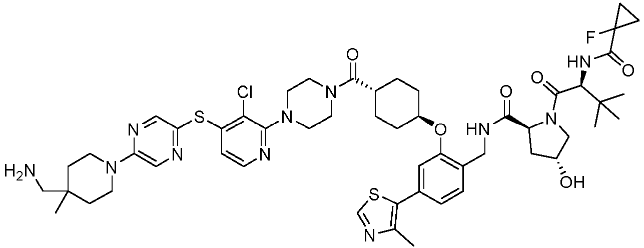

Example 26 was synthesized in the same way as Example 11, using tert-butyl ((1-(5-((3-chloro-2-(piperazin-1-yl)pyridin-4-yl)thio)pyrazin-2-yl)-4-methylpiperidin-4-yl)methyl)carbamate (Intermediate 34) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11).

### Example 27. (2S,4R)-1-((S)-2-acetamido-3,3-dimethylbutanoyl)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide

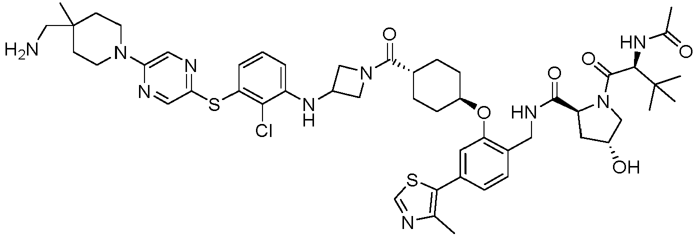

Example 27 was synthesized in the same way as Example 11, using (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65).

### Example 28. (2S,4R)-1-((S)-2-acetamido-3,3-dimethylbutanoyl)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide

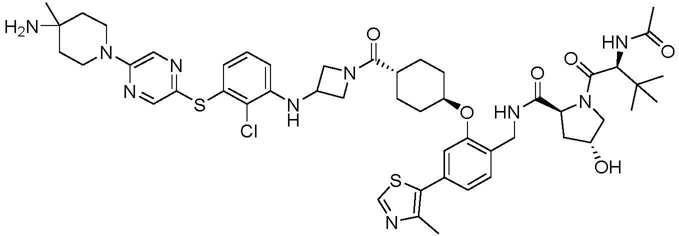

Example 28 was synthesized in the same way as Example 11, using tert-butyl (1-(5-((3-(azetidin-3-ylamino)-2-chlorophenyl)thio)pyrazin-2-yl)-4-methylpiperidin-4-yl)carbamate (Intermediate 12) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 29. (25,4R)-1-((S)-2-acetamido-3,3-dimethylbutanoyl)-N-(2-(((1r,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide

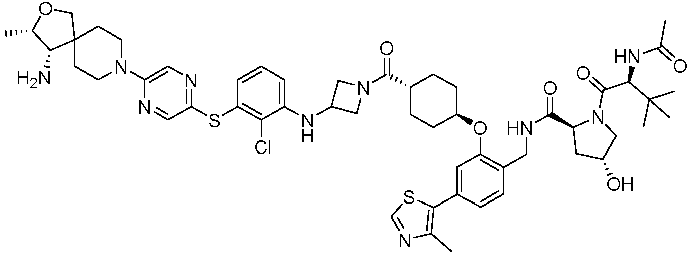

Example 29 was synthesized in the same way as Example 11, using tert-butyl ((3S,4S)-8-(5-((3-(azetidin-3-ylamino)-2-chlorophenyl)thio)pyrazin-2-yl)-3-methyl-2-oxa-8-azaspiro[4.5]decan-4-yl)carbamate (Intermediate 13) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 30. (2S,4R)-1-((S)-2-acetamido-3,3-dimethylbutanoyl)-N-(2-(((1R,4S)-4-(3-((3-((5-((S)-1-amino-1,3-dihydrospiro [indene-2,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide

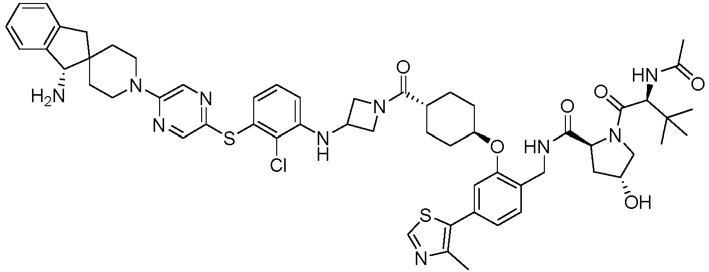

Example 30 was synthesized in the same way as Example 11, using (S)-tert-butyl (1'-(5-((3-(azetidin-3-ylamino)-2-chlorophenyl)thio)pyrazin-2-yl)-1,3-dihydrospiro[indene-2,4'-piperidin]-1-yl)carbamate (Intermediate 14) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 31. (2S,4R)-1-((S)-2-acetamido-3,3-dimethylbutanoyl)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide

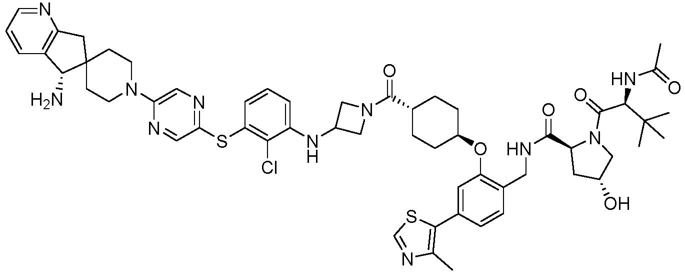

Example 31 was synthesized in the same way as Example 11, using N-((S)-1'-(5-((3-(azetidin-3-ylamino)-2-chlorophenyl)thio)pyrazin-2-yl)-5,7-dihydrospiro[cyclopenta[b]pyridine-6,4'-piperidin]-5-yl)-2-methylpropane-2-sulfinamide (Intermediate 15) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 32. (2S,4R)-1-((S)-2-acetamido-3,3-dimethylbutanoyl)-N-(2-(((1R,4S)-4-(3-(4-((5-((3S,4S)-4-amino-3-methyl-2-oxa-8-azaspiro[4.5]decan-8-yl)pyrazin-2-yl)thio)indolin-1-yl)azetidine-1-carbonyl)cyclohexyl)oxy)-4-(4-methylthiazol-5-yl)benzyl)-4-hydroxypyrrolidine-2-carboxamide

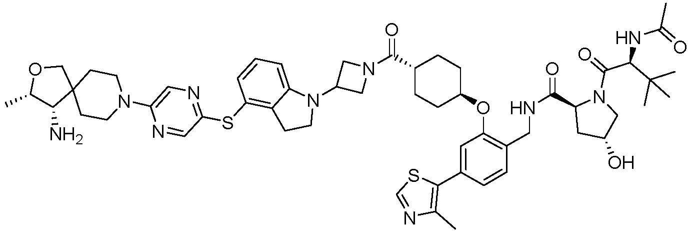

Example 32 was synthesized in the same way as Example 11, using (R)-N-((3S,4S)-8-(5-((1-(azetidin-3-yl)indolin-4-yl)thio)pyrazin-2-yl)-3-methyl-2-oxa-8-azaspiro[4.5]decan-4-yl)-2-methylpropane-2-sulfinamide (Intermediate 22) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 33. (2S,4R)-1-((S)-2-acetamido-3,3-dimethylbutanoyl)-N-(2-(((1R,4S)-4-(4-(4-((5-((3S,4S)-4-amino-3-methyl-2-oxa-8-azaspiro[4.5]decan-8-yl)pyrazin-2-yl)thio)indolin-1-yl)piperidine-1-carbonyl)cyclohexyl)oxy)-4-(4-methylthiazol-5-yl)benzyl)-4-hydroxypyrrolidine-2-carboxamide

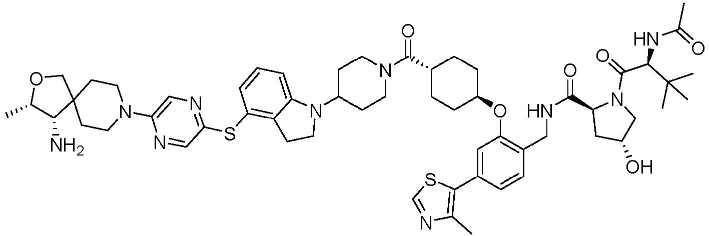

Example 33 was synthesized in the same way as Example 11, using (R)-2-methyl-N-((3S,45)-3-methyl-8-(5-((1-(piperidin-4-yl)indolin-4-yl)thio)pyrazin-2-yl)-2-oxa-8-azaspiro[4.5]decan-4-yl)propane-2-sulfinamide (Intermediate 23) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 34. (2S,4R)-1-((S)-2-acetamido-3,3-dimethylbutanoyl)-N-(2-(((1R,4S)-4-(3-(8-((5-(4-(aminomethyl)-4-methylpiperidin-1-yl)pyrazin-2-yl)thio)-2,3-dihydro-4H-benzo[b][1,4]oxazin-4-yl)azetidine-1-carbonyl)cyclohexyl)oxy)-4-(4-methylthiazol-5-yl)benzyl)-4-hydroxypyrrolidine-2-carboxamide

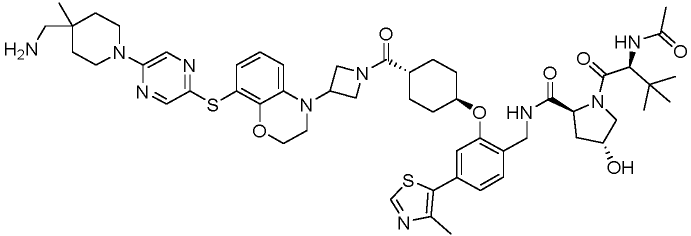

Example 34 was synthesized in the same way as Example 11, using tert-butyl ((1-(5-((4-(azetidin-3-yl)-3,4-dihydro-2H-benzo[b][1,4]oxazin-8-yl)thio)pyrazin-2-yl)-4-methylpiperidin-4-yl)methyl)carbamate (Intermediate 24) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 35. (2S,4R)-1-((S)-2-acetamido-3,3-dimethylbutanoyl)-N-(2-(((1r,4S)-4-(4-(8-((5-(4-(aminomethyl)-4-methylpiperidin-1-yl)pyrazin-2-yl)thio)-2,3-dihydro-4H-benzo[b][1,4]oxazin-4-yl)piperidine-1-carbonyl)cyclohexyl)oxy)-4-(4-methylthiazol-5-yl)benzyl)-4-hydroxypyrrolidine-2-carboxamide

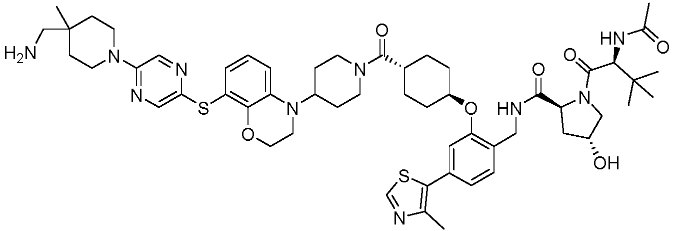

Example 35 was synthesized in the same way as Example 11, using tert-butyl ((4-methyl-1-(5-((4-(piperidin-4-yl)-3,4-dihydro-2H-benzo[b][1,4]oxazin-8-yl)thio)pyrazin-2-yl)piperidin-4-yl)methyl)carbamate (Intermediate 25) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 36. (2S,4R)-1-((S)-2-acetamido-3,3-dimethylbutanoyl)-N-(2-(((1R,4S)-4-(4-(8-((5-((3S,4S)-4-amino-3-methyl-2-oxa-8-azaspiro[4.5]decan-8-yl)pyrazin-2-yl)thio)-2H-benzo[b][1,4]oxazin-4(3H)-yl)piperidine-1-carbonyl)cyclohexyl)oxy)-4-(4-methylthiazol-5-yl)benzyl)-4-hydroxypyrrolidine-2-carboxamide

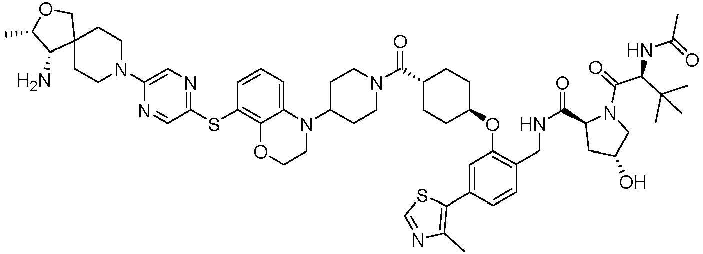

Example 36 was synthesized in the same way as Example 11, using tert-butyl ((3S,45)-3-methyl-8-(5-((4-(piperidin-4-yl)-3,4-dihydro-2H-benzo[b][1,4]oxazin-8-yl)thio)pyrazin-2-yl)-2-oxa-8-azaspiro[4.5]decan-4-yl)carbamate (Intermediate 26) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 37. (2S,4R)-1-((S)-2-acetamido-3,3-dimethylbutanoyl)-N-(2-(((1r,4S)-4-(4-(4-((5-(4-(aminomethyl)-4-methylpiperidin-1-yl)pyrazin-2-yl)thio)-3-fluoro-1H-indol-1-yl)piperidine-1-carbonyl)cyclohexyl)oxy)-4-(4-methylthiazol-5-yl)benzyl)-4-hydroxypyrrolidine-2-carboxamide

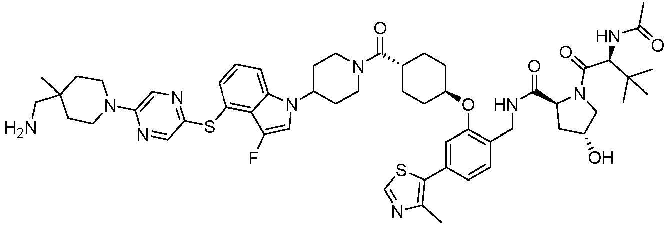

Example 37 was synthesized in the same way as Example 11, using tert-butyl ((1-(5-((3-fluoro-1-(piperidin-4-yl)-1H-indol-4-yl)thio)pyrazin-2-yl)-4-methylpiperidin-4-yl)methyl)carbamate (Intermediate 27) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 38. (2S,4R)-1-((S)-2-acetamido-3,3-dimethylbutanoyl)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide

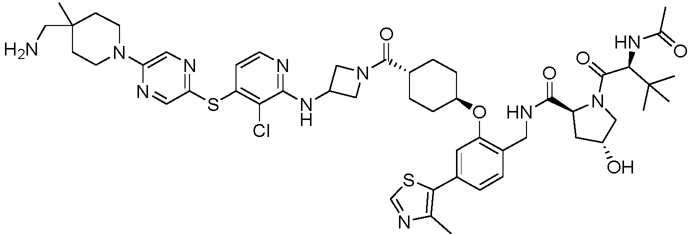

Example 38 was synthesized in the same way as Example 11, using tert-butyl ((1-(5-((2-(azetidin-3-ylamino)-3-chloropyridin-4-yl)thio)pyrazin-2-yl)-4-methylpiperidin-4-yl)methyl)carbamate (Intermediate 28) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 39. (2S,4R)-1-((S)-2-acetamido-3,3-dimethylbutanoyl)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide

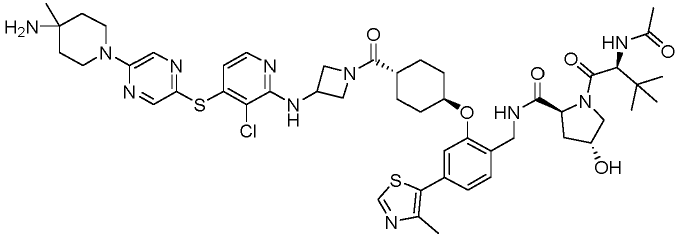

Example 39 was synthesized in the same way as Example 11, using tert-butyl (1-(5-((2-(azetidin-3-ylamino)-3-chloropyridin-4-yl)thio)pyrazin-2-yl)-4-methylpiperidin-4-yl)carbamate (Intermediate 29) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 40. (2S,4R)-1-((S)-2-acetamido-3,3-dimethylbutanoyl)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide

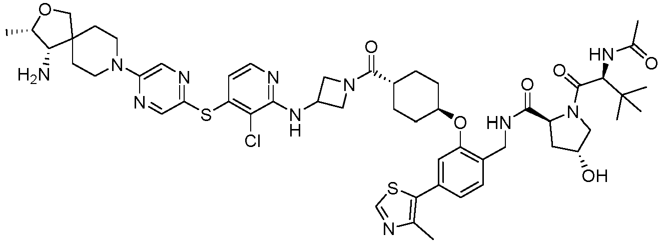

Example 40 was synthesized in the same way as Example 11, using tert-butyl ((3S,4S)-8-(5-((2-(azetidin-3-ylamino)-3-chloropyridin-4-yl)thio)pyrazin-2-yl)-3-methyl-2-oxa-8-azaspiro[4.5]decan-4-yl)carbamate (Intermediate 30) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 41. (2S,4R)-1-((S)-2-acetamido-3,3-dimethylbutanoyl)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide

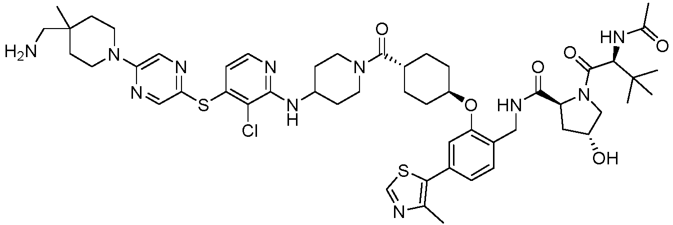

Example 41 was synthesized in the same way as Example 11, using tert-butyl ((1-(5-((3-chloro-2-(piperidin-4-ylamino)pyridin-4-yl)thio)pyrazin-2-yl)-4-methylpiperidin-4-yl)methyl)carbamate (Intermediate 31) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65) , respectively.

### Example 42. (2S,4R)-1-((S)-2-acetamido-3,3-dimethylbutanoyl)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide

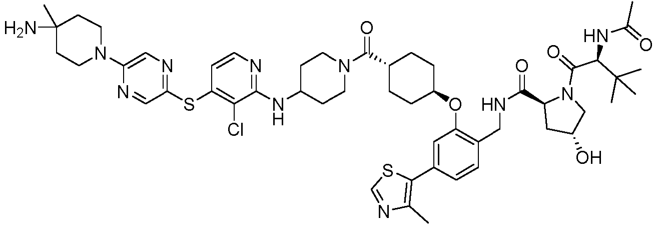

Example 42 was synthesized in the same way as Example 11, using tert-butyl (1-(5-((3-chloro-2-(piperidin-4-ylamino)pyridin-4-yl)thio)pyrazin-2-yl)-4-methylpiperidin-4-yl)carbamate (Intermediate 32) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1 S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 43. (2S,4R)-1-((S)-2-acetamido-3,3-dimethylbutanoyl)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide

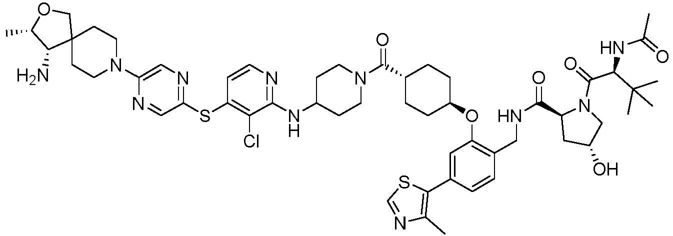

Example 43 was synthesized in the same way as Example 11, using tert-butyl ((3S,4S)-8-(5-((3-chloro-2-(piperidin-4-ylamino)pyridin-4-yl)thio)pyrazin-2-yl)-3-methyl-2-oxa-8-azaspiro[4.5]decan-4-yl)carbamate (Intermediate 33) and (1S,4r)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 44. (25,4R)-1-((S)-2-acetamido-3,3-dimethylbutanoyl)-N-(2-(((1r,4S)-4-(3-((4-((5-(4-(aminomethyl)-4-methylpiperidin-1-yl)-6-(hydroxymethyl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide

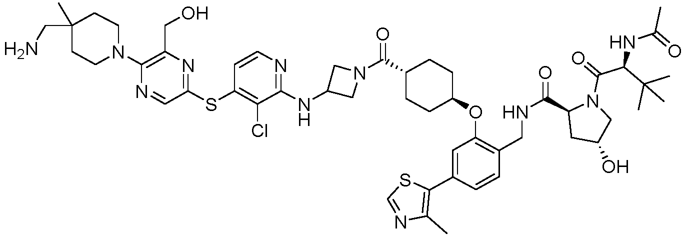

Example 44 was synthesized in the same way as Example 11, using tert-butyl ((1-(5-((2-(azetidin-3-ylamino)-3-chloropyridin-4-yl)thio)-3-(hydroxymethyl)pyrazin-2-yl)-4-methylpiperidin-4-yl)methyl)carbamate (Intermediate 49) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 45. (25,4R)-1-((S)-2-acetamido-3,3-dimethylbutanoyl)-N-(2-(((1r,4S)-4-(4-((4-((5-(4-(aminomethyl)-4-methylpiperidin-1-yl)-6-(hydroxymethyl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide

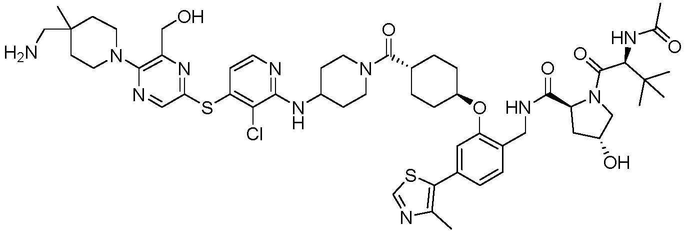

Example 45 was synthesized in the same way as Example 11, using tert-butyl ((1-(5-((3-chloro-2-(piperidin-4-ylamino)pyridin-4-yl)thio)-3-(hydroxymethyl)pyrazin-2-yl)-4-methylpiperidin-4-yl)methyl)carbamate (Intermediate 50) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 46. (2S,4R)-1-((S)-2-acetamido-3,3-dimethylbutanoyl)-N-(2-(((1r,4S)-4-(4-(3-((5-(4-(aminomethyl)-4-methylpiperidin-1-yl)pyrazin-2-yl)thio)-2-chlorophenyl)piperidine-1-carbonyl)cyclohexyl)oxy)-4-(4-methylthiazol-5-yl)benzyl)-4-hydroxypyrrolidine-2-carboxamide

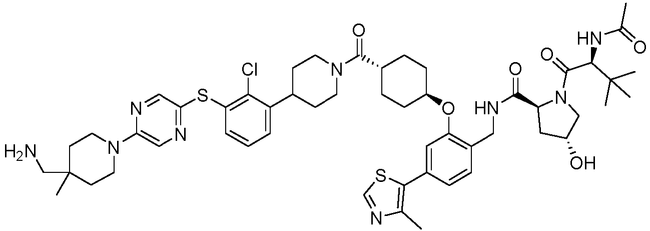

Example 46 was synthesized in the same way as Example 11, using tert-butyl ((1-(5-((2-chloro-3-(piperidin-4-yl)phenyl)thio)pyrazin-2-yl)-4-methylpiperidin-4-yl)methyl)carbamate (Intermediate 55) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 47. (2S,4R)-1-((S)-2-acetamido-3,3-dimethylbutanoyl)-N-(2-(((1r,4S)-4-(4-(4-((5-(4-(aminomethyl)-4-methylpiperidin-1-yl)pyrazin-2-yl)thio)-3-chloropyridin-2-yl)piperidine-1-carbonyl)cyclohexyl)oxy)-4-(4-methylthiazol-5-yl)benzyl)-4-hydroxypyrrolidine-2-carboxamide

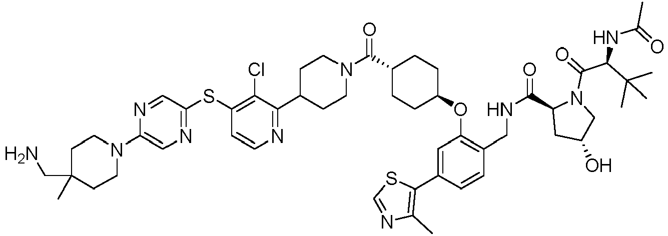

Example 47 was synthesized in the same way as Example 11, using tert-butyl ((1-(5-((3-chloro-2-(piperidin-4-yl)pyridin-4-yl)thio)pyrazin-2-yl)-4-methylpiperidin-4-yl)methyl)carbamate (Intermediate 56) and (1S,4R)-4-(2-(((2S,4R)-1-((S)-2-acetamido-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 66) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 48. (2S,4R)-1-((S)-2-acetamido-3,3-dimethylbutanoyl)-N-((S)-1-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)phenyl)ethyl)-4-hydroxypyrrolidine-2-carboxamide

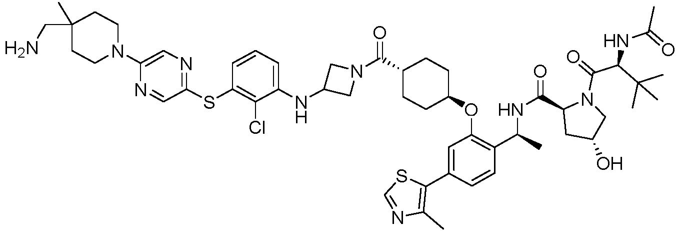

Example 48 was synthesized in the same way as Example 11, using (1S,4R)-4-(2-((S)-1-((2S,4R)-1-((S)-2-acetamido-3,3-dimethylbutanoyl)-4-hydroxypyrrolidine-2-carboxamido)ethyl)-5-(4-methylthiazol-5-yl)phenoxy)cyclohexane-1-carboxylic acid (Intermediate 67) instead of (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65).

### Example 49. (2S,4R)-1-((S)-2-acetamido-3,3-dimethylbutanoyl)-N-((S)-1-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)phenyl)ethyl)-4-hydroxypyrrolidine-2-carboxamide

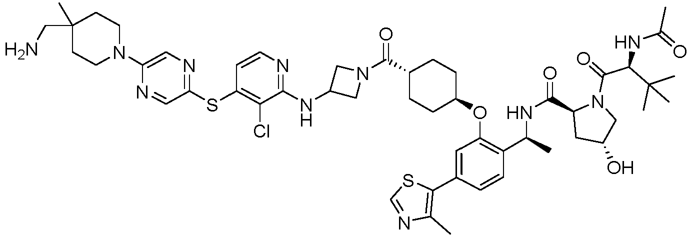

Example 49 was synthesized in the same way as Example 11, using tert-butyl ((1-(5-((2-(azetidin-3-ylamino)-3-chloropyridin-4-yl)thio)pyrazin-2-yl)-4-methylpiperidin-4-yl)methyl)carbamate (Intermediate 28) and (1S,4R)-4-(2-((S)-1-((2S,4R)-1-((S)-2-acetamido-3,3-dimethylbutanoyl)-4-hydroxypyrrolidine-2-carboxamido)ethyl)-5-(4-methylthiazol-5-yl)phenoxy)cyclohexane-1-carboxylic acid (Intermediate 67) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-Fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 50. (2S,4R)-N-(2-(2-(4-(3-((3-((5-(4-(aminomethyl)-4-methylpiperidin-1-yl)pyrazin-2-yl)thio)-2-chlorophenyl)amino)azetidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

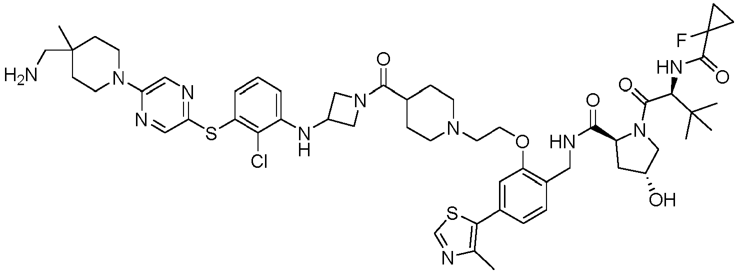

Example 50 was synthesized in the same way as Example 11, using 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylic acid (Intermediate 70) instead of (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65).

### Example 51. (2S,4R)-N-(2-(2-(4-(4-((3-((5-(4-(aminomethyl)-4-methylpiperidin-1-yl)pyrazin-2-yl)thio)-2-chlorophenyl)amino)piperidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

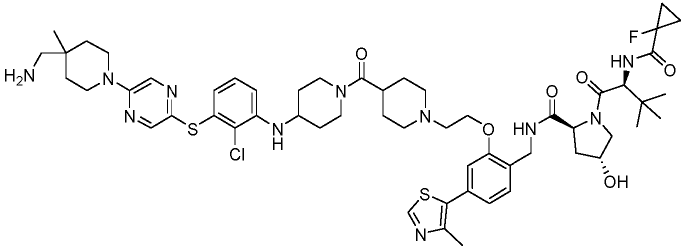

Example 51 was synthesized in the same way as Example 11, using tert-butyl ((1-(5-((2-chloro-3-(piperidin-4-ylamino)phenyl)thio)pyrazin-2-yl)-4-methylpiperidin-4-yl)methyl)carbamate (Intermediate 17) and 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylic acid (Intermediate 70) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 52. (2S,4R)-N-(2-(2-(4-(4-(4-((5-(4-(aminomethyl)-4-methylpiperidin-1-yl)pyrazin-2-yl)thio)-3-chloropyridin-2-yl)piperazine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

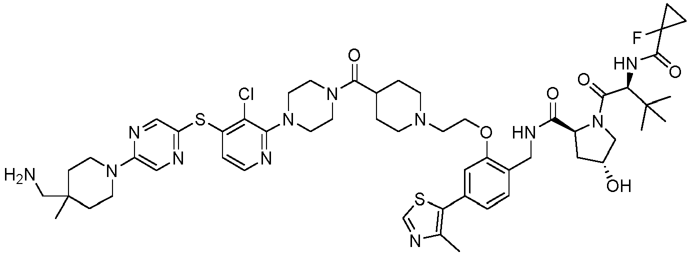

Example 52 was synthesized in the same way as Example 11, using tert-butyl ((1-(5-((3-chloro-2-(piperazin-1-yl)pyridin-4-yl)thio)pyrazin-2-yl)-4-methylpiperidin-4-yl)methyl)carbamate (Intermediate 34) and 1-(2-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)ethyl)piperidine-4-carboxylic acid (Intermediate 70) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 53. (2S,4R)-N-(2-(2-(4-(1-(4-((5-(4-(aminomethyl)-4-methylpiperidin-1-yl)pyrazin-2-yl)thio)-3-chloropyridin-2-yl)azetidine-3-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

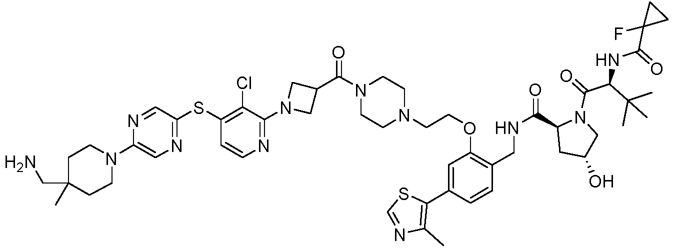

Example 53 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)azetidine-3-carboxylic acid (Intermediate 39) and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (Intermediate 71) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 54. (2S,4R)-N-(2-(2-(4-(1-(4-((5-(4-(aminomethyl)-4-methylpiperidin-1-yl)pyrazin-2-yl)thio)-3-chloropyridin-2-yl)piperidine-4-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

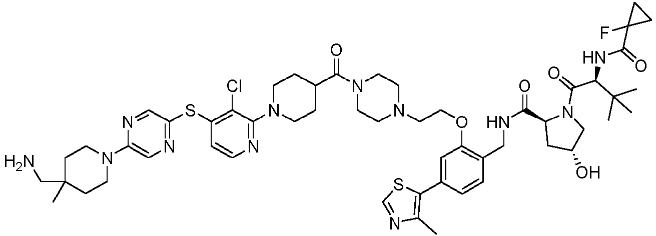

Example 54 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperazin-1-yl)ethoxy)benzyl)pyrrolidine-2-carboxamide (Intermediate 71) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 55. (2S,4R)-N-(2-(2-(1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

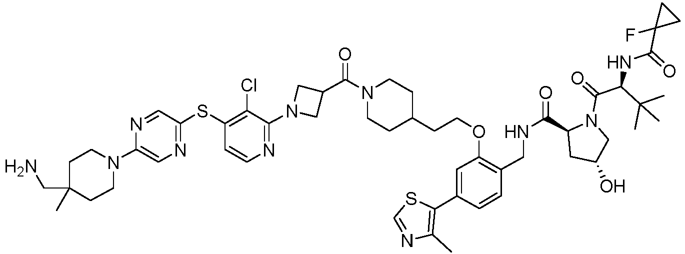

Example 55 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)azetidine-3-carboxylic acid (Intermediate 39) and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide (Intermediate 72) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 56. (2S,4R)-N-(2-(2-(1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

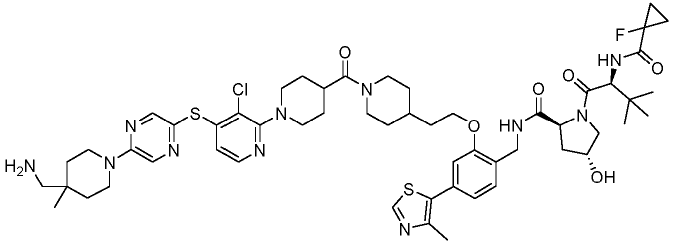

Example 56 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-(4-(4-methylthiazol-5-yl)-2-(2-(piperidin-4-yl)ethoxy)benzyl)pyrrolidine-2-carboxamide (Intermediate 72) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 57. (2S,4R)-N-(2-((7-(1-(4-((5-(4-(aminomethyl)-4-methylpiperidin-1-yl)pyrazin-2-yl)thio)-3-chloropyridin-2-yl)piperidine-4-carbonyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

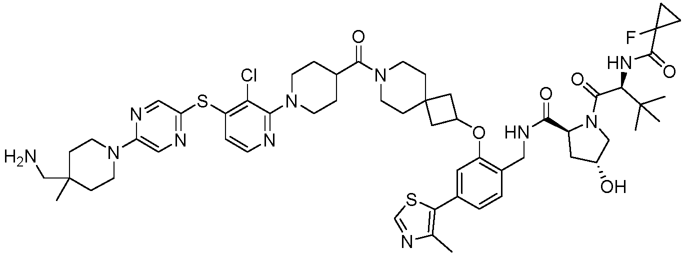

Example 57 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-N-(2-((7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 73) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 58. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

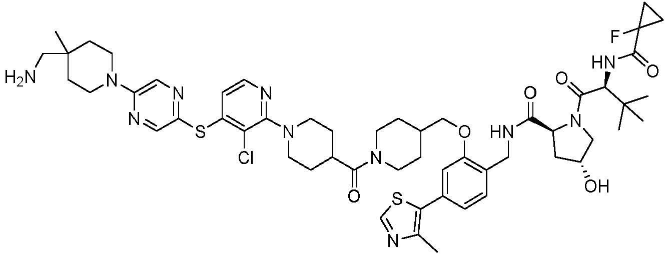

Example 58 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-ylmethoxy)benzyl)pyrrolidine-2-carboxamide (Intermediate 74) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 59. (2S,4R)-N-(2-((1-((1s,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

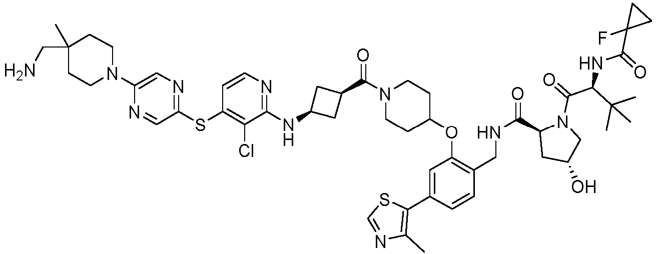

Example 59 was synthesized in the same way as Example 11, using (1S,3S)-3-((4-((5-(4-(((tert-butoxycarbonyl)amino)methyl)-4-methylpiperidin-1-yl)pyrazin-2-yl)thio)-3-chloropyridin-2-yl)amino)cyclobutene-1-carboxylic acid (Intermediate 35) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 60. (2S,4R)-N-(2-((1-((1S,3R)-3-((4-((5-(4-amino-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

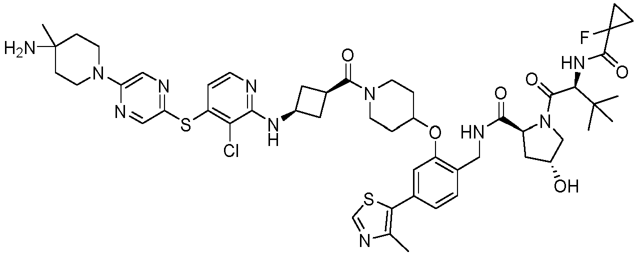

Example 60 was synthesized in the same way as Example 11, using (1S,3S)-3-((4-((5-(4-((tert-butoxycarbonyl)amino)-4-methylpiperidin-1-yl)pyrazin-2-yl)thio)-3-chloropyridin-2-yl)amino)cyclobutane-1-carboxylic acid (Intermediate 36) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 61. (2S,4R)-N-(2-((1-((1r,3S)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

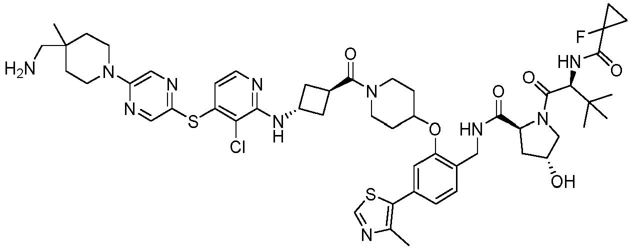

Example 61 was synthesized in the same way as Example 11, using (1R,3R)-3-((4-((5-(4-(((tert-butoxycarbonyl)amino)methyl)-4-methylpiperidin-1-yl)pyrazin-2-yl)thio)-3-chloropyridin-2-yl)amino)cyclobutene-1-carboxylic acid (Intermediate 37) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 62. (2S,4R)-N-(2-((1-((1R,4S)-4-((4-((5-(4-(aminomethyl)-4-methylpiperidin-1-yl)pyrazin-2-yl)thio)-3-chloropyridin-2-yl)amino)cyclohexanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

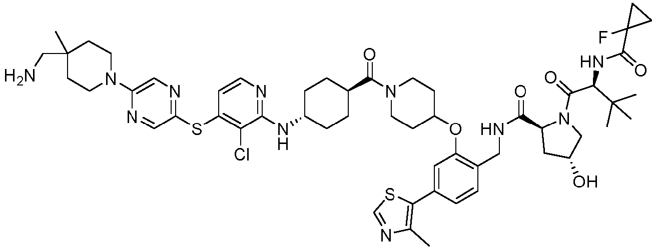

Example 62 was synthesized in the same way as Example 11, using (1R,4R)-4-((4-((5-(4-(((tert-butoxycarbonyl)amino)methyl)-4-methylpiperidin-1-yl)pyrazin-2-yl)thio)-3-chloropyridin-2-yl)amino)cyclohexane-1-carboxylic acid (Intermediate 38) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 63. (2S,4R)-N-(2-((1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

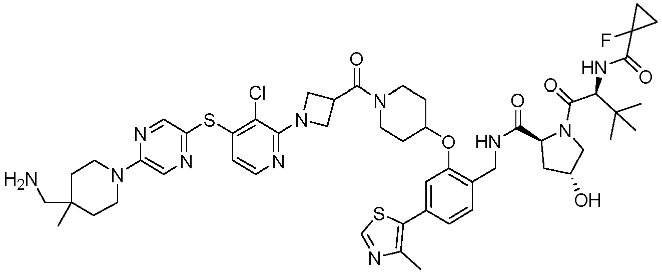

Example 63 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)azetidine-3-carboxylic acid (Intermediate 39) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 64. (2S,4R)-N-(2-((1-(2-(1-(4-((5-(4-(aminomethyl)-4-methylpiperidin-1-yl)pyrazin-2-yl)thio)-3-chloropyridin-2-yl)azetidin-3-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

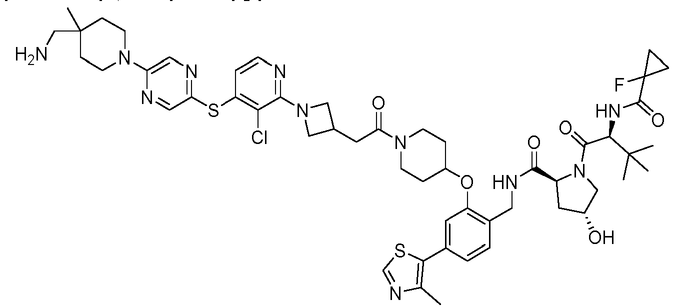

Example 64 was synthesized in the same way as Example 11, using 2-(1-(4-((5-(4-(((tert-butoxycarbonyl)amino)methyl)-4-methylpiperidin-1-yl)pyrazin-2-yl)thio)-3-chloropyridin-2-yl)azetidin-3-yl)acetic acid (Intermediate 40) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 65. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

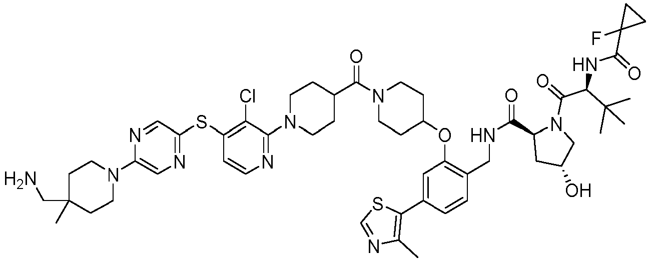

Example 65 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 66. (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

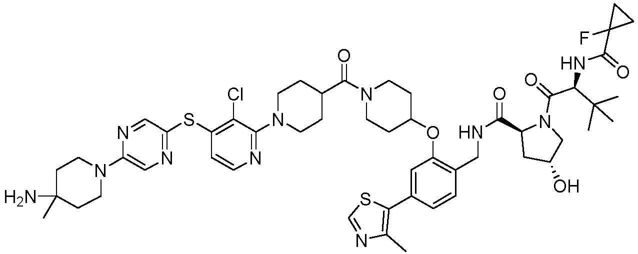

Example 66 was synthesized in the same way as Example 11, using 1-(4-((5-(4-((tert-butoxycarbonyl)amino)-4-methylpiperidin-1-yl)pyrazin-2-yl)thio)-3-chloropyridin-2-yl)piperidine-4-carboxylic acid (Intermediate 42) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 67. (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

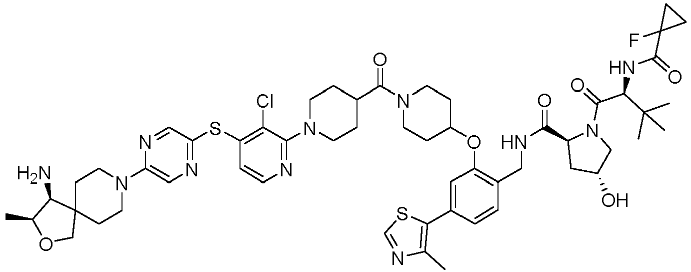

Example 67 was synthesized in the same way as Example 11, using 1-(4-((5-((3S,4S)-4-((tert-butoxycarbonyl)amino)-3-methyl-2-oxa-8-azaspiro[4.5]decan-8-yl)pyrazin-2-yl)thio)-3-chloropyridin-2-yl)piperidine-4-carboxylic acid (Intermediate 43) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 68. (2S,4R)-N-(2-((1-(1-(4-((5-(4-(aminomethyl)-4-methylpiperidin-1-yl)pyrazin-2-yl)thio)-3-chloropyridin-2-yl)-4-methylpiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

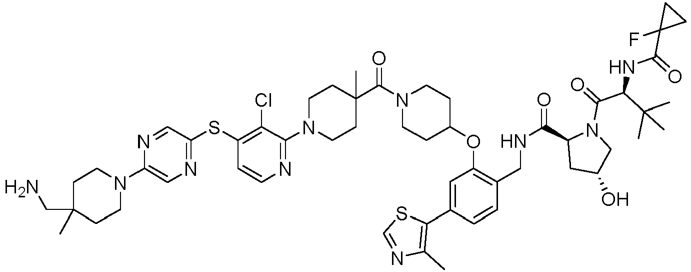

Example 68 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)-4-methylpiperidine-4-carboxylic acid (Intermediate 44) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 69. (2S,4R)-N-(2-((1-(1-(4-((5-(4-(aminomethyl)-4-methylpiperidin-1-yl)pyrazin-2-yl)thio)-3-chloropyridin-2-yl)-4-fluoropiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

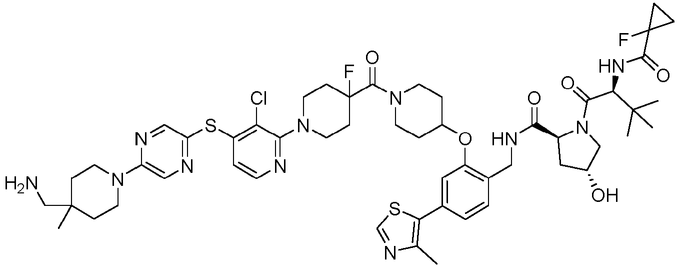

Example 69 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)-4-fluoropiperidine-4-carboxylic acid (Intermediate 45) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 70. (2S,4R)-N-(2-((1-(1-(4-((5-(4-(aminomethyl)-4-methylpiperidin-1-yl)pyrazin-2-yl)thio)-3-chloropyridin-2-yl)-4-hydroxypiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

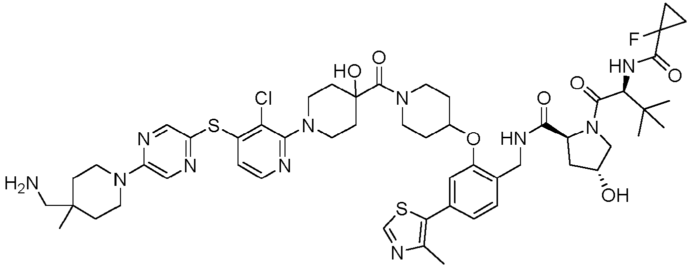

Example 70 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)-4-hydroxypiperidine-4-carboxylic acid (Intermediate 46) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 71. (2S,4R)-N-(2-((1-(2-(1-(4-((5-(4-(aminomethyl)-4-methylpiperidin-1-yl)pyrazin-2-yl)thio)-3-chloropyridin-2-yl)piperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

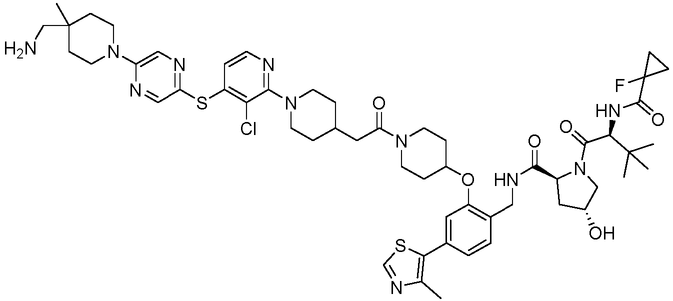

Example 71 was synthesized in the same way as Example 11, using 2-(1-(4-((5-(4-(((tert-butoxycarbonyl)amino)methyl)-4-methylpiperidin-1-yl)pyrazin-2-yl)thio)-3-chloropyridin-2-yl)piperidin-4-yl)acetic acid (Intermediate 47) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 72. (2S,4R)-N-(2-((1-(2-(1-(4-((5-(4-(aminomethyl)-4-methylpiperidin-1-yl)pyrazin-2-yl)thio)-3-chloropyridin-2-yl)-4-hydroxypiperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

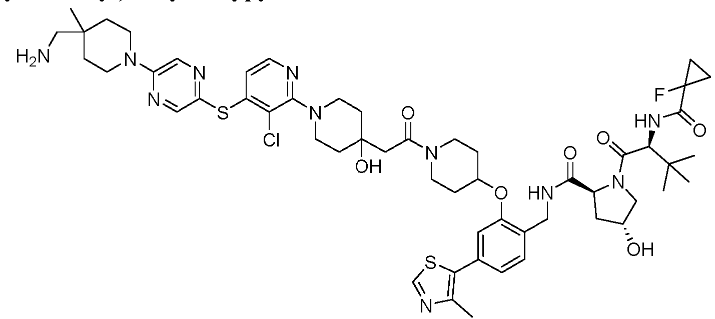

Example 72 was synthesized in the same way as Example 11, using 2-(1-(4-((5-(4-(((tert-butoxycarbonyl)amino)methyl)-4-methylpiperidin-1-yl)pyrazin-2-yl)thio)-3-chloropyridin-2-yl)-4-hydroxypiperidin-4-yl)acetic acid (Intermediate 48) and (2S,4R)-1-((S)-2-(1-fluorocyclopropane-1-carboxamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 75) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 73. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)azetidin-3-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide

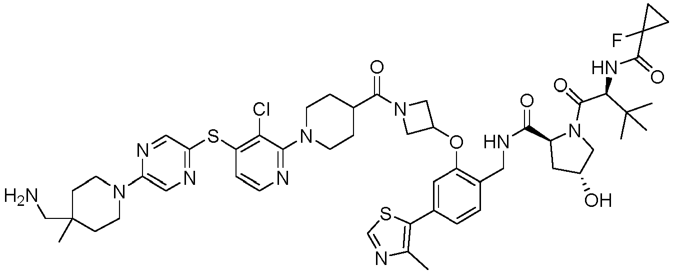

Example 73 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-N-(2-(azetidin-3-yloxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide (Intermediate 76) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 74. (2S,4R)-1-((S)-2-acetamido-3,3-dimethylbutanoyl)-N-(2-((1-((1S,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-4-hydroxypyrrolidine-2-carboxamide

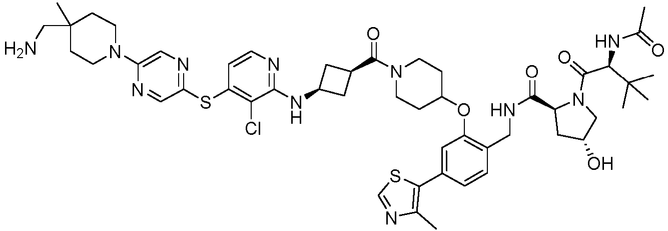

Example 74 was synthesized in the same way as Example 11, using (1S,3S)-3-((4-((5-(4-(((tert-butoxycarbonyl)amino)methyl)-4-methylpiperidin-1-yl)pyrazin-2-yl)thio)-3-chloropyridin-2-yl)amino)cyclobutane-1-carboxylic acid (Intermediate 35) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 75. (2S,4R)-1-((S)-2-acetamido-3,3-dimethylbutanoyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

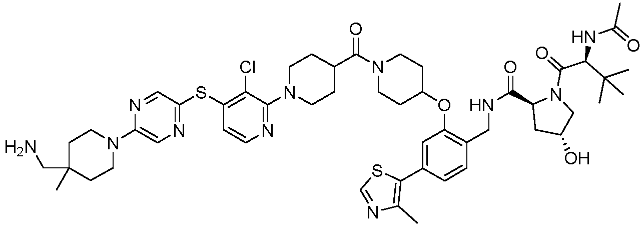

Example 75 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 76. (2S,4R)-1-((S)-2-acetamido-3,3-dimethylbutanoyl)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

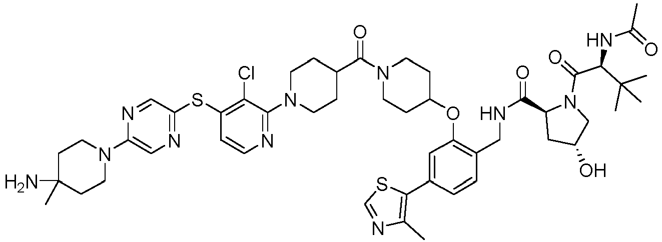

Example 76 was synthesized in the same way as Example 11, using 1-(4-((5-(4-((tert-butoxycarbonyl)amino)-4-methylpiperidin-1-yl)pyrazin-2-yl)thio)-3-chloropyridin-2-yl)piperidine-4-carboxylic acid (Intermediate 42) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 77. (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

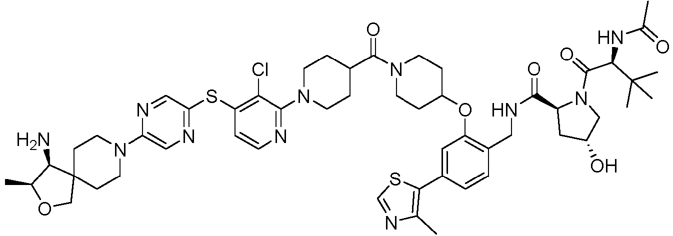

Example 77 was synthesized in the same way as Example 11, using 1-(4-((5-((3S,4S)-4-((tert-butoxycarbonyl)amino)-3-methyl-2-oxa-8-azaspiro[4.5]decan-8-yl)pyrazin-2-yl)thio)-3-chloropyridin-2-yl)piperidine-4-carboxylic acid (Intermediate 43) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 78. (2S,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

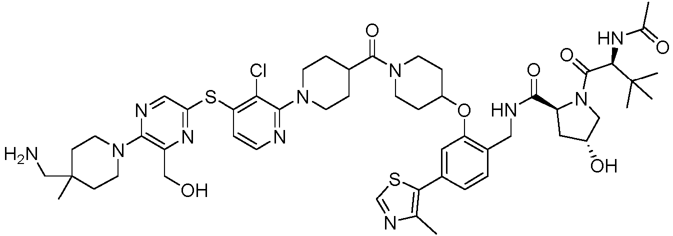

Example 78 was synthesized in the same way as Example 11, using 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid (Intermediate 51) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 79. (2S,4R)-1-((S)-2-acetamido-3,3-dimethylbutanoyl)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

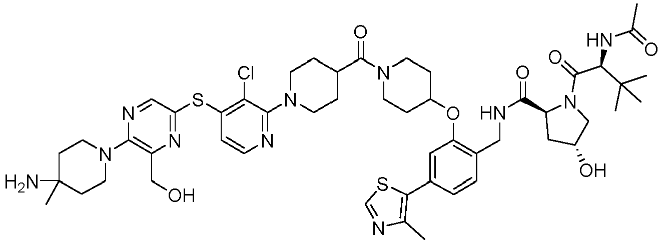

Example 79 was synthesized in the same way as Example 11, using 1-(4-((5-(4-((tert-butoxycarbonyl)amino)-4-methylpiperidin-1-yl)-6-(hydroxymethyl)pyrazin-2-yl)thio)-3-chloropyridin-2-yl)piperidine-4-carboxylic acid (Intermediate 52) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 80. (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

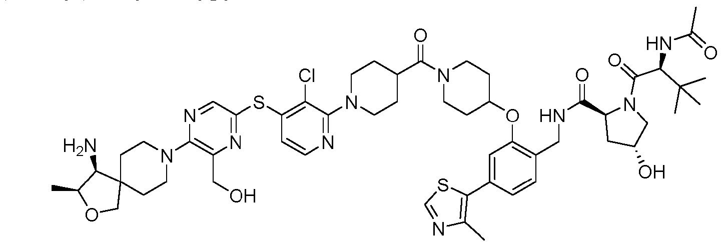

Example 80 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 81. (2S,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)-3-methylpyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

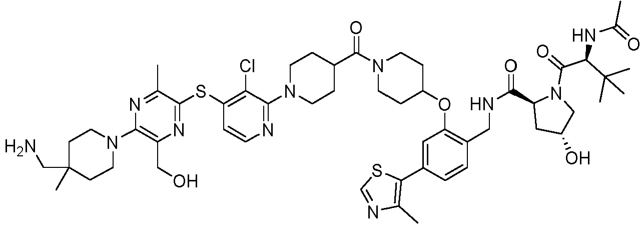

Example 81 was synthesized in the same way as Example 11, using 1-(4-((5-(4-(((tert-butoxycarbonyl)amino)methyl)-4-methylpiperidin-1-yl)-6-(hydroxymethyl)-3-methylpyrazin-2-yl)thio)-3-chloropyridin-2-yl)piperidine-4-carboxylic acid (Intermediate 54) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 82. (2S,4R)-1-((S)-2-acetamido-3,3-dimethylbutanoyl)-N-(2-((1-(1-(3-((5-(4-(aminomethyl)-4-methylpiperidin-1-yl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide

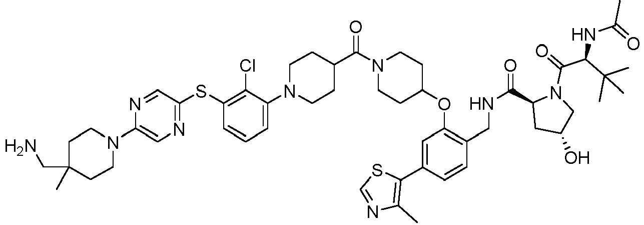

Example 82 was synthesized in the same way as Example 11, using 1-(3-((5-(4-(((tert-butoxycarbonyl)amino)methyl)-4-methylpiperidin-1-yl)pyrazin-2-yl)thio)-2-chlorophenyl)piperidine-4-carboxylic acid (Intermediate 57) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 83. (2S,4R)-1-((S)-2-acetamido-3,3-dimethylbutanoyl)-N-(2-((1-(1-(3-((5-(4-(aminomethyl)-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide

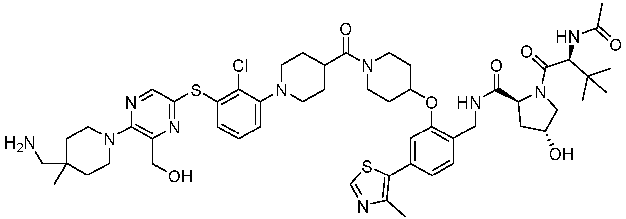

Example 83 was synthesized in the same way as Example 11, using 1-(3-((5-(4-(((tert-butoxycarbonyl)amino)methyl)-4-methylpiperidin-1-yl)-6-(hydroxymethyl)pyrazin-2-yl)thio)-2-chlorophenyl)piperidine-4-carboxylic acid (Intermediate 58) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 84. (2S,4R)-1-((S)-2-acetamido-3,3-dimethylbutanoyl)-N-(2-((1-(1-(3-((5-(4-amino-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide

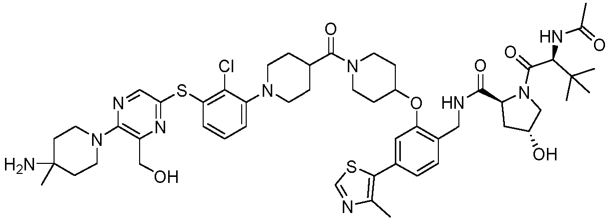

Example 84 was synthesized in the same way as Example 11, using 1-(3-((5-(4-((tert-butoxycarbonyl)amino)-4-methylpiperidin-1-yl)-6-(hydroxymethyl)pyrazin-2-yl)thio)-2-chlorophenyl)piperidine-4-carboxylic acid (Intermediate 59) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 85. (2S,4R)-1-((S)-2-acetamido-3,3-dimethylbutanoyl)-N-(2-((1-(1-(3-((5-((3S,4S)-4-amino-3-methyl-2-oxa-8-azaspiro[4.5]decan-8-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide

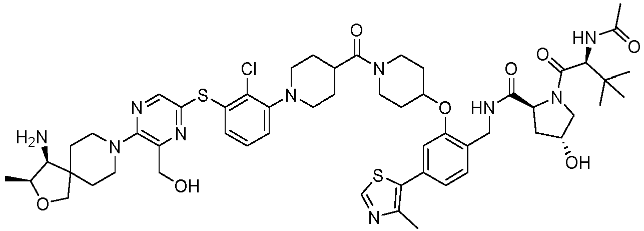

Example 85 was synthesized in the same way as Example 11, using 1-(3-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-2-chlorophenyl)piperidine-4-carboxylic acid (Intermediate 60) and (2S,4R)-1-((S)-2-acetamido-3,3-dimethylbutanoyl)-4-hydroxy-N-(4-(4-methylthiazol-5-yl)-2-(piperidin-4-yloxy)benzyl)pyrrolidine-2-carboxamide (Intermediate 77) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 86. (2S,4R)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-1-((S)-2-(1-fluorocyclopropane-1-carboxamido)-3,3-dimethylbutanoyl)-4-hydroxypyrrolidine-2-carboxamide

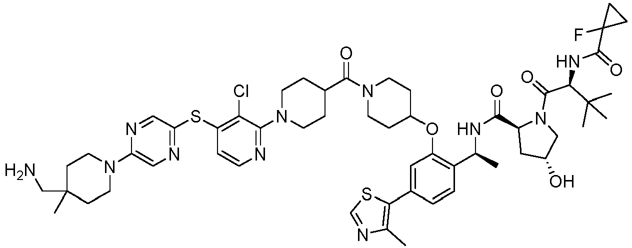

Example 86 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(1-fluorocyclopropane-1-carboxamido)-3,3-dimethylbutanoyl)-4-hydroxy-N-((S)-1-(4-(4-methylthiazol-5-yl)-2-(piperidin-4-yloxy)phenyl)ethyl)pyrrolidine-2-carboxamide (Intermediate 78) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 87. (2S,4R)-1-((S)-2-acetamido-3,3-dimethylbutanoyl)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-4-hydroxypyrrolidine-2-carboxamide

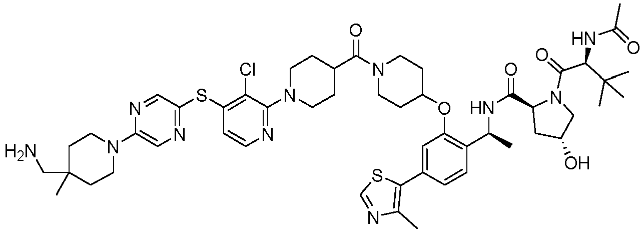

Example 87 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-acetamido-3,3-dimethylbutanoyl)-4-hydroxy-N-((S)-1-(4-(4-methylthiazol-5-yl)-2-(piperidin-4-yloxy)phenyl)ethyl)pyrrolidine-2-carboxamide (Intermediate 79) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 88. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(2-cyanoacetamido)-3,3-dimethylbutanoyl)-4-hydroxypyrrolidine-2-carboxamide

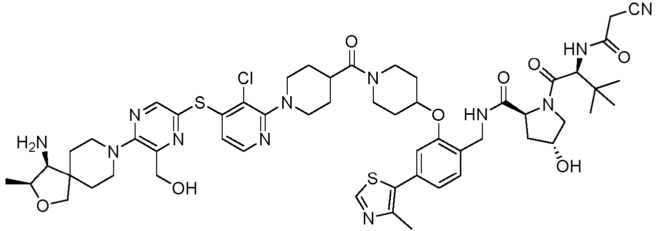

Example 88 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-1-((S)-2-(2-cyanoacetamido)-3,3-dimethylbutanoyl)-4-hydroxy-N-(4-(4-methylthiazol-5-yl)-2-(piperidin-4-yloxy)benzyl)pyrrolidine-2-carboxamide (Intermediate 80) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 89. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-2-(2-hydroxyacetamido)-3,3-dimethylbutanoyl)pyrrolidine-2-carboxamide

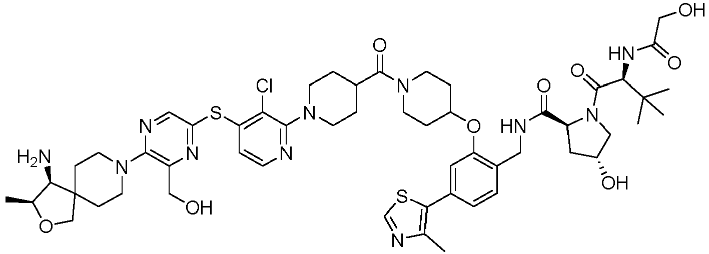

Example 89 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-4-hydroxy-1-((S)-2-(2-hydroxyacetamido)-3,3-dimethylbutanoyl)-N-(4-(4-methylthiazol-5-yl)-2-(piperidin-4-yloxy)benzyl)pyrrolidine-2-carboxamide (Intermediate 81) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 90. (2S,4R)-1-(acetyl-L-valyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

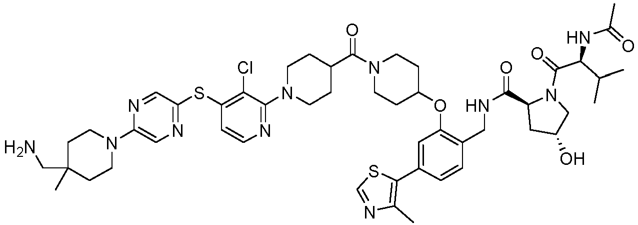

Example 90 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-acetamido-3-methylbutanoyl)-4-hydroxy-N-(4-(4-methylthiazol-5-yl)-2-(piperidin-4-yloxy)benzyl)pyrrolidine-2-carboxamide (Intermediate 82) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 91. (2S,4R)-1-((S)-2-acetamido-2-cyclopropylacetyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide

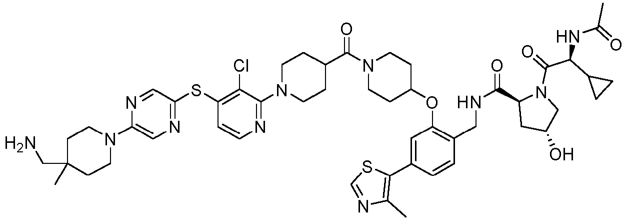

Example 91 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-acetamido-2-cyclopropylacetyl)-4-hydroxy-N-(4-(4-methylthiazol-5-yl)-2-(piperidin-4-yloxy)benzyl)pyrrolidine-2-carboxamide (Intermediate 83) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 92. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P1)

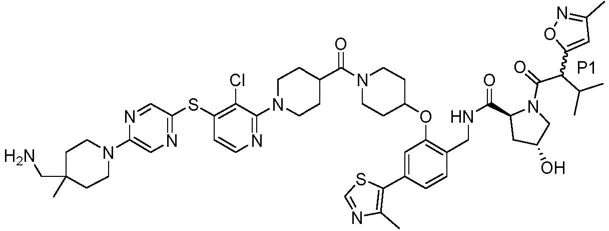

Example 92 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P1) (Intermediate 84) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 93. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

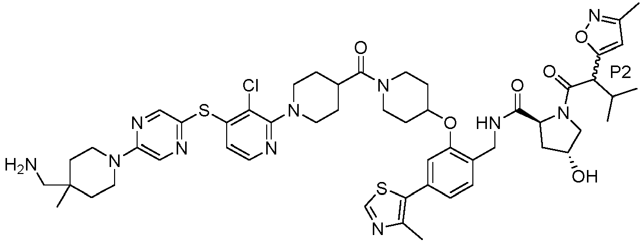

Example 93 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 94. (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

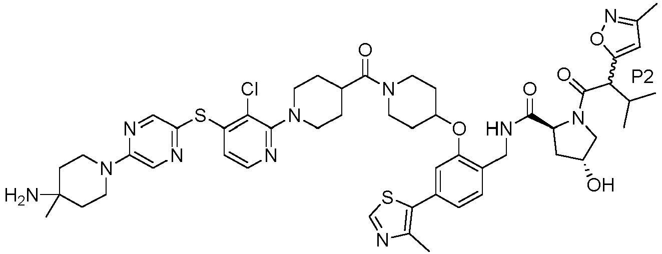

Example 94 was synthesized in the same way as Example 11, using 1-(4-((5-(4-((tert-butoxycarbonyl)amino)-4-methylpiperidin-1-yl)pyrazin-2-yl)thio)-3-chloropyridin-2-yl)piperidine-4-carboxylic acid (Intermediate 42) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 95. (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

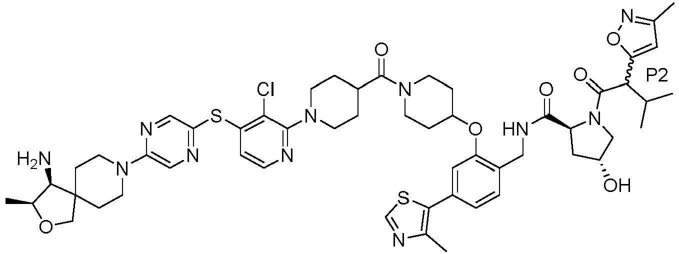

Example 95 was synthesized in the same way as Example 11, using 1-(4-((5-((3S,4S)-4-((tert-butoxycarbonyl)amino)-3-methyl-2-oxa-8-azaspiro[4.5]decan-8-yl)pyrazin-2-yl)thio)-3-chloropyridin-2-yl)piperidine-4-carboxylic acid (Intermediate 43) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 96. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

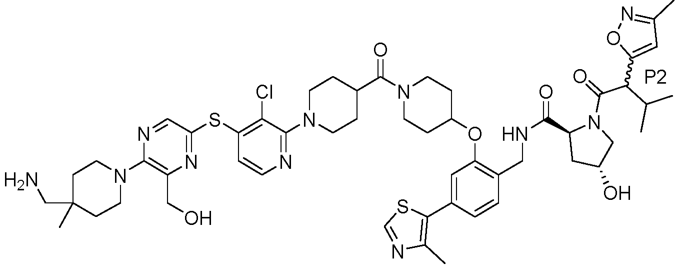

Example 96 was synthesized in the same way as Example 11, using 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid (Intermediate 51) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 97. (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

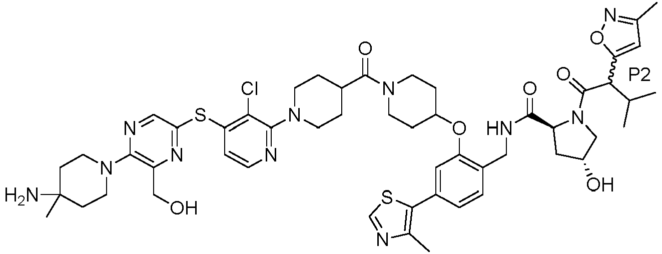

Example 97 was synthesized in the same way as Example 11, using 1-(4-((5-(4-((tert-butoxycarbonyl)amino)-4-methylpiperidin-1-yl)-6-(hydroxymethyl)pyrazin-2-yl)thio)-3-chloropyridin-2-yl)piperidine-4-carboxylic acid (Intermediate 52) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 98. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

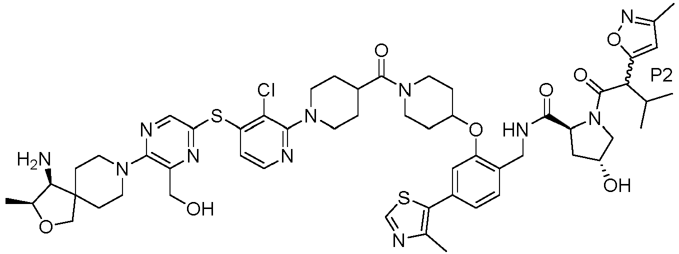

Example 98 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-4-hydroxy-1-(3-methyl-2-(3-methylisoxazol-5-yl)butanoyl)-N-(4-(4-methylthiazol-5-yl)-2-(piperidin-4-yloxy)benzyl)pyrrolidine-2-carboxamide (P2) (Intermediate 85) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 99. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(4-methyl-1H-pyrazol-1-yl)butanoyl)pyrrolidine-2-carboxamide (P1)

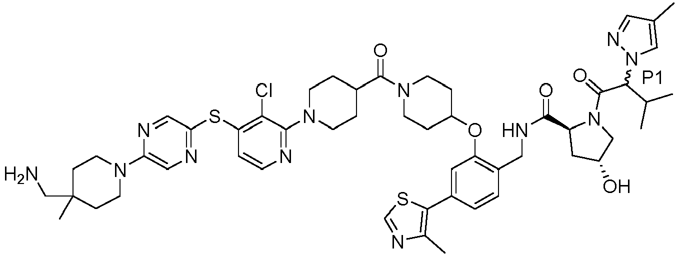

Example 99 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-4-hydroxy-1-(3-methyl-2-(4-methyl-1H-pyrazol-1-yl)butanoyl)-N-(4-(4-methylthiazol-5-yl)-2-(piperidin-4-yloxy)benzyl)pyrrolidine-2-carboxamide (P1) (Intermediate 86) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 100. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(4-methyl-1H-pyrazol-1-yl)butanoyl)pyrrolidine-2-carboxamide (P2)

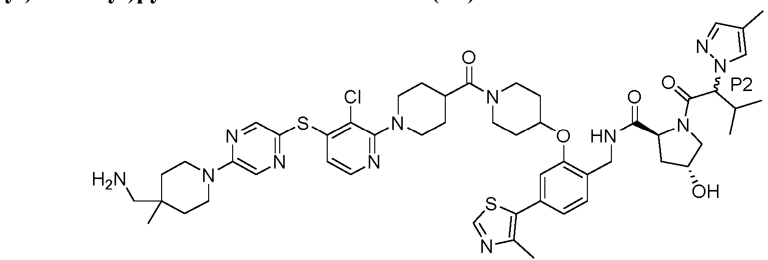

Example 100 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-4-hydroxy-1-(3-methyl-2-(4-methyl-1H-pyrazol-1-yl)butanoyl)-N-(4-(4-methylthiazol-5-yl)-2-(piperidin-4-yloxy)benzyl)pyrrolidine-2-carboxamide (P2) (Intermediate 87) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 101. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

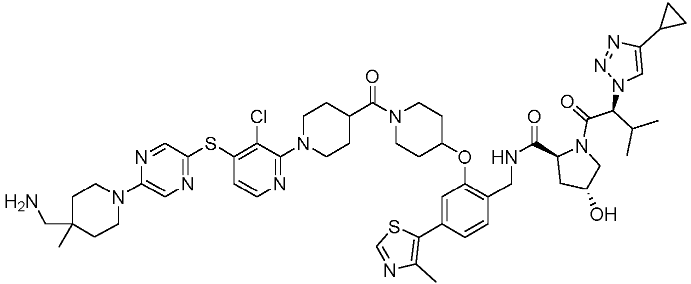

Example 101 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 102. (2S,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

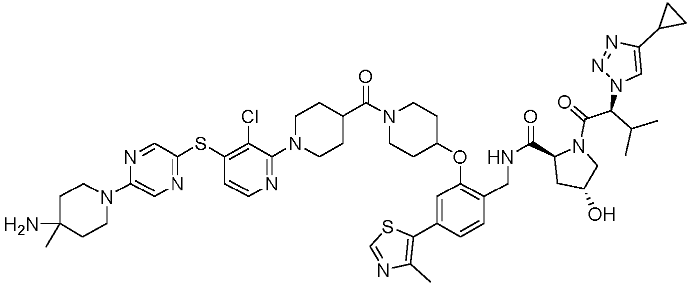

Example 102 was synthesized in the same way as Example 11, using 1-(4-((5-(4-((tert-butoxycarbonyl)amino)-4-methylpiperidin-1-yl)pyrazin-2-yl)thio)-3-chloropyridin-2-yl)piperidine-4-carboxylic acid (Intermediate 42) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 103. (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

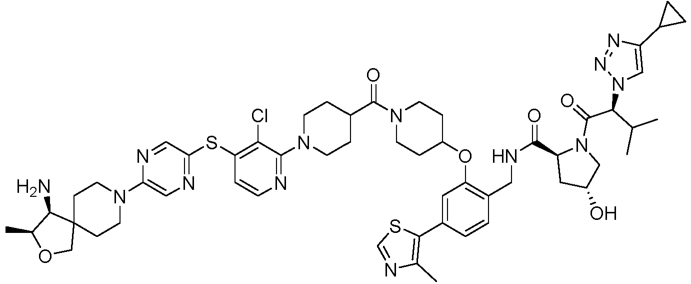

Example 103 was synthesized in the same way as Example 11, using 1-(4-((5-((3S,4S)-4-((tert-butoxycarbonyl)amino)-3-methyl-2-oxa-8-azaspiro[4.5]decan-8-yl)pyrazin-2-yl)thio)-3-chloropyridin-2-yl)piperidine-4-carboxylic acid (Intermediate 43) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 104. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

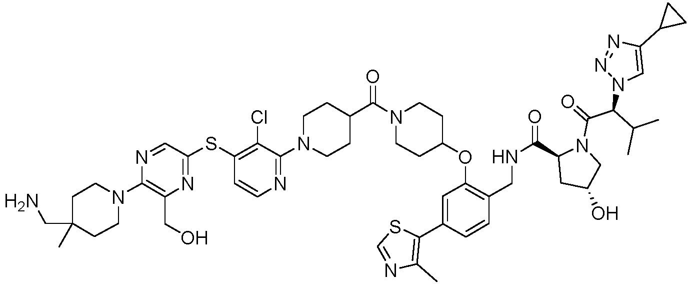

Example 104 was synthesized in the same way as Example 11, using 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid (Intermediate 51) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 105. (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

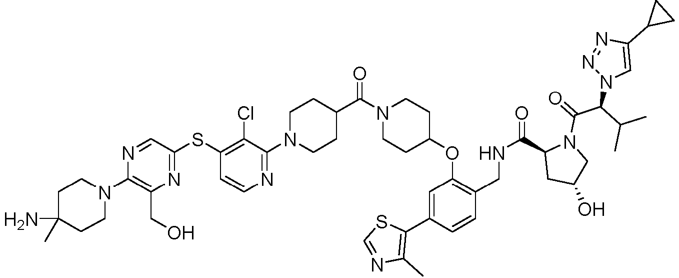

Example 105 was synthesized in the same way as Example 11, using 1-(4-((5-(4-((tert-butoxycarbonyl)amino)-4-methylpiperidin-1-yl)-6-(hydroxymethyl)pyrazin-2-yl)thio)-3-chloropyridin-2-yl)piperidine-4-carboxylic acid (Intermediate 52) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 106. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide

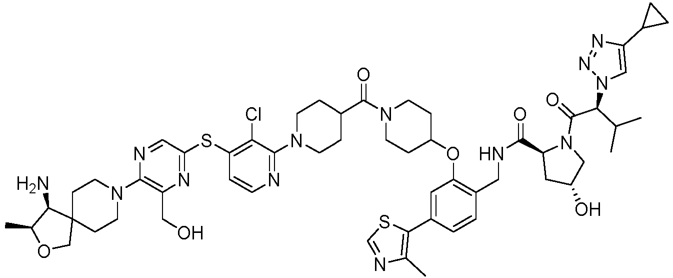

Example 106 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-1-((S)-2-(4-cyclopropyl-1H-1,2,3-triazol-1-yl)-3-methylbutanoyl)-4-hydroxy-N-(4-(4-methylthiazol-5-yl)-2-(piperidin-4-yloxy)benzyl)pyrrolidine-2-carboxamide (Intermediate 88) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 107. (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3,3-dimethylbutanoyl)-4-hydroxypyrrolidine-2-carboxamide

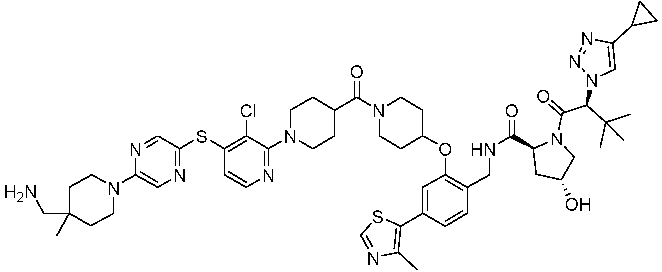

Example 107 was synthesized in the same way as Example 11, using 1-(4-((5-(4-(((tert-butoxycarbonyl)amino)methyl)-4-methylpiperidin-1-yl)pyrazin-2-yl)thio)-3-chloropyridin-2-yl)piperidine-4-carboxylic acid (Intermediate 41) and (2S,4R)-1-((S)-2-(4-cyclopropyl-1H-1,2,3-triazol-1-yl)-3,3-dimethylbutanoyl)-4-hydroxy-N-(4-(4-methylthiazol-5-yl)-2-(piperidin-4-yloxy)benzyl)pyrrolidine-2-carboxamide (Intermediate 89) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 108. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide

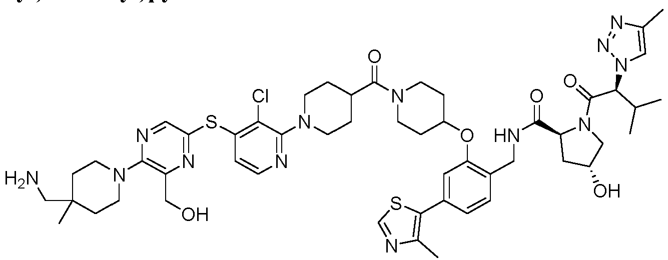

Example 108 was synthesized in the same way as Example 11, using 1-(4-((5-(4-(((tert-butoxycarbonyl)amino)methyl)-4-methylpiperidin-1-yl)-6-(hydroxymethyl)pyrazin-2-yl)thio)-3-chloropyridin-2-yl)piperidine-4-carboxylic acid (Intermediate 51) and (2S,4R)-4-hydroxy-1-((S)-3-methyl-2-(4-methyl-1H-1,2,3-triazol-1-yl)butanoyl)-N-(4-(4-methylthiazol-5-yl)-2-(piperidin-4-yloxy)benzyl)pyrrolidine-2-carboxamide (Intermediate 90) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 109. (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide

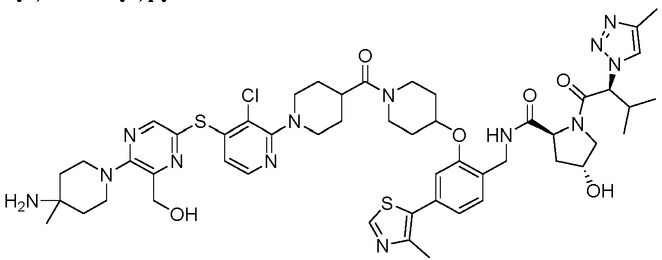

Example 109 was synthesized in the same way as Example 11, using 1-(4-((5-(4-((tert-butoxycarbonyl)amino)-4-methylpiperidin-1-yl)-6-(hydroxymethyl)pyrazin-2-yl)thio)-3-chloropyridin-2-yl)piperidine-4-carboxylic acid (Intermediate 52) and (2S,4R)-4-hydroxy-1-((S)-3-methyl-2-(4-methyl-1H-1,2,3-triazol-1-yl)butanoyl)-N-(4-(4-methylthiazol-5-yl)-2-(piperidin-4-yloxy)benzyl)pyrrolidine-2-carboxamide (Intermediate 90) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 110. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide

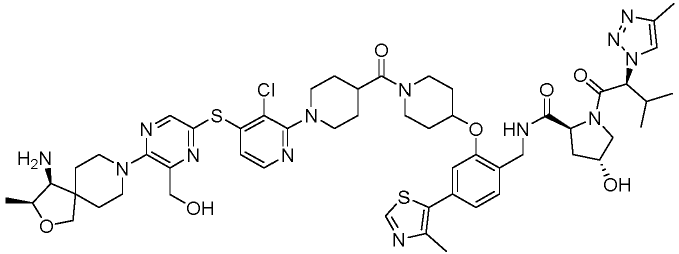

Example 110 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-4-hydroxy-1-((S)-3-methyl-2-(4-methyl-1H-1,2,3-triazol-1-yl)butanoyl)-N-(4-(4-methylthiazol-5-yl)-2-(piperidin-4-yloxy)benzyl)pyrrolidine-2-carboxamide (Intermediate 90) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 111. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide

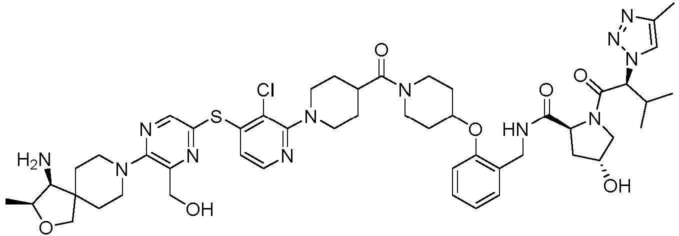

Example 111 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-4-hydroxy-1-((S)-3-methyl-2-(4-methyl-1H-1,2,3-triazol-1-yl)butanoyl)-N-(2-(piperidin-4-yloxy)benzyl)pyrrolidine-2-carboxamide (Intermediate 91) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 112. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide

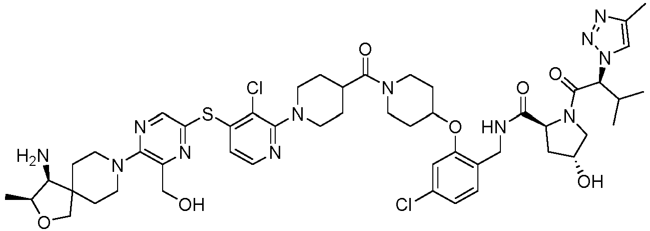

Example 112 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-N-(4-chloro-2-(piperidin-4-yloxy)benzyl)-4-hydroxy-1-((S)-3-methyl-2-(4-methyl-1H-1,2,3-triazol-1-yl)butanoyl)pyrrolidine-2-carboxamide (Intermediate 92) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 113. (2S,4R)-N-(2-((1-(1-(4-((5-((3S,4S)-4-amino-3-methyl-2-oxa-8-azaspiro [4.5] decan-8-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide

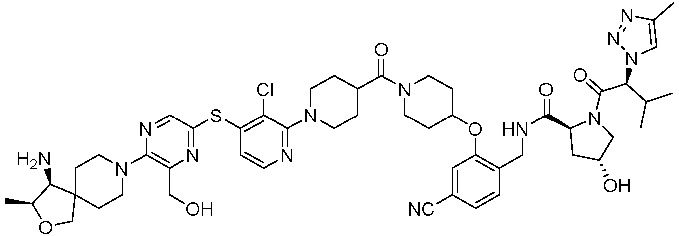

Example 113 was synthesized in the same way as Example 11, using 1-(4-((5-((3S,4S)-4-((tert-butoxycarbonyl)amino)-3-methyl-2-oxa-8-azaspiro[4.5]decan-8-yl)-6-(hydroxymethyl)pyrazin-2-yl)thio)-3-chloropyridin-2-yl)piperidine-4-carboxylic acid (Intermediate 53) and (2S,4R)-N-(4-cyano-2-(piperidin-4-yloxy)benzyl)-4-hydroxy-1-((S)-3-methyl-2-(4-methyl-1H-1,2,3-triazol-1-yl)butanoyl)pyrrolidine-2-carboxamide (Intermediate 93) instead of tert-butyl ((1-(5-((3-(azetidin-3-ylamino)-2-chlorophenyl)thio)pyrazin-2-yl)-4-methylpiperidin-4-yl)methyl)carbamate (Intermediate 11) and (1S,4R)-4-(2-(((2S,4R)-1-((S)-2-(1-fluorocyclopropane-1-carboxamido)-3,3-dimethylbutanoyl)-4-hydroxypyrrolidine-2-carboxamido)methyl)-5-(4-methylthiazol-5-yl)phenoxy)cyclohexane-1-carboxylic acid (Intermediate 65), respectively.

### Example 114. (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-(3-methyl-2-(3-methylisoxazol-5-yl)butanoyl)pyrrolidine-2-carboxamide (P1)

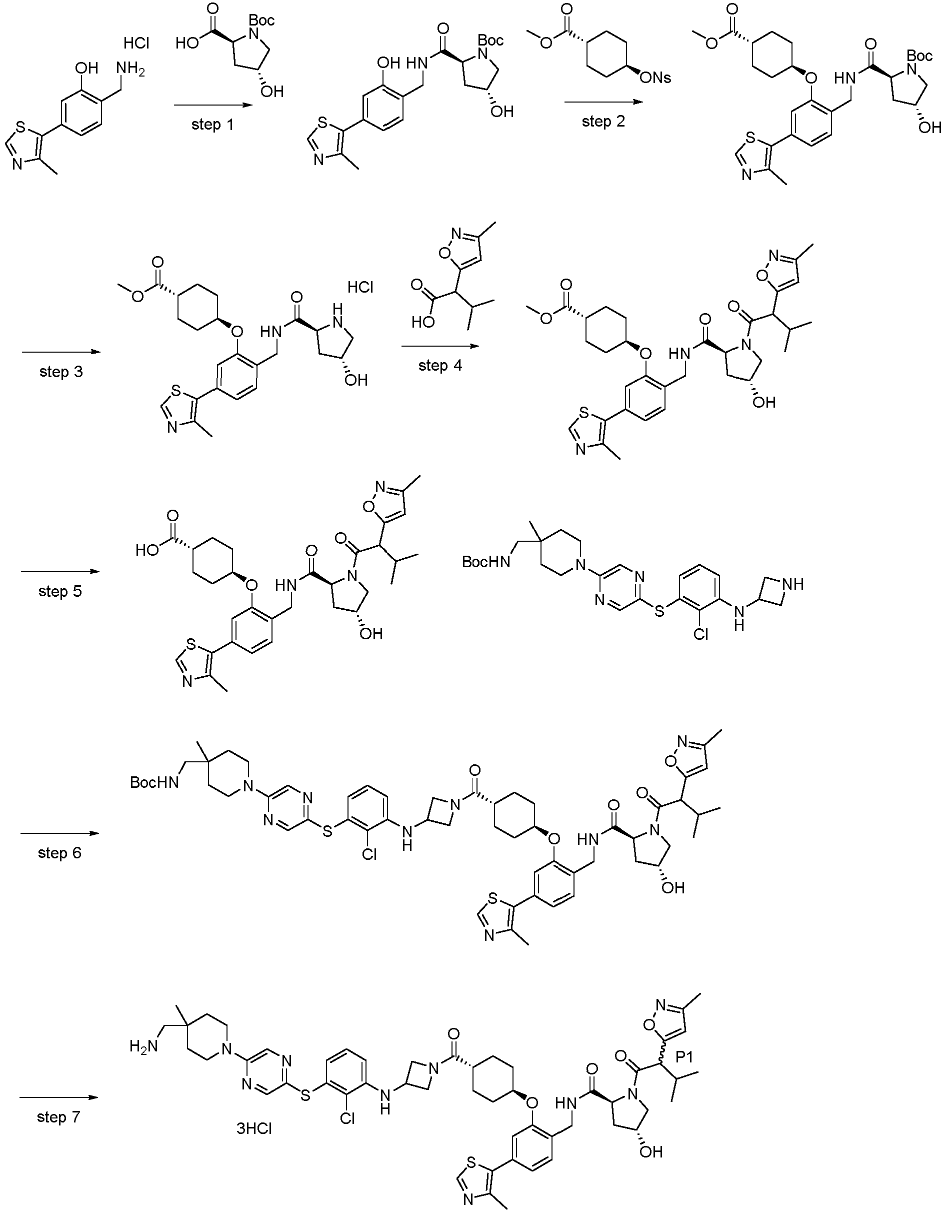

### Step 1: Synthesis of tert-butyl (2S,4R)-4-hydroxy-2-((2-hydroxy-4-(4-methylthiazol-5-yl)benzyl)carbamoyl)pyrrolidine-1-carboxylate

(2-(Aminomethyl)-5-(4-methylthiazol-5-yl)phenol hydrochloride (500 mg, 1.95 mmol), (2S,4R)-1-(tert-butoxycarbonyl)-4-hydroxypyrrolidine-2-carboxylic acid (450 mg, 1.95 mmol), EDCI (448 mg, 2.34 mmol), HOBt (316 mg, 2.24 mmol), and DIPEA (1.36 ml, 7.79 mmol) were suspended in a mixed solvent of DCM:DMF (2.2:1, 9.5 ml) and stirred at room temperature for 16 hours. Distilled water (20 ml) was added to the reaction solution, and the mixture was extracted with DCM (20 ml x 2). The organic layer was dried over anhydrous sodium sulfate, filtered, and concentrated under reduced pressure. The obtained residue was subjected to MPLC (0-7% MeOH/DCM) to obtain 588 mg (70%) of an ivory solid.

### Step 2: Synthesis of tert-butyl (2S,4R)-4-hydroxy-2-((2-(((1R,4S)-4-(methoxycarbonyl)cyclohexyl)oxy)-4-(4-methylthiazol-5-yl)benzyl)carbamoyl)pyrrolidine-1-carboxylate

tert-Butyl (2S,4R)-4-hydroxy-2-((2-hydroxy-4-(4-methylthiazol-5-yl)benzyl)carbamoyl)pyrrolidine-1-carboxylate (450 mg, 1.04 mmol), (1S,4S)-methyl 4-(((2-nitrophenyl)sulfonyl)oxy)cyclohexane-1-carboxylate (1.07 g, 3.11 mmol), and Cs₂CO₃ (676 g, 2.08 mmol) were suspended in DMF (1.7 ml) and stirred at 50 °C for 16 hours. Distilled water (20 ml) was added to the reaction solution, and extracted with EtOAc (20 ml). The organic layer was washed with brine (20 ml), dried over anhydrous sodium sulfate, filtered, and concentrated under reduced pressure. The obtained residue was subjected to MPLC (0-5% MeOH/DCM) to give 278 mg (48%) of an ivory solid.

### Step 3: Synthesis of methyl (1S,4R)-4-(2-(((2S,4R)-4-hydroxypyrrolidine-2-carboxamido)methyl)-5-(4-methylthiazol-5-yl)phenoxy)cyclohexane-1-carboxylate hydrochloride

tert-Butyl (2S,4R)-4-hydroxy-2-((2-(((1R,4S)-4-(methoxycarbonyl)cyclohexyl)oxy)-4-(4-methylthiazol-5-yl)benzyl)carbamoyl)pyrrolidine-1-carboxylate (140 mg, 0.24 mmol) was suspended in DCM (2.4 ml), 4 M HCl in dioxane (0.61 ml) was added, and the mixture was stirred at room temperature for 1 hour. The reaction solution was concentrated under reduced pressure to obtain 149 mg (quant.) of a white solid.

### Step 4: Synthesis of methyl (1S,4R)-4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carboxylate

Methyl (1S,4R)-4-(2-(((2S,4R)-4-hydroxypyrrolidine-2-carboxamido)methyl)-5-(4-methylthiazol-5-yl)phenoxy)cyclohexane-1-carboxylate hydrochloride (149 mg, 0.24 mmol), 3-methyl-2-(3-methylisoxazol-5-yl)butanoic acid (44.7 mg, 0.24 mmol), EDCI (112 mg, 0.59 mmol), HOBt (80 mg, 0.59 mmol), and DIPEA (0.34 ml, 1.95 mmol) were suspended in a mixed solvent of DCM:DMF (2:1, 1.2 ml) and stirred at room temperature for 16 hours. Distilled water (10 ml) was added to the reaction solution, and the mixture was extracted with DCM (10 ml). The organic layer was dried over anhydrous sodium sulfate, filtered and concentrated under reduced pressure. The obtained residue was subjected to MPLC (0-5% MeOH/DCM) to give 83 mg (53%) of a white solid.

### Step 5: Synthesis of (1S,4R)-4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carboxylic acid

Methyl (1S,4R)-4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carboxylate (89.6 mg, 0.14 mmol) was suspended in a mixed solvent of THF: MeOH: H₂O (3:2:1, 0.6 ml), and LiOH H₂O (12 mg, 0.28 mmol) was added. The mixture was stirred at room temperature for 2 hours. The reaction solution was adjusted to pH 2 with 1 M HCl (aq.) and extracted with DCM (10 ml x 2). The organic layer was dried over anhydrous sodium sulfate, filtered and concentrated under reduced pressure to obtain 82.1 mg (94%) of a white solid.

### Step 6: Synthesis of tert-butyl ((1-(5-((2-chloro-3-((1-((1S,4R)-4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carbonyl)azetidin-3-yl)amino)phenyl)thio)pyrazin-2-yl)-4-methylpiperidin-4-yl)methyl)carbamate

(1S,4R)-4-(2-(((2S,4R)-4-Hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carboxylic acid (70 mg, 0.11 mmol), tert-butyl ((1-(5-((3-(azetidin-3-ylamino)-2-chlorophenyl)thio)pyrazin-2-yl)-4-methylpiperidin-4-yl)methyl)carbamate (Intermediate11, 110 mg, crude, about 0.18 mmol), EDCI (26 mg, 0.13 mmol), HOBt (18 mg, about 0.13 mmol), and DIPEA (59 µl, 0.34 mmol) were suspended in DMF (0.37 ml), and the mixture was stirred at room temperature for 16 hours. Distilled water (10 ml) was added to the reaction solution, and extracted with EtOAc (10 ml). The organic layer was washed with brine (10 ml), dried over anhydrous sodium sulfate, filtered, and concentrated under reduced pressure. The obtained residue was subjected to MPLC (0-5% MeOH/DCM) to give 96 mg (76%) of an ivory solid.

### Step 7: Synthesis of (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-(3-methyl-2-(3-methylisoxazol-5-yl)butanoyl)pyrrolidine-2-carboxamide (P1)

tert-Butyl ((1-(5-((2-chloro-3-((1-((1S,4R)-4-(2-(((2S,4R)-4-hydroxy-1-(3-methyl-2-(3-methylisoxazol-5-yl)butanoyl)pyrrolidine-2-carboxamido)methyl)-5-(4-methylthiazol-5-yl)phenoxy)cyclohexane-1-carbonyl)azetidin-3-yl)amino)phenyl)thio)pyrazin-2-yl)-4-methylpiperidin-4-yl)methyl)carbamate (85 mg, 0.08 mmol) was suspended in DCM (7.6 ml), 4 M HCl in dioxane (0.19 ml) was added, and the mixture was stirred at room temperature for 1 hour. After adding sat. NaHCO₃ (aq.) (5 ml) to the reaction solution, it was extracted with DCM (5 ml x 3). The organic layer was dried over anhydrous magnesium sulfate, filtered and concentrated under reduced pressure. The obtained residue was subjected to MPLC (amine-silica, 0-3% MeOH/DCM) to give 65 mg (84%) of an ivory solid mixture. The mixture was purified by chiral HPLC (column: Chiralcel IF column, B 90% (A: n-hexane, B: 0.1% IPAm in EtOH and ACN)), reverse phase HPLC (column: Phenomenex Luna C18, B 20-50% (A: 0.05% HCl in H₂O, B: ACN)) to obtain 9.3 mg (14%, 98.9%de) of an ivory solid.

### Example 115. (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-(3-methyl-2-(3-methylisoxazol-5-yl)butanoyl)pyrrolidine-2-carboxamide (P2)

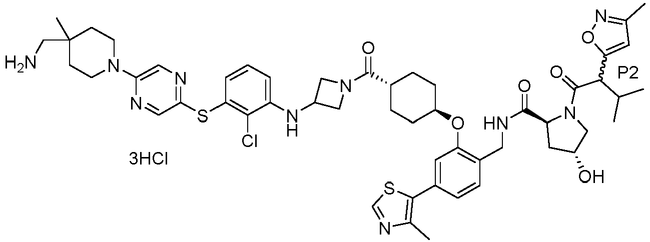

Example 15 (ivory solid, 10.3 mg (16%, 97.1%de)) was obtained by separation in Step 7 of the synthesis of Example 114.

The following Examples 116 to 124 were synthesized using the same method as the synthesis of Example 11.

| |
|---|
| Example 116. (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| 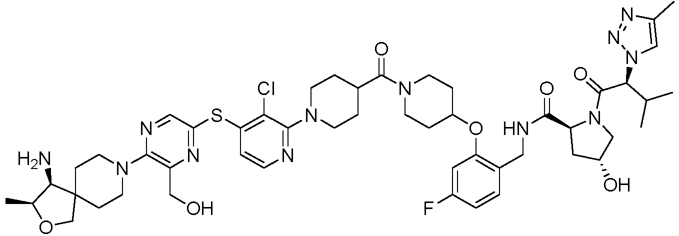 |
| Example 117. (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| 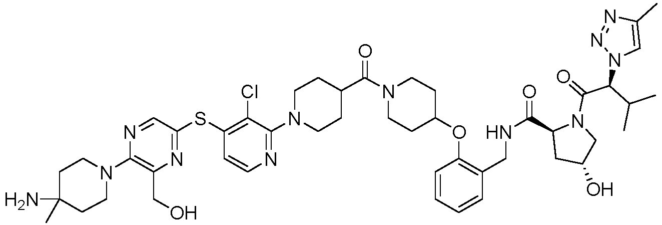 |
| Example 118. (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| 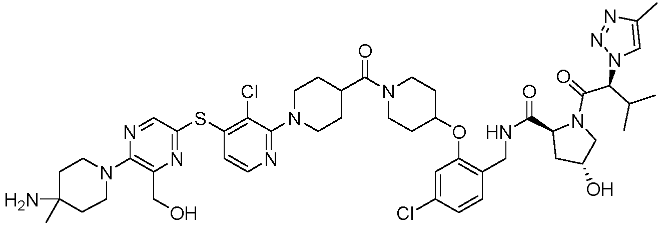 |
| Example 119. (2S, 4R)-N-(2-((1-(1-(4-((5-(4-amino-4-methylpiperidin-1-yl)-6-(hydroxymethyl)pyrazin-2-yk)thio)-3-chloropyridine-4-carbonyl)piperidine-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| 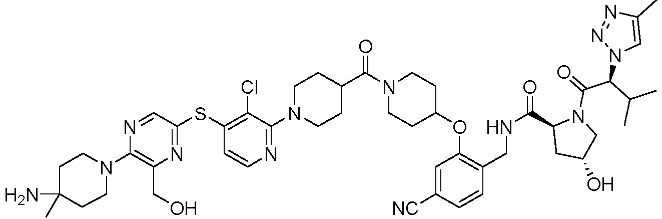 |
| Example 120. (2S,4R)-4-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| 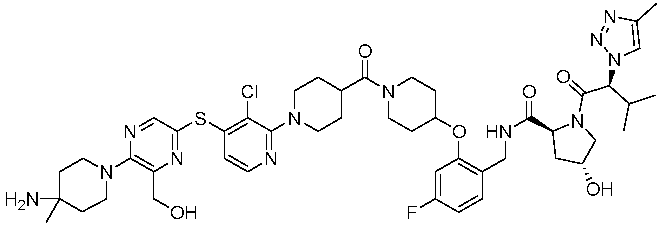 |
| Example 121. (2S,4R)-N-(2-((1-(1-(4-((5-(4-(aminomethyl)-4-methylpiperidi-1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolodone-2-carboxamide |
| 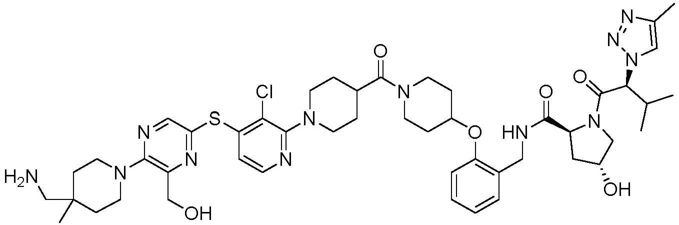 |
| Example 122. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| 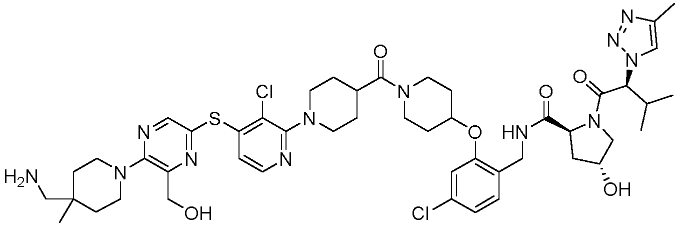 |
| Example 123. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| 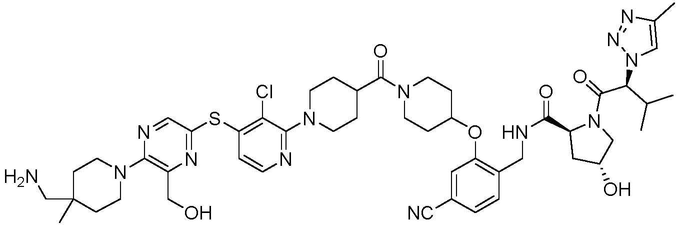 |
| Example 124. (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| 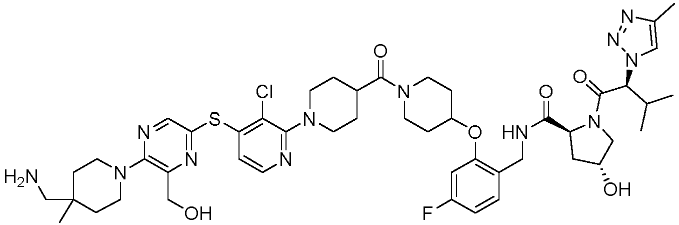 |

The NMR and mass results of the Example compounds obtained above are summarized in Table 1 below.

**[Table 1]**

| Example No. | NMR and Mass Data |
|---|---|
| 1 | ¹H NMR(600 MHz, CDCl₃) δ 8.68 (s, 1H), 8.16 (dd, J=4.2, 1.4 Hz, 1H), 8.14 (d, J=1.4 Hz, 1H), 7.31 (d, J=7.7 Hz, 1H), 7.09-7.05 (m, 1H), 7.03 (dd, J=8.9, 3.3 Hz, 1H), 6.99-6.97 (m, 1H), 6.95 (dd, J=7.6, 1.6 Hz, 1H), 6.86 (d, J=1.5 Hz, 1H), 6.48 (dd, J=8.2, 1.2 Hz, 1H), 6.33 (dd, J=7.9, 1.3 Hz, 1H), 4.70-4.65 (m, 1H), 4.57-4.52 (m, 3H), 4.49-4.42 (m, 2H), 4.39 (dd, J=14.5, 5.1 Hz, 1H), 4.05-3.96 (m, 2H), 3.92-3.86 (m, 2H), 3.68-3.63 (m, 1H), 3.39-3.34 (m, 2H), 3.09-3.05 (m, 2H), 2.99-2.94 (m, 2H), 2.58 (s, 2H), 2.56-2.46 (m, 4H), 2.37-2.29 (m, 2H), 2.03-1.99 (m, 2H), 1.95-1.83 (m, 4H), 1.80-1.77 (m, 2H), 1.57-1.53 (m, 6H), 1.47-1.43 (m, 2H), 1.41-1.33 (m, 4H), 1.32-1.25 (m, 4H), 1.02 (s, 3H), 0.93 (s, 9H). m/z 1075.52 [M+H]⁺. |
| 2 | ¹H NMR (400 MHz, CDCl₃) δ 8.68 (s, 1H), 8.17 (d, J=1.4 Hz, 1H), 8.14 (d, J=1.4 Hz, 1H), 7.34 (d, J=7.7 Hz, 1H), 7.30 (t, J=6.0 Hz, 1H), 7.03-6.96 (m, 3H), 6.89 (d, J=1.4 Hz, 1H), 6.48 (dd, J=8.2, 1.1 Hz, 1H), 6.33 (dd, J=7.9, 1.2 Hz, 1H), 5.18 (t, J=4.5 Hz, 1H), 4.71 (t, J=7.7 Hz, 1H), 4.53-4.43 (m, 4H), 4.22-4.14 (m, 2H), 4.00-3.93 (m, 1H), 3.93-3.84 (m, 2H), 3.62 (dd, J=11.2, 3.9 Hz, 1H), 3.40-3.33 (m, 2H), 3.19-3.16 (m, 2H), 2.94-2.89 (m, 2H), 2.70-2.67 (m, 4H), 2.58 (s, 3H), 2.56-2.48 (m, 8H), 2.09-2.02 (m, 1H), 1.72-1.52 (m, 1H), 1.58-1.52 (m, 2H), 1.48-1.42 (m, 2H), 1.32-1.26 (m, 4H), 1.03 (s, 3H), 0.93 (s, 9H). m/z 1034.54 [M+H]⁺. |
| 3 | ¹H NMR (400 MHz, CDCl₃) δ 8.69 (s, 1H), 8.16 (d, J=1.2 Hz, 1H), 8.15 (d, J=1.2 Hz, 1H), 7.40 (t, J=6.0 Hz, 1H), 7.35 (d, J=7.7 Hz, 1H), 7.06 (dd, J=8.7, 3.3 Hz, 1H), 7.02-6.93 (m, 2H), 6.87 (d, J=1.4 Hz, 1H), 6.49 (d, J=7.4 Hz, 1H), 6.30 (dd, J=7.9, 1.0 Hz, 1H), 4.90 (t, J=5.2 Hz, 1H), 4.57-4.52 (m, 2H), 4.51-4.46 (m, 3H), 4.22-4.10 (m, 2H), 3.97-3.88 (m, 3H), 3.79-3.72 (m, 1H), 3.63 (dd, J=11.1, 3.7 Hz, 1H), 3.56-3.46 (m. 2H), 3.40-3.33 (m, 6H), 3.00-2.87 (m, 3H), 2.78-2.72 (m, 1H), 2.59 (s, 2H), 2.53 (s, 3H), 2.41-2.34 (m, 1H), 2.09-2.03 (m, 2H), 1.59-1.52 (m, 2H), 1.48-1.42 (m, 2H), 1.38-1.25 (m, 4H), 1.03 (s, 3H), 0.96 (s, 9H). m/z 1048.48 [M+H]⁺. |
| 4 | ¹H NMR (400 MHz, CDCl₃) δ 8.69 (s, 1H), 8.18 (d, J=1.4 Hz, 1H), 8.14 (d, J=1.4 Hz, 1H), 7.35 (m, 2H), 7.06-6.94 (m, 3H), 6.88 (d, J=1.5 Hz, 1H), 6.37 (dd, J=8.0, 1.9 Hz, 2H), 5.69 (t, J=4.0 Hz, 1H), 4.69 (t, J=7.7 Hz, 1H), 4.56-4.38 (m, 4H), 4.18 (t, J=5.4 Hz, 2H), 4.01-3.84 (m, 5H), 3.73 (m, 2H), 3.61 (dd, J=11.2, 3.8 Hz, 1H), 3.49 (m, 2H), 3.36 (ddd, J=13.5, 10.0, 3.6 Hz, 2H), 3.04-2.86 (m, 2H), 2.66 (dt, J=18.0, 4.9 Hz, 4H), 2.58 (s, 2H), 2.57-2.50 (m, 4H), 2.08-2.00 (m, 1H), 1.58-1.42 (m, 4H), 1.36-1.24 (m, 4H), 1.03 (s, 3H), 0.92 (s, 9H). m/z 1048.49 [M+H]⁺. |
| 5 | ¹H NMR (400 MHz, CDCl₃) δ 8.67 (s, 1H), 8.16 (d, J=1.2 Hz, 1H), 8.13 (d, J=1.2 Hz, 1H), 7.30 (d, J=7.7 Hz, 1H), 7.21-7.17 (m, 1H), 7.05-6.86 (m, 4H), 6.40 (d, J=6.6 Hz, 1H), 6.33 (d, J=8.3 Hz, 1H), 4.73 (t, J=7.6 Hz, 1H), 4.61 (d, J=6.6 Hz, 1H), 4.57-4.38 (m, 4H), 4.25-4.08 (m, 2H), 4.03 (d, J=11.6 Hz, 1H), 3.93-3.77 (m, 4H), 3.61 (dd, J=11.3, 3.8 Hz, 1H), 3.41-3.33 (m, 2H), 2.90 (t, J=6.9 Hz, 2H), 2.63-2.55 (m, 3H), 2.52 (s, 3H), 2.39 (d, J=6.7 Hz, 2H), 2.27-2.15 (m, 2H), 2.11-2.03 (m, 1H), 1.96-1.86 (m, 2H), 1.61-1.41 (m, 9H), 1.36-1.22 (m, 4H), 1.03 (s, 3H), 0.94 (s, 9H). m/z 1045.64 [M+H]⁺. |
| 6 | ¹H NMR (400 MHz, CDCl₃) δ 8.67 (s, 1H), 8.17-8.15 (m, 1H), 8.14-8.12 (m, 1H), 7.31 (d, J=7.6 Hz, 1H), 7.20-7.17 (m, 1H), 7.02-6.97 (m, 2H), 6.95-6.89 (m, 2H), 6.51-6.49 (m, 1H), 6.36-6.32 (m, 1H), 4.73 (t, J=7.7 Hz, 1H), 4.56-4.50 (m, 2H), 4.47-4.43 (m, 2H), 4.37-4.31 (m, 1H), 4.25-4.17 (m, 1H), 4.05-4.02 (m, 1H), 3.92-3.86 (m, 2H), 3.61 (dd, J=11.4, 3.9 Hz, 2H), 3.46-3.32 (m, 5H), 3.08 (s, 1H), 2.88-2.77 (m, 2H), 2.64-2.55 (m, 3H), 2.53 (s, 3H), 2.26-2.18 (m, 5H), 2.09-2.03 (m, 4H), 2.00-1.93 (m, 3H), 1.52-1.43 (m, 6H), 1.37-1.29 (m, 4H), 1.03 (s, 3H), 0.95 (s, 9H). m/z 1073.64 [M+H]⁺. |
| 7 | ¹H NMR (400 MHz, CDCl₃) δ 8.68 (s, 1H), 8.25 (d, J=1.2 Hz, 1H), 8.19 (d, J=1.2 Hz, 1H), 7.74 (d, J=5.5 Hz, 1H), 7.33 (d, J=7.7 Hz, 1H), 7.24-7.20 (m, 1H), 7.00-6.94 (m, 2H), 6.87 (d, J=1.2 Hz, 1H), 5.91 (d, J=5.5 Hz, 1H), 4.84 (d, J=7.8 H, 1Hz), 4.74 (t, J=7.7 Hz, 1H), 4.54-4.42 (m, 5H), 4.04 (d, J=11.3 Hz, 1H), 3.96-3.86 (m, 3H), 3.60 (dd, J=11.4, 3.7 Hz, 1H), 3.45-3.38 (m, 2H), 2.62 (s, 2H), 2.60-2.50 (m, 2H), 2.52 (s, 3H), 2.17-2.08 (m, 2H), 2.10-2.05 (m, 1H), 1.96-1.88 (m, 4H), 1.59-1.53 (m, 8H), 1.51-1.45 (m, 4H), 1.37-1.31 (m, 5H), 1.22-1.17 (m, 3H), 1.05 (s, 3H), 0.93 (s, 9H). m/z 1074.57 [M+H]⁺. |
| 8 | ¹H NMR (400 MHz, CDCl₃) δ 8.68 (s, 1H), 8.26 (d, J=1.2 Hz, 1H), 8.21 (d, J=1.2 Hz, 1H), 7.88-7.73 (m, 1H), 7.37-7.32 (m, 2H), 7.01 (dd, J=8.3, 3.1 Hz, 1H), 6.96 (dd, J=7.7, 1.4 Hz, 1H), 6.89 (s, 1H), 6.22-5.96 (m, 1H), 4.75-4.69 (m, 1H), 4.55-4.40 (m, 4H), 4.19-4.15 (m, 2H), 4.03-3.93 (m, 3H), 3.74 (d, J=12.2 Hz, 2H), 3.62 (dd, J=11.2, 3.6 Hz, 2H), 3.45-3.33 (m, 2H), 2.94-2.88 (m, 2H), 2.77 (t, J=11.7 Hz, 2H), 2.80-2.64 (m, 2H), 2.60-2.55 (m, 3H), 2.53-2.38 (m, 7H), 2.25 (d, J=7.2 Hz, 2H), 2.11-2.06 (m, 1H), 1.87-1.82 (m, 2H), 1.56-1.54 (m, 4H), 1.52-1.42 (m, 3H), 1.40-1.34 (m, 2H), 1.32-1.25 (m, 4H), 1.05 (s, 3H), 0.93 (s, 9H). m/z 1089.60 [M+H]⁺. |
| 9 | ¹H NMR (400 MHz, CDCl₃) δ 8.68 (s, 1H), 8.26 (d, J=1.3 Hz, 1H), 8.21 (d, J=1.3 Hz, 1H), 7.88 (d, J=5.4 Hz, 1H), 7.33 (d, J=7.7 Hz, 1H), 7.23 (t, J=6.0 Hz, 1H), 7.03 (dd, J=8.5, 3.3 Hz, 1H), 6.94 (dd, J=7.7, 1.5 Hz, 1H), 6.88 (d, J=1.3 Hz, 1H), 6.22 (d, J=5.4 Hz, 1H), 4.74 (t, J=7.8 Hz, 1H), 4.55 - 4.42 (m, 5H), 4.06 - 4.03 (m, 1H), 3.99 - 3.93 (m, 2H), 3.77 - 3.74 (m, 2H), 3.63 - 3.59 (m, 1H), 3.46 - 3.39 (m, 2H), 2.82-2.66 (m, 4H), 2.62 - 2.58 (m, 2H), 2.57 - 2.55 (m, 1H), 2.53 (s, 3H), 2.41-2.22 (m, 4H), 2.12-2.03 (m, 3H), 1.92 - 1.84 (m, 3H), 1.58 - 1.55 (m, 4H), 1.51-1.45 (m, 2H), 1.42 - 1.39 (m, 2H), 1.35 - 1.25 (m, 4H), 1.05 (s, 3H), 0.95 (s, 9H). m/z 1060.48 [M+H]⁺. |
| 10 | ¹H NMR (400 MHz, CDCl₃) δ 8.68 (s, 1H), 8.26 (d, J=1.3 Hz, 1H), 8.21 (d, J=1.3 Hz, 1H), 7.88 (d, J=5.4 Hz, 1H), 7.34 (d, J=7.7 Hz, 1H), 7.26-7.20 (m, 1H), 7.04 (dd, J=8.7, 3.4 Hz, 1H), 6.95 (dd, J=7.7, 1.4 Hz, 1H), 6.87 (s, 1H), 6.21 (d, J=5.4 Hz, 1H), 4.74 (t, J=7.7 Hz, 1H), 4.59-4.39 (m, 5H), 4.04 (d, J=11.5 Hz, 1H), 4.00-3.90 (m, 2H), 3.64-3.38 (m, 5H), 3.31-3.21 (m, 2H), 2.98-2.87 (m, 2H), 2.67-2.56 (m, 5H), 2.52 (s, 3H), 2.44 (s, 2H), 2.12-1.87 (m, 7H), 1.58-1.40 (m, 6H), 1.38-1.26 (m, 4H), 1.05 (s, 3H), 0.95 (s, 9H). m/z 1076.62 [M+H]⁺. |
| 11 | ¹H NMR(400 MHz, CDCl₃) δ 8.69 (s, 1H), 8.19 (d, J=1.2 Hz, 1H), 8.15 (s, 1H), 7.31 (d, J=7.8 Hz, 1H), 7.25-7.22 (m, 1H), 7.03-6.96 (m, 2H), 6.94 (d, J=7.8 Hz, 1H), 6.89 (s, 1H), 6.44 (d, J=7.8 Hz, 1H), 6.24 (d, J=8.0 Hz, 1H), 4.76-4.70 (m, 2H), 4.59-4.22 (m, 8H), 4.05-3.84 (m, 5H), 3.60 (dd, J=11.3, 3.8 Hz, 1H), 3.38 (t, J=9.7 Hz, 2H), 2.62-2.50 (m, 6H), 2.35-2.20 (m, 3H), 2.10-1.98 (m, 1H), 1.76-1.41 (m, 10H), 1.37-1.28 (m, 4H), 1.04 (s, 3H), 0.93 (s, 9H). m/z 1059.52 [M+H]⁺. |
| 12 | ¹H NMR (400 MHz, CDCl₃) δ 8.69 (s, 1H), 8.18 (d, J=1.4 Hz 1H), 8.16 (d, J=1.3 1H), 7.31 (d, J=7.7 Hz, 1H), 7.25-7.23 (m, 1H), 7.02-6.98 (m, 2H), 6.94 (dd, J=7.7, 1.5 Hz, 1H), 6.89 (d, J=1.3 Hz, 1H) 6.45 (dd, J=7.9, 1.1 Hz, 1H), 6.24 (dd, J=8.1, 1.0 Hz, 1H), 4.73 (t, J=7.3 Hz, 2H), 4.56-4.52 (m, 2H), 4.50-4.45 (m, 2H), 4.42-4.37 (m, 2H), 4.33-4.25 (m, 2H), 4.03 (d, J=11.4 Hz, 1H), 4.00-3.96 (m, 1H), 3.88 (dd, J=10.2, 4.7 Hz, 1H), 3.77-3.71 (m, 2H), 3.68-3.64 (m, 2H), 3.62-3.58 (m, 1H), 2.62-2.56 (m, 1H), 2.53 (s, 3H), 2.33-2.20 (m, 3H), 2.09-2.04 (m, 1H), 1.94-1.84 (m, 2H), 1.76-1.51 (m, 10H), 1.35-1.25 (m, 5H), 0.94 (s, 9H). m/z 1045.55 [M+H]⁺. |
| 13 | ¹H NMR (400 MHz, CDCl₃) δ 8.69 (s, 1H), 8.18 (s, 1H), 8.16 (s, 1H), 7.31 (d, J=7.7 Hz, 1H), 7.24 (t, J=6.0 Hz, 1H), 7.06-6.96 (m, 2H), 6.94 (d, J=7.7 Hz, 1H), 6.89 (s, 1H), 6.50-6.42 (m, 1H), 6.25 (d, J=8.0 Hz, 1H), 4.77-4.69 (m, 2H), 4.59-4.36 (m, 6H), 4.34-4.22 (m, 2H), 4.22-4.14 (m, 1H), 4.05-3.85 (m, 5H), 3.81 (d, J=8.8 Hz, 1H), 3.69 (d, J=8.8 Hz, 1H), 3.64-3.55 (m, 1H), 3.51-3.40 (m, 1H), 3.39-3.31 (m, 1H), 2.99 (d, J=4.5 Hz, 1H), 2.63-2.49 (m, 4H), 2.36-2.22 (m, 3H), 2.12-2.02 (m, 1H), 1.94-1.82 (m, 4H), 1.63-1.43 (m, 6H), 1.37-1.22 (m, 7H), 0.93 (s, 9H). m/z 1101.53 [M+H]⁺. |
| 14 | ¹H NMR (400 MHz, CDCl₃) δ 8.69 (s, 1H), 8.19-8.17 (m, 2H), 7.34 - 7.30 (m, 2H), 7.24 - 7.22 (m, 4H), 7.03 - 6.99 (m, 2H), 6.94 (dd, J=7.7, 1.5 Hz, 1H), 6.89 (s, 1H), 6.46 (dd, J=7.4, 3.7 Hz, 1H), 6.25 (d, J=8.1 Hz, 1H), 4.75 - 4.71 (m, 2H), 4.54-4.41 (m, 7H), 4.31-4.26 (m, 2H), 4.23 - 4.19 (m, 2H), 4.05-3.96 (m, 3H), 3.88 (dd, J=10.3, 4.7 Hz, 1H), 3.59 (dd, J=11.4, 3.7 Hz, 1H), 3.28 - 3.19 (m, 2H), 3.10 (d, J=15.6 Hz, 1H), 2.75 (d, J=15.6 Hz, 1H), 2.64-2.55 (m, 1H), 2.53 (s, 3H), 2.29 - 2.22 (m, 2H), 2.11-2.04 (m, 1H), 1.89 - 1.83 (m, 4H), 1.81 - 1.75 (m, 4H), 1.42 - 1.39 (m, 2H), 1.36 - 1.22 (m, 4H), 0.93 (s, 9H). m/z 1133.30 [M+H]⁺. |
| 15 | ¹H NMR (400 MHz, CDCl₃) δ 8.68 - 8.67 (m, 1H), 8.43 (d, J=4.8 Hz, 1H), 8.20 - 8.16 (m, 2H), 7.64 (d, J=7.5 Hz, 1H), 7.31 (d, J=7.7 Hz, 1H), 7.25-7.22 (m, 1H), 7.17 - 7.14 (m, 1H), 7.04-6.86 (m, 5H), 6.49 - 6.46 (m, 1H), 6.25 (d, J=8.0 Hz, 1H), 4.75 - 4.72 (m, 2H), 4.55 - 4.42 (m, 6H), 4.29 - 4.26 (m, 4H), 4.03-3.97 (m, 3H), 3.90 - 3.86 (m, 1H), 3.62 - 3.58 (m, 1H), 3.27 - 3.19 (m, 3H), 2.91-2.85 (m, 1H), 2.64-2.59 (m, 1H), 2.57 - 2.52 (m, 3H), 2.37-2.20 (m, 5H), 2.09 - 2.04 (m, 2H), 1.89 - 1.86 (m, 3H), 1.81 - 1.65 (m, 3H), 1.42-1.37 (m, 4H), 0.93 (s, 9H). m/z 1134.40 [M+H]⁺. |
| 16 | ¹H NMR (400 MHz, CDCl₃) δ 8.69 (s, 1H), 8.00 (s, 1H), 7.68 (s, 1H), 7.31 (d, J=7.6 Hz, 1H), 7.29 -7.24 (m, 1H), 7.13 (t, J=8.2 Hz, 1H), 7.05-6.99 (m, 1H), 6.94 (d, J=7.7 Hz, 1H), 6.89 (s, 1H), 6.51 (d, J=8.2 Hz, 1H), 6.24 (d, J=8.2 Hz, 1H), 4.77-4.69 (m, 2H), 4.61-4.24 (m, 8H), 4.07-3.97 (m, 2H), 3.90 (dd, J=10.2, 4.4 Hz, 1H), 3.77-3.69 (m, 2H), 3.60 (dd, J=11.2, 3.8 Hz, 1H), 3.31-3.21 (m, 2H), 2.68-2.54 (m, 3H), 2.53 (s, 3H), 2.37-2.21 (m, 3H), 2.12-2.02 (m, 1H), 1.75-1.63 (m, 6H), 1.62-1.42 (m, 4H), 1.36-1.27 (m, 4H), 1.03 (s, 3H), 0.93 (s, 9H). m/z 1043.54 [M+H]⁺. |
| 17 | ¹H NMR (400 MHz, CDCl₃) δ 8.69 (s, 1H), 8.17 (d, J=1.2Hz, 1H), 8.15 (d, J=1.2Hz, 1H), 7.31 (d, J=7.7 Hz, 1H), 7.26-7.21 (m, 1H), 7.03-6.88 (m, 4H), 6.53 (d, J=8.3 Hz, 1H), 6.36 (d, J=7.8 Hz, 1H), 4.74 (t, J=7.7 Hz, 1H), 4.60-4.36 (m, 5H), 4.35-4.24 (m, 2H), 4.04 (d, J=11.6 Hz, 1H), 3.96-3.84 (m, 3H), 3.63-3.51 (m, 2H), 3.42-3.32 (m, 2H), 3.29-3.19 (m, 1H), 2.98-2.87 (m, 1H), 2.68-2.49 (m, 7H), 2.37-2.24 (m, 2H), 2.15-2.00 (m, 3H), 1.95-1.85 (m, 2H), 1.54-1.37 (m, 10H), 1.37-1.20 (m, 4H), 1.03 (s, 3H), 0.93 (s, 9H). m/z 1087.51 [M+H]⁺. |
| 18 | ¹H NMR(400 MHz, CDCl₃) δ 8.68 (s, 1H), 8.16 (d, J=1.2 Hz, 1H), 8.14 (d, J=1.4 Hz, 1H), 7.31 (d, J=7.7 Hz, 1H), 7.23 (t, J=5.9 Hz, 1H), 7.05-6.97 (m, 2H), 6.94 (dd, J=7.7, 1.5 Hz, 1H), 6.89 (s, 1H), 6.52 (d, J=8.0 Hz, 1H), 6.42 (dd, J=7.9, 1.2 Hz, 1H), 4.73 (t, J=7.7 Hz, 1H), 4.54-4.38 (m, 5H), 4.33-4.28 (m, 1H), 4.17-4.12 (m, 1H), 4.03 (d, J=11.3 Hz, 1H), 3.94-3.87 (m, 3H), 3.75 (dd, J=10.0, 5.1 Hz, 1H), 3.60 (dd, J=11.3, 3.8 Hz, 1H), 3.46-3.34 (m, 3H), 3.02-2.89 (m, 1H), 2.62-2.55 (m, 3H), 2.53 (s, 3H), 2.33-2.21 (m, 3H), 2.09-2.20 (m, 1H), 1.89-1.83 (m, 2H), 1.74-1.67 (m, 2H), 1.57-1.42 (m, 8H), 1.33-1.29 (m, 4H), 1.03 (s, 3H), 0.93 (s, 9H). m/z 1073.49 [M+H]⁺. |
| 19 | ¹H NMR (400 MHz, CDCl₃) δ 8.68 (s, 1H), 8.16 (d, J=1.6 Hz, 1H), 8.14 (d, J=1.2 Hz, 1H), 7.31 (d, J=7.7 Hz, 1H), 7.25-7.21 (s, 1H), 7.04-6.87 (m, 4H), 6.49 (d, J=8.1 Hz, 1H), 6.38 (d, J=8.1 Hz, 1H), 4.76-4.65 (m, 2H), 4.57-4.35 (m, 5H), 4.32-4.24 (m, 1H), 4.07-3.84 (m, 4H), 3.60 (dd, J=11.4, 3.6 Hz, 1H), 3.41-3.31 (m, 2H), 3.18-2.98 (m, 3H), 2.64-2.50 (m, 7H), 2.37-2.23 (m, 2H), 2.11-2.01 (m, 2H), 1.91-1.82 (m, 2H), 1.59-1.40 (m, 13H), 1.36-1.15 (m, 4H), 1.03 (m, 3H), 0.93 (s, 9H). m/z 1101.57 [M+H]⁺. |
| 20 | ¹H NMR (400 MHz, CDCl₃) δ 8.69 (s, 1H), 8.25 (d, J=1.3 Hz, 1H), 8.20 (d, J=1.2 Hz, 1H), 7.74 (d, J=5.5 Hz, 1H), 7.31 (d, J=7.7 Hz, 1H), 7.24-7.22 (m, 1H), 7.01 (dd, J=8.3, 3.6 Hz, 1H), 6.94 (dd, J=7.7, 1.5 Hz, 1H), 6.89 (s, 1H), 6.04 (d, J=5.5 Hz, 1H), 5.29-5.28 (m, 1H), 4.78-4.69 (m, 2H), 4.57-4.36 (m, 6H), 4.30-4.25 (m, 1H), 4.04-3.89 (m, 4H), 3.60 (dd, J=11.3, 3.8 Hz, 1H), 3.47-3.39 (m, 2H), 2.62-2.53 (m, 3H), 2.52 (s, 3H), 2.31-2.21 (m, 3H), 2.09-2.03 (m, 1H), 1.91-1.87 (m, 2H), 1.75-1.65 (m, 6H), 1.50-1.45 (m, 4H), 1.37-1.23 (m, 4H), 1.05 (s, 3H), 0.93 (s, 9H). m/z 1060.53 [M+H]⁺. |
| 21 | ¹H NMR (400 MHz, CDCl₃) δ 8.69 (s, 1H), 8.25 (s, 1H), 8.22 (s, 1H), 7.74 (d, J=5.5 Hz, 1H), 7.31 (d, J=7.6 Hz, 1H), 7.26-7.20 (m, 1H), 7.01 (dd, J=8.4, 3.3 Hz, 1H), 6.93 (d, J=7.8 Hz, 1H), 6.89 (s, 1H), 6.04 (d, J=5.5 Hz, 1H), 5.29 (d, J=5.8 Hz, 1H), 4.77-4.69 (m, 2H), 4.57-4.36 (m, 6H), 4.33-4.23 (m, 1H), 4.06-3.88 (m, 3H), 3.87-3.76 (m, 2H), 3.73-3.56 (m, 3H), 2.65-2.54 (m, 1H), 2.53 (s, 3H), 2.35-2.20 (m, 3H), 2.11-2.01 (m, 1H), 1.94-1.83 (m, 2H), 1.74-1.65 (m, 8H), 1.38-1.26 (m, 4H), 1.24 (s, 3H), 0.93 (s, 9H). m/z 1046.52 [M+H]⁺. |
| 22 | ¹H NMR (400 MHz, CDCl₃) δ 8.68 (s, 1H), 8.26 (s, 1H), 8.22 (s, 1H), 7.74 (d, J=5.5 Hz, 1H), 7.30 (d, J=7.6 Hz, 1H), 7.23 (t, J=5.9 Hz, 1H), 7.01 (dd, J=8.3, 3.3 Hz, 1H), 6.93 (d, J=7.7 Hz, 1H), 6.89 (s, 1H), 6.04 (dd, J=5.5, 0.9 Hz, 1H), 5.30 - 5.27 (m ,1H), 4.79-4.64 (m, 2H), 4.58-4.35 (m, 6H), 4.32-4.23 (m, 1H), 4.23-4.14 (m, 1H), 4.05-3.86 (m, 5H), 3.82 (d, J=8.8 Hz, 1H), 3.70 (d, J=8.9 Hz, 1H), 3.60 (dd, J=11.3, 3.5 Hz, 1H), 3.56-3.45 (m, 1H), 3.45-3.34 (m, 1H), 3.00 (d, J=4.3 Hz, 1H), 2.63-2.54 (m, 1H), 2.53 (s, 3H), 2.34-2.18 (m, 3H), 2.11-2.02 (m, 1H), 1.96-1.82 (m, 3H), 1.80-1.70 (m, 7H), 1.36-1.21 (m, 7H), 0.93 (s, 9H). m/z 1102.57 [M+H]⁺. |
| 23 | ¹H NMR (400 MHz, CDCl₃) δ 8.68 (s, 1H), 8.25 (d, J=1.3 Hz, 1H), 8.19 (d, J=1.3 Hz, 1H), 7.75 (d, J=5.5 Hz, 1H), 7.31 (d, J=7.6 Hz, 1H), 7.23 (t, J=6.0 Hz, 1H), 7.03-6.89 (m, 3H), 5.97 (d, J=5.5 Hz, 1H), 4.87 (d, J=7.6 Hz, 1H), 4.74 (t, J=7.7 Hz, 1H), 4.61-4.35 (m, 4H), 4.34-4.25 (m, 1H), 4.23-4.13 (m, 1H), 4.03 (d, J=11.4 Hz, 1H), 3.99-3.87 (m, 3H), 3.60 (dd, J=11.4, 3.8 Hz, 1H), 3.42 (ddd, J=13.5, 9.8, 3.5 Hz, 2H), 3.30-3.18 (m, 1H), 2.85 (t, J=11.7 Hz, 1H), 2.65-2.55 (m, 4H), 2.53 (s, 3H), 2.37-2.24 (m, 2H), 2.24-2.15 (m, 1H), 2.12-2.02 (m, 1H), 1.95-1.85 (m, 2H), 1.79-1.68 (m, 2H), 1.55-1.37 (m, 10H), 1.36-1.23 (m, 4H), 1.04 (s, 3H), 0.93 (s, 9H). m/z 1088.63 [M+H]⁺. |
| 24 | ¹H NMR (400 MHz, CDCl₃) δ 8.69 (s, 1H), 8.25 (d, J=1.3 Hz, 1H), 8.21 (d, J=1.3 Hz, 1H), 7.75 (d, J=5.5 Hz, 1H), 7.31 (d, J=7.7 Hz, 1H), 7.24 (t, J=6.1 Hz, 1H), 7.05-6.97 (m, 1H), 6.96-6.89 (m, 2H), 5.96 (d, J=5.5 Hz, 1H), 4.87 (d, J=7.6 Hz, 1H), 4.73 (t, J=7.7 Hz, 1H), 4.60-4.36 (m, 4H), 4.34-4.24 (m, 1H), 4.24-4.12 (m, 1H), 4.03 (d, J=11.5 Hz, 1H), 3.92 (d, J=13.6 Hz, 1H), 3.86-3.77 (m, 2H), 3.72-3.57 (m, 3H), 3.24 (t, J=11.4 Hz, 1H), 2.85 (t, J=12.0 Hz, 1H), 2.67-2.55 (m, 2H), 2.53 (s, 3H), 2.37-2.24 (m, 2H), 2.24-2.15 (m, 1H), 2.14-2.03 (m, 3H), 1.96-1.85 (m, 2H), 1.83-1.68 (m, 4H), 1.57-1.47 (m, 4H), 1.47-1.38 (m, 2H), 1.36-1.26 (m, 4H), 1.24 (s, 3H), 0.93 (s, 9H). m/z 1074.50 [M+H]⁺. |
| 25 | ¹H NMR (400 MHz, CDCl₃) δ 8.69 (s, 1H), 8.26 (d, J=1.3 Hz, 1H), 8.21 (d, J=1.3 Hz, 1H), 7.75 (d, J=5.5 Hz, 1H), 7.31 (d, J=7.7 Hz, 1H), 7.24 - 7.22 (m, 1H), 7.01-6.99 (m, 1H), 6.94 (dd, J=7.7, 1.5 Hz, 1H), 6.91 (s, 1H), 5.96 (d, J=5.5 Hz, 1H), 4.88 (d, J=7.7 Hz, 1H), 4.74 (t, J=7.7 Hz, 1H), 4.58-4.54 (m, 2H), 4.51-4.46 (m, 2H), 4.40 (dd, J=14.6, 5.4 Hz, 1H), 4.33-4.26 (m, 1H), 4.22-4.16 (m, 2H), 4.03 (d, J=11.5 Hz, 1H), 4.00-3.91 (m, 3H), 3.82 (d, J=8.8 Hz, 1H), 3.70 (d, J=8.8 Hz, 1H), 3.60 (dd, J=11.3, 3.8 Hz, 1H), 3.54-3.48 (m, 1H), 3.44-3.37 (m, 1H), 3.24 (t, J=11.9 Hz, 1H), 3.01 (d, J=4.6 Hz, 1H), 2.84 (t, J=11.6 Hz, 1H), 2.64-2.57 (m, 3H), 2.53 (s, 3H), 2.37-2.27 (m, 2H), 2.22-2.17 (m, 1H), 2.10-2.04 (m, 2H), 1.94-1.87 (m, 4H), 1.81-1.73 (m, 4H), 1.72-1.67 (m, 3H), 1.43-1.28 (m, 4H), 1.25 (d, J=6.4 Hz, 3H), 0.93 (s, 9H). m/z 1130.44 [M+H]⁺. |
| 26 | ¹H NMR (400 MHz, CDCl₃) δ 8.68 (s, 1H), 8.27 (d, J=1.3 Hz, 1H), 8.21 (d, J=1.2 Hz, 1H), 7.90 (d, J=5.3 Hz, 1H), 7.31 (d, J=7.7 Hz, 1H), 7.26-7.20 (m, 1H), 7.03-6.98 (m, 1H), 6.96-6.90 (m, 2H), 6.31 (d, J=5.3 Hz, 1H), 4.74 (t, J=7.7 Hz, 1H), 4.59-4.37 (m, 4H), 4.35-4.26 (m, 1H), 4.06-3.92 (m, 3H), 3.82-3.65 (m, 4H), 3.60 (dd, J=11.3, 3.8 Hz, 1H), 3.43 (ddd, J=13.4, 9.9, 3.6 Hz, 2H), 3.36-3.25 (m, 4H), 2.70-2.57 (m, 3H), 2.53 (s, 3H), 2.31 (t, J=15.3 Hz, 2H), 2.07 (dd, J=13.9, 8.7 Hz, 1H), 1.98-1.87 (m, 3H), 1.86-1.71 (m, 2H), 1.54-1.41 (m, 6H), 1.37-1.22 (m, 4H), 1.06 (s, 3H), 0.93 (s, 9H). m/z 1074.62 [M+H]⁺. |
| 27 | ¹H NMR (400 MHz, CDCl₃) δ 8.68 (s, 1H), 8.18 (d, J=1.3 Hz, 1H), 8.14 (d, J=1.3 Hz, 1H), 7.30 (d, J=7.7 Hz, 1H), 7.14 (t, J=5.9 Hz, 1H), 7.00 (t, J=8.0 Hz, 1H), 6.93 (dd, J=7.7, 1.4 Hz, 1H), 6.89 (s, 1H), 6.45 (d, J=7.9 Hz, 1H), 6.24 (d, J=7.4 Hz, 1H), 6.04 (d, J=8.7 Hz, 1H), 4.74-4.66 (m, 2H), 4.57-4.34 (m, 6H), 4.33-4.22 (m, 2H), 4.08 (d, J=11.4 Hz, 1H), 3.97 (dd, J=8.5, 4.5 Hz, 1H), 3.94-3.84 (m, 3H), 3.57 (dd, J=11.4, 3.7 Hz, 1H), 3.38 (ddd, J=13.4, 9.9, 3.5 Hz, 2H), 2.61-2.54 (m, 3H), 2.52 (s, 3H), 2.35-2.19 (m, 3H), 2.13-2.01 (m, 1H), 1.99 (s, 3H), 1.94-1.84 (m, 2H), 1.60-1.40 (m, 8H), 1.03 (s, 3H), 0.89 (s, 9H). m/z 1015.55 [M+H]⁺. |
| 28 | ¹H NMR (400 MHz, CDCl₃) δ 8.68 (s, 1H), 8.18 (d, J=1.3 Hz, 1H), 8.16 (d, J=1.3 Hz, 1H), 7.33-7.28 (m, 1H), 7.15 (t, J=5.9 Hz, 1H), 7.02 - 6.98 (m, 1H), 6.93 (dd, J=7.7, 1.5 Hz, 1H), 6.88 (d, J=1.3 Hz, 1H), 6.44 (dd, J=8.0, 1.2 Hz, 1H), 6.24 (d, J=7.1 Hz, 1H), 6.07 (d, J=8.7 Hz, 1H), 4.73 - 4.69 (m, 2H), 4.57-4.46 (m, 4H), 4.43-4.35 (m, 2H), 4.32 - 4.24 (m, 2H), 4.10 - 4.08 (m, 1H), 3.98 (dd, J=8.5, 4.6 Hz, 1H), 3.88 (dd, J=10.2, 4.7 Hz, 1H), 3.78-3.71 (m, 2H), 3.68-3.61 (m, 2H), 3.57 (dd, J=11.4, 3.6 Hz, 1H), 2.59-2.54 (m, 1H), 2.52 (s, 3H), 2.32-2.22 (m, 3H), 2.12 - 2.06 (m, 1H), 2.00 (s, 3H), 1.93 - 1.87 (m, 2H), 1.58-1.42 (m, 8H), 1.23 (s, 3H), 0.89 (s, 9H). m/z 1001.55 [M+H]⁺. |
| 29 | ¹H NMR (400 MHz, CDCl₃) δ 8.69 (s, 1H), 8.18 (d, J=1.3 Hz, 1H), 8.16 (d, J=1.1 Hz, 1H), 7.30 (d, J=7.7 Hz, 1H), 7.15 (t, J=5.9 Hz, 1H), 7.01 (t, J=8.0 Hz, 1H), 6.94 (dd, J=7.7, 1.5 Hz, 1H), 6.88 (s, 1H), 6.45 (d, J=8.0 Hz, 1H), 6.25 (d, J=8.1 Hz, 1H), 6.09 (d, J=8.8 Hz, 1H), 4.78-4.65 (m, 2H), 4.63-4.24 (m, 8H), 4.24-4.14 (m, 1H), 4.09 (d, J=11.5 Hz, 1H), 3.99-3.85 (m, 4H), 3.81 (d, J=8.8 Hz, 1H), 3.69 (d, J=8.8 Hz, 1H), 3.57 (dd, J=11.4, 3.6 Hz, 1H), 3.52-3.40 (m, 1H), 3.40-3.25 (m, 1H), 2.99 (d, J=4.5 Hz, 1H), 2.59-2.47 (m, 4H), 2.34-2.19 (m, 3H), 2.14-2.03 (m, 1H), 2.00 (s, 3H), 1.97-1.80 (m, 3H), 1.79-1.67 (m, 5H), 1.56-1.41 (m, 2H), 1.24 (d, J=6.4 Hz, 3H), 0.89 (s, 9H). m/z 1057.55 [M+H]⁺. |
| 30 | ¹H NMR (400 MHz, CDCl₃) δ 8.69 (s, 1H), 8.21-8.15 (m, 2H), 7.35 - 7.33 (m, 1H), 7.30 (d, J=7.8 Hz, 1H), 7.25-7.21 (m, 3H), 7.16 - 7.13 (m, 1H), 7.02 (t, J=8.1 Hz, 1H), 6.96-6.91 (m, 1H), 6.88 (s, 1H), 6.45 (d, J=7.9 Hz, 1H), 6.25 (d, J=7.9 Hz, 1H), 6.12 (d, J=8.6 Hz, 1H), 4.71 (dd, J=17.9, 6.8 Hz, 2H), 4.57 - 4.37 (m, 6H), 4.32-4.18 (m, 4H), 4.08 (d, J=11.7 Hz, 1H), 4.00 (s, 2H), 3.88 (dd, J=10.4, 4.7 Hz, 1H), 3.57 (dd, J=11.5, 3.6 Hz, 1H), 3.28 - 3.20 (m, 2H), 3.10 (d, J=15.6 Hz, 1H), 2.75 (d, J=15.5 Hz, 1H), 2.57 - 2.52 (s, 4H), 2.31 - 2.22 (m, 3H), 2.11 - 2.03 (m, 1H), 2.00 (S, 3H), 1.91 - 1.83 (m, 4H), 1.62-1.44 (m, 4H), 1.45-1.36 (m, 2H), 0.89 (s, 9H). m/z 1089.54 [M+H]⁺. |
| 31 | ¹H NMR (400 MHz, CDCl₃) δ 8.69 (s, 1H), 8.43 (d, J=4.9 Hz, 1H), 8.19 - 8.16 (m, 2H), 7.64 (d, J=7.5 Hz, 1H), 7.31-7.29 (m, 1H), 7.17 - 7.14 (m, 2H), 7.04-7.00 (m, 1H), 6.93 (dd, J=7.7, 1.4 Hz, 1H), 6.88 (s, 1H), 6.47 (d, J=8.0 Hz, 1H), 6.25 (d, J=8.1 Hz, 1H), 6.01 (d, J=8.7 Hz, 1H), 4.73 - 4.69 (m, 2H), 4.55-4.40 (m, 6H), 4.29-4.26 (m, 4H), 4.10 (d, J=11.6 Hz, 1H), 4.03 (s, 1H), 4.00-3.96 (m, 1H), 3.90 - 3.86 (m, 1H), 3.57 (dd, J=11.5, 3.6 Hz, 1H), 3.28-3.19 (m, 3H), 2.88 (d, J=16.4 Hz, 1H), 2.62-2.53 (m, 1H), 2.53 (s, 3H), 2.36-2.20 (m, 4H), 2.12-2.02 (m, 1H), 2.00 (s, 3H), 1.92-1.83 (m, 4H), 1.80-1.69 (m, 2H), 1.42-1.29 (m, 3H), 0.88 (s, 9H). m/z 1090.42 [M+H]⁺. |
| 32 | ¹H NMR (400 MHz, CDCl₃) δ 8.69 (s, 1H), 8.08 (s, 1H), 8.05 (s, 1H), 7.30 (d, J=7.7 Hz, 1H), 7.19-7.13 (m, 1H), 6.99 (t, J=7.8 Hz, 1H), 6.94 (dd, J=7.7, 1.5 Hz, 1H), 6.88 (s, 1H), 6.64 (d, J=7.9 Hz, 1H), 6.25 (d, J=7.8 Hz, 1H), 6.03 (d, J=8.5 Hz, 1H), 4.71 (t, J=7.8 Hz, 1H), 4.56-4.34 (m, 5H), 4.32-4.15 (m, 6H), 4.10 (d, J=12.6 Hz, 1H), 3.90-3.81 (m, 2H), 3.80 (d, J=8.7 Hz, 1H), 3.68 (d, J=8.8 Hz, 1H), 3.60-3.54 (m, 1H), 3.49-3.36 (m, 3H), 3.34-3.26 (m, 1H), 3.02-2.95 (m, 3H), 2.62-2.53 (m, 1H), 2.53 (s, 3H), 2.36-2.22 (m, 3H), 2.12-2.05 (m, 1H), 2.00 (s, 3H), 1.93-1.80 (m, 3H), 1.77-1.64 (m, 5H), 1.56-1.43 (m, 2H), 1.23 (d, J=6.4 Hz, 3H), 0.89 (s, 9H). m/z 1049.56 [M+H]⁺. |
| 33 | ¹H NMR (400 MHz, CDCl₃) δ 8.69 (s, 1H), 8.08 (s, 1H), 8.03 (s, 1H), 7.30 (d, J=7.7 Hz, 1H), 7.17 (t, J=6.0 Hz, 1H), 6.99 (t, J=7.8 Hz, 1H), 6.94 (d, J=7.7 Hz, 1H), 6.90 (s, 1H), 6.53 (d, J=8.2 Hz, 1H), 6.31 (d, J=7.8 Hz, 1H), 6.03 (d, J=8.4 Hz, 1H), 4.84-4.77 (m, 1H), 4.72 (t, J=7.8 Hz, 1H), 4.57-4.35 (m, 4H), 4.32-4.22 (m, 1H), 4.22-4.14 (m, 1H), 4.14 -4.01 (m, 2H), 3.90-3.82 (m, 2H), 3.80 (d, J=8.8 Hz, 1H), 3.68 (d, J=8.8 Hz, 1H), 3.63-3.54 (m, 2H), 3.44-3.33 (m, 3H), 3.33-3.25 (m, 1H), 3.18-3.08 (m, 1H), 2.98 (d, J=4.5 Hz, 1H), 2.97- 2.90 (m, 2H), 2.65-2.55 (m, 3H), 2.53 (s, 3H), 2.37-2.23 (m, 2H), 2.14-2.06 (m, 1H), 2.00 (s, 3H), 1.94-1.81 (m, 5H), 1.80-1.69 (m, 5H), 1.59-1.47 (m, 4H), 1.24 (d, J=6.4 Hz, 3H), 0.89 (s, 9H). m/z 1077.52 [M+H]⁺. |
| 34 | ¹H NMR (400 MHz, CDCl₃) δ 8.69 (s, 1H), 8.10 (s, 2H), 7.30 (d, J=7.7 Hz, 1H), 7.22-7.17 (m, 1H), 6.93 (d, J=8.1 Hz, 1H), 6.88 (s, 1H), 6.72 (t, J=8.0 Hz, 1H), 6.61 (d, J=8.1 Hz, 1H), 6.32 (d, J=8.1 Hz, 1H), 6.12 (d, J=8.1 Hz, 1H), 4.71 (t, J=7.9 Hz, 1H), 4.55 - 4.50 (m ,1H) 4.46-4.41 (m, 5H), 4.40-4.30 (m, 2H), 4.29-4.24 (m, 2H), 4.21 - 4.17 (m, 1H), 4.14-4.06 (m, 2H), 3.89 - 3.83 (m, 2H), 3.59 (dd, J=11.3, 3.6 Hz, 1H), 3.36 - 3.22 (m, 4H), 2.65 (s, 2H), 2.57-2.54 (m, 1H), 2.52 (s, 3H), 2.33-2.25 (m, 5H), 2.13-2.09 (m, 1H), 1.99 (s, 3H), 1.92-1.86 (m, 2H), 1.73-1.66 (m, 2H), 1.56-1.49 (m, 4H), 1.06 (s, 3H), 0.90 (s, 9H). m/z 1023.59 [M+H]⁺. |
| 35 | ¹H NMR (400 MHz, CDCl₃) δ 8.69 (s, 1H), 8.09 (s, 1H), 8.09 (s, 1H), 7.30 (d, J=7.7 Hz, 1H), 7.22-7.16 (m, 1H), 6.94 (dd, J=7.7, 1.4 Hz, 1H), 6.90 (s, 1H), 6.74 (t, J=7.9 Hz, 1H), 6.67 (d, J=7.7 Hz, 1H), 6.49 (d, J=7.6 Hz, 1H), 6.08 (d, J=8.5 Hz, 1H), 4.85 - 4.82 (m, 1H), 4.71 (t, J=7.9 Hz, 1H), 4.56 - 4.52 (m, 1H), 4.51 - 4.41 (m, 3H), 4.32 - 4.25 (m, 3H), 4.11-4.03 (m, 2H), 3.87 - 3.79 (m, 3H), 3.59 (dd, J=11.3, 3.6 Hz, 1H), 3.37 - 3.30 (m, 2H), 3.27-3.21 (m, 2H), 3.17 - 3.11 (m, 1H), 2.69-2.57 (m, 5H), 2.53 (s, 3H), 2.34-2.24 (m, 2H), 2.13-2.08 (m, 1H), 2.00 (s, 3H), 1.93-1.83 (m, 4H), 1.78-1.68 (m, 2H), 1.62-1.45 (m, 8H), 1.05 (s, 3H), 0.90 (s, 9H). m/z 1051.69 [M+H]⁺. |
| 36 | ¹H NMR (400 MHz, CDCl₃) δ 8.69 (s, 1H), 8.11 (s, 1H), 8.09 (s, 1H), 7.30 (d, J=7.8 Hz, 1H), 7.16 (t, J=5.9 Hz, 1H), 6.94 (d, J=8.7 Hz, 1H), 6.90 (s, 1H), 6.75 (t, J=8.0 Hz, 1H), 6.67 (d, J=8.1 Hz, 1H), 6.49 (d, J=7.5 Hz, 1H), 6.04 (d, J=8.4 Hz, 1H), 4.84 (d, J=13.6 Hz, 1H), 4.72 (t, J=7.8 Hz, 1H), 4.57-4.52 (m, 1H), 4.52-4.36 (m, 3H), 4.32-4.23 (m, 3H), 4.23-4.15 (m, 1H), 4.14-4.04 (m, 2H), 3.93-3.77 (m, 4H), 3.69 (d, J=8.7 Hz, 1H), 3.61-3.55 (m, 1H), 3.46-3.36 (m, 1H), 3.34-3.21 (m, 3H), 3.15 (t, J=12.7 Hz, 1H), 2.98 (d, J=4.5 Hz, 1H), 2.65-2.56 (m, 3H), 2.53 (s, 3H), 2.38-2.22 (m, 2H), 2.16-2.05 (m, 1H), 2.00 (s, 3H), 1.96-1.80 (m, 6H), 1.80-1.70 (m, 4H), 1.61-1.44 (m, 4H), 1.24 (d, J=6.4 Hz, 3H), 0.89 (s, 9H). m/z 1093.73 [M+H]⁺. |
| 37 | ¹H NMR (400 MHz, CDCl₃) δ 8.69 (s, 1H), 8.10-8.06 (m, 2H), 7.31 (d, J=7.7 Hz, 1H), 7.23 (dd, J=8.3, 1.8 Hz, 1H), 7.17-7.10 (m, 2H), 7.03 (d, J=7.2 Hz, 1H), 6.96-6.92 (m, 2H), 6.90 (s, 1H), 6.05 (d, J=8.6 Hz, 1H), 4.95-4.84 (m, 1H), 4.71 (t, J=7.8 Hz, 1H), 4.61-4.35 (m, 5H), 4.35-4.22 (m, 1H), 4.17-4.02 (m, 2H), 3.89-3.78 (m, 2H), 3.61-3.54 (m, 1H), 3.38-3.21 (m, 3H), 2.78-2.67 (m, 1H), 2.67-2.60 (m, 1H), 2.60-2.55 (m, 3H), 2.53 (s, 3H), 2.39-2.25 (m, 2H), 2.20-2.05 (m, 3H), 2.00 (s, 3H), 1.96-1.87 (m, 2H), 1.86-1.74 (m, 4H), 1.58-1.47 (m, 4H), 1.47-1.39 (m, 2H), 1.01 (s, 3H), 0.89 (s, 9H). m/z 1051.69 [M+H]⁺. |
| 38 | ¹H NMR (400 MHz, CDCl₃) δ 8.68 (s, 1H), 8.25 (s, 1H), 8.20 (s, 1H), 7.74 (d, J=5.5 Hz, 1H), 7.30 (d, J=7.7 Hz, 1H), 7.16 (t, J=5.8 Hz, 1H), 6.96-6.91 (m, 1H), 6.88 (s, 1H), 6.11 (d, J=8.8 Hz, 1H), 6.04 (d, J=5.5 Hz, 1H), 5.29 (m, 1H), 4.77-4.66 (m, 2H), 4.60-4.33 (m, 6H), 4.33-4.20 (m, 1H), 4.08 (d, J=11.4 Hz, 1H), 4.05-3.87 (m, 4H), 3.57 (dd, J=11.3, 3.6 Hz, 1H), 3.52-3.35 (m, 2H), 2.63-2.51 (m, 6H), 2.34-2.20 (m, 3H), 2.13-2.05 (m, 1H), 2.00 (s, 3H), 1.94-1.85 (m, 2H), 1.63-1.40 (m, 8H), 1.05 (s, 3H), 0.89 (s, 9H). m/z 1016.56 [M+H]⁺. |
| 39 | ¹H NMR (400 MHz, CDCl₃) δ 8.69 (s, 1H), 8.26 (d, J=1.2 Hz, 1H), 8.22 (d, J=1.2 Hz, 1H), 7.74 (d, J=5.5 Hz, 1H), 7.30 (d, J=7.7 Hz, 1H), 7.17 (t, J=5.8 Hz, 1H), 6.93 (dd, J=7.7, 1.4 Hz, 1H), 6.88 (s, 1H), 6.08 (d, J=8.6 Hz, 1H), 6.04 (d, J=5.5 Hz, 1H), 5.29-5.28 (m, 1H), 4.76-4.67 (m, 2H), 4.60-4.33 (m, 6H), 4.31-4.21 (m, 1H), 4.08 (d, J=11.5 Hz, 1H), 4.04-3.95 (m, 1H), 3.95-3.88 (m, 1H), 3.87-3.77 (m, 2H), 3.75-3.63 (m, 2H), 3.58 (dd, J=11.3, 3.6 Hz, 1H), 2.61-2.48 (m, 4H), 2.37-2.19 (m, 3H), 2.12-2.04 (m, 1H), 2.00 (s, 3H), 1.95-1.84 (m, 2H), 1.64-1.42 (m, 8H), 1.24 (s, 3H), 0.89 (s, 9H). m/z 1002.60 [M+H]⁺. |
| 40 | ¹H NMR (400 MHz, CDCl₃) δ 8.68 (s, 1H), 8.26 (d, J=1.2 Hz, 1H), 8.22 (d, J=1.1 Hz, 1H), 7.74 (d, J=5.5 Hz, 1H), 7.30 (d, J=7.7 Hz, 1H), 7.18 (t, J=5.6 Hz, 1H), 6.95-6.91 (m, 1H), 6.88 (s, 1H), 6.10 (d, J=7.1 Hz, 1H), 6.04 (d, J=5.5 Hz, 1H), 5.32-5.29 (m, 1H), 4.77-4.67 (m, 2H), 4.58-4.35 (m, 6H), 4.31-4.17 (m, 2H), 4.08 (d, J=11.4 Hz, 1H), 4.05-3.88 (m, 4H), 3.85 (d, J=8.8 Hz, 1H), 3.71 (d, J=8.8 Hz, 1H), 3.57 (dd, J=11.3, 3.7 Hz, 1H), 3.53-3.45 (m, 1H), 3.44-3.34 (m, 1H), 3.04 (d, J=4.0 Hz, 1H), 2.61-2.49 (m, 4H), 2.35-2.18 (m, 3H), 2.12-2.03 (m, 1H), 2.00 (s, 3H), 1.97-1.83 (m, 3H), 1.80-1.64 (m, 7H), 1.30-1.26 (m, 3H), 0.89 (s, 9H). m/z 1058.58 [M+H]⁺. |
| 41 | ¹H NMR (400 MHz, CDCl₃) δ 8.69 (s, 1H), 8.25 (s, 1H), 8.20 (s, 1H), 7.75 (d, J=5.5 Hz, 1H), 7.31 (d, J=7.7 Hz, 1H), 7.18 (t, J=5.8 Hz, 1H), 6.93 (d, J=7.8 Hz, 1H), 6.90 (s, 1H), 6.08 (d, J=8.6 Hz, 1H), 5.96 (d, J=5.5 Hz, 1H), 4.88 (d, J=7.6 Hz, 1H), 4.72 (t, J=7.8 Hz, 1H), 4.60-4.53 (m, 2H), 4.49-4.45 (m, 2H), 4.39 (dd, J=14.6, 5.1 Hz, 1H), 4.31-4.24 (m, 1H), 4.22-4.15 (m, 1H), 4.11-4.07 (m, 1H), 3.99-3.91 (m, 3H), 3.58 (dd, J=11.4, 3.6 Hz, 1H), 3.49-3.39 (m, 2H), 3.28-3.22 (m, 1H), 2.87-2.82 (m, 1H), 2.64-2.56 (m, 4H), 2.53 (s, 3H), 2.33-2.20 (m, 4H), 2.12-2.07 (m, 2H), 2.00 (s, 3H), 1.93-1.84 (m, 2H), 1.80-1.68 (m, 2H), 1.63-1.47 (m, 5H), 1.43-1.37 (d, 2H), 1.07 (s, 3H), 0.89 (s, 9H). m/z 1044.63 [M+H]⁺. |
| 42 | ¹H NMR (400 MHz, CDCl₃) δ 8.69 (s, 1H), 8.25 (d, J=1.3 Hz, 1H), 8.21 (d, J=1.3 Hz, 1H), 7.75 (d, J=5.5 Hz, 1H), 7.31 (d, J=7.7 Hz, 1H), 7.17 (t, J=5.9 Hz, 1H), 6.94 (dd, J=7.7, 1.5 Hz, 1H), 6.90 (d, J=1.3 Hz, 1H), 6.08 (d, J=8.7 Hz, 1H), 5.96 (d, J=5.5 Hz, 1H), 4.88 (d, J=7.6 Hz, 1H), 4.71 (t, J=7.8 Hz, 1H), 4.58-4.53 (m, 2H), 4.51-4.45 (m, 2H), 4.39 (dd, J=14.7, 5.2 Hz, 1H), 4.31-4.24 (m, 1H), 4.24-4.18 (m, 1H), 4.11 (d, J=11.5 Hz, 1H), 3.92 (d, J=14.4 Hz, 1H), 3.83-3.77 (m, 2H), 3.72-3.66 (m, 2H), 3.58 (dd, J=11.4, 3.7 Hz, 1H), 3.25 (t, J=11.8 Hz, 1H), 2.85 (t, J=11.5 Hz, 1H), 2.61-2.54 (m, 1H), 2.53 (s, 3H), 2.33-2.17 (m, 3H), 2.12-2.07 (m, 2H), 2.00 (s, 3H), 1.93-1.87 (m, 2H), 1.71-1.58 (m, 8H), 1.46-1.40 (m, 2H), 1.25 (s, 3H), 0.89 (s, 9H). m/z 1030.58 [M+H]⁺. |
| 43 | ¹H NMR (400 MHz, CDCl₃) δ 8.69 (s, 1H), 8.26 (d, J=1.2 Hz, 1H), 8.22 (d, J=1.3 Hz, 1H), 7.75 (d, J=5.5 Hz, 1H), 7.30 (d, J=7.7 Hz, 1H), 7.17 (t, J=5.8 Hz, 1H), 6.94 (dd, J=7.7, 1.4 Hz, 1H), 6.90 (s, 1H), 6.06 (d, J=8.6 Hz, 1H), 5.96 (d, J=5.5 Hz, 1H), 4.88 (d, J=7.6 Hz, 1H), 4.72 (t, J=7.8 Hz, 1H), 4.58-4.51 (m, , 2H), 4.47 (dd, J=11.9, 7.7 Hz, 2H), 4.39 (dd, J=14.7, 5.2 Hz, 1H), 4.31-4.24 (m, 1H), 4.19-4.17 (m, 2H), 4.13-4.08 (m, 1H), 4.01-3.91 (m, 3H), 3.82 (d, J=8.8 Hz, 1H), 3.70 (d, J=8.8 Hz, 1H), 3.58 (dd, J=11.4, 3.6 Hz, 1H), 3.55-3.51 (dd, J=8.9, 4.4 Hz, 1H), 3.44-3.38 (m, 1H), 3.25 (t, J=11.9 Hz, 1H), 3.01 (d, J=4.5 Hz, 1H), 2.85 (t, J=11.5 Hz, 1H), 2.63-2.55 (m, 2H), 2.53 (s, 3H), 2.34-2.20 (m, 4H), 2.12-2.07 (m, 2H), 2.00 (s, 3H), 1.94-1.87 (m, 4H), 1.79-1.67 (m, 5H), 1.42 (d, J=11.2 Hz, 2H), 1.25 (d, J=6.4 Hz, 3H), 0.89 (s, 9H). m/z 1086.57 [M+H]⁺. |
| 44 | ¹H NMR (400 MHz, DMSO-d6) δ 8.99 (s, 1H), 8.40 (t, J=5.3 Hz, 1H), 8.30 (s, 1H), 7.98-7.90 (m, 1H), 7.77 (d, J=5.5 Hz, 1H), 7.44 (d, J=7.8 Hz, 1H), 7.13-7.09 (m, 2H), 6.89 (d, J=9.0 Hz, 1H), 6.00 (d, J=5.5 Hz, 1H), 5.50-5.42 (m, 1H), 5.12 (d, J=3.4 Hz, 1H), 4.70-4.59 (m, 1H), 4.55-4.37 (m, 6H), 4.35 - 4.31 (m, 1H), 4.31-4.24 (m, 1H), 4.16-4.07 (m, 3H), 3.88 - 3.85 (m, 1H), 3.70-3.58 (m, 4H), 3.17 (d, J=3.8 Hz, 1H), 2.68-2.54 (m, 2H), 2.47-2.42 (m, 3H), 2.30-2.22 (m, 1H), 2.16-2.08 (m, 2H), 2.05 - 1.98 (m, 1H), 1.95 - 1.90 (m, 1H), 1.89 (s,3H), 1.79-1.72 (m, 2H), 1.60-1.49 (m, 4H), 1.48-1.41 (m, 2H), 1.39 - 1.34 (m, 2H), 0.96 (s, 3H), 0.92 (s, 9H). m/z 1046.54 [M+H]⁺. |
| 45 | ¹H NMR (400 MHz, DMSO-d6) δ 8.98 (s, 1H), 8.40 (t, J=5.9 Hz, 1H), 8.29 (s, 1H), 7.95 (d, J=9.4 Hz, 1H), 7.76 (d, J=5.4 Hz, 1H), 7.45 (d, J=7.8 Hz, 1H), 7.10 (s, 1H), 6.89 (dd, J=7.9, 1.2 Hz, 1H), 6.22 (d, J=7.9 Hz, 1H), 5.92 (d, J=5.4 Hz, 1H), 5.44 (t, J=5.8 Hz, 1H), 5.12 (d, J=3.6 Hz, 1H), 4.55-4.39 (m, 6H), 4.37 - 4.33 (m, 1H), 4.32 - 4.26 (m, 1H), 4.17 - 4.11 (m, 2H), 4.02 - 3.99 (m, 1H), 3.69-3.59 (m, 4H), 3.14 - 3.08 (m, 1H), 2.70 - 2.58 (m, 2H), 2.46 (s, 3H), 2.43 (s, 2H), 2.17 - 2.09 (m, 2H), 2.07-2.00 (m, 1H), 1.95 - 1.91 (m , 1H), 1.89 (s, 3H), 1.85 - 1.79 (m, 2H), 1.76 - 1.70 (m, 2H), 1.61-1.48 (m, 8H), 1.42 - 1.31 (m, 3H), 0.94 (s, 3H), 0.93 (s, 9H). m/z 1074.59 [M+H]⁺. |
| 46 | ¹H NMR (400 MHz, CDCl₃) δ 8.69 (s, 1H), 8.21 (s, 1H), 8.18 (s, 1H), 7.31 (d, J=7.7 Hz, 1H), 7.17 (t, J=5.8 Hz, 1H), 7.09 (t, J=7.8 Hz, 1H), 7.01 (d, J=7.7 Hz, 1H), 6.94 (dd, J=7.7, 1.5 Hz, 1H), 6.90 (s, 1H), 6.82 (d, J=7.7 Hz, 1H), 6.06 (d, J=8.6 Hz, 1H), 4.88-4.70 (m, 1H), 4.72 (t, J=7.8 Hz, 1H), 4.60-4.33 (m, 4H), 4.33-4.23 (m, 1H), 4.12-4.02 (m, 2H), 3.96-3.88 (m, 2H), 3.62-3.55 (m, 1H), 3.48-3.28 (m, 3H), 3.22 (t, J=12.3 Hz, 1H), 2.72-2.62 (m, 2H), 2.62-2.54 (m, 3H), 2.53 (s, 3H), 2.37-2.24 (m, 2H), 2.13-2.05 (m, 1H), 2.00 (s, 3H), 1.96-1.86 (m, 4H), 1.81-1.66 (m, 4H), 1.62-1.42 (m, 6H), 1.04 (s, 3H), 0.89 (s, 9H). m/z 1028.69 [M+H]⁺. |
| 47 | ¹H NMR (400 MHz, CDCl₃) δ 8.69 (s, 1H), 8.27 (s, 1H), 8.23 (s, 1H), 8.13 (s, 1H), 7.30 (d, J=7.7 Hz, 1H), 7.19 (t, J=5.9 Hz, 1H), 6.93 (dd, J=7.7, 1.4 Hz, 1H), 6.90 (s, 1H), 6.50 (d, J=5.3 Hz, 1H), 6.07 (d, J=8.6 Hz, 1H), 4.83-4.76 (m, 1H), 4.72 (t, J=7.8 Hz, 1H), 4.55-4.42 (m, 3H), 4.42-4.35 (m, 1H), 4.32-4.23 (m, 1H), 4.12-4.04 (m, 2H), 4.02-3.92 (m, 2H), 3.61- 3.55 (m, 1H), 3.51-3.39 (m, 3H), 3.28-3.16 (m, 1H), 2.71 (t, J=14.1 Hz, 1H), 2.66-2.56 (m, 4H), 2.53 (s, 3H), 2.35-2.23 (m, 2H), 2.13-2.05 (m, 1H), 2.00 (s, 3H), 1.96-1.85 (m, 6H), 1.79-1.66 (m, 4H), 1.63-1.56 (m, 2H), 1.54-1.45 (m, 2H), 1.06 (s, 3H), 0.89 (s, 9H). m/z 1029.58 [M+H]⁺. |
| 48 | ¹H NMR (400 MHz, CDCl₃) δ 8.68 (s, 1H), 8.19 (s, 1H), 8.16 (s, 1H), 7.43 - 7.38 (m, 1H), 7.25 (s, 1H), 7.01 - 6.96 (m, 2H), 6.91 (s, 1H), 6.45 - 6.40 (m, 1H), 6.23 (d, J=8.5 Hz, 1H), 6.13 (d, J=8.9 Hz, 1H), 5.32-5.28 (m, 1H), 4.72-4.65 (m, 2H), 4.54-4.50 (m, 3H), 4.42 - 4.39 (m, 1H), 4.33-4.28 (m, 2H), 4.11 - 4.09 (m, 1H), 4.00-3.90 (m, 4H), 3.62 - 3.59 (m, 1H), 3.42 - 3.36 (m, 2H), 3.07 - 2.58 (m, 2H), 2.54-2.46 (m, 4H), 2.33-2.20 (m, 4H), 2.08-2.01 (m, 4H), 1.92-1.85 (m, 2H), 1.74-1.67 (m, 6H), 1.44 - 1.42 (m, 3H), 1.06 - 1.03 (m, 12H). m/z 1029.55 [M+H]⁺. |
| 49 | ¹H NMR(400 MHz, CDCl₃) δ 8.68 (s, 1H), 8.25 (s, 1H), 8.20 (s, 1H), 7.73 (d, J=5.5 Hz, 1H), 7.42 (d, J=7.9 Hz, 1H), 7.24 (s, 1H), 6.97 (d, J=7.8 Hz, 1H), 6.92 (s, 1H), 6.15 (d, J=8.5 Hz, 1H), 6.04 (d, J=5.5 Hz, 1H), 5.30 - 5.27 (m, 1H), 4.75-4.65 (m, 2H), 4.57-4.51 (m, 3H), 4.43-4.36 (m, 1H), 4.34-4.29 (m, 1H), 4.11 - 4.08 (m, 1H), 4.01-3.90 (m, 5H), 3.63 - 3.60 (m, 1H), 3.46 - 3.39 (m, 2H), 2.60 (s, 2H), 2.54-2.41 (m, 5H), 2.33-2.17 (m, 4H), 2.07-2.01 (m, 4H), 1.92-1.85 (m, 2H), 1.76-1.68 (m, 2H), 1.51-1.45 (m, 4H), 1.43 (d, J=6.9 Hz, 3H), 1.05 (s, 12H). m/z 1030.56 [M+H]⁺. |
| 50 | ¹H NMR (400 MHz, CDCl₃) δ 8.68 (s, 1H), 8.17 (d, J=1.4 Hz, 1H), 8.15 (s, 1H), 7.33 (d, J=7.7 Hz, 1H), 7.24 (s, 1H), 7.13-7.09 (m, 1H), 7.00-6.95 (m, 2H), 6.87 (s, 1H), 6.40 (t, J=7.6 Hz, 1H), 6.20 (d, J=7.9 Hz, 1H), 4.74-4.64 (m, 2H), 4.60 (d, J=8.8 Hz, 1H), 4.57-4.44 (m, 4H), 4.42-4.33 (m, 1H), 4.33-4.21 (m, 1H), 4.21-4.05 (m, 2H), 3.98-3.81 (m, 5H), 3.66 (dd, J=10.8, 3.2 Hz, 1H), 3.41-3.35 (m, 2H), 3.16-3.07 (m, 2H), 2.93-2.87 (m, 1H), 2.79-2.74 (m, 1H), 2.54 (s, 3H), 2.38-2.16 (m, 3H), 2.07-2.00 (m, 3H), 1.93-1.79 (m, 3H), 1.73-1.65 (m, 3H), 1.47-1.43 (m, 3H), 1.36-1.21 (m, 4H), 1.03 (s, 3H), 0.97 (s, 9H). m/z 1088.41 [M+H]⁺. |
| 51 | ¹H NMR (400 MHz, CDCl₃) δ 8.68 (s, 1H), 8.17 (s, 1H), 8.15 (s, 1H), 7.33 (d, J=7.7 Hz, 1H), 7.24 (d, J=5.8 Hz, 1H), 7.15-7.06 (m, 1H), 7.02-6.93 (m, 2H), 6.88 (d, J=1.1 Hz, 1H), 6.50 (dd, J=7.8, 2.3 Hz, 1H), 6.33 (t, J=7.3 Hz, 1H), 4.71-4.65 (m, 1H), 4.61 (d, J=8.8 Hz, 1H), 4.58-4.38 (m, 4H), 4.34-4.29 (m, 1H), 4.20-4.07 (m, 2H), 3.97-3.83 (m, 4H), 3.67 (dd, J=11.1, 3.7 Hz, 1H), 3.57-3.53 (m, 1H), 3.45-3.32 (m, 2H), 3.26-3.21 (m, 1H), 3.17-3.05 (m, 2H), 2.99-2.83 (m, 2H), 2.80-2.76 (m, 1H), 2.54 (s, 4H), 2.38-2.22 (m, 2H), 2.14-2.05 (m, 4H), 1.98-1.84 (m, 2H), 1.74-1.67 (m. 4H), 1.66-1.62 (m, 4H), 1.44-1.37 (m, 2H), 1.33-1.28 (m, 4H), 1.05 (d, J=12.7 Hz, 3H), 0.97 (s, 9H). m/z 1116.42 [M+H]⁺. |
| 52 | ¹H NMR (400 MHz, CDCl₃) δ 8.67 (s, 1H), 8.26 (d, J=1.2 Hz, 1H), 8.21 (d, J=1.2 Hz, 1H), 7.88 (d, J=5.4 Hz, 1H), 7.33 (d, J=7.7 Hz, 1H), 7.25-7.20 (m, 1H), 7.08 (dd, J=8.8, 3.4 Hz, 1H), 6.96 (dd, J=7.6, 1.4 Hz, 1H), 6.89 (s, 1H), 6.30 (d, J=5.4 Hz, 1H), 4.70 (t, J=8.2 Hz, 1H), 4.60 (d, J=8.5 Hz, 1H), 4.56-4.42 (m, 3H), 4.19-4.10 (m, 2H), 4.02-3.89 (m, 3H), 3.84-3.75 (m, 1H), 3.71-3.59 (m, 5H), 3.43 (ddd, J=13.4, 9.9, 3.6 Hz, 2H), 3.38-3.19 (m, 5H), 3.19-3.06 (m, 2H), 2.96-2.86 (m, 1H), 2.86-2.74 (m, 1H), 2.63-2.50 (m, 6H), 2.41-3.32 (m, 1H), 2.28-2.19 (m, 1H), 2.12 (dd, J=24.6, 12.0 Hz, 2H), 2.05-1.83 (m, 2H), 1.72 (t, J=12.5 Hz, 2H), 1.52-1.38 (m, 2H), 1.36-1.17 (m, 4H), 1.05 (s, 3H), 0.96 (s, 9H). m/z 1103.82 [M+H]⁺. |
| 53 | ¹H NMR (400 MHz, CDCl₃) δ 8.68 (s, 1H), 8.25 (s, 1H), 8.20 (s, 1H), 7.75 (d, J=5.4 Hz, 1H), 7.34-7.30 (m, 2H), 7.02-6.97 (m, 2H), 6.88 (s, 1H), 6.04-6.02 (m, 1H), 4.70 (t, J=7.7 Hz, 1H), 4.54-4.49 (m, 4H), 4.41-4.36 (m, 4H), 4.17 (t, J=5.3 Hz, 2H), 3.99-3.94 (m, 3H), 3.67-3.56 (m, 4H), 3.49-3.37 (m, 4H), 2.98-2.87 (m, 2H), 2.65-2.57 (m, 6H), 2.56-2.51 (m, 4H), 2.11-2.04 (m, 1H), 1.63-1.45 (m, 4H), 1.29-1.25 (m, 4H), 1.08 (s, 3H), 0.92 (s, 9H). m/z 1075.54 [M+H]⁺. |
| 54 | ¹H NMR (400 MHz, CDCl₃) δ 8.69 (s, 1H), 8.26 (s, 1H), 8.20 (s, 1H), 7.86 (d, J=5.3 Hz, 1H), 7.35-7.30 (m, 2H), 7.02-6.97 (m, 2H), 6.89 (s, 1H), 6.24 (d, J=5.4 Hz, 1H), 4.72 (t, J=7.7 Hz, 1H), 4.60-4.37 (m, 4H), 4.19 (t, J=5.3 Hz, 2H), 4.00-3.95 (m, 2H), 3.81 (d, J=12.3 Hz, 2H), 3.73-3.53 (m, 5H), 3.48-3.42 (m, 2H), 3.09 (s, 1H), 2.99-2.89 (m, 2H), 2.86-2.79 (m, 2H), 2.68-2.57 (m, 4H), 2.55-2.51 (m, 4H), 2.10-2.02 (m, 3H), 1.80-1.77 (m, 2H), 1.74-1.69 (m, 2H), 1.62-1.45 (m, 5H), 1.35-1.26 (m, 4H), 1.08 (s, 3H), 0.93 (s, 9H). m/z 1103.54 [M+H]⁺. |
| 55 | ¹H NMR (400 MHz, CDCl₃) δ 8.68 (s, 1H), 8.25 (s, 1H), 8.20 (s, 1H), 7.76 (d, J=5.4 Hz, 1H), 7.35-7.28 (m, 2H), 7.02-6.93 (m, 2H), 6.87 (s, 1H), 6.03 (dd, J=5.4, 2.3 Hz, 1H), 4.73 (t, J=6.3 Hz, 1H), 4.62 (d, J=13.6 Hz, 1H), 4.57-4.35 (m, 8H), 4.13-3.89 (m, 5H), 3.67-3.54 (m, 3H), 3.50-3.37 (m, 2H), 3.10-3.01 (m, 1H), 2.69-2.54 (m, 4H), 2.53 (s, 3H), 2.12-2.03 (m, 1H), 1.77-1.64 (m, 6H), 1.64-1.43 (m, 5H), 1.37-1.16 (m, 4H), 1.08 (s, 3H), 0.91 (s, 9H). m/z 1074.62 [M+H]⁺. |
| 56 | ¹H NMR (400 MHz, CDCl₃) δ 8.69 (s, 1H), 8.26 (d, J=1.2 Hz, 1H), 8.21 (d, J=1.2 Hz, 1H), 7.87 (d, J=5.4 Hz, 1H), 7.35-7.29 (m, 2H), 7.03-6.94 (m, 2H), 6.87 (d, J=1.3 Hz, 1H), 6.23 (d, J=5.4 Hz, 1H), 4.77-4.63 (m, 2H), 4.58-4.38 (m, 4H), 4.12-3.91 (m, 6H), 3.81 (d, J=11.9 Hz, 2H), 3.61 (dd, J=11.2, 3.6 Hz, 1H), 3.43 (ddd, J=13.5, 10.0, 3.6 Hz, 2H), 3.15-3.03 (m, 1H), 2.83 (t, J=11.7 Hz, 2H), 2.72-2.55 (m, 5H), 2.53 (s, 3H), 2.14-1.97 (m, 3H), 1.93-1.73 (m, 7H), 1.53-1.42 (m, 6H), 1.38-1.17 (m, 4H), 1.05 (s, 3H), 0.92 (s, 9H). m/z 1102.57 [M+H]⁺. |
| 57 | ¹H NMR (400 MHz, CDCl₃) δ 8.68 (s, 1H), 8.26 (d, J=1.2 Hz, 1H), 8.21 (d, J=1.2 Hz, 1H), 7.87 (d, J=5.4 Hz, 1H), 7.38-7.30 (m, 2H), 7.03-6.93 (m, 2H), 6.67 (s, 1H), 6.23 (d, J=5.4 Hz, 1H), 4.79-4.72 (m, 2H), 4.57-4.40 (m, 4H), 4.05-3.91 (m, 3H), 3.82 (d, J=11.8 Hz, 2H), 3.66-3.38 (m, 7H), 2.88-2.76 (s, 2H), 2.69-2.55 (m, 4H), 2.54-2.43 (m, 5H), 2.20-2.14 (m, 1H), 2.11-1.97 (m, 4H), 1.81-1.71 (m, 2H), 1.61-1.43 (m, 8H), 1.37-1.23 (m, 4H), 1.05 (s, 3H), 0.94 (s, 9H). m/z 1114.58 [M+H]⁺. |
| 58 | ¹H NMR (400 MHz, CDCl₃) δ 8.68 (s, 1H), 8.26 (d, J=1.2 Hz, 1H), 8.21 (d, J=1.2 Hz, 1H), 7.87 (d, J=5.4 Hz, 1H), 7.33 (d, J=7.7 Hz, 1H), 7.22-7.16 (m, 1H), 7.02-6.95 (m, 2H), 6.85 (d, J=1.3 Hz, 1H), 6.24 (d, J=5.4 Hz, 1H), 4.80-4.68 (m, 2H), 4.61-4.49 (m, 3H), 4.45-4.33 (m, 1H), 4.09-3.79 (m, 8H), 3.62 (dd, J=11.0, 3.9 Hz, 1H), 3.43 (ddd, J=13.5, 9.9, 3.6 Hz, 2H), 3.20-3.07 (m, 1H), 2.86 (t, J=12.2 Hz, 2H), 2.77-2.54 (m, 5H), 2.52 (s, 3H), 2.23-1.99 (m, 6H), 1.94-1.75 (m, 4H), 1.60-1.43 (m, 4H), 1.37-1.23 (m, 4H), 1.05 (s, 3H), 0.91 (s, 9H). m/z 1088.60 [M+H]⁺. |
| 59 | ¹H NMR (400 MHz, DMSO-d₆) δ 8.98 (s, 1H), 8.47-8.44 (m, 1H), 8.42 (d, J=1.2 Hz, 1H), 8.26 (d, J=1.3 Hz, 1H), 7.72 (d, J=5.4 Hz, 1H), 7.42 (d, J=7.8 Hz, 1H), 7.27 (dd, J=9.2, 2.5 Hz, 1H), 7.08 (s, 1H), 6.95 (dd, J=7.8, 1.2 Hz, 1H), 6.60 (d, J=7.2 Hz, 1H), 5.83 (d, J=5.4 Hz, 1H), 5.16 (d, J=3.5 Hz, 1H), 4.78 (m, 1H), 4.59 (d, J=9.2 Hz, 1H), 4.50 (t, J=8.2 Hz, 1H), 4.41-4.34 (m, 2H), 4.27 (t, J=5.8 Hz, 2H), 3.92-3.84 (m, 2H), 3.67-3.58 (m, 4H), 3.50-3.35 (m, 5H), 3.07-3.00 (m, 2H), 2.45 (s, 3H), 2.27-2.21 (m, 2H), 2.10-2.04 (m, 1H), 1.92-1.87 (m, 4H), 1.67-1.59 (m, 2H), 1.52-1.42 (m, 2H), 1.40-1.30 (m, 4H), 1.23-1.20 (m, 2H), 0.95 (s, 12H). m/z 1060.50 [M+H]⁺. |
| 60 | ¹H NMR (400 MHz, CDCl₃) δ 8.69 (s, 1H), 8.25 (d, J=1.4 Hz, 1H), 8.21 (d, J=1.2 Hz, 1H), 7.73 (d, J=5.5 Hz, 1H), 7.35 (dd, J=7.7, 4.6 Hz, 1H), 7.30-7.20 (m, 1H), 7.02-6.96 (m, 2H), 6.86 (s, 1H), 5.94 (d, J=5.5 Hz, 1H), 5.23 (d, J=7.7 Hz, 1H), 4.71 (dd, J=13.6, 7.6 Hz, 1H), 4.65 - 4.61 (m, 1H), 4.57-4.38 (m, 4H), 4.01 (d, J=10.4 Hz, 1H), 3.84-3.72 (m, 4H), 3.71 - 3.61 (m, 4H), 3.60 - 3.57 (m, 1H), 3.43-3.40 (m, 1H), 3.03 - 2.97 (m, 1H), 2.74 - 2.68 (m, 2H), 2.61-2.52 (m, 1H), 2.52 (s, 3H), 2.29-2.21 (m, 2H), 2.09 - 2.03 (m, 1H), 1.95 - 1.84 (m, 4H), 1.70 - 1.64 (m, 2H), 1.37 - 1.25 (m, 6H), 1.24 (s, 3H), 0.93 (d, J=13.4 Hz, 9H). m/z 1046.54 [M+H]⁺. |
| 61 | ¹H NMR (400 MHz, DMSO-d₆) δ 8.98 (s, 1H), 8.49-8.44 (brs, 1H), 8.47 (d, J=0.8 Hz, 1H), 8.29 (d, J=0.8 Hz, 1H), 7.73 (d, J=5.4 Hz, 1H), 7.42 (dd, J=7.8, 3.8 Hz, 1H), 7.27 (dd, J=9.2, 2.6 Hz, 1H), 7.08 (s, 1H), 6.95 (d, J=8.8 Hz, 1H), 6.67 (d, J=6.9 Hz, 1H), 5.83 (d, J=5.4 Hz, 1H), 5.17 (d, J=3.6 Hz, 1H), 4.80-4.75 (m, 1H), 4.59 (d, J=9.3 Hz, 1H), 4.50 (t, J=8.2 Hz, 1H), 4.42 (dd, J=14.8, 7.7 Hz, 1H), 4.35-4.31 (m, 1H), 4.27 (t, J=6.2 Hz, 2H), 3.97-3.89 (m, 2H), 3.73-3.58 (m, 4H), 3.53-3.43 (m, 5H), 2.79 (s, 2H), 2.45 (s, 3H), 2.34-2.25 (m, 2H), 2.10-2.01 (m, 1H), 1.93-1.84 (m, 2H), 1.70-1.65 (m, 2H), 1.58-1.52 (m, 2H), 1.47-1.44 (m, 2H), 1.40-1.33 (m, 2H), 1.23-1.20 (m, 4H), 1.09 (s, 3H), 0.95 (s, 9H). m/z 1060.51 [M+H]⁺. |
| 62 | ¹H NMR (400 MHz, DMSO-d₆) δ 8.99 (s, 1H), 8.48-8.45 (m, 2H), 8.29 (d, J=1.2 Hz, 1H), 7.75 (d, J=5.4 Hz, 1H), 7.46-7.41 (m, 2H), 7.27 (dd, J=9.1, 2.7 Hz, 1H), 7.09 (d, J=0.9 Hz, 1H), 6.96 (dd, J=7.8, 1.1 Hz, 1H), 5.99 (d, J=7.9 Hz, 1H), 5.80 (d, J=5.4 Hz, 1H), 5.17 (d, J=3.6 Hz, 1H), 4.81-4.76 (m, 1H), 4.59 (d, J=9.2 Hz, 1H), 4.51 (t, J=8.2 Hz, 1H), 4.37-4.32 (m, 1H), 4.27 (t, J=6.0 Hz, 2H), 3.97-3.91 (m, 2H), 3.88-3.80 (m, 1H), 3.76-3.57 (m, 3H), 3.50-3.43 (m, 5H), 2.78 (s, 2H), 2.68-2.54 (m, 1H), 2.46 (s, 3H), 2.10-2.05 (m, 1H), 1.99-1.82 (m, 5H), 1.75-1.70 (m, 2H), 1.58-1.53 (m, 2H), 1.47-1.44 (m, 6H), 1.38-1.33 (m, 2H), 1.21-1.19 (m, 2H), 1.09 (s, 3H), 0.96 (s, 9H). m/z 1088.51 [M+H]⁺. |
| 63 | ¹H NMR (400 MHz, CDCl₃) δ 8.69 (s, 1H), 8.25 (d, J=1.2 Hz, 1H), 8.20 (d, J=1.2 Hz, 1H), 7.77 (dd, J=5.4, 1.3 Hz, 1H), 7.37-7.30 (m, 1H), 7.24 - 7.22 (m, 1H), 7.02-6.97 (m, 2H), 6.87 (s, 1H), 6.04 (dd, J=5.4, 1.2 Hz, 1H), 4.75-4.70 (m, 1H), 4.68-4.64 (m, 1H), 4.55-4.41 (m, 8H), 4.02-3.93 (m, 3H), 3.89-3.80 (m, 1H), 3.75-3.57 (m, 4H), 3.42-3.39 (m, 2H), 3.37-3.30 (m, 1H), 2.61 (s, 2H), 2.58-2.55 (m, 1H), 2.53 (s, 3H), 2.09-2.04 (m, 1H), 2.02-1.89 (m, 4H), 1.50-1.46 (m, 4H), 1.37-1.25 (m, 4H), 1.05 (s, 3H), 0.93 (d, J=14.7 Hz, 9H). m/z 1046.50 [M+H]⁺. |
| 64 | ¹H NMR (400 MHz, CDCl₃) δ 8.69 (s, 1H), 8.25 (d, J=1.3 Hz, 1H), 8.20 (d, J=1.3 Hz, 1H), 7.75 (d, J=5.4 Hz, 1H), 7.35 (dd, J=7.7, 3.5 Hz, 1H), 7.33-7.30 (m, 1H), 7.03-6.97 (m, 2H), 6.87 (s, 1H), 5.99 (d, J=5.4 Hz, 1H), 4.75-4.70 (m, 1H), 4.66-4.62 (m, 1H), 4.56-4.44 (m, 6H), 4.05-3.89 (m, 5H), 3.83-3.58 (m, 4H), 3.54-3.48 (m, 1H), 3.45-3.39 (m, 2H), 3.10-3.03 (m, 1H), 2.76 (d, J=7.3 Hz, 2H), 2.63 (s, 2H), 2.61-2.56 (m, 1H), 2.52 (s, 3H), 2.10-1.83 (m, 5H), 1.63-1.55 (m, 2H), 1.52-1.47 (m, 2H), 1.34-1.31 (m, 3H), 1.28-1.24 (m, 1H), 1.07 (s, 3H), 0.93 (d, J=16.8 Hz, 9H). m/z 1060.54 [M+H]⁺. |
| 65 | ¹H NMR (400 MHz, CDCl₃) δ 8.69 (s, 1H), 8.26 (d, J=1.2 Hz, 1H), 8.21 (d, J=1.2 Hz, 1H), 7.88 (d, J=5.3 Hz, 1H), 7.35 (d, J=7.8 Hz, 1H), 7.30-7.19 (m, 1H), 7.00-6.97 (m, 2H), 6.89 (s, 1H), 6.24 (d, J=5.4 Hz, 1H), 4.78-4.70 (m, 1H), 4.70-4.62 (m, 1H), 4.63-4.36 (m, 5H), 4.07-3.99 (m, 1H), 3.98-3.93 (m, 2H), 3.85-3.82 (m, 3H), 3.78-3.66 (m, 3H), 3.66-3.49 (m, 2H), 3.46-3.40 (m, 2H), 2.88-2.82 (m, 2H), 2.74-2.66 (m, 1H), 2.61-2.56 (m, 3H), 2.53 (s, 3H), 2.10-2.07 (m, 4H), 1.99-1.90 (m, 2H), 1.84-1.77 (m, 2H), 1.51-1.43 (m, 2H), 1.36-1.31 (m, 2H), 1.29-1.24 (m, 3H), 1.05 (s, 3H), 0.96-0.91 (m, 9H). m/z 1074.57 [M+H]⁺. |
| 66 | ¹H NMR (400 MHz, CDCl₃) δ 8.69 (s, 1H), 8.27 (d, J=1.2 Hz, 1H), 8.23 (d, J=1.2 Hz, 1H), 7.88 (d, J=5.4 Hz, 1H), 7.37-7.34 (m, 1H), 7.32-7.21 (m, 1H), 7.03-6.97 (m, 2H), 6.88 (s, 1H), 6.24 (d, J=5.4 Hz, 1H), 4.76 - 4.70 (m, 1H), 4.66 (brs, 1H), 4.62-4.39 (m, 4H), 4.03 (t, J=9.2 Hz, 1H), 3.84-3.78 (m, 6H), 3.72-3.66 (m, 3H), 3.65-3.52 (m, 2H), 2.85 (t, J=11.6 Hz, 2H), 2.72 - 2.68 (m, 1H), 2.60-2.57 (m, 1H), 2.53 (s, 3H), 2.11-2.05 (m , 5H), 1.97-1.94 (m, 2H), 1.88-1.75 (m, 4H), 1.35-1.26 (m, 6H), 1.24 (s, 3H), 0.94 (d, J=22.7 Hz, 9H). m/z 1060.51 [M+H]⁺. |
| 67 | ¹H NMR (400 MHz, CDCl₃) δ 8.69 (s, 1H), 8.27 (d, J=1.4 Hz, 1H), 8.23 (d, J=1.4 Hz, 1H), 7.88 (d, J=5.4 Hz, 1H), 7.36-7.33 (m, 1H), 7.32-7.20 (m, 1H), 7.02-6.97 (m, 2H), 6.88 (s, 1H), 6.23 (d, J=5.3 Hz 1H), 4.76 - 4.70 (m, 1H), 4.69-4.63 (m, 1H), 4.60-4.38 (m, 4H), 4.23-4.17 (m, 1H), 4.05-3.91 (m, 3H), 3.85-3.77 (m, 6H), 3.71 - 3.69 (m, 2H), 3.63 - 3.56 (m, 2H), 3.54 - 3.48 (m, 1H), 3.44 - 3.38 (m, 1H), 3.02 - 3.01 (m, 1H), 2.88 - 2.82 (m, 2H), 2.74 - 2.68 (m, 1H), 2.64 - 2.55 (m, 1H), 2.53 (s, 3H), 2.10-2.05 (m, 4H), 1.98-1.87 (m, 2H), 1.82-1.76 (m, 4H), 1.72-1.67 (m, 2H), 1.37 - 1.31 (m, 4H), 1.28-1.24 (m, 3H), 0.93 (d, J=22.5 Hz, 9H). m/z 1116.43 [M+H]⁺. |
| 68 | ¹H NMR (400 MHz, CDCl₃) δ 8.69 (s, 1H), 8.26 (d, J=1.3 Hz, 1H), 8.21 (d, J=1.3 Hz, 1H), 7.85 (d, J=5.4 Hz, 1H), 7.35 (d, J=7.7 Hz, 1H), 7.21 (t, J=5.9 Hz, 1H), 7.01-6.96 (m, 2H), 6.87 (d, J=1.3 Hz, 1H), 6.21 (d, J=5.4 Hz, 1H), 4.71 (t, J=7.6 Hz, 1H), 4.68-4.61 (m, 1H), 4.57-4.41 (m, 4H), 4.02-3.91 (m, 3H), 3.91-3.81 (m, 1H), 3.79 - 3.73 (m, 3H), 3.63 (dd, J=11.2, 4.1 Hz, 1H), 3.46 - 3.40 (m, 4H), 3.25 - 3.18 (m, 2H), 2.60 (s, 2H), 2.57 - 2.54 (m, 1H), 2.53 (s, 3H), 2.39-2.30 (m, 2H), 2.10-2.03 (m, 1H), 2.01-1.85 (m, 4H), 1.74-1.68 (m, 2H), 1.52-1.42 (m, 2H), 1.37 (s, 3H), 1.35-1.23 (m, 6H), 1.05 (s, 3H), 0.93 (s, 9H). m/z 1088.57 [M+H]⁺. |
| 69 | ¹H NMR (400 MHz, CDCl₃) δ 8.69 (s, 1H), 8.27 (d, J=1.3 Hz, 1H), 8.22 (d, J=1.3 Hz, 1H), 7.88 (d, J=5.4 Hz, 1H), 7.36 (d, J=7.8 Hz, 1H), 7.32-7.27 (m, 1H), 7.03-6.95 (m, 2H), 6.88 (s, 1H), 6.25 (d, J=5.3 Hz, 1H), 4.79-4.63 (m, 2H), 4.59-4.37 (m, 4H), 4.06-3.78 (m, 6H), 3.77-3.57 (m, 4H), 3.48-3.38 (m, 2H), 3.22 (t, J=11.7 Hz, 2H), 2.68-2.40 (m, 8H), 2.16-1.88 (m, 7H), 1.72-1.43 (m, 4H), 1.38-1.24 (m, 4H), 1.05 (s, 3H), 0.93 (s, 9H). m/z 1092.52 [M+H]⁺. |
| 70 | ¹H NMR (400 MHz, CDCl₃) δ 8.69 (s, 1H), 8.26 (d, J=1.3 Hz, 1H), 8.21 (d, J=1.3 Hz, 1H), 7.87 (d, J=5.4 Hz, 1H), 7.36 (d, J=7.8 Hz, 1H), 7.26-7.19 (m, 1H), 7.03-6.95 (m, 2H), 6.88 (s, 1H), 6.22 (d, J=5.4 Hz, 1H), 4.75-4.65 (m, 2H), 4.57-4.42 (m, 4H), 4.05-3.82 (m, 7H), 3.74-3.57 (m, 3H), 3.47-3.38 (m, 2H), 3.33 (t, J=12.0 Hz, 2H), 2.62-2.49 (m, 6H), 2.42-2.29 (m, 2H), 2.11-1.92 (m, 5H), 1.75 (d, J=13.5 Hz, 2H), 1.60-1.43 (m, 4H), 1.34-1.24 (m, 4H), 1.05 (s, 3H), 0.95 (s, 9H). m/z 1090.60 [M+H]⁺. |
| 71 | ¹H NMR (400 MHz, CDCl₃) δ 8.69 (s, 1H), 8.26 (d, J=1.3 Hz, 1H), 8.21 (d, J=1.3 Hz, 1H), 7.87 (d, J=5.3 Hz, 1H), 7.38-7.32 (m, 1H), 7.25-7.21 (m, 1H), 7.03-6.95 (m, 2H), 6.88 (s, 1H), 6.21 (d, J=5.3 Hz, 1H), 4.77-4.61 (m, 2H), 4.60-4.36 (m, 4H), 4.05-3.92 (m, 3H), 3.86-3.67 (m, 5H), 3.67-3.58 (m, 1H), 3.58-3.47 (m, 1H), 3.47-3.35 (m, 2H), 2.84 (t, J=11.5 Hz, 2H), 2.63-2.54 (m, 3H), 2.53 (s, 3H), 2.40-2.31 (m, 2H), 2.13-1.91 (m, 6H), 1.87 (d, J=13.1 Hz, 2H), 1.63-1.42 (m, 6H), 1.38-1.22 (m, 4H), 1.05 (s, 3H), 0.93 (d, J=18.0 Hz, 9H). m/z 1088.52 [M+H]⁺. |
| 72 | ¹H NMR (400 MHz, CDCl₃) δ 8.69 (s, 1H), 8.26 (d, J=1.3 Hz, 1H), 8.21 (d, J=1.3 Hz, 1H), 7.88 (d, J=5.3 Hz, 1H), 7.36 (dd, J=7.8, 3.2 Hz, 1H), 7.30 (t, J=5.9 Hz, 1H), 7.04-6.97 (m, 2H), 6.87 (s, 1H), 6.21 (d, J=5.3 Hz, 1H), 4.75-4.65 (m, 2H), 4.57-4.41 (m, 4H), 4.04-3.94 (m, 3H), 3.91-3.63 (m, 4H), 3.62 - 3.56 (m, 1H), 3.54 - 3.50 (m, 2H), 3.46 - 3.39 (m, 2H), 3.35 - 3.30 (m, 2H), 2.63 (s, 2H), 2.63 - 2.52 (m, 1H), 2.53 (s, 3H), 2.10-2.05 (m, 1H), 1.97-1.90 (m, 6H), 1.63-1.47 (m, 8H), 1.36-1.29 (m, 4H), 1.07 (s, 3H), 0.94 (d, J=15.5 Hz, 9H). m/z 1104.49 [M+H]⁺. |
| 73 | ¹H NMR (400 MHz, CDCl₃) δ 8.69 (s, 1H), 8.26 (d, J=1.2 Hz, 1H), 8.21 (d, J=1.2 Hz, 1H), 7.86 (d, J=5.4 Hz, 1H), 7.39 (t, J=7.5 Hz, 1H), 7.33-7.29 (m, 1H), 7.09-7.00 (m, 2H), 6.54 (s, 1H), 6.23 (d, J=5.4 Hz, 1H), 5.07-5.01 (m, 1H), 4.72 (t, J=7.7 Hz, 1H), 4.64-4.59 (m, 1H), 4.55-4.53 (m, 2H), 4.52-4.49 (m, 2H), 4.48-4.42 (m, 1H), 4.41-4.31 (m, 1H), 4.19-4.10 (m, 1H), 4.04-3.93 (m, 3H), 3.81 (d, J=12.4 Hz, 2H), 3.64 (dd, J=11.2, 3.7 Hz, 1H), 3.46-3.39 (m, 2H), 2.85-2.77 (m, 2H), 2.62 (s, 2H), 2.56-2.50 (m, 4H), 2.42-2.34 (m, 1H), 2.13-2.08 (m, 1H), 2.05-2.02 (m, 2H), 1.82-1.77 (m, 2H), 1.62-1.55 (m, 2H), 1.50-1.46 (m, 2H), 1.33-1.26 (m, 4H), 1.06 (s, 3H), 0.96 (d, J=13.5 Hz, 9H). m/z 1046.46 [M+H]⁺. |
| 74 | ¹H NMR (400 MHz, CDCl₃) δ 8.68 (s, 1H), 8.25 (d, J=1.3 Hz, 1H), 8.19 (d, J=1.3 Hz, 1H), 7.72 (d, J=5.5 Hz, 1H), 7.36 - 7.33 (m, 1H), 7.31 - 7.22 (m, 1H), 6.99 - 6.95 (m, 1H), 6.86 (s, 1H), 6.18 (dd, J=39.9, 8.8 Hz, 1H), 5.95 (dd, J=5.5, 1.6 Hz, 1H), 5.23 (d, J=7.6 Hz, 1H), 4.71 - 4.65 (m, 1H), 4.63 - 4.59 (m, 1H), 4.54 - 4.40 (m, 5H), 4.05 (t, J=11.5 Hz, 1H), 3.98 - 3.92 (m, 2H), 3.81-3.72 (m, 2H), 3.67 - 3.59 (m, 1H), 3.58 - 3.53 (m, 1H), 3.45 - 3.38 (m, 3H), 3.03 - 2.97 (m, 1H), 2.75-2.67 (m, 2H), 2.60 (s, 2H), 2.58 - 2.54 (m, 1H), 2.52 (s, 3H), 2.30-2.21 (m, 2H), 2.09 - 2.06 (m, 1H), 2.04 - 2.01 (m, 3H), 1.97-1.84 (m, 4H), 1.58-1.54 (m, 2H), 1.50 - 1.44 (m, 2H), 1.04 (s, 3H), 0.90 (d, J=4.7 Hz, 9H). m/z 1016.52 [M+H]⁺. |
| 75 | ¹H NMR (400 MHz, CDCl₃) δ 8.69 (s, 1H), 8.26 (d, J=0.9 Hz, 1H), 8.21 (d, J=1.1 Hz, 1H), 7.87 (d, J=5.3 Hz, 1H), 7.37-7.34 (m, 2H), 6.97 (d, J=7.9 Hz, 1H), 6.88 (s, 1H), 6.26-6.07 (m, 2H), 4.74-4.63 (m, 2H), 4.57-4.41 (m, 4H), 4.08 (d, J=11.3 Hz, 1H), 3.98 - 3.95 (m, 2H), 3.85-3.73 (m, 4H), 3.60-3.52 (m, 2H), 3.50-3.39 (m, 2H), 3.08 (s, 2H), 2.90-2.80 (m, 3H), 2.73-2.67 (m, 1H), 2.62 - 2.56 (m, 1H), 2.53 (s, 3H), 2.14-1.94 (m, 9H), 1.81 - 1.69 (m, 5H), 1.55-1.47 (m, 2H), 1.08 (s, 3H), 0.91 (d, J=12.0 Hz, 9H). m/z 1030.56 [M+H]⁺. |
| 76 | ¹H NMR (400 MHz, CDCl₃) δ 8.69 (s, 1H), 8.26 (d, J=1.1 Hz, 1H), 8.23 (d, J=1.1 Hz, 1H), 7.87 (d, J=5.3 Hz, 1H), 7.37 - 7.34 (m, 2H), 6.97 (d, J=7.9 Hz, 1H), 6.88 (s, 1H), 6.24 - 6.08 (m, 2H), 4.74-4.62 (m, 2H), 4.56-4.41 (m, 4H), 4.08 - 4.05 (m, 1H), 3.84-3.78 (m, 7H), 3.72-3.65 (m, 2H), 3.60 - 3.56 (m, 2H), 2.87 - 2.82 (m, 2H), 2.73-2.67 (m, 1H), 2.61-2.52 (m, 1H), 2.52 (s, 3H), 2.09-2.05 (m, 5H), 2.01 - 1.94 (m, 5H), 1.85 - 1.77 (m, 2H), 1.70-1.63 (m, 4H), 1.24 (s, 3H), 0.91 (d, J=11.6 Hz, 9H). m/z 1016.43 [M+H]⁺. |
| 77 | ¹H NMR (400 MHz, CDCl₃) δ 8.69 (s, 1H), 8.27 (d, J=1.2 Hz, 1H), 8.23 (d, J=1.2 Hz, 1H), 7.88 (d, J=5.3 Hz, 1H), 7.37 - 7.34 (m, 2H), 6.97 (d, J=7.8 Hz, 1H), 6.88 (s, 1H), 6.26-6.08 (m, 2H), 4.74-4.66 (m, 2H), 4.57-4.40 (m, 4H), 4.22-4.17 (m, 1H), 4.08 - 4.06 (m, 1H), 4.01-3.91 (m, 2H), 3.85-3.75 (m, 5H), 3.74-3.67 (m, 2H), 3.60-3.48 (m, 3H), 3.44 - 3.38 (m, 1H), 3.01 (d, J=4.6 Hz, 1H), 2.88 - 2.82 (m, 2H), 2.73 - 2.67 (m, 1H), 2.61-2.55 (m, 1H), 2.52 (s, 3H), 2.13-2.04 (m, 5H), 2.01 - 1.95 (m, 5H), 1.94-1.87 (m, 2H), 1.81 - 1.69 (m, 4H), 1.25 - 1.23 (m, 3H), 0.91 (d, J=11.9 Hz, 9H). m/z 1072.36 [M+H]⁺. |
| 78 | ¹H NMR (400 MHz, DMSO-d6) δ 8.99 (s, 1H), 8.42 (t, J=6.0 Hz, 1H), 8.32 (s, 1H), 7.98-7.90 (m, 1H), 7.48 (d, J=5.3 Hz, 1H), 7.10 (s, 1H), 6.91 (d, J=8.7 Hz, 1H), 6.32 (d, J=5.3 Hz, 1H), 5.46 (t, J=5.7 Hz, 1H), 5.12 (d, J=3.4 Hz, 1H), 4.85-4.74 (m, 1H), 4.61-4.40 (m, 4H), 4.40-4.27 (m, 2H), 4.21 (dd, J=16.5, 5.4 Hz, 1H), 3.79-3.58 (m, 8H), 3.55 - 3.46 (m, 2H), 2.94-2.84 (m, 4H), 2.46 (m, 5H), 2.06-1.94 (m, 4H), 1.89 (s, 3H), 1.80-1.67 (m, 6H), 1.59 - 1.52 (m, 2H), 1.38 - 1.29 (m, 2H), 0.95 (s, 3H), 0.92 (s, 9H). m/z 1060.52 [M+H]⁺. |
| 79 | ¹H NMR (400 MHz, CDCl₃) δ 8.69 (s, 1H), 8.28 (s, 1H), 7.90 (d, J=5.3 Hz, 1H), 7.40-7.32 (m, 2H), 6.97 (d, J=7.6 Hz, 1H), 6.88 (s, 1H), 6.28 (d, J=5.3 Hz, 1H), 6.25-6.01 (m, 1H), 4.76-4.62 (m, 4H), 4.59-4.42 (m, 4H), 4.08 (d, J=10.9 Hz, 1H), 3.88-3.71 (m, 5H), 3.61-3.51 (m, 2H), 3.47-3.33 (m, 4H), 2.92-2.81 (m, 2H), 2.77-2.67 (m, 1H), 2.64-2.55 (m, 1H), 2.53 (s, 3H), 2.12-1.93 (m, 10H), 1.87-1.76 (m, 2H), 1.76-1.67 (m, 4H), 1.23 (s, 3H), 0.91 (d, J=13.1 Hz, 9H). m/z 1068.61 [M+Na]⁺. |
| 80 | ¹H NMR (400 MHz, CDCl₃) δ 8.69 (s, 1H), 8.29 (s, 1H), 7.91 (d, J=5.3 Hz, 1H), 7.41-7.30 (m, 2H), 6.97 (d, J=7.6 Hz, 1H), 6.88 (s, 1H), 6.29 (d, J=5.3 Hz, 1H), 6.24 - 6.05 (m, 1H), 4.77-4.70 (m, 1H), 4.68 (s, 2H), 4.67 - 4.62 (m, 1H) 4.59-4.39 (m, 4H), 4.23-4.13 (m, 1H), 4.08 (d, J=11.5 Hz, 1H), 3.90-3.75 (m, 6H), 3.71 - 3.69 (m, 1H), 3.64-3.50 (m, 4H), 3.28-3.19 (m, 1H), 3.18-3.07 (m, 1H), 3.01 (d, J=4.5 Hz, 1H), 2.94-2.79 (m. 2H), 2.78-2.64 (m. 1H), 2.64-2.55 (m, 1H), 2.53 (s, 3H), 2.13 - 2.09 (m, 1H), 2.05 (s, 3H), 2.02-1.92 (m, 6H), 1.86-1.70 (m, 6H), 1.24 (d, J=6.4 Hz, 3H), 0.91 (d, J=13.2 Hz, 9H). m/z 1102.64 [M+H]⁺. |
| 81 | ¹H NMR (400 MHz, CDCl₃) δ 8.69 (s, 1H), 7.89 (d, J=5.3 Hz, 1H), 7.39 - 7.34 (m, 2H), 6.97 (d, J=8.1 Hz, 1H), 6.88 (s, 1H), 6.23-6.03 (m, 2H), 4.76-4.65 (m, 2H), 4.63 (s, 2H), 4.58 - 4.38 (m, 4H), 4.08 (d, J=11.2 Hz, 1H), 3.92-3.70 (m, 5H), 3.60 - 3.56 (m, 2H), 3.50 - 3.44 (m, 2H), 3.28-3.17 (m, 2H), 2.92-2.80 (m, 2H), 2.76-2.67 (m, 1H), 2.63-2.57 (m, 3H), 2.53 (s, 6H), 2.15-2.05 (m, 5H), 2.03-1.97 (m, 5H), 1.85 - 1.78 (m, 2H), 1.65-1.60 (m, 2H), 1.50 - 1.46 (m, 2H), 1.03 (s, 3H), 0.91 (d, J=13.3 Hz, 9H). m/z 1074.70 [M+H]⁺. |
| 82 | ¹H NMR (400 MHz, CDCl₃) δ 8.69 (s, 1H), 8.20 (s, 1H), 8.16 (s, 1H), 7.40-7.28 (m, 2H), 7.05 (t, J=7.8 Hz, 1H), 6.97 (d, J=7.7 Hz, 1H), 6.91-6.82 (m, 2H), 6.64 (d, J=7.8 Hz, 1H), 6.26-6.04 (m, 1H), 4.76-4.61 (m, 2H), 4.58-4.38 (m, 4H), 4.07 (d, J=11.1 Hz, 1H), 3.95-3.87 (m, 2H), 3.86-3.66 (m, 3H), 3.63-3.49 (m, 2H), 3.47-3.33 (m, 4H), 2.75-2.63 (m, 3H), 2.62-2.54 (m, 3H), 2.53 (s, 3H), 2.17-1.92 (m, 10H), 1.87-1.75 (m, 2H), 1.61-1.51 (m, 2H), 1.51-1.41 (m, 2H), 1.03 (s, 3H), 0.91 (d, J=13.1 Hz, 9H). m/z 1051.63 [M+Na]⁺. |
| 83 | ¹H NMR (400 MHz, CDCl₃) δ 8.69 (s, 1H), 8.04 (s, 1H), 7.43 - 7.34 (m, 2H), 7.14 (t, J=7.8 Hz, 1H), 6.99 - 6.97 (m, 3H), 6.88 (s, 1H), 6.21 (dd, J=74.3, 9.2 Hz, 1H), 4.75 - 4.67 (m, 2H), 4.62 (s, 2H), 4.58-4.52 (m, 2H), 4.49 - 4.45 (m, 2H), 4.07 (d, J=11.5 Hz, 1H), 3.89-3.68 (m, 3H), 3.62-3.68 (m, 2H), 3.47 - 3.36 (m, 2H), 3.32-3.27 (m, 2H), 3.16 - 3.09 (m, 2H), 2.76-2.63 (m, 3H), 2.60 - 2.57 (m, 2H), 2.56-2.55 (m, 1H), 2.53 (s, 3H), 2.13 - 2.12 (m, 1H), 2.09 - 2.05 (m, 3H), 2.02 - 1.98 (m, 4H), 1.84 - 1.78 (m, 4H), 1.63 - 1.56 (m, 2H), 1.49-1.41 (m, 2H), 1.00 (s, 3H), 0.91 (d, J=13.1 Hz, 9H). m/z 1059.89 [M+H]⁺. |
| 84 | ¹H NMR (400 MHz, CDCl₃) δ 8.69 (s, 1H), 8.04 (s, 1H), 7.41 - 7.34 (m, 2H), 7.15 (t, J=7.9 Hz, 1H), 7.00 - 6.97 (m, 3H), 6.89 (s, 1H), 6.31-6.09 (m, 1H), 4.73 - 4.67 (m, 2H), 4.63 (s, 2H), 4.55-4.44 (m, 4H), 4.09 (d, J=11.2 Hz, 1H), 3.91-3.67 (m, 4H), 3.58-3.55 (m, 1H), 3.47-3.37 (m, 2H), 3.33-3.18 (m, 4H), 2.77-2.64 (m, 3H), 2.61-2.55 (m, 1H), 2.53 (s, 3H), 2.11-1.98 (m, 12H), 1.83-1.77 (m, 2H), 1.71-1.69 (m, 2H), 1.21 (s, 3H), 0.91 (d, J=13.7 Hz, 9H). m/z 1045.85 [M+H]⁺. |
| 85 | ¹H NMR (400 MHz, CDCl₃) δ 8.69 (s, 1H), 8.02 (s, 1H), 7.43-7.32 (m, 2H), 7.16 (t, J=7.9 Hz, 1H), 7.05-6.95 (m, 3H), 6.88 (s, 1H), 6.31-6.05 (m, 1H), 4.75-4.65 (m, 2H), 4.63 (s, 2H), 4.58-4.38 (m, 4H), 4.22-4.11 (m, 1H), 4.08 (d, J=12.1 Hz, 1H), 3.88-3.65 (m, 5H), 3.61-3.51 (m, 2H), 3.47-3.28 (m, 4H), 3.15-3.05 (m, 1H), 3.04-2.95 (m, 2H), 2.79-2.62 (m, 3H), 2.62-2.54 (m, 1H), 2.53 (s, 3H), 2.16-1.86 (m, 12H), 1.83-1.68 (m, 4H), 1.23 (d, J=6.4 Hz, 3H), 0.91 (d, J=14.0 Hz, 9H). m/z 1101.81 [M+H]⁺. |
| 86 | ¹H NMR (400 MHz, CDCl₃) δ 8.69 (s, 1H), 8.26 (d, J=1.2 Hz, 1H), 8.21 (d, J=1.3 Hz, 1H), 7.87 (d, J=5.3 Hz, 1H), 7.49 (dd, J=33.9, 8.5 Hz, 1H), 7.32 (d, J=7.5 Hz, 1H), 7.09 (s, 1H), 7.02 (d, J=9.1 Hz, 1H), 6.90 (s, 1H), 6.23 (d, J=5.4 Hz, 1H), 5.41-5.37 (m, 1H), 4.75 - 4.69 (m, 2H), 4.59 (d, J=5.3 Hz, 1H), 4.53 (s, 1H), 4.05 (t, J=12.6 Hz, 1H), 3.99 - 3.94 (m, 2H), 3.84 - 3.69 (m, 5H), 3.65 - 3.49 (m, 2H), 3.46 - 3.39 (m, 2H), 2.85 (t, J=11.7 Hz, 2H), 2.74 - 2.66 (m, 1H), 2.60 (s, 2H), 2.58 - 2.50 (m, 4H), 2.09 - 1.92 (m, 5H), 1.86 - 1.76 (m, 2H), 1.58 - 1.55 (m, 3H), 1.51 - 1.45 (m, 6H), 1.39 - 1.23 (m, 4H), 1.09 (s, 9H), 1.05 (s, 3H). m/z 1088.44 [M+H]⁺. |
| 87 | ¹H NMR (400 MHz, CDCl₃) δ 8.68 (s, 1H), 8.26 (s, 1H), 8.21 (s, 1H), 7.86 (d, J=4.8 Hz, 1H), 7.69-7.52 (m, 1H), 7.30 (d, J=7.7 Hz, 1H), 7.00 (t, J=8.1 Hz, 1H), 6.91 (s, 1H), 6.43-6.14 (m, 2H), 5.39-5.32 (m, 1H), 4.75-4.67 (m, 2H), 4.64-4.52 (m, 2H), 4.09 (dd, J=22.3, 12.1 Hz, 1H), 3.99-3.93 (m, 3H), 3.88-3.78 (m, 4H), 3.64-3.49 (m, 3H), 3.47-3.39 (m, 2H), 2.87-2.82 (m, 2H), 2.76-2.68 (m, 1H), 2.61 (s, 2H), 2.58-2.56 (m, 1H), 2.53 (s, 3H), 2.10-1.97 (m, 8H), 1.80-1.44 (m, 10H), 1.05 (m, 12H). m/z 1044.59 [M+H]⁺. |
| 88 | ¹H NMR (400 MHz, CDCl₃) δ 8.69 (s, 1H), 8.28 (s, 1H), 7.93-7.86 (m, 1H), 7.53-7.19 (m, 1H), 7.39-7.32 (m, 1H), 7.17-6.85 (m, 1H), 6.97 (d, J=7.7 Hz, 1H), 6.90 (s, 1H), 6.33-6.27 (m, 1H), 4.78-4.61 (m, 4H), 4.59-4.47 (m, 3H), 4.45-4.30 (m, 1H), 4.22-4.13 (m, 1H), 4.00-3.74 (m, 6H), 3.74-3.65 (m, 2H), 3.65-3.48 (m, 6H), 3.28-3.20 (m, 1H), 3.17-3.07 (m, 1H), 3.01 (d, J=4.6 Hz, 1H), 2.93 - 2.81 (m, 2H), 2.77-2.68 (m, 1H), 2.64-2.54 (m, 1H), 2.53 (s, 3H), 2.15-1.89 (m, 9H), 1.84-1.69 (m, 4H), 1.24 (d, J=6.4 Hz, 3H), 0.98 - 0.86 (m, 9H). m/z 1127.04 [M+H]⁺. |
| 89 | ¹H NMR (400 MHz, CDCl₃) δ 8.69 (s, 1H), 8.29 (s, 1H), 7.94-7.83 (m, 2H), 7.43-7.41 (m, 1H), 7.38-7.35 (m, 1H), 6.97 (d, J=7.7 Hz, 1H), 6.90 (s, 1H), 6.30 (d, J=5.3 Hz, 1H), 4.74-4.65 (m, 4H), 4.52-4.47 (m, 3H), 4.35 - 4.30 (m, 1H), 4.20 - 4.17 (m, 2H), 4.05 - 4.01 (m, 3H), 3.86-3.77 (m, 5H), 3.70 (d, J=8.8 Hz, 1H), 3.66 - 3.60 (m, 1H), 3.58 - 3.49 (m, 4H), 3.26 - 3.21 (m, 1H), 3.16 - 3.10 (m, 1H), 3.00 (d, J=4.6 Hz, 1H), 2.90-2.84 (m, 2H), 2.75 - 2.68 (m, 1H), 2.64 - 2.56 (m, 1H), 2.52 (s, 3H), 2.23 - 2.20 (s, 1H), 2.10-1.91 (m, 8H), 1.85-1.76 (m, 4H), 1.24 (d, J=6.4 Hz, 3H), 0.93 - 0.89 (m, 9H). m/z 1118.92 [M+H]⁺. |
| 90 | ¹H NMR (400 MHz, CDCl₃) δ 8.69 (s, 1H), 8.26 (s, 1H), 8.21 (s, 1H), 7.87 (d, J=4.8 Hz, 1H), 7.52-7.31 (m, 2H), 7.00-6.95 (m, 1H), 6.89 (s, 1H), 6.43-6.05 (m, 2H), 4.74- 4.67 (m, 2H), 4.55 - 4.54 (m, 1H), 4.49-4.34 (m, 2H), 4.17-4.10 (m, 1H), 3.99 - 3.91 (m, 2H), 3.85-3.66 (m, 5H), 3.62-3.49 (m, 2H), 3.47-3.39 (m, 2H), 3.08 - 2.81 (m, 3H), 2.75-2.68 (m, 1H), 2.65-2.56 (m, 3H), 2.53 (s, 3H), 2.12 - 2.06 (m, 4H), 2.01-1.91 (m, 7H), 1.84 - 1.78 (m, 2H), 1.61 - 1.55 (m, 2H), 1.51-1.42 (m, 2H), 1.05 (s, 3H), 0.96 - 0.88 (m, 3H), 0.84 (d, J=6.7 Hz, 3H). m/z 1016.65 [M+H]⁺. |
| 91 | ¹H NMR (400 MHz, CDCl₃) δ 8.69 (s, 1H), 8.26 (s, 1H), 8.21 (s, 1H), 7.88 (d, J=5.2 Hz, 1H), 7.41 - 7.30 (m, 2H), 6.97 (d, J=7.5 Hz, 1H), 6.89 (s, 1H), 6.52 - 6.29 (m, 1H), 6.23 (d, J=5.3 Hz, 1H), 4.77-4.72 (m, 1H), 4.70-4.64 (m, 1H), 4.58-4.39 (m, 3H), 4.23 - 4.14 (m, 1H), 4.00-3.94 (m, 3H), 3.84-3.71 (m, 5H), 3.62-3.53 (m, 2H), 3.46 - 3.39 (m, 2H), 2.88 - 2.82 (m, 2H), 2.76 - 2.65 (m, 1H), 2.60 - 2.56 (m, 3H), 2.52 (s, 3H), 2.14-2.10 (m, 1H), 2.04 (s, 3H), 1.99-1.90 (m, 4H), 1.83 - 1.74 (m, 2H), 1.62-1.53 (m, 4H), 1.50-1.45 (m, 2H), 1.20 - 1.12 (m, 1H), 1.05 (s, 3H), 0.57-0.43 (m, 2H), 0.42-0.27 (m, 2H). m/z 1014.52 [M+H]⁺. |
| 92 | ¹H NMR (400 MHz, CDCl₃) δ 8.69 (s, 1H), 8.26 (d, J=1.3 Hz, 1H), 8.21 (d, J=1.3 Hz, 1H), 7.87 (d, J=5.4 Hz, 1H), 7.31-7.26 (m, 1H), 7.20 (t, J=6.2 Hz, 1H), 7.01-6.93 (m, 1H), 6.89 (s, 1H), 6.24 (d, J=5.4 Hz, 1H), 5.92 (d, J=29.3 Hz, 1H), 4.77-4.71 (m, 1H), 4.69-4.57 (m, 2H), 4.51-4.32 (m, 2H), 4.00-3.92 (m, 2H), 3.88-3.68 (m, 5H), 3.68-3.59 (m, 2H), 3.59-3.50 (m, 2H), 3.47-3.38 (m, 2H), 2.85 (t, J=11.4 Hz, 2H), 2.76-2.67 (m, 1H), 2.67-2.62 (m, 1H), 2.60 (s, 2H) 2.53 (s, 3H), 2.47-2..36 (m, 1H), 2.17 (d, J=14.3 Hz, 3H), 2.07-1.93 (m, 6H), 1.86-1.75 (m, 3H), 1.63-1.54 (m, 2H), 1.53-1.42 (m, 2H), 1.07-1.00 (m, 6H), 0.87 (d, J=5.5 Hz, 3H). m/z 1040.61 [M+H]⁺. |
| 93 | ¹H NMR (400 MHz, CDCl₃) δ 8.69 (s, 1H), 8.26 (d, J=1.3 Hz, 1H), 8.21 (d, J=1.3 Hz, 1H), 7.87 (d, J=5.4 Hz, 1H), 7.39-7.31 (m, 2H), 6.99 (dd, J=7.8, 1.5 Hz, 1H), 6.89 (s, 1H), 6.24 (d, J=5.4 Hz, 1H), 6.08 (d, J=8.9 Hz, 1H), 4.70-4.59 (m, 3H), 4.56-4.37 (m, 2H), 3.99 - 3.93 (m, 2H), 3.84-3.72 (m, 5H), 3.63-3.54 (m, 3H), 3.49 - 3.39 (m, 2H), 2.88 - 2.83 (m, 2H), 2.78-2.67 (m, 1H), 2.60 (s, 3H), 2.53 (s, 3H), 2.42-2.30 (m, 1H), 2.27 (s, 3H), 2.12-2.00 (m, 4H), 2.00-1.88 (m, 4H), 1.86 - 1.77 (m, 2H), 1.61-1.56 (m, 2H), 1.50-1.44 (m, 2H), 1.10-0.99 (m, 3H), 0.93 - 0.86 (m, 3H), 0.83 - 0.82 (m, 3H). m/z 1040.66 [M+H]⁺. |
| 94 | ¹H NMR (400 MHz, CDCl₃) δ 8.69 (s, 1H), 8.26 (d, J=1.3 Hz, 1H), 8.23 (d, J=1.4 Hz, 1H), 7.87 (d, J=5.4 Hz, 1H), 7.41-7.30 (m, 2H), 6.99 (dd, J=7.7, 1.5 Hz, 1H), 6.89 (s, 1H), 6.24 (d, J=5.4 Hz, 1H), 6.08 (d, J=8.9 Hz, 1H), 4.70-4.36 (m, 5H), 3.88-3.74 (m, 7H), 3.74-3.62 (m, 3H), 3.62-3.51 (m, 3H), 2.85 (t, J=11.8 Hz, 2H), 2.76-2.67 (m, 1H), 2.63-2.55 (m, 1H), 2.53 (s, 3H), 2.40-2.29 (m, 1H), 2.27 (s, 3H), 2.13-1.91 (m, 7H), 1.85-1.76 (m, 2H), 1.73-1.48 (m, 4H), 1.24 (m, 3H), 0.93-0.81 (m, 6H). m/z 1026.68 [M+H]⁺. |
| 95 | ¹H NMR (400 MHz, CDCl₃) δ 8.69 (s, 1H), 8.27 (d, J=1.3 Hz,1H), 8.23 (d, J=1.3 Hz, 1H), 7.87 (d, J=5.3 Hz, 1H), 7.41-7.31 (m, 2H), 6.99 (dd, J=7.8, 1.5 Hz, 1H), 6.89 (s, 1H), 6.23 (d, J=5.4 Hz, 1H), 6.08 (d, J=9.1 Hz, 1H), 4.70 - 4.51 (m, 4H), 4.47-4.38 (m, 1H), 4.23-4.17 (m, 1H), 4.01-3.91 (m, 2H), 3.84-3.76 (m, 5H), 3.72 - 3.66 (m, 2H), 3.62 - 3.49 (m, 5H), 3.45-3.38 (m, 1H), 3.01 (d, J=4.6 Hz, 1H), 2.86 (t, J=11.6 Hz, 2H), 2.77-2.67 (m, 1H), 2.66-2.56 (m, 1H), 2.53 (s, 3H), 2.40-2.31 (m, 1H), 2.27 (s, 3H), 2.13-2.00 (m, 4H), 1.98 - 1.87 (m, 4H), 1.83-1.67 (m, 5H), 1.25 (d, J=6.4 Hz, 3H), 0.93-0.86 (m, 3H), 0.85-0.82 (m, 3H). m/z 1082.60 [M+H]⁺. |
| 96 | ¹H NMR (400 MHz, CDCl₃) δ 8.69 (s, 1H), 8.28 (s, 1H), 7.90 (d, J=5.3 Hz, 1H), 7.35-7.33 (m, 2H), 6.99 (d, J=8.9 Hz, 1H), 6.89 (s, 1H), 6.28 (d, J=5.3 Hz, 1H), 6.08 (d, J=9.3 Hz, 1H), 4.67 (s, 2H), 4.66 - 4.60 (m, 2H), 4.57-4.50 (m, 1H), 4.46 - 4.37 (m, 1H), 3.87-3.76 (m, 6H), 3.73 - 3.66 (m, 1H), 3.60 - 3.54 (m, 3H), 3.50-3.45 (m, 2H), 3.28-3.21 (m, 2H), 2.90 - 2.84 (m, 2H), 2.75-2.69 (m, 1H), 2.63 - 2.62 (m, 1H), 2.60 (s, 2H), 2.53 (s, 3H), 2.38-2.30 (m, 1H), 2.27 (s, 3H), 2.05-1.95 (m, 5H), 1.85-1.80 (m, 2H), 1.63-1.60 (m, 4H), 1.51-1.45 (m, 2H), 1.03 (s, 3H), 0.93 - 0.82 (m, 6H). m/z 1070.71 [M+H]⁺. |
| 97 | ¹H NMR (400 MHz, CDCl₃) δ 8.69 (s, 1H), 8.28 (s, 1H), 7.90 (d, J=5.3 Hz, 1H), 7.39-7.29 (m, 2H), 6.99 (dd, J=7.7, 1.4 Hz, 1H), 6.89 (s, 1H), 6.28 (d, J=5.3 Hz, 1H), 6.07 (d, J=8.9 Hz, 1H), 4.71-4.66 (m, 3H), 4.66-4.58 (m, 2H), 4.57-4.35 (m, 2H), 3.90-3.74 (m, 5H), 3.73-3.65 (m, 1H), 3.62-3.51 (m, 3H), 3.47-3.32 (m, 4H), 2.87 (t, J=12.0 Hz, 2H), 2.78-2.67 (m, 1H), 2.63-2.53 (m, 1H), 2.53 (s, 3H), 2.39-2.29 (m, 1H), 2.27 (s, 3H), 2.12-1.92 (m, 7H), 1.87-1.78 (m, 2H), 1.76-1.68 (m, 2H), 1.63-1.54 (m, 2H), 1.24 (s, 3H), 0.95-0.80 (m, 6H). m/z 1056.71 [M+H]⁺. |
| 98 | ¹H NMR (400 MHz, CDCl₃) δ 8.69 (s, 1H), 8.29 (s, 1H), 7.91 (d, J=5.3 Hz, 1H), 7.40-7.28 (m, 2H), 7.02-6.96 (m, 1H), 6.89 (s, 1H), 6.29 (d, J=5.3 Hz, 1H), 6.08 (d, J=9.5 Hz, 1H), 4.68 (s, 2H), 4.66 - 4.60 (m, 3H), 4.57 - 4.50 (m, 1H), 4.46 - 4.36 (m, 1H), 4.22-4.16 (m, 1H), 3.87-3.77 (m, 6H), 3.70 (d, J=8.8 Hz, 1H), 3.62-3.48 (m, 6H), 3.27 - 3.20 (m, 1H), 3.16-3.09 (m, 1H), 3.01 (d, J=4.6 Hz, 1H), 2.88 (t, J=12.0 Hz, 2H), 2.76 - 2.68 (m, 1H), 2.65 - 2.57 (m, 1H), 2.53 (s, 3H), 2.39 - 2.29 (m, 1H), 2.27 (s, 3H), 2.12 - 2.03 (m, 4H), 1.98-1.91 (m, 3H), 1.85-1.75 (m, 4H), 1.74-1.69 (m, 2H), 1.24 (d, J=6.4 Hz, 3H), 0.93-0.86 (m, 3H), 0.83 - 0.82 (m, 3H). m/z 1112.58 [M+H]⁺. |
| 99 | ¹H NMR (400 MHz, CDCl₃) δ 8.69 (s, 1H), 8.26 (d, J=1.1 Hz, 1H), 8.21 (d, J=1.3 Hz, 1H), 7.87 (d, J=5.4 Hz, 1H), 7.38 (d, J=4.6 Hz, 1H), 7.34 - 7.24 (m, 3H), 6.99 (dd, J=7.7, 1.4 Hz, 1H), 6.89 (s, 1H), 6.23 (d, J=5.4 Hz, 1H), 4.70-4.59 (m, 3H), 4.55-4.39 (m, 3H), 3.99 - 3.92 (m, 3H), 3.85-3.68 (m, 6H), 3.63-3.53 (m, 1H), 3.49-3.39 (m, 2H), 2.88 - 2.82 (m, 2H), 2.73 - 2.68 (m, 1H), 2.60 (s, 2H), 2.57 - 2.55 (m, 1H), 2.53 (s, 3H), 2.49 - 2.44 (m, 1H), 2.13 - 2.09 (m, 1H), 2.06 (s, 3H), 2.00-1.95 (m, 6H), 1.82 - 1.79 (m, 2H), 1.62-1.56 (m, 2H), 1.50-1.42 (m, 2H), 1.08 - 1.05 (m, 3H), 0.89 (dd, J=22.9, 6.4 Hz, 3H), 0.77 (d, J=4.1 Hz, 3H). m/z 1039.45 [M+H]⁺. |
| 100 | ¹H NMR (400 MHz, CDCl₃) δ 8.69 (s, 1H), 8.26 (d, J=1.2 Hz, 1H), 8.21 (d, J=1.2 Hz, 1H), 7.88 (d, J=5.4 Hz, 1H), 7.60 - 7.52 (m, 1H), 7.25 - 7.17 (m, 2H), 7.08-6.90 (m, 3H), 6.24 (d, J=5.3 Hz, 1H), 4.83-4.78 (m, 1H), 4.65 - 4.56 (m, 2H), 4.53 - 4.49 (m, 1H), 4.47-4.38 (m, 2H), 3.99 - 3.93 (m, 2H), 3.84-3.74 (m, 5H), 3.64-3.50 (m, 2H), 3.46 - 3.39 (m, 2H), 3.31 - 3.12 (m, 1H), 2.88 - 2.82 (m, 2H), 2.73 - 2.65 (m, 1H), 2.60 (s, 2H), 2.53 (s, 3H), 2.49-2.35 (m, 1H), 2.17 - 2.06 (m, 2H), 2.05-2.03 (m, 2H), 1.99 (s, 3H), 1.96 - 1.88 (m, 4H), 1.83-1.77 (m, 2H), 1.62-1.55 (m, 2H), 1.51-1.46 (m, 2H), 1.05 - 1.03 (m, 6H), 0.74 (d, J=6.6 Hz, 3H). m/z 1039.39 [M+H]⁺. |
| 101 | ¹H NMR (400 MHz, CDCl₃) δ 8.69 (s, 1H), 8.26 (d, J=1.3 Hz, 1H), 8.21 (d, J=1.3 Hz, 1H), 7.87 (d, J=5.4 Hz, 1H), 7.56 (d, J=9.7 Hz, 1H), 7.35 (d, J=8.0 Hz, 1H), 7.04-6.88 (m, 3H), 6.24 (d, J=5.3 Hz, 1H), 5.08 (d, J=10.5 Hz, 1H), 4.72-4.37 (m, 5H), 3.98 - 3.93 (m, 2H), 3.85-3.75 (m, 7H), 3.61-3.50 (m, 1H), 3.46 - 3.39 (m, 2H), 2.88 - 2.83 (m, 2H), 2.76-2.64 (m, 1H), 2.60 (s, 2H), 2.53 (s, 3H), 2.51-2.39 (m, 2H), 2.07 - 1.99 (m, 5H), 1.97 - 1.89 (m, 3H), 1.85-1.76 (m, 2H), 1.61 - 1.55 (m, 2H), 1.50 - 1.45 (m, 2H), 1.05 (s, 3H), 0.99 - 0.95 (m, 2H), 0.94-0.92 (m, 3H), 0.85-0.80 (m, 2H), 0.73 (d, J=6.6 Hz, 3H). m/z 1066.52 [M+H]⁺. |
| 102 | ¹H NMR (400 MHz, CDCl₃) δ 8.69 (s, 1H), 8.26 (d, J=1.2 Hz, 1H), 8.23 (d, J=1.3 Hz, 1H), 7.87 (d, J=5.3 Hz, 1H), 7.56 (d, J=9.5 Hz, 1H), 7.35 (d, J=7.8 Hz, 1H), 7.02-6.88 (m, 3H), 6.24 (d, J=5.3 Hz, 1H), 5.09 (d, J=10.4 Hz, 1H), 4.71-4.63 (m, 1H), 4.63 - 4.52 (m, 3H), 4.52-4.39 (m, 1H), 3.94-3.87 (m, 1H), 3.87-3.74 (m, 8H), 3.72-3.64 (m, 2H), 3.61-3.51 (m, 1H), 2.85 (t, J=11.4 Hz, 2H), 2.78-2.65 (m, 1H), 2.53 (s, 3H), 2.51-2.40 (m, 2H), 2.11-1.89 (m, 8H), 1.86-1.75 (m, 2H), 1.71-1.62 (m, 2H), 1.61-1.52 (m, 2H), 1.24 (s, 3H), 1.01-0.91 (m, 5H), 0.84-0.80 (m, 2H), 0.73 (d, J=6.4 Hz, 3H). m/z 1052.69 [M+H]⁺. |
| 103 | ¹H NMR (400 MHz, CDCl₃) δ 8.69 (s, 1H), 8.27 (d, J=1.2 Hz, 1H), 8.23 (d, J=1.2 Hz, 1H), 7.87 (d, J=5.4 Hz, 1H), 7.57 (d, J=9.7 Hz, 1H), 7.35 (d, J=7.2 Hz, 1H), 7.04-6.87 (m, 3H), 6.23 (d, J=5.4 Hz, 1H), 5.09 (d, J=10.4 Hz, 1H), 4.73-4.64 (m, 1H), 4.64-4.51 (m, 3H), 4.51-4.38 (m, 1H), 4.24-4.15 (m, 1H), 4.02-3.88 (m, 3H), 3.88-3.72 (m, 7H), 3.70 (d, J=8.8 Hz, 1H), 3.63-3.49 (m, 2H), 3.44 - 3.38 (m, 1H), 3.01 (d, J=4.5 Hz, 1H), 2.88 - 2.82 (m, 2H), 2.78-2.65 (m, 1H), 2.53 (s, 3H), 2.51-2.39 (m, 2H), 2.09-1.99 (m, 5H), 1.99-1.86 (m, 5H), 1.86-1.75 (m, 4H), 1.25 (d, J=6.4 Hz, 3H), 1.01-0.95 (m, 2H), 0.93 (dd, J=8.5, 1.9 Hz, 3H), 0.85-0.80 (m, 2H), 0.73 (d, J=6.3 Hz, 3H). m/z 1108.66 [M+H]⁺. |
| 104 | ¹H NMR (400 MHz, CDCl₃) δ 8.69 (s, 1H), 8.28 (s, 1H), 7.90 (d, J=5.3 Hz, 1H), 7.57 (d, J=9.9 Hz, 1H), 7.35 (d, J=8.0 Hz, 1H), 7.05-6.92 (m, 2H), 6.90 (s, 1H), 6.28 (d, J=5.3 Hz, 1H), 5.09 (d, J=10.4 Hz, 1H), 4.73-4.64 (m, 3H), 4.64-4.53 (m, 3H), 4.52-4.38 (m, 1H), 3.98-3.70 (m, 7H), 3.61-3.52 (m, 1H), 3.52-3.44 (m, 2H), 3.30-3.20 (m, 2H), 2.87 (t, J=12.1 Hz, 2H), 2.77-2.67 (m, 1H), 2.61 (s, 2H), 2.53 (s, 3H), 2.51-2.39 (m, 2H), 2.17-1.88 (m, 8H), 1.88-1.78 (m, 2H), 1.67-1.55 (m, 2H), 1.53-1.44 (m, 2H), 1.03 (s, 3H), 1.01-0.91 (m, 5H), 0.86-0.78 (m, 2H), 0.73 (d, J=6.3 Hz, 3H). m/z 1118.70 [M+Na]⁺. |
| 105 | ¹H NMR (400 MHz, CDCl₃) δ 8.69 (s, 1H), 8.28 (s, 1H), 7.90 (d, J=5.3 Hz, 1H), 7.56 (d, J=10.4 Hz, 1H), 7.35 (d, J=7.1 Hz, 1H), 7.00 (dd, J=7.7, 1.4 Hz, 1H), 6.99 - 6.92 (m, 1H), 6.90 (s, 1H), 6.28 (d, J=5.3 Hz, 1H), 5.08 (d, J=10.5 Hz, 1H), 4.68 (s, 2H), 4.62 - 4.53 (m, 3H), 4.50 - 4.40 (m, 1H), 3.93 - 3.64 (m, 8H), 3.61 - 3.56 (m, 1H), 3.47-3.34 (m, 4H), 2.90 - 2.84 (m, 2H), 2.76 - 2.69 (m, 1H), 2.53 (s, 3H), 2.50 - 2.45 (m, 1H), 2.08-1.92 (m, 9H), 1.83 - 1.80 (m, 2H), 1.75 - 1.68 (m, 2H), 1.61-1.57 (m, 2H), 1.24 (s, 3H), 1.00-0.92 (m, 5H), 0.84 - 0.81 (m, 2H), 0.73 (d, J=6.0 Hz, 3H). m/z 1082.72 [M+H]+ |
| 106 | ¹H NMR (400 MHz, CDCl₃) δ 8.69 (s, 1H), 8.29 (s, 1H), 7.90 (d, J=5.3 Hz, 1H), 7.56 (d, J=10.5 Hz, 1H), 7.35 (d, J=7.9 Hz, 1H), 7.04-6.92 (m, 2H), 6.90 (s, 1H), 6.29 (d, J=5.3 Hz, 1H), 5.09 (d, J=10.5 Hz, 1H), 4.68 (s, 2H), 4.67-4.38 (m, 5H), 4.23-4.14 (m, 1H), 3.97-3.73 (m, 8H), 3.71 - 3.69 (m, 2H), 3.57-3.47 (m, 2H), 3.27-3.19 (m, 1H), 3.19-3.08 (m, 1H), 3.01 (d, J=4.6 Hz, 1H), 2.87 (t, J=12.1 Hz, 2H), 2.79-2.66 (m, 1H), 2.53 (s, 3H), 2.51-2.39 (m, 2H), 2.11 - 1.99 (m, 5H), 1.98-1.90 (m, 5H), 1.85-1.77 (m, 2H), 1.77-1.68 (m, 2H), 1.24 (d, J=6.4 Hz, 3H), 1.02-0.96 (m, 2H), 0.96-0.91 (m, 3H), 0.85-0.80 (m, 2H), 0.73 (d, J=6.1 Hz, 3H). m/z 1138.75 [M+H]⁺. |
| 107 | ¹H NMR (400 MHz, CDCl₃) δ 8.68 (s, 1H), 8.25 (d, J=1.1 Hz, 1H), 8.20 (d, J=1.2 Hz, 1H), 7.86 (d, J=5.4 Hz, 1H), 7.79 (d, J=6.2 Hz, 1H), 7.42-7.34 (m, 1H), 6.99 (dd, J=7.7, 1.4 Hz, 1H), 6.92-6.76 (m, 2H), 6.23 (d, J=5.3 Hz, 1H), 5.37 (s, 1H), 4.78-4.39 (m, 5H), 4.01-3.89 (m, 2H), 3.82 (m, 7H), 3.62-3.50 (m, 1H), 3.45 - 3.38 (m, 2H), 2.87 - 2.81 (m, 2H), 2.74-2.65 (m, 1H), 2.59 (s, 2H), 2.52 (s, 3H), 2.50-2.39 (m, 1H), 2.10-2.00 (m, 5H), 1.98-1.87 (m, 3H), 1.82-1.74 (m, 2H), 1.61-1.57 (m, 2H), 1.49 - 1.36 (m, 2H), 1.03 (s, 3H), 0.97 (d, J=11.0 Hz, 9H), 0.92 (dd, J=8.5, 2.7 Hz, 2H), 0.84 - 0.79 (m, 2H). m/z 1080.52 [M+H]⁺. |
| 108 | ¹H NMR (400 MHz, CDCl₃) δ 8.69 (s, 1H), 8.27 (s, 1H), 7.90 (d, J=5.3 Hz, 1H), 7.59 (d, J=9.6 Hz, 1H), 7.35 (d, J=7.9 Hz, 1H), 7.01 - 6.99 (m, 2H), 6.91 (s, 1H), 6.30 (d, J=5.3 Hz, 1H), 5.08 (d, J=10.5 Hz, 1H), 4.71 - 4.69 (m, 1H), 4.67 (s, 2H), 4.62 - 4.49 (m, 3H), 4.46-4.38 (m, 1H), 3.96 - 3.88 (s, 1H), 3.86-3.74 (m, 6H), 3.61 - 3.52 (m, 1H), 3.47 - 3.45 (m, 2H), 3.27 - 3.21 (m, 2H), 2.92 - 2.82 (m, 2H), 2.75 - 2.68 (m, 1H), 2.64 (s, 2H), 2.53 (s, 3H), 2.50 - 2.42 (m, 2H), 2.34 (s, 3H), 2.09 - 1.93 (m, 9H), 1.85 - 1.77 (m, 4H), 1.05 (s, 3H), 1.00-0.95 (m, 3H), 0.75 (d, J=6.6 Hz, 3H). m/z 1070.93 [M+H]⁺. |
| 109 | ¹H NMR (400 MHz, CDCl₃) δ 8.69 (s, 1H), 8.28 (s, 1H), 7.90 (d, J=5.3 Hz, 1H), 7.61 (d, J=8.6 Hz, 1H), 7.35 (d, J=6.5 Hz, 1H), 7.08-6.95 (m, 2H), 6.90 (s, 1H), 6.29 (d, J=5.3 Hz, 1H), 5.10 (d, J=10.5 Hz, 1H), 4.68 (s, 1H), 4.67 (s, 2H), 4.59 - 4.44 (m, 4H), 3.96 - 3.92 (m, 1H), 3.86 - 3.74 (m, 6H), 3.61 - 3.54 (m, 1H), 3.47-3.35 (m, 4H), 2.90 - 2.84 (m, 2H), 2.74 - 2.69 (m, 1H), 2.53 (s, 3H), 2.49 - 2.41 (m, 2H), 2.33 (s, 3H), 2.09 - 1.96 (m, 9H), 1.82 - 1.78 (m, 4H), 1.24 (s, 3H), 1.01 - 0.96 (m, 3H), 0.75 (d, J=6.6 Hz, 3H). m/z 1056.93 [M+H]⁺. |
| 110 | ¹H NMR (400 MHz, CDCl₃) δ 8.70 (s, 1H), 8.29 (s, 1H), 7.91 (d, J=5.3 Hz, 1H), 7.59 (d, J=10.1 Hz, 1H), 7.35 (d, J=6.9 Hz, 1H), 7.03-6.92 (m, 2H), 6.91 (s, 1H), 6.29 (d, J=5.3 Hz, 1H), 5.08 (d, J=10.5 Hz, 1H), 4.68 (s, 2H), 4.63-4.49 (m, 4H), 4.46 - 1.38 (m, 1H), 4.22 - 4.16 (m, 1H), 3.93 - 3.90 (m, 1H), 3.87 - 3.82 (m, 1H), 3.81-3.78 (m, 6H), 3.70 (d, J=8.8 Hz, 1H), 3.60-3.47 (m, 3H), 3.27 - 3.21 (m, 1H), 3.16-3.11 (m, 1H), 3.01 (d, J=4.5 Hz, 1H), 2.91 - 2.85 (m, 2H), 2.76-2.68 (m, 1H), 2.53 (s, 3H), 2.50 - 2.44 (m, 2H), 2.34 (s, 3H), 2.10-1.92 (m, 9H), 1.85 - 1.76 (m, 4H), 1.24 (d, J=6.4 Hz, 3H), 0.97 (dd, J=14.7, 6.4 Hz, 3H), 0.74 (d, J=6.3 Hz, 3H). m/z 1112.79 [M+H]⁺. |
| 111 | ¹H NMR (400 MHz, CDCl₃) δ 8.29 (s, 1H), 7.91 (d, J=5.3 Hz, 1H), 7.60 (d, J=9.8 Hz, 1H), 7.25-7.22 (m, 1H), 6.94-6.87 (m, 3H), 6.29 (d, J=5.3 Hz, 1H), 5.08 (d, J=10.5 Hz, 1H), 4.68 (s, 2H), 4.66-4.50 (m, 4H), 4.47-4.36 (m, 1H), 4.22-4.16 (m, 1H), 3.92-3.74 (m, 9H), 3.70 (d, J=8.8 Hz, 1H), 3.55 - 3.51 (m, 2H), 3.27-3.20 (m, 1H), 3.16 - 3.10 (m, 1H), 3.01 (d, J=4.6 Hz, 1H), 2.91 - 2.85 (m, 2H), 2.76 - 2.68 (m, 1H), 2.50 - 2.42 (m, 2H), 2.33 (s, 3H), 2.08 - 1.92 (m, 9H), 1.85 - 1.79 (m, 5H), 1.24 (d, J=6.4 Hz, 3H), 0.96 (dd, J=17.1, 6.5 Hz, 3H), 0.74 (d, J=5.4 Hz, 3H). m/z 1015.95 [M+H]⁺. |
| 112 | ¹H NMR (400 MHz, CDCl₃) δ 8.29 (s, 1H), 7.90 (d, J=5.3 Hz, 1H), 7.60 (d, J=10.1 Hz, 1H), 7.23 (d, J=5.6 Hz, 1H), 7.00 - 6.90 (m, 2H), 6.85 (d, J=1.8 Hz, 1H), 6.29 (d, J=5.3 Hz, 1H), 5.09 (d, J=10.5 Hz, 1H), 4.68 (s, 2H), 4.64-4.55 (m, 2H), 4.52-4.31 (m, 3H), 4.22 - 4.16 (m, 1H), 3.94-3.84 (m, 4H), 3.82 - 3.74 (m, 5H), 3.70 (d, J=8.8 Hz, 1H), 3.56-3.51 (m, 2H), 3.27-3.20 (m, 1H), 3.16-3.10 (m, 1H), 3.01 (d, J=4.6 Hz, 1H), 2.90 - 2.84 (m, 2H), 2.76-2.68 (m, 1H), 2.48 - 2.40 (m, 2H), 2.33 (s, 3H), 2.06 - 1.98 (m, 4H), 1.97-1.91 (m, 4H), 1.86 - 1.76 (m, 5H), 1.24 (d, J=6.5 Hz, 3H), 0.96 (dd, J=12.4, 6.5 Hz, 3H), 0.74 (d, J=6.6 Hz, 3H). m/z 1049.94 [M+H]⁺. |
| 113 | ¹H NMR (400 MHz, CDCl₃) δ 8.29 (s, 1H), 7.90 (d, J=5.3 Hz, 1H), 7.63-7.57 (m, 1H), 7.47-7.40 (m, 1H), 7.24 (d, J=8.9 Hz, 1H), 7.12-7.04 (m, 2H), 6.29 (d, J=5.3 Hz, 1H), 5.11 (d, J=10.4 Hz, 1H), 4.68 (s, 2H), 4.67-4.61 (m, 1H), 4.61-4.46 (m, 4H), 4.24-4.11 (m, 1H), 3.98-3.91 (m, 1H), 3.90-3.75 (m, 6H), 3.74-3.66 (m, 2H), 3.58-3.46 (m, 3H), 3.31-3.20 (m, 1H), 3.17-3.09 (m, 1H), 3.01 (d, J=4.6 Hz, 1H), 2.88 (t, J=11.5 Hz, 2H), 2.76-2.66 (m, 1H), 2.53-2.35 (m, 2H), 2.33 (s, 3H), 2.13-1.96 (m, 6H), 1.97-1.87 (m, 3H), 1.87-1.70 (m, 4H), 1.24 (d, J=6.4 Hz, 3H), 1.01-0.94 (m, 3H), 0.76 (d, J=6.7 Hz, 3H). m/z 1040.91 [M+H]⁺. |
| 114 | ¹H NMR (400 MHz, DMSO-*d₆*) δ 8.99 (s, 1H), 8.42 (s, 1H), 8.33-8.25 (m, 1H), 8.20 (s, 1H), 7.78-7.67 (m, 3H), 7.24 (d, J=7.7 Hz, 1H), 7.10 (s, 1H), 7.02 (t, J=7.9 Hz, 1H), 6.99-6.91 (m, 1H), 6.39 (d, J=8.1 Hz, 1H), 6.23 (s, 1H), 6.15 (d, J=7.2 Hz, 1H), 4.56-4.48 (m, 1H), 4.48-4.38 (m, 2H), 4.36-4.31 (m, 1H), 4.31-4.21 (m, 3H), 4.21-4.12 (m, 2H), 4.12-4.04 (m, 1H), 3.96-3.87 (m, 3H), 3.85 (d, J=8.3 Hz, 1H), 3.82-3.75 (m, 1H), 3.62-3.4i8 (m, 1H), 3.28 - 3.18 (m, 2H), 2.83-2.76 (m, 2H), 2.58-2.53 (m, 1H), 2.46 (s, 3H), 2.30-2.21 (m, 3H), 2.14 (s, 3H), 2.11-1.99 (m, 1H), 1.94-1.85 (m, 1H), 1.81-1.69 (m, 2H), 1.60-1.37 (m, 8H), 1.07 (s, 3H), 0.96 (d, J=6.8 Hz, 3H), 0.79 (d, J=6.8 Hz, 3H). m/z 1025.84 [M+H]⁺. |
| 115 | ¹H NMR (400 MHz, DMSO-*d₆*) δ 9.00 (s, 1H), 8.42 (s, 1H), 8.38-8.32 (m, 1H), 8.20 (s, 1H), 7.93-7.80 (m, 3H), 7.33 (d, J=7.8 Hz, 1H), 7.11 (s, 1H), 7.02 (t, J=8.1 Hz, 1H), 6.97 (d, J=7.5 Hz, 1H), 6.38 (d, J=7.8 Hz, 1H), 6.23 (s, 1H), 6.15 (d, J=7.8 Hz, 1H), 4.59-4.48 (m, 1H), 4.48-4.41 (m, 1H), 4.40-4.32 (m, 2H), 4.32-4.13 (m, 5H), 4.12-4.00 (m, 1H), 3.97-3.85 (m, 3H), 3.83-3.74 (m, 2H), 3.69-3.63 (m, 1H), 3.47-3.33 (m, 2H), 2.83-2.72 (m, 2H), 2.53-2.51 (m, 1H), 2.46 (s, 3H), 2.32-2.21 (m, 3H), 2.20 (s, 3H), 2.05-1.98 (m, 1H), 1.95-1.87 (m, 1H), 1.80-1.71 (m, 2H), 1.59-1.37 (m, 8H), 1.08 (s, 3H), 0.94 (d, J=6.5 Hz, 3H), 0.76 (d, J=6.6 Hz, 3H).m/z 1025.88 [M+H]⁺. |

### Evaluation of the compounds of the present disclosure

### Experimental Example 1: Measurement of SHP2 protein degradation in MV-4-11 cells

MV-4-11 cells were treated with the synthesized Example compounds, and the amount of SHP2 protein present in the cells was measured using western blotting detection. The protocol for the experiment conducted using MV-4-11 cells is as follows.

[Culture] MV-4-11 cells were resuspended in IMDM MEDIUM (Hyclone, SH30228.01), 5% FBS (Hyclone, SV30207.02), 1% Penicillin-streptomycin (Welgene, LS 202-02) media and seeded at 1 x 10⁶/mL in 12-well plates (1 mL each).

[Compound treatment] 12-well plates were treated as follows. 10 mM stock dissolved in DMSO was serially diluted 1/100 or 1/10 with media, and the compounds were treated to the cells at a final concentration of 0.01, 0.1, 1 µM or 0.001, 0.01, 0.1 µM, and harvested after 24 hours. For the negative control group, DMSO diluted 1/100 or 1/1000 in media was added (3 µL DMSO + 297 µL media or 1 µL DMSO + 999 µL media).

[Harvest] Each well was pipetted with a 1 mL pipette, collected in a 1.5 mL microtube, and centrifuged (500 g, 5 min, 4 °C). The supernatant was removed, washed with PBS, and centrifuged again (500 g, 5 min, 4 °C). The supernatant was removed and a pellet was prepared.

[Cell lysis and sample preparation] Lysis buffer was prepared as follows. RIPA buffer (Biosesang, RC2038-050-00) + 0.5 mM PMSF (SIGMA, P7626) + 1x Protease/Phosphatase Inhibitor (Cell signaling, 5872S) were added at 50 µL per tube and incubated on ice for 30 minutes (vortexed at 0 and 30 minutes). After sonication (10 sec pulse, 30 sec rest, 5 cycles, 70% amplification) and centrifugation (15,000 g, 15 min, 4 °C), only the supernatant was transferred to a microtube. RIPA buffer 7.5 µL and sample 2.5 µL were added to a 96-well plate to dilute to 1/4, and then BCA Protein Assay Kit (iNtRON, 21071) A and B were mixed in a 50:1 ratio and 200 µL each was added. After that, the plate was incubated at 37 °C for 30 minutes and left at room temperature for 10 minutes. After shaking for 10 seconds with a SYNERGY H1 microplate reader from BioTek, the absorbance was measured at 562 nm. The protein was quantified using the measured value to prepare a sample, which was then incubated at 70 °C for 10 minutes. The sample buffer used at this time is a mixture of NuPAGE or Bolt 4x sample buffer (Invitrogen) and each 10x sample reducing agent according to the gel to be used, and RIPA buffer was used for protein dilution.

[Western blotting detection] Equal amounts of samples were loaded onto NuPAGE or Bolt Bis-tris 4-12% gradient gels and run (200 V, 35 min). This was transferred to 0.2 mm NC membranes using Trans-blot Turbo (BIO-RAD) (1.3 A constant, 25 V limit, 15 min), and was blocked at room temperature for 1 hour with Skim milk or Intercept Blocking Buffer (LI-COR, 927-60001):0.1% TBST = 1:1. Anti-SHPTP2 Mouse (1:500 in 2% BSA/0.2% TBST, size: 70 kDa, Santacruz sc-7384) was incubated overnight at 4°C, and Anti-GAPDH Rabbit (1:2,000 in 2% BSA/0.2% TBST, size: 37 kDa, CST #2118) was incubated at room temperature for 1.5 hours or 3 hours. After washing three times for 5 minutes each with 0.2% TBST washing buffer, secondary antibodies Anti-Mouse IgG (1:5,000 in 2% BSA/0.2% TBST, CST) and IRDye^{®} 680RD Goat anti-Rabbit IgG Secondary Antibody (1:10,000 in 2% BSA/0.2% TBST, LI-COR 926-68071) were added, and incubated on a rocker at room temperature for 45 minutes. The resultants were washed five times for 5 minutes each with 0.2% TBST washing buffer, and detected using LI-COR's Odyssey. GAPDH was detected with a 700 nm channel, and SHPTP2 was detected using SuperSignal West Pico PLUS Chemiluminescent Substrate.

### Experimental Example 2: Measurement of SHP2 protein degradation in NCI-H358 cells

H358 cells were treated with the synthesized Example compounds, and the amount of SHP2 protein present in the cells was measured using western blotting detection. The protocol for the experiment using H358 cells is as follows.

[Culture] H358 cells were resuspended in RPMI1640 with 25 mM HEPES (Hyclone, SH30255.01), 10% FBS (Hyclone, SV30207.02), 1% Penicillin-streptomycin (Welgene, LS 202-02) media, seeded at 3 x 10⁵/mL in 12-well plates (1 mL each), and cultured for 24 hours.

[Compound treatment] Before compound treatment, the media was changed to 1 mL media, and then the 12-well plate was treated as follows. The 10 mM stock dissolved in DMSO was serially diluted 1/100 or 1/10 with the media, and the compound was treated to the cells at a final concentration of 0.001, 0.01, and 0.1 µM, and harvested after 24 hours. For the negative control group, DMSO diluted 1/1000 in the media was added (1 µL DMSO + 999 µL media).

[Harvest] 24 hours after compound treatment, each well was scraped with a cell lifter (SPL, 90040), pipetted with a 1 mL pipette, collected in a 1.5 mL microtube, and centrifuged (500 g, 5 min, 4 °C). The supernatant was removed, washed with PBS, and centrifuged again (500 g, 5 min, 4 °C). Afterwards, the supernatant was removed to prepare a pellet.

[Cell lysis and sample preparation] Lysis buffer was prepared as follows. RIPA buffer (Biosesang, RC2038-050-00) + 0.5 mM PMSF (SIGMA, P7626) + 1x Protease/Phosphatase Inhibitor (Cell signaling, 5872S) were added at 30 µL per tube, vortexed, and incubated on ice for 30 minutes. Afterwards, sonication was performed (10 sec pulse, 30 sec rest, 5 cycles, 70% amplification) and centrifugation was performed (15,000 g, 15 min, 4 °C), and only the supernatant was transferred to a microtube. A 96-well plate was diluted to 1/4 with 7.5 µL of RIPA buffer and 2.5 µL of sample, and 200 µL each was added after mixing A and B of the BCA Protein Assay Kit (iNtRON, 21071) in a ratio of 50:1. The plate was incubated at 37 °C for 30 minutes and left at room temperature for 10 minutes. After shaking for 10 seconds with a SYNERGY H1 microplate reader from BioTek, the absorbance was measured at 562 nm. The protein was quantified using the measured value to make a sample, which was then incubated at 70 °C for 10 minutes. The sample buffer used here was a mixture of NuPAGE or Bolt 4x sample buffer (Invitrogen) and each 10x sample reducing agent depending on the gel to be used, and RIPA buffer was used for protein dilution.

[Western blotting detection] Equal amounts of samples were loaded onto NuPAGE or Bolt Bis-tris 4-12% gradient gel and run (200 V, 35 min). This was transferred to 0.2 mm NC membrane using Trans-blot Turbo (BIO-RAD) (1.3 A constant, 25 V limit, 15 min), and blocked with 5% skim milk/0.2% TBST for 45 min at room temperature. Anti-SHPTP2 Mouse (1: 1,000 in 5% skim milk/0.2% TBST, size: 70 kDa, Santa cruz sc-7384) and Anti-GAPDH Rabbit (1:2,000 in 5% skim milk or 5% BSA/0.2% TBST, size: 37 kDa, CST #2118) were added and incubated overnight at 4°C. After washing three times with 0.2% TBST washing buffer for 5 minutes each, secondary antibodies Anti-Mouse IgG (1:5,000 in 5% skim milk/0.2% TBST, CST) and IRDye^{®} 680RD Goat anti-Rabbit IgG Secondary Antibody (1:10,000 in 5% skim milk/0.2% TBST, LI-COR 926-68071) were added, and incubated on a rocker at room temperature for 45 minutes. The resultants were washed five times with 0.2% TBST washing buffer for 5 minutes each, and detected using LI-COR's Odyssey. GAPDH was detected with a 700 nm channel, and SHPTP2 was detected using SuperSignal West Pico PLUS Chemiluminescent Substrate.

The results of evaluating the SHP2 protein degradation ability of the compounds of the examples according to Experimental Examples 1 and 2 are summarized in Table 2 below. As shown in Table 2 below, the compounds of the present disclosure exhibited excellent activity in degrading SHP2 protein.

**[Table 2]**

| Example No. | Cell-line | SHP2 % degradation | |
|---|---|---|---|
| | | 0.01 µM | 0.1 µM |
| 1 | MV-4-11 | -33.4 | 6.5 |
| 2 | MV-4-11 | 11.8 | 80.3 |
| 3 | MV-4-11 | 19 | 90.3 |
| 4 | MV-4-11 | 42.9 | 95.6 |
| 5 | MV-4-11 | 22.3 | 62.0 |
| 6 | MV-4-11 | 14.8 | 56.9 |
| 7 | MV-4-11 | 10.1 | 55.4 |
| 8 | MV-4-11 | 11.7 | 81.9 |
| 9 | MV-4-11 | 4.0 | 81.1 |
| 10 | H358 | -8.0 | 93.9 |
| 11 | MV-4-11 | 83 | 97.9 |
| 12 | MV-4-11 | 83.7 | 98.6 |
| 13 | MV-4-11 | 93.8 | 99.5 |
| 14 | MV-4-11 | 1.2 | 85.1 |
| 15 | H358 | 93.5 | 99.6 |
| 16 | MV-4-11 | -30.1 | 94.3 |
| 17 | MV-4-11 | 64.4 | 96.6 |
| 18 | MV-4-11 | 63.2 | 98.4 |
| 19 | MV-4-11 | 50.4 | 95.7 |
| 20 | MV-4-11 | 78.8 | 98.2 |
| 21 | MV-4-11 | 81.1 | 99.7 |
| 22 | H358 | 95.7 | 97.1 |
| 23 | MV-4-11 | 74.2 | 99.8 |
| 24 | MV-4-11 | 83.1 | 100.0 |
| 25 | H358 | 94.2 | 97.0 |
| 26 | MV-4-11 | 52.3 | 96.1 |
| 27 | MV-4-11 | 95.8 | 99.9 |
| 28 | H358 | 86.9 | 98.5 |
| 29 | MV-4-11 | 97.2 | 99.9 |
| 30 | MV-4-11 | 86.1 | 99.9 |
| 31 | H358 | 76.3 | 99.4 |
| 32 | H358 | 54.4 | 98.5 |
| 33 | H358 | 96.6 | 99.1 |
| 34 | H358 | -36.9 | 73.0 |
| 35 | H358 | 66.8 | 97.7 |
| 36 | H358 | 91.0 | 100.0 |
| 37 | H358 | 75.8 | 99.1 |
| 38 | MV-4-11 | 97.7 | 99.5 |
| 39 | H358 | 83.6 | 98.1 |
| 40 | H358 | 94.2 | 95.9 |
| 41 | MV-4-11 | 98.2 | 99.7 |
| 42 | H358 | 95.2 | 98.7 |
| 43 | H358 | 94.3 | 95.9 |
| 44 | H358 | 88.5 | 99.1 |
| 45 | H358 | 97.0 | 99.4 |
| 46 | H358 | 33.8 | 93.2 |
| 47 | H358 | -34.6 | 50.6 |
| 48 | H358 | 88.8 | 98.3 |
| 49 | H358 | 87.1 | 99.4 |
| 50 | MV-4-11 | 67.3 | 97.6 |
| 51 | MV-4-11 | 32.9 | 92.6 |
| 52 | MV-4-11 | 20.5 | 91.9 |
| 53 | MV-4-11 | 70.4 | 99.4 |
| 54 | MV-4-11 | 69.4 | 99.3 |
| 55 | MV-4-11 | 27.5 | 95.0 |
| 56 | MV-4-11 | 55.8 | 98.6 |
| 57 | MV-4-11 | 65.7 | 96.8 |
| 58 | MV-4-11 | 82.0 | 97.7 |
| 59 | MV-4-11 | 81.7 | 99.5 |
| 60 | MV-4-11 | 81.5 | 99.3 |
| 61 | MV-4-11 | 80.3 | 99.3 |
| 62 | MV-4-11 | 75.6 | 98.9 |
| 63 | H358 | 53.0 | 99.0 |
| 64 | H358 | 87.9 | 99.8 |
| 65 | MV-4-11 | 85.2 | 98.1 |
| 66 | MV-4-11 | 78.6 | 99.6 |
| 67 | H358 | 94.5 | 96.9 |
| 68 | MV-4-11 | -22.1 | 95.7 |
| 69 | MV-4-11 | 19.9 | 99.8 |
| 70 | MV-4-11 | 84.5 | 99.9 |
| 71 | MV-4-11 | 60.0 | 99.4 |
| 72 | MV-4-11 | 91.5 | 99.8 |
| 73 | MV-4-11 | 65.8 | 97.7 |
| 74 | MV-4-11 | 97.0 | 99.8 |
| 75 | MV-4-11 | 97.0 | 99.3 |
| 76 | H358 | 90.0 | 98.7 |
| 77 | H358 | 94.4 | 98.0 |
| 78 | H358 | 98.0 | 99.4 |
| 79 | H358 | 96.1 | 99.9 |
| 80 | H358 | 99.6 | 99.5 |
| 81 | H358 | 85.6 | 99.6 |
| 82 | H358 | 94.9 | 97.4 |
| 83 | H358 | 97.8 | 99.7 |
| 84 | H358 | 92.4 | 99.9 |
| 85 | H358 | 98.3 | 99.9 |
| 86 | MV-4-11 | 81.9 | 99.9 |
| 87 | MV-4-11 | 95.8 | 100.0 |
| 88 | H358 | 97.0 | 99.9 |
| 89 | H358 | 98.8 | 99.9 |
| 90 | H358 | 70.5 | 99.2 |
| 91 | H358 | 53.2 | 98.5 |
| 92 | H358 | 30.1 | 80.2 |
| 93 | H358 | 99.9 | 99.9 |
| 94 | H358 | 95.5 | 100.0 |
| 95 | H358 | 99.2 | 99.7 |
| 96 | H358 | 98.9 | 99.9 |
| 97 | H358 | 95.0 | 99.7 |
| 98 | H358 | 99.6 | 99.7 |
| 99 | H358 | -4.8 | 91.5 |
| 100 | H358 | -65.7 | -18.7 |
| 101 | H358 | 96.3 | 97.4 |
| 102 | H358 | 99.4 | 99.8 |
| 103 | H358 | 99.9 | 99.7 |
| 104 | H358 | 98.6 | 98.0 |
| 105 | H358 | 98.4 | 97.9 |
| 106 | H358 | 99.1 | 97.3 |
| 107 | H358 | 96.5 | 99.2 |
| 108 | H358 | 99.1 | 99.6 |
| 109 | H358 | 99.0 | 99.4 |
| 110 | H358 | 99.8 | 99.8 |
| 111 | H358 | 98.0 | 99.9 |
| 112 | H358 | 93.8 | 99.7 |
| 113 | H358 | 96.2 | 99.8 |
| 114 | H358 | 48.3 | 88.1 |
| 115 | H358 | 98.3 | 99.2 |

## Claims

1. A compound of Chemical Formula 1: 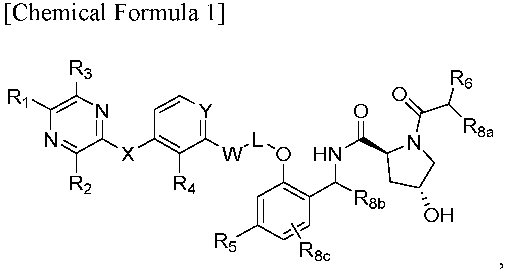
an optical isomer, a stereoisomer, an isotope variant, a hydrate, a solvate, or a pharmaceutically acceptable salt thereof,
in the Chemical Formula 1,
R₁ is 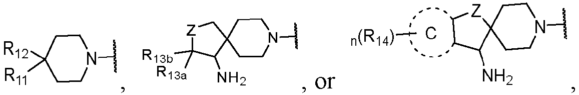
R₁₁ is H, C₁₋₆alkyl, hydroxy, halogen, C₁₋₆hydroxyalkyl, C₁₋₆haloalkyl, C₃₋₁₀cycloalkyl, heterocycle, aryl, or heteroaryl, wherein one or more hydrogens in the aryl and heteroaryl are optionally and each independently substituted with C₁₋₆alkyl, hydroxy, halogen, C₁₋₃hydroxyalkyl, C₁₋₃haloalkyl, or C₁₋₃alkoxy,
R₁₂ is amino, C₁₋₆aminoalkyl, or C₁₋₆alkylamino,
R₁₃ₐ and R_{13b} are each independently H, C₁₋₆alkyl, hydroxy, halogen, C₁₋₆hydroxyalkyl, C₁₋₆haloalkyl, or C₁₋₆alkoxy,
R₁₄ is each independently H, C₁₋₆alkyl, hydroxyl, halogen, or CN,
Z is each independently -O-, -S-, -NH-, or -CH₂-,
Ring C is aryl or heteroaryl,
n is an integer from 0 to 5,
R₂ is H, C₁₋₆alkyl, or -NH₂,
R₃ is H, C₁₋₆alkyl, C₁₋₆hydroxyalkyl, C₁₋₆haloalkyl, -C(O)R₉ₐ, -C(O)OR₉ₐ, -C(O)NR₉ₐR_{9b}, or CN,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₆alkyl, halogen, or C₁₋₃haloalkyl,
R₅ is H, C₁₋₆alkyl, C₂₋₆alkynyl, halogen, -CN, or heteroaryl, wherein one or more hydrogens in the heteroaryl are optionally substituted with C₁₋₆alkyl, halogen, or C₁₋₃haloalkyl,
R₆ is -NHC(O)R₇ or heteroaryl, wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, C₃₋₁₀cycloalkyl, or heterocycle, wherein one or more hydrogens in the cycloalkyl and heterocycle are optionally and each independently substituted with C₁₋₆alkyl, halogen, or -CN,
R₈ₐ is H, C₁₋₆alkyl, or C₃₋₆cycloalkyl,
R_{8b} and R_{8c} are each independently H, C₁₋₆alkyl, C₁₋₆alkoxy, or halogen,
X is -S-, -O-, -N(R₉ₐ)-, -CH₂-, -CHCH-, -CC-, -CH₂O-, or -OCH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 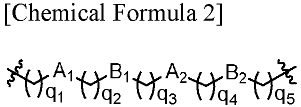
in the Chemical Formula 2,
A₁ and A₂ are each independently direct bond, C₃₋₁₀cycloalkyl, heterocycle, aryl, or heteroaryl, wherein one or more hydrogens in the cycloalkyl, heterocycle, aryl, or heteroaryl are optionally and each independently substituted with C₁₋₆alkyl, halogen, C₁₋₃haloalkyl, -OH, or =O,
B₁ and B₂ are each independently direct bond, -O-, -N(R₁₀)- -C(O)-, -C(O)N(R₁₀)-, or - N(R₁₀)C(O)-, wherein R₁₀ is each independently H or C₁₋₆alkyl, and
q₁, q₂, q₃, q₄, and q₅ are each independently an integer from 0 to 10.

2. The compound of Claim 1, an optical isomer, a stereoisomer, an isotope variant, a hydrate, a solvate, or a pharmaceutically acceptable salt thereof, in the Chemical Formula 1,
R₁ is 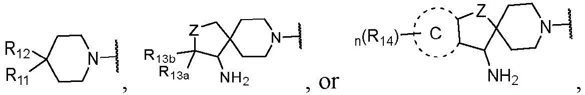
R₁₁ is H, C₁₋₆alkyl, hydroxy, halogen, C₁₋₆hydroxyalkyl, or C₁₋₆haloalkyl,
R₁₂ is amino, C₁₋₆aminoalkyl, or C₁₋₆alkylamino,
R₁₃ₐ and R_{13b} are each independently H, C₁₋₆alkyl, hydroxy, halogen, C₁₋₆hydroxyalkyl, C₁₋₆haloalkyl, or C₁₋₆alkoxy,
R₁₄ is each independently H, C₁₋₆alkyl, hydroxyl, halogen, or CN,
Z is each independently -O-, -S-, -NH-, or -CH₂-,
Ring C is aryl or heteroaryl,
n is an integer from 0 to 5,
R₂ is H, C₁₋₆alkyl, or -NH₂,
R₃ is H, C₁₋₆alkyl, C₁₋₆hydroxyalkyl, -C(O)NR₉ₐR_{9b}, or CN,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₆alkyl, halogen, or C₁₋₃haloalkyl,
R₅ is H, C₁₋₆alkyl, C₂₋₆alkynyl, halogen, -CN, or heteroaryl, wherein one or more hydrogens in the heteroaryl are optionally substituted with C₁₋₆alkyl, halogen, or C₁₋₃haloalkyl,
R₆ is -NHC(O)R₇ or heteroaryl, wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, C₃₋₁₀cycloalkyl, or heterocycle, wherein one or more hydrogens in the cycloalkyl and heterocycle are optionally and each independently substituted with C₁₋₆alkyl, halogen, or -CN,
R₈ₐ is H, C₁₋₆alkyl, or C₃₋₆cycloalkyl,
R_{8b} and R_{8c} are each independently H, C₁₋₆alkyl, C₁₋₆alkoxy, or halogen,
X is -S-, -O-, -N(R₉ₐ)-, or -CH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 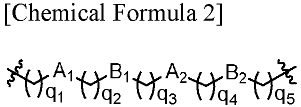
in the Chemical Formula 2,
A₁ and A₂ are each independently direct bond, C₃₋₁₀cycloalkyl, heterocycle, aryl, or heteroaryl, wherein one or more hydrogens in the cycloalkyl, heterocycle, aryl, or heteroaryl are optionally and each independently substituted with C₁₋₆alkyl, halogen, C₁₋₃haloalkyl, -OH, or =O,
B₁ and B₂ are each independently direct bond, -O-, -N(R₁₀)-, -C(O)-, -C(O)N(R₁₀)-, or - N(R₁₀)C(O)-, wherein R₁₀ is each independently H or C₁₋₆alkyl, and
q₁, q₂, q₃, q₄, and q₅ are each independently an integer from 0 to 10.

3. The compound of Claim 2, an optical isomer, a stereoisomer, an isotope variant, a hydrate, a solvate, or a pharmaceutically acceptable salt thereof, in the Chemical Formula 1,
R₁ is 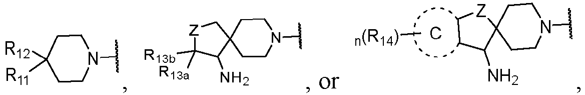
R₁₁ is H, C₁₋₆alkyl, hydroxy, halogen, C₁₋₃hydroxyalkyl, or C₁₋₃haloalkyl,
R₁₂ is amino, C₁₋₃aminoalkyl, or C₁₋₃alkylamino,
R₁₃ₐ and R_{13b} are each independently H, C₁₋₆alkyl, hydroxy, halogen, C₁₋₃hydroxyalkyl, C₁₋₃haloalkyl, or C₁₋₃alkoxy,
R₁₄ is each independently H, C₁₋₃alkyl, hydroxyl, halogen, or CN,
Z is each independently -O-, -NH-, or -CH₂-,
Ring C is aryl or heteroaryl,
n is an integer from 0 to 3,
R₂ is H, C₁₋₆alkyl, or -NH₂,
R₃ is H, C₁₋₆alkyl, C₁₋₆hydroxyalkyl, -C(O)NR₉ₐR_{9b}, or CN,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₃alkyl, halogen, or C₁₋₃haloalkyl,
R₅ is H, C₁₋₆alkyl, acetylenyl, halogen, -CN, or heteroaryl, wherein one or more hydrogens in the heteroaryl are optionally substituted with C₁₋₆alkyl, halogen, or C₁₋₃haloalkyl,
R₆ is -NHC(O)R₇ or heteroaryl, wherein one or more hydrogens in the heteroaryl are optionally substituted with C₁₋₆alkyl, halogen, C₁₋₃haloalkyl, or C₃₋₆cycloalkyl, and wherein R₇ is C₁₋₆alkyl, C₁₋₆haloalkyl, C₁₋₆hydroxyalkyl, C₁₋₆cyanoalkyl, or C₃₋₁₀cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₆alkyl, or halogen,
X is -S-, -O-, -N(R₉ₐ)-, or -CH₂-,
Y is CH or N,
W is direct bond, -O-, or -N(R₉ₐ)-,
R₉ₐ and R_{9b} are each independently H, C₁₋₆alkyl, or C₁₋₆haloalkyl,
L is the following Chemical Formula 2, 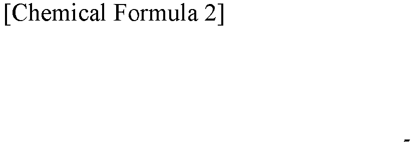 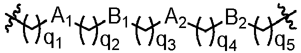
in the Chemical Formula 2,
A₁ and A₂ are each independently direct bond, C₃₋₁₀cycloalkyl, or heterocycle, wherein one or more hydrogens in the cycloalkyl and heterocycle are optionally and each independently substituted with C₁₋₆alkyl, halogen, C₁₋₃haloalkyl, -OH, or =O,
B₁ and B₂ are each independently direct bond, -O-, -N(R₁₀)-, or -C(O)-, wherein R₁₀ is each independently H or C₁₋₆alkyl, and
q₁, q₂, q₃, q₄, and q₅ are each independently an integer from 0 to 5.

4. The compound of Claim 3, an optical isomer, a stereoisomer, an isotope variant, a hydrate, a solvate, or a pharmaceutically acceptable salt thereof, in the Chemical Formula 1,
R₁ is 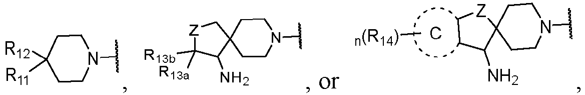
R₁₁ is H, C₁₋₆alkyl, hydroxy, or C₁₋₃hydroxyalkyl,
R₁₂ is amino or C₁₋₃aminoalkyl,
R₁₃ₐ and R_{13b} are each independently H, C₁₋₆alkyl, hydroxy, C₁₋₃hydroxyalkyl, or C₁₋₃alkoxy,
R₁₄ is each independently H, C₁₋₃alkyl, hydroxyl, halogen, or CN,
Z is each independently -O-, -NH-, or -CH₂-,
Ring C is aryl or heteroaryl,
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₃alkyl, halogen, or C₁₋₃haloalkyl,
R₅ is H, C₁₋₆alkyl, acetylenyl, halogen, -CN, thiazole, pyrazole, pyrrole, oxazole, or triazole, wherein one or more hydrogens in the thiazole, pyrazole, pyrrole, oxazole, and triazole are optionally substituted with C₁₋₆alkyl, halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond, -O-, or -N(R₉ₐ)-,
R₉ₐ and R_{9b} are each independently H, C₁₋₃alkyl, or C₁₋₃haloalkyl,
L is the following Chemical Formula 2, 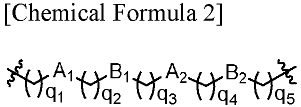
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, C₁₋₃haloalkyl, -OH, or =O,
B₁ and B₂ are each independently direct bond, -O-, or -C(O)-, and
q₁, q₂, q₃, q₄, and q₅ are each independently an integer from 0 to 5.

5. The compound of Claim 4, an optical isomer, a stereoisomer, an isotope variant, a hydrate, a solvate, or a pharmaceutically acceptable salt thereof, in the Chemical Formula 1,
R₁ is 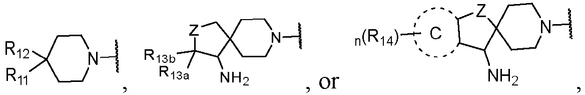
R₁₁ is H, C₁₋₆alkyl, or hydroxy,
R₁₂ is amino or C₁₋₃aminoalkyl,
R₁₃ₐ and R_{13b} are each independently H, C₁₋₆alkyl, hydroxy, or C₁₋₃hydroxyalkyl,
R₁₄ is each independently H, C₁₋₃alkyl, or halogen,
Z is each independently -O- or -CH₂-,
Ring C is aryl or heteroaryl,
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, C₁₋₆haloalkyl, or C₁₋₆alkoxy, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₃alkyl or halogen,
R₅ is H, C₁₋₆alkyl, acetylenyl, halogen, -CN, thiazole, pyrazole, or pyrrole, wherein one or more hydrogens in the thiazole, pyrazole, and pyrrole are optionally substituted with C₁₋₆alkyl, halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with C₁₋₃alkyl, halogen, or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond, -O-, or -N(R₉ₐ)-,
R₉ₐ and R_{9b} are each independently H, C₁₋₃alkyl, or C₁₋₃haloalkyl,
L is the following Chemical Formula 2, 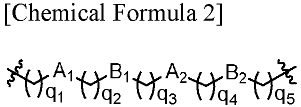
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, -OH, or =O,
B₁ and B₂ are each independently direct bond or -C(O)-, and
q₁, q₂, q₃, q₄, and q₅ are each independently an integer from 0 to 3.

6. The compound of Claim 5, an optical isomer, a stereoisomer, an isotope variant, a hydrate, a solvate, or a pharmaceutically acceptable salt thereof, in the Chemical Formula 1,
R₁ is 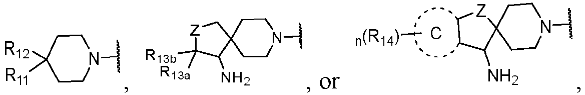
R₁₁ is H or C₁₋₆alkyl,
R₁₂ is amino or C₁₋₃aminoalkyl,
R₁₃ₐ and R_{13b} are each independently H or C₁₋₆alkyl,
R₁₄ is each independently H or C₁₋₃alkyl,
Z is each independently -O- or -CH₂-,
Ring C is aryl or heteroaryl,
n is an integer from 0 to 3,
R₂ is H, C₁₋₆alkyl, or -NH₂,
R₃ is H, C₁₋₆alkyl, or C₁₋₆hydroxyalkyl,
R₄ is H, C₁₋₆alkyl, halogen, or C₁₋₆haloalkyl, or is connected to R₉ₐ of W, which is -N(R₉ₐ)-, to form a heterocycle or heteroaryl, wherein one or more hydrogens in the heterocycle and heteroaryl are optionally and each independently substituted with C₁₋₃alkyl or halogen,
R₅ is H, C₁₋₆alkyl, halogen, -CN, thiazole, or pyrazole, wherein one or more hydrogens in the thiazole and pyrazole are optionally substituted with C₁₋₆alkyl, halogen, or C₁₋₃haloalkyl,
R₆ is -NHC(O)R₇, triazole, pyrazole, or isoxazole, wherein one or more hydrogens in the triazole, pyrazole, and isoxazole are optionally substituted with C₁₋₆alkyl, halogen, C₁₋₃haloalkyl, or C₃₋₆cycloalkyl, and wherein R₇ is C₁₋₃alkyl, C₁₋₃haloalkyl, C₁₋₃hydroxyalkyl, C₁₋₃cyanoalkyl, or C₃₋₆cycloalkyl, wherein one or more hydrogens in the cycloalkyl are optionally and each independently substituted with halogen or -CN,
R₈ₐ is H, C₁₋₆alkyl, or C₃₋₆cycloalkyl,
R_{8b} and R_{8c} are each independently H, C₁₋₃alkyl, or halogen,
X is -S- or -O-,
Y is CH or N,
W is direct bond or -N(R₉ₐ)-,
R₉ₐ and R_{9b} are each independently H, C₁₋₃alkyl, or C₁₋₃haloalkyl,
L is the following Chemical Formula 2, 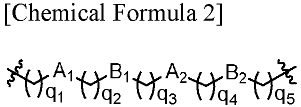
in the Chemical Formula 2,
A₁ and A₂ are each independently direct bond, cyclohexane, cyclobutane, piperidine, piperazine, azetidine, or 7-azaspiro[3.5]nonane, wherein one or more hydrogens in the cyclohexane, cyclobutane, piperidine, piperazine, azetidine, and 7-azaspiro[3.5]nonane are optionally and each independently substituted with C₁₋₆alkyl, halogen, -OH, or =O,
B₁ and B₂ are each independently direct bond or -C(O)-, and
q₁, q₂, q₃, q₄, and q₅ are each independently an integer from 0 to 3.

7. The compound of Claim 1, an optical isomer, a stereoisomer, an isotope variant, a hydrate, a solvate, or a pharmaceutically acceptable salt thereof, wherein the compound is any one of the following compounds:
| IUPAC Name |
|---|
| (28,4R)-N-(2-((6-(4-(((3-((5-(4-(aminomethyl)-4-methylpiperidin-1-yl)pyrazin-2-yl)thio)-2-chlorophenyl)amino)methyl)piperidin-1-yl)hexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(2-((3-((5-(4-(aminomethyl)-4-methylpiperidin-1-yl)pyrazin-2-yl)thio)-2-chlorophenyl)amino)ethyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(2-((3-((5-(4-(aminomethyl)-4-methylpiperidin-1-yl)pyrazin-2-yl)thio)-2-chlorophenyl)amino)ethyl)-3-oxopiperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-((3-((5-(4-(aminomethyl)-4-methylpiperidin-1-yl)pyrazin-2-yl)thio)-2-chlorophenyl)glycyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-((3-((3-((5-(4-(aminomethyl)-4-methylpiperidin-1-yl)pyrazin-2-yl)thio)-2-chlorophenyl)amino)azetidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-((4-((3-((5-(4-(aminomethyl)-4-methylpiperidin-1-yl)pyrazin-2-yl)thio)-2-chlorophenyl)amino)piperidin-1-yl)methyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(((1R,4S)-4-((4-((5-(4-(aminomethyl)-4-methylpiperidin-1-yl)pyrazin-2-yl)thio)-3-chloropyridin-2-yl)amino)cyclohexyl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-((1-(4-((5-(4-(aminomethyl)-4-methylpiperidin-1-yl)pyrazin-2-yl)thio)-3-chloropyridin-2-yl)piperidin-4-yl)methyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-((1-((1-(4-((5-(4-(aminomethyl)-4-methylpiperidin-1-yl)pyrazin-2-yl)thio)-3-chloropyridin-2-yl)piperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-((1-((1-(4-((5-(4-(aminomethyl)-4-methylpiperidin-1-yl)pyrazin-2-yl)thio)-3-chloropyridin-2-yl)-4-hydroxypiperidin-4-yl)methyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((S)-1-amino-1,3-dihydrospiro[indene-2,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((3-((5-(4-(aminomethyl)-4-methylpiperidin-1-yl)pyrazin-2-yl)oxy)-2-chlorophenyl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((3-((5-(4-(aminomethyl)-4-methylpiperidin-1-yl)pyrazin-2-yl)thio)-2-chlorophenyl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-(((3-((5-(4-(aminomethyl)-4-methylpiperidin-1-yl)pyrazin-2-yl)thio)-2-chlorophenyl)amino)methyl)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-(((3-((5-(4-(aminomethyl)-4-methylpiperidin-1-yl)pyrazin-2-yl)thio)-2-chlorophenyl)amino)methyl)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(((1R,4S)-4-(4-(4-((5-(4-(aminomethyl)-4-methylpiperidin-1-yl)pyrazin-2-yl)thio)-3-chloropyridin-2-yl)piperazine-1-carbonyl)cyclohexyl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-(4-amino-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(3-((3-((5-((3S,4S)-4-amino-3-methyl-2-oxa-8-azaspiro[4.5]decan-8-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-((S)-1-amino-1,3-dihydrospiro[indene-2,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((3-((5-((S)-5-amino-5,7-dihydrospiro[cyclopenta[b]pyridine-6,4'-piperidin]-1'-yl)pyrazin-2-yl)thio)-2-chlorophenyl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-(4-((5-((3S,4S)-4-amino-3-methyl-2-oxa-8-azaspiro[4.5]decan-8-yl)pyrazin-2-yl)thio)indolin-1-yl)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(4-((5-((3S,4S)-4-amino-3-methyl-2-oxa-8-azaspiro[4.5]decan-8-yl)pyrazin-2-yl)thio)indolin-1-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-(8-((5-(4-(aminomethyl)-4-methylpiperidin-1-yl)pyrazin-2-yl)thio)-2,3-dihydro-4H-benzo[b][1,4]oxazin-4-yl)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(4-(8-((5-(4-(aminomethyl)-4-methylpiperidin-1-yl)pyrazin-2-yl)thio)-2,3-dihydro-4H-benzo[b][1,4]oxazin-4-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(8-((5-((3S,4S)-4-amino-3-methyl-2-oxa-8-azaspiro[4.5]decan-8-yl)pyrazin-2-yl)thio)-2H-benzo[b][1,4]oxazin-4(3H)-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1r,4S)-4-(4-(4-((5-(4-(aminomethyl)-4-methylpiperidin-1-yl)pyrazin-2-yl)thio)-3-fluoro-1H-indol-1-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-amino-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-((3S,4S)-4-amino-3-methyl-2-oxa-8-azaspiro[4.5]decan-8-yl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-(aminomethyl)-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-amino-4-methylpiperidin-1-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-((3S,4S)-4-amino-3-methyl-2-oxa-8-azaspiro[4.5]decan-8-yl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(3-((4-((5-(4-(aminomethyl)-4-methylpiperidin-1-yl)-6-(hydroxymethyl)pyrazin-2-yl)thio)-3-chloropyridin-2-yl)amino)azetidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-((4-((5-(4-(aminomethyl)-4-methylpiperidin-1-yl)-6-(hydroxymethyl)pyrazin-2-yl)thio)-3-chloropyridin-2-yl)amino)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(3-((5-(4-(aminomethyl)-4-methylpiperidin-1-yl)pyrazin-2-yl)thio)-2-chlorophenyl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-(((1R,4S)-4-(4-(4-((5-(4-(aminomethyl)-4-methylpiperidin-1-yl)pyrazin-2-yl)thio)-3-chloropyridin-2-yl)piperidine-1-carbonyl)cyclohexyl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-((S)-1-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)phenyl)ethyl)-4-hydroxypyrrolidine-2-carboxamide |
| (2S,4R)-1-((S)-2-acetamido-3,3-dimethylbutanoyl)-N-((S)-1-(2-(((1R,4S)-4-(3-((4-((5-(4-(aminomethyl)-4-methylpiperidin-1-yl)pyrazin-2-yl)thio)-3-chloropyridin-2-yl)amino)azetidine-1-carbonyl)cyclohexyl)oxy)-4-(4-methylthiazol-5-yl)phenyl)ethyl)-4-hydroxypyrrolidine-2-carboxamide |
| (2S,4R)-N-(2-(2-(4-(3-((3-((5-(4-(aminomethyl)-4-methylpiperidin-1-yl)pyrazin-2-yl)thio)-2-chlorophenyl)amino)azetidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(4-((3-((5-(4-(aminomethyl)-4-methylpiperidin-1-yl)pyrazin-2-yl)thio)-2-chlorophenyl)amino)piperidine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(4-(4-((5-(4-(aminomethyl)-4-methylpiperidin-1-yl)pyrazin-2-yl)thio)-3-chloropyridin-2-yl)piperazine-1-carbonyl)piperidin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(1-(4-((5-(4-(aminomethyl)-4-methylpiperidin-1-yl)pyrazin-2-yl)thio)-3-chloropyridin-2-yl)azetidine-3-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(4-(1-(4-((5-(4-(aminomethyl)-4-methylpiperidin-1-yl)pyrazin-2-yl)thio)-3-chloropyridin-2-yl)piperidine-4-carbonyl)piperazin-1-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-(2-(1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-(2-(1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)ethoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((7-(1-(4-((5-(4-(aminomethyl)-4-methylpiperidin-1-yl)pyrazin-2-yl)thio)-3-chloropyridin-2-yl)piperidine-4-carbonyl)-7-azaspiro[3.5]nonan-2-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)methoxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1s,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1S,3R)-3-((4-((5-(4-amino-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1r,3S)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-((1R,4S)-4-((4-((5-(4-(aminomethyl)-4-methylpiperidin-1-yl)pyrazin-2-yl)thio)-3-chloropyridin-2-yl)amino)cyclohexanecarbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-((1-(1-(4-((5-(4-(aminomethyl)-4-methylpiperidin-1-yl)pyrazin-2-yl)thio)-3-chloropyridin-2-yl)azetidine-3-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-((1-(2-(1-(4-((5-(4-(aminomethyl)-4-methylpiperidin-1-yl)pyrazin-2-yl)thio)-3-chloropyridin-2-yl)azetidin-3-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (28,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-methylpiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-fluoropiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)-4-hydroxypiperidine-4-carbonyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(2-(1-(4-((5-(4-(aminomethyl)-4-methylpiperidin-1-yl)pyrazin-2-yl)thio)-3-chloropyridin-2-yl)piperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(2-(1-(4-((5-(4-(aminomethyl)-4-methylpiperidin-1-yl)pyrazin-2-yl)thio)-3-chloropyridin-2-yl)-4-hydroxypiperidin-4-yl)acetyl)piperidin-4-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)azetidin-3-yl)oxy)-4-(4-methylthiazol-5-yl)benzyl)-1-((S)-2-(1-fluorocyclopropane-1-carboxamido)-3,3-dimethylbutanoyl)-4-hydroxypyrrolidine-2-carboxamide |
| (2S,4R)-1-((S)-2-acetamido-3,3-dimethylbutanoyl)-N-(2-((1-((1S,3R)-3-((4-((5-(4-(aminomethyl)-4-methylpiperidin-1-yl)pyrazin-2-yl)thio)-3-chloropyridin-2-yl)amino)cyclobutanecarbonyl)piperidin-4-yl)oxy)-4-(4-methylthiazol-5-yl)benzyl)-4-hydroxypyrrolidine-2-carboxamide |
| (28,4R)-1-((S)-2-acetamido-3,3-dimethylbutanoyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (28,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(4-((5-(4-(aminomethyl)-4-methylpiperidin-1-yl)-6-(hydroxymethyl)-3-methylpyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (28,4R)-1-((S)-2-acetamido-3,3-dimethylbutanoyl)-N-(2-((1-(1-(3-((5-(4-(aminomethyl)-4-methylpiperidin-1-yl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (28,4R)-1-((S)-2-acetamido-3,3-dimethylbutanoyl)-N-(2-((1-(1-(3-((5-(4-(aminomethyl)-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-(4-amino-4-methylpiperidin-1-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-3,3-dimethylbutanoyl)-N-(2-((1-(1-(3-((5-((3S,4S)-4-amino-3-methyl-2-oxa-8-azaspiro[4.5]decan-8-yl)-6-(hydroxymethyl)pyrazin-2-yl)thio)-2-chlorophenyl)piperidine-4-carbonyl)piperidin-4-yl)oxy)-4-(4-methylthiazol-5-yl)benzyl)-4-hydroxypyrrolidine-2-carboxamide |
| (2S,4R)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-1-((S)-2-(1-fluorocyclopropane-1-carboxamido)-3,3-dimethylbutanoyl)-4-hydroxypyrrolidine-2-carboxamide |
| (28,4R)-1-((S)-2-acetamido-3,3-dimethylbutanoyl)-N-((S)-1-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)phenyl)ethyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(2-cyanoacetamido)-3,3-dimethylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-2-(2-hydroxyacetamido)-3,3-dimethylbutanoyl)pyrrolidine-2-carboxamide |
| (2S,4R)-1-(acetyl-L-valyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-1-((S)-2-acetamido-2-cyclopropylacetyl)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (28,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl- 1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((R)-3-methyl-2-(4-methyl-1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-(3-methyl-2-(4-methyl-1H-pyrazol-1-yl)butanoyl)pyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (28,4R)-N-(2-((1-(1-(4-((5-(4-amino-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3-methylbutanoyl)-4-hydroxypyrrolidine-2-carboxamide |
| (2S,4R)-N-(2-((1-(1-(4-((5-(4-(aminomethyl)-4-methylpiperidin-1-yl)pyrazin-2-yl)thio)-3-chloropyridin-2-yl)piperidine-4-carbonyl)piperidin-4-yl)oxy)-4-(4-methylthiazol-5-yl)benzyl)-1-((S)-2-(4-cyclopropyl-1H-1,2,3-triazol-1-yl)-3,3-dimethylbutanoyl)-4-hydroxypyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin- 1-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-(4-methylthiazol-5-yl)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-((S)-3-methyl-2-(3-methylisoxazol-5-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-((R)-3-methyl-2-(3-methylisoxazol-5-yl)butanoyl)pyrrolidine-2-carboxamide |
| (2S,4R)-N-(2-(((1R,4S)-4-(3-((3-((5-(4-(aminomethyl)-4-methylpiperidin-1-yl)pyrazin-2-yl)thio)-2-chlorophenyl)amino)azetidine-1-carbonyl)cyclohexyl)oxy)-4-(4-methylthiazol-5-yl)benzyl)-4-hydroxy-1-(3-methyl-2-(3-methylisoxazol-5-yl)butanoyl)pyrrolidine-2-carboxamide |
| (2S,4R)-N-(2-((1-(1-(4-((5-((3S,4S)-4-amino-3-methyl-2-oxa-8-azaspiro[4.5]decan-8-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin- 1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1 H-1,2,3-triazol-1-yl)butanoyl)pyrrolidine-2-carboxamide |
| (2S,4R)-N-(2-((1-(1-(4-((5-(4-amino-4-methylpiperidin-1-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1 H-1,2,3-triazol-1-yl)butanoyl)pyrrolidine-2-carboxamide |
| (2S,4R)-N-(2-((1-(1-(4-((5-(4-amino-4-methylpiperidin- 1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1H-1,2,3-triazol-1-yl)butanoyl)pyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)benzyl)-4-hydroxy-1-((S)-3-methyl-2-(4-methyl-1H-1,2,3-triazol-1-yl)butanoyl)pyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-chlorobenzyl)-4-hydroxy-1-((S)-3-methyl-2-(4-methyl-1 H-1,2,3-triazol-1-yl)butanoyl)pyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-cyanobenzyl)-4-hydroxy-1-((S)-3-methyl-2-(4-methyl-1H-1,2,3-triazol-1-yl)butanoyl)pyrrolidine-2-carboxamide |
| (28,4R)-N-(2-((1-(1-(4-((5-(4-(aminomethyl)-4-methylpiperidin-1-yl)-6-(hydroxymethyl)pyrazin-2-yl)thio)-3-chloropyridin-2-yl)piperidine-4-carbonyl)piperidin-4-yl)oxy)-4-fluorobenzyl)-4-hydroxy-1-((S)-3-methyl-2-(4-methyl-1 H-1,2,3-triazol-1-yl)butanoyl)pyrrolidine-2-carboxamide |

8. A composition comprising a compound of any one of Claims 1 to 7, an optical isomer, a stereoisomer, an isotope variant, a hydrate, a solvate, or a pharmaceutically acceptable salt thereof, and a pharmaceutically acceptable carrier.

9. A pharmaceutical composition for degrading or inhibiting Src homology 2 domain-containing phosphatase (SHP2), comprising as an effective agent a compound of any one of Claims 1 to 7, an optical isomer, a stereoisomer, an isotope variant, a hydrate, a solvate, or a pharmaceutically acceptable salt thereof.

10. The pharmaceutical composition of Claim 9, wherein the pharmaceutical composition for degrading or inhibiting Src homology 2 domain-containing phosphatase (SHP2) is for treating or preventing cancer, cancer metastasis, cardiovascular disease, immune disorders, eye disorders, Noonan syndrome, or leopard syndrome.

11. The pharmaceutical composition of Claim 10, wherein the cancer is at least one selected from blood cancer, acute lymphoblastic leukemia, chronic lymphoblastic leukemia, acute myeloid leukemia, monocytic leukemia, chronic myeloid leukemia, chronic lymphocytic leukemia, mixed lineage leukemia, NUT-midline cancer, multiple myeloma, small cell lung cancer, non-small cell lung cancer, neuroblastoma, lymphoma, cervical cancer, head and neck cancer, head cancer, esophageal cancer, stomach cancer, pancreatic cancer, liver cancer, melanoma, colorectal cancer, colon cancer, prostate cancer, breast cancer, ovarian cancer, bladder cancer, glioma, sarcoma, papillary thyroid carcinoma and combinations thereof.

12. A method for treating or preventing a Src homology 2 domain-containing phosphatase (SHP2)-related disease, comprising administering to a subject in need thereof a therapeutically effective amount of a compound of any one of Claims 1 to 7, an optical isomer, a stereoisomer, an isotope variant, a hydrate, a solvate, or a pharmaceutically acceptable salt thereof.

13. The method of Claim 12, wherein the Src homology 2 domain-containing phosphatase (SHP2)-related disease is cancer, cancer metastasis, cardiovascular disease, immune disorders, eye disorders, Noonan syndrome, or leopard syndrome.

14. The method of Claim 13, wherein the cancer is at least one selected from blood cancer, acute lymphoblastic leukemia, chronic lymphoblastic leukemia, acute myeloid leukemia, monocytic leukemia, chronic myeloid leukemia, chronic lymphocytic leukemia, mixed lineage leukemia, NUT-midline cancer, multiple myeloma, small cell lung cancer, non-small cell lung cancer, neuroblastoma, lymphoma, cervical cancer, head and neck cancer, head cancer, esophageal cancer, stomach cancer, pancreatic cancer, liver cancer, melanoma, colorectal cancer, colon cancer, prostate cancer, breast cancer, ovarian cancer, bladder cancer, glioma, sarcoma, papillary thyroid carcinoma and combinations thereof.

15. A method for inhibiting or degrading Src homology 2 domain-containing phosphatase (SHP2), comprising administering to a subject in need thereof a therapeutically effective amount of a compound of any one of claims 1 to 7, an optical isomer, a stereoisomer, an isotopic variant, a hydrate, a solvate, or a pharmaceutically acceptable salt thereof.

16. A method for inhibiting or degrading Src homology 2 domain-containing phosphatase (SHP2) in a patient or a biological sample, comprising contacting the patient or the biological sample with a compound of any one of claims 1 to 7, an optical isomer, a stereoisomer, an isotopic variant, a hydrate, a solvate, or a pharmaceutically acceptable salt thereof.

17. Use of a compound of any one of claims 1 to 7, an optical isomer, a stereoisomer, an isotopic variant, a hydrate, a solvate, or a pharmaceutically acceptable salt thereof, for the production of a medicament for degrading Src homology 2 domain-containing phosphatase (SHP2) in a patient or biological sample.

18. Use of a compound of any one of claims 1 to 7, an optical isomer, a stereoisomer, an isotopic variant, a hydrate, a solvate, or a pharmaceutically acceptable salt thereof, for the production of a medicament for treating a Src homology 2 domain-containing phosphatase (SHP2)-related disease.
